# EUROPEAN PATENT APPLICATION

(11) **EP 4 233 834 A2**
(43) Date of publication of application: **30.08.2023**
(21) Application number: 23178930.6
(22) Date of filing: 20.11.2019
(51) Int. Cl.: A61K 8/49

(54) **ARYL-ANILINE AND HETEROARYL-ANILINE COMPOUNDS FOR TREATMENT OF BIRTHMARKS**

(30) Priority: 20.11.2018 US 201862769844 P
(62) Divisional of application: 19887611.2
(71) Applicant: NFlection Therapeutics, Inc., Chestnut Hill, Massachusetts 02467 (US); The Board of Trustees of the Leland Stanford Junior University, Stanford, CA 94305 (US)
(72) Inventor: Kincaid,, John, Chestnut Hill, 02467 (US); Sarin,, Kavita, Yang, Los Altos, 94024 (US)
(74) Representative: J A Kemp LLP

(57) **Abstract**

Provided herein are compounds and pharmaceutical compositions thereof for treating a birthmark in a subject in need thereof, wherein the compound is according to any one of formula (I), (II), (III), (IV) and (V) 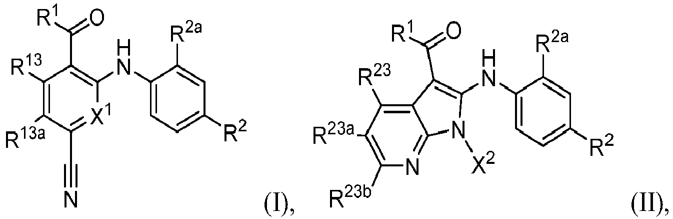 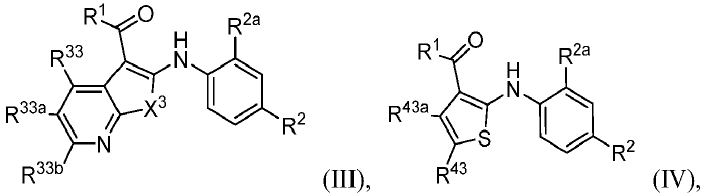 and 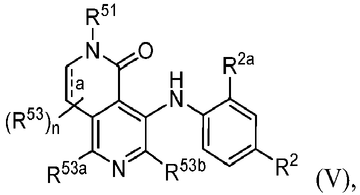 wherein X¹, X², X³, R¹, R², R^{2a}, R¹³, R^{13a}, R²³, R^{23a}, R^{23b}, R³³, R^{33a}, R^{33b}, R⁴³, R^{43a}, R⁵¹, R⁵³, R^{53a}, R^{53b}, bond "a", and subscript n are described herein.

## Description

### CROSS-REFERENCES TO RELATED APPLICATIONS

This application claims priorty to U. S. Provisional Application No. 62/769,844, filed November 20, 2018, the content of which is incorporated herein in its entirety for all purposes.

### BACKGROUND OF THE INVENTION

Benign cutaneous tumors of the vascular, keratinocytic, and melanocytic compartments often occur at birth or during childhood. These lesions, referred in this application as "birthmarks", can cause cosmetic distress, disfigurement and social anxiety. In some cases, these lesions can predispose individuals to functional impairment or future malignancies. These birthmarks can be sporadic or arise as part of an underlying neurocutaneous syndrome.

Vascular birthmarks include, for example port wine stain/capillary malformation, angiomas, lobular capillary hemangiomas, arteriovascular malformation, lymphatic malformation, vascular malformation, hemangiomas, and other angioma. Keratinocytic nevi refers to Keratinocytic epidermal nevi and nevi sebacei. Melanocytic nevi (commonly known as moles) include, for example congenital nevi, multiple lentigines (which can occur in syndromes such as LEOPARD), ephiledes (freckles), and nevus spiilus.

Neurocutaneous syndromes, also referred to as birthmarks, such as port-wine stains, are associated with congenital low-flow vascular malformations (capillary malformation) in the skin which, if left untreated, can hypertrophy and develop nodularity (Minkis, K. et al, Lasers Surg Med. (2009) 41(6): pp423-426). Laser therapy is typically used for treatment of port-wine stains, but often without full resolution. Epidermal nevi are common cutaneous mosaic disorders, subdivided into keratinocytic and organoid nevi. Organoid nevi include nevus sebaceus (NS). Immunolabelling of NS is reportedly associated with increased phosphorylated ERK staining (Aslam, A, et al., Clinical and Experimental Dermatology (2014) 39: pp 1-6). Non-organoid keratinocytic epidermal nevus (KEN) is characterized by benign congenital hyperpigmented skin lesions. Epidermal nevi with localized epidermal thickening are present at birth or become visible during childhood. Other cutaneous disorders that also occur in childhood birthmarks include nevus cellular nevus, lobulary capillary hemangioma, congenital nevi, ephiledes (freckles), multiple lentigines (which can occur in multiple syndromes including LEOPARD syndrome), capillary angioma, nevus spilus, arterio-venous malformations, lymphatic malformations, and congenital melanocytic nevus. Lentigines can occur in childhood (in syndromes such as LEOPARD syndrome), which has mutations that activate RAS/MAPK pathway, as well as can be acquired in adults. In some cases birthmarks are not amenable to surgical excision and/or laser treatment. In some cases birthmarks, when untreated, can progress to lesions and/or proliferative skin conditions.

Modulation of ERK/MEK pathways may have a therapeutic effect on birthmarks. RAS mutations have been reported in mosaic RASopathies i.e. non-organoid KEN, and sebaceous nevus (Farschtschi S, et al., BMC Medical Genetics. (2015);16: pp 6; and Sun, B.K. et. Al, Journal of Investigative Dermatology, (2013); 3: pp824-827). Thus, inhibition of Ras signaling pathway, including the Ras-MAPK cascade, may be useful in treating birthmarks.

Four distinct MAPK cascades have been identified and named according to their MAPK module. See Akinleye et al. Journal of Hematology & Oncology 6:27, 2013. MEK proteins belong to a family of enzymes that lie upstream to their specific MAPK targets in each of the four MAP kinase signaling pathways. Two of these MEK proteins, MEK1 and MEK2, are closely related and participate in this signaling pathway cascade. Inhibitors of MEK1 and MEK2 have been shown to effectively inhibit MEK signaling downstream of Ras (Rice et al. Medicinal Chemistry Letters 3:416-421, 2012), and thus provide a rationale for targeting MEK in the treatment of birthmarks.

Currently available MEK pathway inhibitors are designed to have oral bioavailability for systemic delivery, but are associated with one or more significant side effects including decreased left ventricular ejection fraction, elevated creatine phosphokinase, pneumonitis, renal failure, diarrhea, infection, uticaria, and maculo-papular rash, all of which are dose limiting or require permanent discontinuation. Moreover, clinical trials have shown one or more side effects with prolonged high-dose administration of MEK inhibitors. (Huang et al. J. Ocul. Pharmacol. Ther. 25:519-530, 2009). For example, PD0325901, a clinically-tested MEK inhibitor, has exhibited one or more neurological side effects associated with ataxia, confusion, and syncope. In addition, a number of other side effects have been observed with systemic exposure to MEK inhibitors including: acneiform rash, CPK elevation, nausea, vomiting, diarrhea, abdominal pain, and fatigue. Thus, there is a need for therapies that treat birthmarks and also limit one or more side effects associated with systemic exposure to MEK/ERK pathway inhibitors.

### BRIEF SUMMARY OF THE INVENTION

In one aspect, provided herein is a method of treating a birthmark. The method includes administering to a subject in need thereof a therapeutically effective amount of a compound selected from the group consisting of formula (I), (II), (III), (IV), and (V): 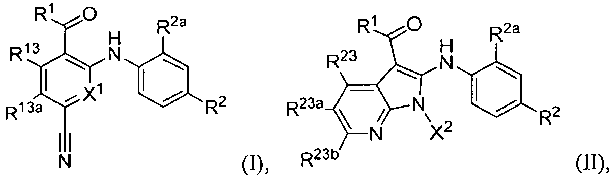 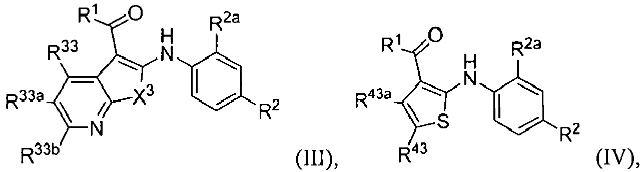 and 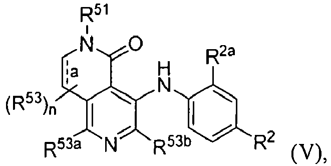 or a N-oxide, stereoisomer, mixture of stereoisomers, and/or a pharmaceutically acceptable salt thereof,
wherein:
X¹ is -CR^{13b} or N; X² is C₁-C₆ alkyl; X³ is S or O;
subscript n is an integer from 0 to 2;
bond "a" is a single or double bond;
R¹ is -OR⁴, -NR⁵R^{5a}, -N(OR^{5b})R^{5a}, or a N-linked heterocycloalkyl which is unsubstituted or substituted with one or two R⁶;
R² is halo, C₁-C₆ alkyl, -S-C₁-C₆ alkyl, C₃-C₈ cycloalkyl, C₂-C₆ alkenyl, or C₂-C₆ alkynyl;
R^{2a} is halo or C₁-C₆ alkyl;
R⁴, R⁵, and R^{5b} are each independently hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶;
R^{5a} is hydrogen or Ci-C6 alkyl;
each R⁶ is independently halo, hydroxy, oxo, C₁-C₆ alkyl, C₃-C₈ cycloalkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl;
R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino;
R¹³, R^{13a}, and R^{13b} are each independently hydrogen, halo, C₁-C₆ alkyl, C₂-C₆ alkenyl, or C₂-C₆ alkynyl;
R²³, R^{23a}, and R^{23b} are each independently hydrogen, halo, C₁-C₆ alkyl, C₂-C₆ alkenyl, C₂-C₆ alkynyl, C₁-C₆ alkoxy, or C₃-C₈ cycloalkoxy;
R³³, R^{33a}, and R^{33b} are each independently hydrogen, halo, C₁-C₆ alkyl, C₂-C₆ alkenyl, C₂-C₆ alkynyl, C₁-C₆ alkoxy, or C₃-C₈ cycloalkoxy;
R⁴³ is cyano, -C(O)NR⁴⁸R^{48a}, or -C(O)R⁴⁶;
R^{43a} is hydrogen, halo, C₁-C₆ alkyl; or
R⁴³ and R^{43a} together form -CH₂CH₂C(O)- or -CH₂CH₂CH₂C(O)-, each of which is unsubstituted or substituted with one or two R⁴⁹;
R⁴⁶ is hydrogen, C₁-C₆ alkyl, C₁-C₆ haloalkyl, C₃-C₈ cycloalkyl or heterocycloalkyl;
R⁴⁸ and R^{48a} are independently hydrogen or C₁-C₆ alkyl; and
each R⁴⁹ is independently C₁-C₆ alkyl, C₃-C₈ cycloalkyl, or C₃-C₈ cycloalkyl-C₁-C₆ alkyl;
R⁵¹ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, R⁷-C(O)-C₁-C₆ alkyl, or -OR⁵⁴, wherein each of the C₃-C₈ cycloalkyl and heterocycloalkyl groups is unsubstituted or substituted with one to six R⁶;
each R⁵³ is independently halo or C₁-C₆ alkyl;
R^{53a} and R^{53b} are each independently hydrogen, halo, C₁-C₆ alkyl, C₂-C₆ alkenyl, or C₂-C₆ alkynyl; and
R⁵⁴ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶.

### BRIEF DESCRIPTION OF THE DRAWINGS

FIG. 1 shows suppression of p-ERK in human epidermal nevus explants after application of topical formulations containing vehicle only, Compound **2.003** or Compound **4.013,** using the protocol described in Example 1. FIG. 1A: untreated; FIG. 1B: vehicle only; FIG. 1C: Compound **2.003;** and FIG. 1D: Compound **4.013.**
FIG. 2 shows foci of hyperproliferation overlapping with p-ERK expression by Ki-67 staining of epidermal nevus, using the protocol described in Example 1.
FIGs. 3 and 4 shows suppression of p-ERK in human nevus sebaceous explants after application of gel topical formulations containing vehicle only, Trametinib, Compound **2.003,** Compound **4.029,** using the protocol described in Example 1.
FIG. 5 shows suppression ofp-ERK in human nevus sebaceous explants after application of gel topical formulations containing vehicle only and Compound **2.003,** using the protocol described in Example 1.
FIG. 6 shows a dose response of Compound **2.003** in suppression of p-ERK in human nevus sebaceous explants after application of gel topical formulations containing vehicle only and Compound **2.003,** using the protocol described in Example 1.
FIGs. 7 and 8 show synthesis Scheme 1-1 and Scheme 1-2 for the preparation of a compound of formula (1a), respectively.
FIG. 9 shows synthesis Scheme 1-3 for the preparation of a compound of formula (1b).
FIGs. 10-14 show synthesies Schemes 11-1 to 11-5 for the preparation of a compound of formula (II), respectively.
FIGs. 15-16 show synthesis Schemes 111-1 and 111-2 for the preparation of a compound of formula (IIIa), respectively.
FIG. 17 shows synthesis Scheme IV-1 for the preparation of a compound of any one of formulae (IVa), (IVb), and (IVc).
FIG. 18 shows synthesis Scheme 1V-2 for the preparation of a compound of any one of formulae (IVd-1), (IVd-2), (IVe-1) and (IVe-2).
FIG. 19 shows synthesis Scheme IV-3 for the the preparation of a compound of formula (1Va).
FIGs. 20-21 show synthesis Scheme V-1 and V-2 for the preparation of a compound of formula (Va), repectively.
FIG. 22 shows synthesis Scheme V-3 for the preparation of a compound of formula (Vb).

### DETAILED DESCRIPTION OF THE INVENTION

### I. GENERAL

Provided herein are methods of using compounds and pharmaceutical compositions comprising the compounds for treating birthmarks.

The birthmarks of the present invention are as defined and described as follows:

In some embodiments, the birthmark is a port-wine stain (capillary malformation). Port-wine stains may be present at birth. Port-wine stains can occur anywhere on the body and the area of affected skin grows in proportion to general growth. Thickening of the lesion or the development of small lumps may occur in adulthood and can interfere with normal function (e.g., where the port-wine stain is near the eye or mouth). Port-wine stains may, in some cases, be part of a syndrome such as Sturge-Weber syndrome or Klippel-Trénaunay-Weber syndrome.

In some embodiments, the birthmark is epidermal nevi. Epidermal nevus is a benign skin growth with localized epidermal thickening that is often present at birth or within the first year of life. It typically appears as one or more oblong or linear growths that are skin colored, brown or gray in color. The surface can be wart-like or velvety with sharp borders. Malignant transformation can occur in some cases in middle aged or elderly subjects. Epidermal nevi are subdivided into keratinocytic and organoid nevi. Organoid nevi include nevus sebaceous (NS). In some embodiments, the birthmark is nevus sebaceous. Non-organoid keratinocytic epidermal nevus (KEN) is characterized by benign congenital hyperpigmented skin lesions. Contemplated within the scope of embodiments presented herein are other types of epidermal nevi, including nevus comedonicus. Nevus comedonicus (NC) is a hamartoma of the pilosebaceous unit that, like other epidermal nevi, typically presents at birth or during childhood. Clinically, NC lesions consist of linear arrays or clusters of dilated, keratin-plugged follicular orifices resembling comedones.

In some embodiments the birthmark is melanocytic nevus, including congenital nevi, blue nevi, and acquired melanocytic nevi. Malignant melanoma occasionally develops from the melanocytic nevus (also known as nevocytic nevus, nevus-cell nevus and commonly as a mole). Reasons for treatment of pigmented nevi (i.e., nevus cellular nevus) include prevention of malignant change, limiting malignant progression, cosmetic improvement, or prevention of other functional or anatomical changes.

In some embodiments, the birthmark is dysplastic nevi. Dysplastic nevi (or atypical moles) are unusual-looking benign moles and may resemble melanoma. People who have atypical moles are at increased risk of developing melanoma in a mole or elsewhere on the body.

In some embodiments, the birthmark is a nevus spilus. Nevus spilus (also known as speckled lentiginous nevus and zosteriform lentiginous nevus) is a skin lesion that presents as a light brown patch of pigmentation, and within this patch, are multiple tiny dark brown spots.

In some embodiments, the birthmark is an arterio-venous malformation in the skin (e.g., blue rubber bleb nevus syndrome) which may present as skin lesions comprised of compressible blue subcutaneous nodules.

In some embodiments, the birthmark is a lymphatic malformation. A lymphatic malformation is a type of vascular nevus or birthmark due to malformed and dilated lymphatic vessels. The cystic hygroma (also called 'cystic lymphangioma' and 'lymphangioma cysticum') is a 'macrocytic' lymphatic malformation, and is composed of large fluid-filled spaces. It appears as a skin colored, red or bluish, somewhat transparent, swelling under the skin. Cavernous lymphangioma can affect any site on the body, including the tongue. Lymphangioma circumscriptum is a 'microcytic' lymphatic malformation. It appears as a cluster of small firm blisters filled with lymph fluid, resembling frogspawn.

In some embodiments, the birthmark is a congenital melanocytic nevus. The congenital melanocytic nevus appears as a circumscribed, light brown to black patch or plaque, heterogeneous in consistency, covering any size surface area and any part of the body. Congenital melanocytic nevus poses a risk for malignancy degeneration.

As used herein, the term 'birthmark' does not include cafe au lait spots.

### II. DEFINITION

The abbreviations used herein have their conventional meaning within the chemical and biological arts.

Where substituent groups are specified by their conventional chemical formulae, written from left to right, they equally encompass the substituents that would result from writing the structure from right to left, e.g., -CH₂O- is meant to include -OCH₂-.

"Alkyl" refers to a straight or branched, saturated, aliphatic radical having the number of carbon atoms indicated (i.e., C₁-C₆ means one to six carbons). Alkyl can include any number of carbons, such as C₁-C₂, C₁-C₃, C₁-C₄, C₁-C₅, C₁-C₆, C₁-C₇, C₁-C₈, C₁-C₉, C₁-C₁₀, C₂-C₃, C₂-C₄, C₂-C₅, C₂-C₆, C₃-C₄, C₃-C₅, C₃-C₆, C₄-C₅, C₄-C₆ and C₅-C₆. For example, C₁-C₆ alkyl includes, but is not limited to, methyl, ethyl, propyl, isopropyl, butyl, isobutyl, sec-butyl, tert-butyl, pentyl, isopentyl, hexyl, etc. Alkyl can also refer to alkyl groups having up to 20 carbons atoms, such as, but not limited to heptyl, octyl, nonyl, decyl, etc.

"Alkylene" refers to a straight or branched, saturated, aliphatic radical having the number of carbon atoms indicated (i.e., C₁-C₆ means one to six carbons), and linking at least two other groups, i.e., a divalent hydrocarbon radical. The two moieties linked to the alkylene can be linked to the same atom or different atoms of the alkylene group. For instance, a straight chain alkylene can be the bivalent radical of -(CH₂)ₙ-, where n is 1, 2, 3, 4, 5 or 6. Representative alkylene groups include, but are not limited to, methylene, ethylene, propylene, isopropylene, butylene, isobutylene, sec-butylene, pentylene and hexylene.

"Alkenyl" refers to a straight chain or branched hydrocarbon having at least 2 carbon atoms and at least one double bond and having the number of carbon atom indicated (i.e., C₂-C₆ means to two to six carbons). Alkenyl can include any number of carbons, such as C₂, C₂-C₃, C₂-C₄, C₂-C₅, C₂-C₆, C₂-C₇, C₂-C₈, C₂-C₉, C₂-C₁₀, C₃, C₃-C₄, C₃-C₅, C₃-C₆, C₄, C₄-C₅, C₄-C₅, C₅, C₅-C₆, and C₆. Alkenyl groups can have any suitable number of double bonds, including, but not limited to, 1, 2, 3, 4, 5 or more. Examples of alkenyl groups include, but are not limited to, vinyl (ethenyl), propenyl, isopropenyl, 1-butenyl, 2-butenyl, isobutenyl, butadienyl, 1-pentenyl, 2-pentenyl, isopentenyl, 1,3-pentadienyl, 1,4-pentadienyl, 1-hexenyl, 2-hexenyl, 3-hexenyl, 1,3-hexadienyl, 1,4-hexadienyl, 1,5-hexadienyl, 2,4-hexadienyl, or 1,3,5-hexatrienyl.

"Alkynyl" refers to either a straight chain or branched hydrocarbon having at least 2 carbon atoms and at least one triple bond and having the number of carbon atom indicated (i.e., C₂-C₆ means to two to six carbons). Alkynyl can include any number of carbons, such as C₂, C₂-C₃, C₂-C₄, C₂-C₅, C₂-C₆, C₂-C₇, C₂-C₈, C₂-C₉, C₂-C₁₀, C₃, C₃-C₄, C₃-C₅, C₃-C₆, C₄, C₄-C₅, C₄-C₆, C₅, C₅-C₆, and C₆. Examples of alkynyl groups include, but are not limited to, acetylenyl, propynyl, 1-butynyl, 2-butynyl, butadiynyl, 1-pentynyl, 2-pentynyl, isopentynyl, 1,3-pentadiynyl, 1,4-pentadiynyl, 1-hexynyl, 2-hexynyl, 3-hexynyl, 1,3-hexadiynyl, 1,4-hexadiynyl, 1,5-hexadiynyl, 2,4-hexadiynyl, or 1,3,5-hexatriynyl.

"Cycloalkyl" refers to a saturated or partially unsaturated, monocyclic, fused bicyclic or bridged polycyclic ring assembly containing from 3 to 12 ring atoms, or the number of atoms indicated. Cycloalkyl can include any number of carbons, such as C₃-C₆, C₄-C₆, C₅-C₆, C₃-C₈, C₄-C₈, C₅-C₈, C₆-C₈, C₃-C₉, C₃-C₁₀, C₃-C₁₁, and C₃-C₁₂. Saturated monocyclic cycloalkyl rings include, for example, cyclopropyl, cyclobutyl, cyclopentyl, cyclohexyl, and cyclooctyl. Saturated bicyclic and polycyclic cycloalkyl rings include, for example, norbornane, [2.2.2] bicyclooctane, decahydronaphthalene and adamantane. Cycloalkyl groups can also be partially unsaturated, having one or more double or triple bonds in the ring. Representative cycloalkyl groups that are partially unsaturated include, but are not limited to, cyclobutene, cyclopentene, cyclohexene, cyclohexadiene (1,3- and 1,4-isomers), cycloheptene, cycloheptadiene, cyclooctene, cyclooctadiene (1,3-, 1,4- and 1,5-isomers), norbornene, and norbornadiene. When cycloalkyl is a saturated monocyclic C₃-C₈ cycloalkyl, exemplary groups include, but are not limited to cyclopropyl, cyclobutyl, cyclopentyl, cyclohexyl, cycloheptyl and cyclooctyl.

"Cycloalkylalkyl" refers to a radical having an alkyl component and a cycloalkyl component, where the alkyl component links the cycloalkyl component to the point of attachment. The alkyl component is as defined above, except that the alkyl component is at least divalent, an alkylene, to link to the cycloalkyl component and to the point of attachment. The alkyl component can include any number of carbons, such as C₁-C₆, C₁-C₂, C₁-C₃, C₁-C₄, C₁-C₅, C₂-C₃, C₂-C₄, C₂-C₅, C₂-C₆, C₃-C₄, C₃-C₅, C₃-C₆, C₄-C₅, C₄-C₆ and C₅-C₆. The cycloalkyl component is as defined above. Exemplary cycloalkyl-alkyl groups include, but are not limited to, cyclopropylmethyl, cyclobutylmethyl, cyclopentylmethyl and cyclohexylmethyl.

"Alkoxy" refers to an alkyl group having an oxygen atom that connects the alkyl group to the point of attachment: alkyl-O-. Alkoxy groups can have any suitable number of carbon atoms, such as C₁-C₆. Alkoxy groups include, for example, methoxy, ethoxy, propoxy, iso-propoxy, butoxy, 2-butoxy, iso-butoxy, sec-butoxy, tert-butoxy, pentoxy, hexoxy, etc.

"Hydroxyalkyl" refers to an alkyl group, as defined above, where at least one of the hydrogen atoms is replaced with a hydroxy group. As for the alkyl group, a hydroxyalkyl group can have any suitable number of carbon atoms, such as C₁-C₆. Exemplary hydroxyalkyl groups include, but are not limited to, hydroxymethyl, hydroxyethyl (where the hydroxy is in the 1- or 2-position), hydroxypropyl (where the hydroxy is in the 1-, 2- or 3-position), hydroxybutyl (where the hydroxy is in the 1-, 2-, 3- or 4-position), hydroxypentyl (where the hydroxy is in the 1-, 2-, 3-, 4- or 5-position), hydroxyhexyl (where the hydroxy is in the 1-, 2-, 3-, 4-, 5- or 6-position), 1,2-dihydroxyethyl, and the like.

"Alkoxyalkyl" refers to a radical having an alkyl component and an alkoxy component, where the alkyl component links the alkoxy component to the point of attachment. The alkyl component is as defined above, except that the alkyl component is at least divalent, an alkylene, to link to the alkoxy component and to the point of attachment. The alkyl component can include any number of carbons, such as C₁-C₂, C₁-C₃, C₁-C₄, C₁-C₅, C₁-C₆, C₂-C₃, C₂-C₄, C₂-C₅, C₂-C₆, C₃-C₄, C₃-C₅, C₃-C₆, C₄-C₅, C₄-C₆ and C₅-C₆. The alkoxy component is as defined above. Examples of the alkoxy-alkyl group include, but are not limited to, 2-ethoxy-ethyl and methoxymethyl.

"Halogen" or "halo" refers to fluoro, chloro, bromo, or iodo.

"Haloalkyl" refers to alkyl, as defined above, where some or all of the hydrogen atoms are replaced with halogen atoms. As for alkyl group, haloalkyl groups can have any suitable number of carbon atoms, such as C₁-C₆. For example, haloalkyl includes trifluoromethyl, fluoromethyl, 2,2,2-trifluoroethyl, etc. In some instances, the term "perfluoro" can be used to define a compound or radical where all the hydrogens are replaced with fluorine. For example, perfluoromethyl refers to 1,1,1-trifluoromethyl.

"Amino" as used herein, and unless otherwise specified, refers to -NH₂.

"Alkylamino" as used herein, and unless otherwise specified, refers to an -NHR radical where R is alkyl as defined herein, or an N-oxide derivative thereof. In some embodiments, alkylamino is C₁-C₆-alkyl-amino. In some embodiments, C₁-C₆-alkyl-amino is methylamino, ethylamino, *n-,* iso-propylamino, *n-, iso-,* tert-butylamino, or methylamino-N-oxide, and the like.

"Dialkylamino" as used herein, and unless otherwise specified, refers to an -NR'R radical where R and R' are independently alkyl as defined herein, or an N-oxide derivative thereof. In some embodiments, dialkylamino is di-C₁-C₆-alkyl-amino. In some embodiments, di-C₁-C₆-alkyl-amino is dimethylamino, methyl-ethylamino, diethylamino, or dimethylamino-N-oxide, and the like.

"Aminoalkyl" as used herein, unless otherwise specified, refers to an alkyl group substituted with one or two NH₂. In some embodiments, aminoalkyl is amino-C₁-C₆-alkyl.

"Alkylaminoalkyl" as used herein, unless otherwise specified, refers to an alkyl group substituted with one or two -NH(alkyl) groups. In some embodiments, alkylaminoalkyl is C₁-C₆-alkyl-amino-C₁-C₆-alkyl.

"Dialkylaminoalkyl" as used herein, unless otherwise specified, refers to an alkyl group substituted with one or two -N(alkyl)₂ groups. In some embodiments, dialkylaminoalkyl is di-C₁-C₆-alkyl-amino-C₁-C₆-alkyl.

"Hydroxyamino" as used herein, unless otherwise specified, refers to -NHOH.

"N-alkylhydroxyamino" as used herein, unless otherwise specified, refers to the amine hydrogen of -NHOH is substituted with alkyl as defined herein. In some embodiments, N-alkyl hydroxyamino is *N*-C₁-C₆ alkyl-hydroxyamino. In some embodiments, *N*-C₁-C₆ alkyl-hydroxyamino is *N*-methylhydroxyamino, N-ethylhydroxyamino, N-(n-, iso-propyl)-hydroxyamino, or *N*-(*n*-, *iso*-, *tert*-butyl)hydroxyamino, and the like.

"Heterocycloalkyl" refers to a saturated ring system having from 3 to 12 ring members and from 1 to 4 heteroatoms of N, O and S. The heteroatoms can also be oxidized, such as, but not limited to, -S(O)- and -S(O)₂-. Heterocycloalkyl groups can include any number of ring atoms, such as, 3 to 6, 4 to 6, 5 to 6, 3 to 8, 4 to 8, 5 to 8, 6 to 8, 3 to 9, 3 to 10, 3 to 11, or 3 to 12 ring members. Any suitable number of heteroatoms can be included in the heterocycloalkyl groups, such as 1, 2, 3, or 4, or 1 to 2, 1 to 3, 1 to 4, 2 to 3, 2 to 4, or 3 to 4. The heterocycloalkyl group can include groups such as aziridinyl, azetidinyl, pyrrolidinyl, piperidinyl, azepanyl, azocanyl, quinuclidinyl, pyrazolidinyl, imidazolidinyl, piperazinyl (1,2-, 1,3- and 1,4-isomers), oxiranyl, oxetanyl, tetrahydrofuranyl, oxanyl (tetrahydropyranyl), oxepanyl, thiiranyl, thietanyl, thiolanyl (tetrahydrothiophenyl), thianyl (tetrahydrothiopyranyl), oxazolidinyl, isoxazolidinyl, thiazolidinyl, isothiazolidinyl, dioxolanyl, dithiolanyl, morpholinyl, thiomorpholinyl, dioxanyl, or dithianyl. The heterocycloalkyl groups can also be fused to aromatic or non-aromatic ring systems to form members including, but not limited to, indoline. Heterocycloalkyl groups can be unsubstituted or substituted. For example, heterocycloalkyl groups can be substituted with C₁-C₆ alkyl or oxo (=O), among many others.

The heterocycloalkyl groups can be linked via any position on the ring. For example, aziridinyl can be 1- or 2- aziridinyl, azetidinyl can be 1- or 2- azetidinyl, pyrrolidinyl can be 1-, 2- or 3-pyrrolidinyl, piperidinyl can be 1-, 2-, 3- or 4-piperidinyl, pyrazolidinyl can be 1-, 2-, 3-, or 4-pyrazolidinyl, imidazolidinyl can be 1-, 2-, 3- or 4-imidazolidinyl, piperazinyl can be 1-, 2-, 3- or 4-piperazinyl, tetrahydrofuranyl can be 1- or 2-tetrahydrofuranyl, oxazolidinyl can be 2-, 3-, 4- or 5-oxazolidinyl, isoxazolidinyl can be 2-, 3-, 4- or 5-isoxazolidinyl, thiazolidinyl can be 2-, 3-, 4- or 5-thiazolidinyl, isothiazolidinyl can be 2-, 3-, 4- or 5- isothiazolidinyl, and morpholinyl can be 2-, 3- or 4-morpholinyl.

"N-linked heterocycloalkyl" or "nitrogen-linked heterocycloalkyl" refers to the heterocycloalkyl group linked via N-position on the ring. For example, N-linked aziridinyl is aziridin-1-yl, N-linked azetidinyl is azetidin-1-yl, N-linked pyrrolidinyl is pyrrolidin-1-yl, N-linked piperidinyl is piperidin-1-yl, N-linked pyrazolidinyl is pyrazolidin-1-yl or pyrazolidin-2-yl, N-linked imidazolidinyl can be imidazolidin-1-yl or imidazolidin-3-yl, N-linked piperazinyl is piperazin-1-yl or piperazin-4-yl, N-linked oxazolidinyl is oxazolidin-3-yl, N-linked isoxazolidiny is isoxazolidin-2-yl, N-linked thiazolidinyl is thiazolidin-3-yl, N-linked isothiazolidinyl is isothiazolidin-2-yl, and N-linked morpholinyl is 4-morpholinyl.

When heterocycloalkyl includes 3 to 8 ring members and 1 to 3 heteroatoms, representative members include, but are not limited to, pyrrolidinyl, piperidinyl, tetrahydrofuranyl, oxanyl, tetrahydrothiophenyl, thianyl, pyrazolidinyl, imidazolidinyl, piperazinyl, oxazolidinyl, isoxzoalidinyl, thiazolidinyl, isothiazolidinyl, morpholinyl, thiomorpholinyl, dioxanyl and dithianyl. Heterocycloalkyl can also form a ring having 5 to 6 ring members and 1 to 2 heteroatoms, with representative members including, but not limited to, pyrrolidinyl, piperidinyl, tetrahydrofuranyl, tetrahydrothiophenyl, pyrazolidinyl, imidazolidinyl, piperazinyl, oxazolidinyl, isoxazolidinyl, thiazolidinyl, isothiazolidinyl, and morpholinyl.

"Protecting group" refers to a compound that renders a functional group unreactive to a particular set of reaction conditions, but that is then removable in a later synthetic step so as to restore the functional group to its original state. Such protecting groups are well known to one of ordinary skill in the art and include compounds that are disclosed in "Protective Groups in Organic Synthesis", 4th edition, T. W. Greene and P. G. M. Wuts, John Wiley & Sons, New York, 2006, which is incorporated herein by reference in its entirety.

"Salt" refers to acid or base salts of the compounds of the present invention. Illustrative examples of pharmaceutically acceptable salts are mineral acid (hydrochloric acid, hydrobromic acid, phosphoric acid, and the like) salts, organic acid (acetic acid, propionic acid, glutamic acid, citric acid and the like) salts, quaternary ammonium (methyl iodide, ethyl iodide, and the like) salts. It is understood that the pharmaceutically acceptable salts are non-toxic. Additional information on suitable pharmaceutically acceptable salts can be found in Remington's Pharmaceutical Sciences, 17th ed., Mack Publishing Company, Easton, Pa., 1985, which is incorporated herein by reference.

Pharmaceutically acceptable salts of the acidic compounds of the present invention are salts formed with bases, namely cationic salts such as alkali and alkaline earth metal salts, such as sodium, lithium, potassium, calcium, magnesium, as well as ammonium salts, such as ammonium, trimethyl-ammonium, diethylammonium, and tris-(hydroxymethyl)-methyl-ammonium salts.

Similarly acid addition salts, such as of mineral acids, organic carboxylic and organic sulfonic acids, e.g., hydrochloric acid, methanesulfonic acid, maleic acid, are also possible provided a basic group, such as pyridyl, constitutes part of the structure.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

"Isomer" refers to compounds with the same chemical formula but which are structurally distinguishable. Certain compounds of the present invention possess asymmetric carbon atoms (optical centers) or double bonds; the racemates, diastereomers, geometric isomers and individual isomers are all intended to be encompassed within the scope of the present invention.

"Tautomer" refers to one of two or more structural isomers which exist in equilibrium and which are readily converted from one form to another.

"Solvate" refers to a compound provided herein or a salt thereof, that further includes a stoichiometric or non-stoichiometric amount of solvent bound by non-covalent intermolecular forces. Where the solvent is water, the solvate is a hydrate.

"Hydrate" refers to a compound that is complexed to at least one water molecule. The compounds of the present invention can be complexed with from 1 to 10 water molecules.

"Substantially free of" or "substantially in the absence of" stereoisomers with respect to a composition refers to a composition that includes at least 85 or 90% by weight, In some embodiments 95%, 98 %, 99% or 100% by weight, of a designated stereoisomer of a compound in the composition. In some embodiments, in the methods and compounds provided herein, the compounds are substantially free of stereoisomers.

"Isolated" with respect to a composition refers to a composition that includes at least 85%, 90%, 95%, 98%, 99% to 100% by weight, of a specified compound, the remainder comprising other chemical species or stereoisomers.

"Composition" as used herein is intended to encompass a product comprising the specified ingredients in the specified amounts, as well as any product, which results, directly or indirectly, from combination of the specified ingredients in the specified amounts. By "pharmaceutically acceptable" it is meant the carrier, diluent or excipient must be compatible with the other ingredients of the formulation and deleterious to the recipient thereof.

"Pharmaceutically acceptable excipient" refers to a substance that aids the administration of an active agent to and absorption by a subject. Pharmaceutical excipients useful in the present invention include, but are not limited to, binders, fillers, disintegrants, lubricants, coatings, sweeteners, flavors and colors. One of skill in the art will recognize that other pharmaceutical excipients are useful in the present invention.

"IC₅₀" refers to an amount, concentration or dosage of a particular test compound that achieves a 50% inhibition of a maximal response in an assay that measures such response.

"Inhibition", "inhibits" and "inhibitor" refer to a compound that prohibits or a method of prohibiting, a specific action or function.

"Administering" refers to oral administration, administration as a suppository, topical contact, parenteral, intravenous, intraperitoneal, intramuscular, intralesional, intranasal or subcutaneous administration, intrathecal administration, or the implantation of a slow-release device e.g., a mini-osmotic pump, to the subject.

"Treat", "treating" and "treatment" refer to any indicia of success in the treatment or amelioration of an injury, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters; including the results of a physical examination, neuropsychiatric exams, and/or a psychiatric evaluation.

"Treating" or "treatment" of a birthmark, in some embodiments, refers to ameliorating the appearance or severity of the birthmark that exists in a subject. In some embodiments, "treating" or "treatment" includes ameliorating at least one physical parameter. In some embodiments, "treating" or "treatment" includes modulating the appearance of a birthmark physically (e.g., stabilization or reduction of a discernible symptom or characteristic of the birthmark, such as the size, volume, color, thickness, and/or texture of the birthmark). In some embodiments, "treating" or "treatment" includes the reduction or elimination of one or more of the following: size, color, thickness, and/or texture of the birthmark.

"Prophylactically treating" and "preventing" malignant progression of a birthmark, in some embodiments, refer to reducing the risk in a subject of the malignant progression of a birthmark. In some embodiments, "prophylactically treating" or "preventing" includes delaying the onset of a malignancy which results from a birthmark. In some embodiments, "prophylactically treating" or "preventing" includes retarding the progression of the birthmark to a malignancy or of ameliorating the one or more symptoms associated with the progression of a birthmark to malignancy.

"Patient" or "subject" refers to a living organism suffering from or prone to a disease or condition that can be treated by administration of a pharmaceutical composition as provided herein. Non-limiting examples include humans, other mammals, bovines, rats, mice, dogs, monkeys, goat, sheep, cows, deer, and other non-mammalian animals. In some embodiments, the patient is human.

"Therapeutically effective amount" or "effective amount" refers to an amount of a compound or of a pharmaceutical composition useful for treating or ameliorating an identified disease or condition (e.g., a birthmark as described herein), or for exhibiting a detectable therapeutic or inhibitory effect. The exact amounts will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

"Therapeutically effective amount" or "Effective amount", in some embodiments, refers to an amount of a compound or composition that, when administered to a subject for treating the appearance of a birthmark, is sufficient to alter the appearance of the birthmark. An "effective amount" can vary depending on, *inter alia,* the compound, the birthmark and its severity, the size of the birthmark to be treated, and the age, weight, *etc.,* of the subject to be treated.

"Topical" means application of a suitable compound (*e.g*. active agent) or composition comprising a compound (*e.g.* active agent) to the skin to treat a birthmark. "Subcutaneous" means application of a suitable compound (*e.g.* active agent) or composition comprising a compound (*e.g.* active agent) to the layers below the epidermis and dermis. "Intradermal" means application of a suitable compound (*e.g.* active agent) or composition comprising a compound (*e.g.* active agent) in the dermal or hypodermal layers. Intralesional" means injection of a suitable compound (*e.g.* active agent) or composition comprising a compound (*e.g.* active agent) at the site of the lesion (*e.g.* birthmark).

In some embodiments, "topical" means application of a suitable compound (*e.g.* active agent) or composition comprising a compound (*e*.*g*. active agent) to the skin with adequate penetration of the epidermis or dermis to treat the birthmark of the epidermis and/or dermis. In some embodiments of topical application, the compound or composition penetrates the epidermis or dermis without significant systemic exposure nor intent to treat or prevent a disease of another organ system. In some embodiments, "subcutaneous" means injection of a suitable compound (*e.g.* active agent) or composition comprising a compound *(*e.g. active agent) into the layers below the epidermis and dermis. In some embodiments, "intradermal" means injection of a compound *(*e.g. active agent) or composition comprising a compound (*e.g.* active agent) into the dermal layers. In some embodiments, "intralesional" means injection of a compound (*e.g.* active agent) or composition comprising a compound (*e.g.* active agent) directly into a lesion, such as a birthmark, with the objective of treating a birthmark or a lesion.

The disclosure provides "soft" MEK inhibitors, compositions comprising "soft" MEK inhibitors, and methods of treating and/or ameliorating a birthmark (*e.g.,* a MEK-inhibitor responsive or MEK-mediated birthmark). For example, the methods described herein provide administration, *e.g.,* local or non-systemic, *e.g.,* topical, subcutaneous, transdermal, intradermal, or intralesional administration, of MEK inhibitors, *e.g.,* "soft" MEK inhibitors, *e.g.,* "soft" MEK inhibitors described herein, whereby one or more side effects exhibited with systemic exposure, *e.g.,* known one or more side effects exhibited with MEK inhibitors designed for systemic delivery, are significantly reduced.

In some embodiments, "soft MEK inhibitor" is a compound which inhibits MEK1 and/or 2 and is characterized by a predictable and controllable metabolism/degradation to non-toxic and biologically less active or inactive (*i*.*e*. does not inhibit, or inhibits to a lesser degree, MEK1 and/or 2) products after they have achieved their therapeutic role in the skin.

"Hard MEK inhibitor" refers to a MEK inhibitor known in the art. In some embodiments, a hard MEK inhibitor is designed for oral bioavailability. This is necessary to deliver therapeutically effective levels of MEK inhibitor to peripheral lesions with systemic delivery. Hard MEK inhibitor include, for example, PD0325901; PD184161; SMK-17; AS703026 (Pimasertib, MSC1936369); RO-4987655; Selumetinib (AZD6244, ARRY142886); Binimetinib (MEK162, ARRY-162, ARRY-438162); Refametinib; Cobimetinib (GDC-0973, XL518); GDC-0623; AZD8330 (ARRY-424704); CI-1040 (PD184352); PD198306; Trametinib; RO-4987655; GDC-0623; TAK-733; WX-554; CH5126766 (also as RO5126766); G-573; Arry 300; SHR 7390; MSC2015103B (also known as AS-703988); CS 3006; and LY 2228820 (also know as Ralimetinib).

While not wishing to be bound by theory, it is believed that soft MEK inhibitors, *e.g.,* such as the "soft" MEK inhibitors described herein, are more metabolically labile than known "hard" MEK inhibitors. Due to their inherent metabolic instability, *e.g.,* for degradation upon reaching the systemic circulation, "soft" MEK inhibitors, *e.g.,* such as the "soft" MEK inhibitors described herein, are dermally active but have low systemic exposure upon local or non-systemic administration, *e.g.,* topical, subcutaneous, intradermal, or intralesional administration, because they rapidly degrade upon exposure to plasma or blood or hepatic metabolic enzymes. Unlike "soft" MEK inhibitors, known MEK inhibitors have been historically designed for oral bioavailability, which requires good stability in plasma or blood and good stability to hepatic metabolism necessary to permit systemic delivery at therapeutically effective or effective levels, and are more prone to one or more unwanted side effects and increased toxicity. As a result, "soft" MEK inhibitors, *e.g.,* such as the soft MEK inhibitors described herein, are less systemically toxic.

"A," "an," or "a(n)", when used in reference to a group of substituents or "substituent group" herein, mean at least one. For example, where a compound is substituted with "an" alkyl or aryl, the compound is optionally substituted with at least one alkyl and/or at least one aryl, wherein each alkyl and/or aryl is optionally different. In another example, where a compound is substituted with "a" subsitutent group, the compound is substituted with at least one substituent group, wherein each subsitutent group is optionally different.

### 111. METHODS

In one aspect, provided herein is a method of treating a birthmark. The method includes administering to a subject in need thereof a therapeutically effective amount or an effective amount of a compound selected from the group consisting of formula (1), (II), (111), (IV), and (V): 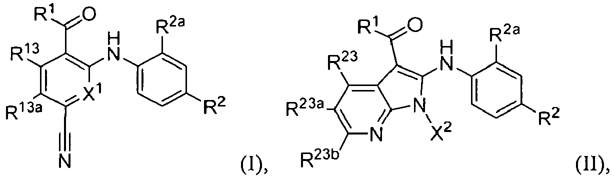 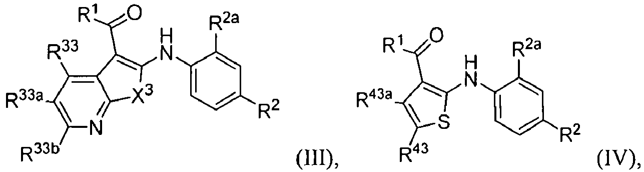 and 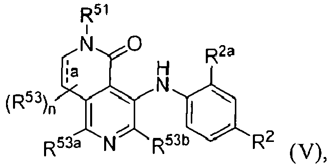 or a N-oxide, stereoisomer, mixture of stereoisomers, and/or a pharmaceutically acceptable salt thereof,
wherein:
X¹ is -CR^{13b} or N; X² is C₁-C₆ alkyl; X³ is S or O;
subscript n is an integer from 0 to 2;
bond "a" is a single or double bond;
R¹ is -OR⁴, -NR⁵R^{5a}, -N(OR^{5b})R^{5a}, or a N-linked heterocycloalkyl which is unsubstituted or substituted with one or two R⁶;
R² is halo, C₁-C₆ alkyl, -S-C₁-C₆ alkyl, C₃-C₈ cycloalkyl, C₂-C₆ alkenyl, or C₂-C₆ alkynyl;
R^{2a} is halo or C₁-C₆ alkyl;
R⁴, R⁵, and R^{5b} are each independently hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶;
R^{5a} is hydrogen or C₁-C₆ alkyl;
each R⁶ is independently halo, hydroxy, oxo, C₁-C₆ alkyl, C₃-C₈ cycloalkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl;
R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino;
R¹³, R^{13a}, and R^{13b} are each independently hydrogen, halo, C₁-C₆ alkyl, C₂-C₆ alkenyl, or C₂-C₆ alkynyl;
R²³, R^{23a}, and R^{23b} are each independently hydrogen, halo, C₁-C₆ alkyl, C₂-C₆ alkenyl, C₂-C₆ alkynyl, C₁-C₆ alkoxy, or C₃-C₈ cycloalkoxy;
R³³, R^{33a}, and R^{33b} are each independently hydrogen, halo, C₁-C₆ alkyl, C₂-C₆ alkenyl, C₂-C₆ alkynyl, C₁-C₆ alkoxy, or C₃-C₈ cycloalkoxy;
R⁴³ is cyano, -C(O)NR⁴⁸R^{48a}, or -C(O)R⁴⁶;
R^{43a} is hydrogen, halo, C₁-C₆ alkyl; or
R⁴³ and R^{43a} together form -CH₂CH₂C(O)- or -CH₂CH₂CH₂C(O)-, each of which is unsubstituted or substituted with one or two R⁴⁹;
R⁴⁶ is hydrogen, C₁-C₆ alkyl, C₁-C₆ haloalkyl, C₃-C₈ cycloalkyl or heterocycloalkyl;
R⁴⁸ and R^{48a} are independently hydrogen or C₁-C₆ alkyl; and
each R⁴⁹ is independently C₁-C₆ alkyl, C₃-C₈ cycloalkyl, or C₃-C₈ cycloalkyl-C₁-C₆ alkyl;
R⁵¹ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, R⁷-C(O)-C₁-C₆ alkyl, or -OR⁵⁴, wherein each of the C₃-C₈ cycloalkyl and heterocycloalkyl groups is unsubstituted or substituted with one to six R⁶;
each R⁵³ is independently halo or C₁-C₆ alkyl;
R^{53a} and R^{53b} are each independently hydrogen, halo, C₁-C₆ alkyl, C₂-C₆ alkenyl, or C₂-C₆ alkynyl; and
R⁵⁴ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶.

In some embodiments, the method includes administering the subject in need thereof a therapeutically effective amount or an effective amount of a compound disclosed herein, e.g., a compound having any one of formula (1), (II), (III), (IV), and (V), and compounds in Tables 1-5, including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt, solvate, prodrug, phosphate, or active metabolite thereof.

In some embodiments, the method includes administering the subject in need thereof a therapeutically effective amount or an effective amount of a compound having formula (I) and compounds in Table 1, including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt or solvate thereof.

In some embodiments, the method includes administering the subject in need thereof a therapeutically effective amount or an effective amount of a compound having formula (II) and compounds in Table 2, including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt or solvate thereof.

In some embodiments, the method includes administering the subject in need thereof a therapeutically effective amount or an effective amount of a compound having formula (III) and compounds in Table 3, including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt or solvate thereof.

In some embodiments, the method includes administering the subject in need thereof a therapeutically effective amount or an effective amount of a compound having formula (IV) and compounds in Table 4, including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt or solvate thereof.

In some embodiments, the method includes administering the subject in need thereof a therapeutically effective amount or an effective amount of a compound having formula (V) and compounds in Table 5, including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt or solvate thereof.

In some embodiments, the birthmark is a port-wine stain (capillary malformation). In some embodiments, provided herein is a method of treating a port-wine stain (capillary malformation) birthmark to reduce the cosmetic disfigurement or progression of the birthmark. In some embodiments, provided herein is a method of prophylactically treating a port-wine stain (capillary malformation) birthmark to prevent the progression of the birthmark, delaying the onset of the birthmark, or delaying the progression of the birthmark.

In some embodiments, the birthmark is epidermal nevi. In some embodiments, provided herein is a method of treating epidermal nevi to reduce the cosmetic disfigurement or progression of the birthmark. In some embodiments, provided herein is a method of prophylactically treating epidermal nevi to prevent the progression of the birthmark, delaying the onset of the birthmark, or delaying the progression of the birthmark.

In some embodiments, the birthmark is nevus sebaceous. In some embodiments, provided herein is a method of treating a nevus sebaceous birthmark to reduce the cosmetic disfigurement or progression of the birthmark. In some embodiments, provided herein is a method of prophylactically treating a nevus sebaceous birthmark to prevent the progression of the birthmark, delaying the onset of the birthmark, or delaying the progression of the birthmark.

In some embodiments the birthmark is nevus cellular nevi. In some embodiments, provided herein is a method of treating nevus cellular nevi to reduce the cosmetic disfigurement or progress of the birthmark. In some embodiments, provided herein is a method of prophylactically treating nevus cellular nevi to prevent the progression of the birthmark, delaying the onset of the birthmark, or delaying the progression of the birthmark.

In some embodiments, the birthmark is dysplastic nevi. In some embodiments, provided herein is a method of treating dysplastic nevi to reduce the cosmetic disfigurement or progression of the birthmark. In some embodiments, provided herein is a method of prophylactically treating dysplastic nevi to prevent the progression of the birthmark, delaying the onset of the birthmark, or delaying the progression of the birthmark.

In some embodiments, the birthmark is a nevus spilus. In some embodiments, provided herein is a method of treating a nevus spilus birthmark to reduce the cosmetic disfigurement or progression of the birthmark. In some embodiments, provided herein is a method of prophylactically treating a nevus spilus birthmark to prevent the progression of the birthmark, delaying the onset of the birthmark, or delaying the progression of the birthmark.

In some embodiments, the birthmark is an arterio-venous malformation in the skin. In some embodiments, provided herein is a method of treating an arterio-venous malformation to reduce the cosmetic disfigurement or progression of the birthmark. In some embodiments, provided herein is a method of prophylactically treating an arterio-venous malformation to prevent the progression of the birthmark, delaying the onset of the birthmark, or delaying the progression of the birthmark.

In some embodiments, the birthmark is a lymphatic malformation. In some embodiments, provided herein is a method of treating a lymphatic malformation to reduce the risk of the cosmetic disfigurement or progression of the birthmark. In some embodiments, provided herein is a method of prophylactically treating a lymphatic malformation to prevent the progression of the birthmark, delaying the onset of the birthmark, or delaying the progression of the birthmark.

In some embodiments, the birthmark is a congenital melanocytic nevus. In some embodiments, provided herein is a method of treating a congenital melanocytic nevus to reduce the risk of cosmetic disfigurement or progression of the birthmark. In some embodiments, provided herein is a method of prophylactically treating a congenital melanocytic nevus to prevent the progression of the birthmark, delaying the onset of the birthmark, or delaying the progression of the birthmark.

In some embodiments, the compounds described herein are used for the reduction of a birthmark in a subject in need thereof.

In some embodiments, the compounds described herein are used for the amelioration of a birthmark in a subject in need thereof

In some embodiments, the compounds described herein are used for prevention of a birthmark (e.g., a MEK-inhibitor responsive or MEK-mediated birthmark) and/or prevention of worsening of a birthmark (e.g., where the birthmark may progress to a proliferative disease) in a subject in need thereof.

In some embodiments, the subject in need thereof is a human.

The birthmark is not cafe au lait spots.

In some embodiments, administering includes contacting the soft MEK inhibitor with the skin of the subject, *e.g.,* an affected region of the skin, *e.g.,* a region of the skin having a birthmark.

In some embodiments, the appearance of a birthmark is reduced, e*.g.,* the size, volume, or the total surface area is reduced, by at least about 15% relative to the reference standard (*e.g.,* the size of the birthmark prior to start of treatment), thereby treating the subject. In some embodiments, the size, volume, or the total surface area on skin is reduced, by at least about 15%, by at least about 20%, by at least about 25%, by at least about 30%, by at least about 35%, by at least about 40%, by at least about 45%, by at least about 50%, by at least about 55%, by at least about 60% relative to the reference standard. In some embodiments, the reference standard is the size of the birthmark prior to start of treatment.

### Assay Methods

Compounds can be assayed for efficacy in treating a birthmark (e.g., a MEK-inhibitor responsive or MEK-mediated birthmark) where the subject is in need thereof according to any assay known to those of skill in the art. Exemplary assay methods are provided elsewhere herein.

### IV. COMPOUNDS

The present invention provides a compound for use in the method for treating a birthmark as defined and decribed herein, wherein the compound is selected from the group consisting of formula (1), (11), (111), (IV), and (V): 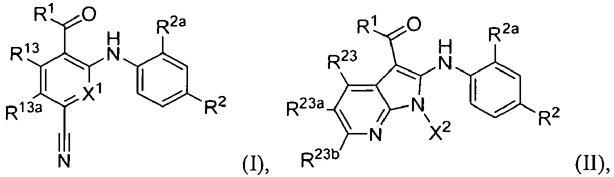 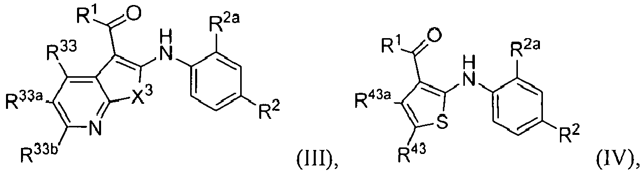 and 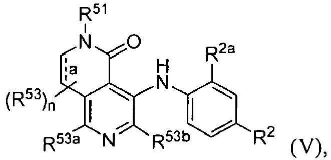 or a N-oxide, stereoisomer, mixture of stereoisomers, and/or a pharmaceutically acceptable salt thereof,
wherein:
X¹ is -CR^{13b} or N; X² is C₁-C₆ alkyl; X³ is S or O;
subscript n is an integer from 0 to 2;
bond "a" is a single or double bond;
R¹ is -OR⁴, -NR⁵R^{5a}, -N(OR^{5b})R^{5a}, or a N-linked heterocycloalkyl which is unsubstituted or substituted with one or two R⁶;
R² is halo, C₁-C₆ alkyl, -S-C₁-C₆ alkyl, C₃-C₈ cycloalkyl, C₂-C₆ alkenyl, or C₂-C₆ alkynyl;
R^{2a} is halo or C₁-C₆ alkyl;
R⁴, R⁵, and R^{5b} are each independently hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶;
R^{5a} is hydrogen or C₁-C₆ alkyl;
each R⁶ is independently halo, hydroxy, oxo, C₁-C₆ alkyl, C₃-C₈ cycloalkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl;
R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino;
R⁸ is hydroxy, C₁-C₃ alkyl, or C₁-C₃ alkoxy;
R¹³, R^{13a}, and R^{13b} are each independently hydrogen, halo, C₁-C₆ alkyl, C₂-C₆ alkenyl, or C₂-C₆ alkynyl;
R²³, R^{23a}, and R^{23b} are each independently hydrogen, halo, C₁-C₆ alkyl, C₂-C₆ alkenyl, C₂-C₆ alkynyl, C₁-C₆ alkoxy, or C₃-C₈ cycloalkoxy;
R³³, R^{33a}, and R^{33b} are each independently hydrogen, halo, C₁-C₆ alkyl, C₂-C₆ alkenyl, C₂-C₆ alkynyl, C₁-C₆ alkoxy, or C₃-C₈ cycloalkoxy;
R⁴³ is cyano, -C(O)NR⁴⁸R^{48a}, or -C(O)R⁴⁶;
R^{43a} is hydrogen, halo, C₁-C₆ alkyl; or
R⁴³ and R^{43a} together form -CH₂CH₂C(O)- or -CH₂CH₂CH₂C(O)-, each of which is unsubstituted or substituted with one or two R⁴⁹;
R⁴⁶ is hydrogen, C₁-C₆ alkyl, C₁-C₆ haloalkyl, C₃-C₈ cycloalkyl or heterocycloalkyl;
R⁴⁸ and R^{48a} are independently hydrogen or C₁-C₆ alkyl; and each R⁴⁹ is independently C₁-C₆ alkyl, C₃-C₈ cycloalkyl, or C₃-C₈ cycloalkyl-C₁-C₆ alkyl;
R⁵¹ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, R⁷-C(O)-C₁-C₆ alkyl, or-OR⁵⁴, wherein each of the C₃-C₈ cycloalkyl and heterocycloalkyl groups is unsubstituted or substituted with one to six R⁶;
each R⁵³ is independently halo or C₁-C₆ alkyl;
R^{53a} and R^{53b} are each independently hydrogen, halo, C₁-C₆ alkyl, C₂-C₆ alkenyl, or C₂-C₆ alkynyl; and
R⁵⁴ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶.

In some embodiments, the cycloalkyl group provided in formula (1), (II), (III), (IV) and (V) is a saturated monocyclic C₃-C₈ cycloalkyl. In some embodiments, the C₃-C₈ cycloalkyl group, as alone or as part of C₃-C₈ cycloalkyl-C₁-C₆ alkyl is cyclopropyl or cyclobutyl. In some embodiments, the C₃-C₈ cycloalkyl group, as alone or as part of C₃-C₈ cycloalkyl-C₁-C₆ alkyl, is unsubstituted. In some embodiments, the C₃-C₈ cycloalkyl group, as alone or as part of C₃-C₈ cycloalkyl-C₁-C₆ alkyl, is substituted with one to six R⁶ and R⁶ is as defined and described herein.

With reference to R⁶ as one or more substituents of the C₃-C₈ cycloalkyl group, in some embodiments, each R⁶ is independently halo, hydroxy, oxo, C₁-C₆ alkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl. In some embodiments, each R⁶ is independently halo, hydroxy, C₁-C₆ alkyl, C₁-C₆ alkoxy, C₁-C₆-hydroxyalkyl, C₁-C₆ haloalkyl, amino, C₁-C₆ alkylamino, or di-(C₁-C₆ alkyl)amino. In some embodiments, each R⁶ is independently halo, hydroxy, C₁-C₆ alkyl, C₁-C₆ alkoxy, or amino. In some embodiments, each R⁶ is independently hydroxy or C₁-C₆ alkyl. In some embodiments, each R⁶ is independently hydroxy or amino.

In some embodiments, the heterocycloalkyl group provided in formula (1), (11), (111), (IV) and (V) is a 3 to 8 membered heterocycloalkyl having 1 to 3 ring member heteroatoms selected from N, O, and S. In some embodiments, heterocycloalkyl is a 3 to 6 membered heterocycloalkyl having 1 to 2 heteroatoms of N or O. In some embodiments, the heterocycloalkyl group, as alone or as part of heterocycloalkyl-C₁-C₆ alkyl, is unsubstituted. In some embodiments, the heterocycloalkyl group, as alone or as part of heterocycloalkyl-C₁-C₆ alkyl, is substituted one to six R⁶ and R⁶ is as defined and described herein. In some embodiments, the N-linked heterocycloalkyl group is substituted one or two R⁶ and R⁶ is as defined and described herein.

With reference to R⁶ as one or more substituents of the heterocycloalkyl group or the N-linked heterocycloalkyl, in some embodiments, each R⁶ is independently halo, hydroxy, oxo, C₁-C₆ alkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl. In some embodiments, each R⁶ is independently halo, hydroxy, oxo, C₁-C₆ alkyl, C₁-C₆ alkoxy, C₁-C₆-hydroxyalkyl, C₁-C₆ haloalkyl, amino, C₁-C₆ alkylamino, or di-(C₁-C₆ alkyl)amino. In some embodiments, each R⁶ is independently halo, hydroxy, oxo, C₁-C₆ alkyl, C₁-C₆ alkoxy, or amino. In some embodiments, each R⁶ is independently hydroxy or C₁-C₆ alkyl. In some embodiments, each R⁶ is independently hydroxy, oxo, or amino. In some embodiments, each R⁶ is independently hydroxy or amino.

In some embodiments, the compounds useful in the present methods are compounds of formula (I).

With reference to formula (I), in some embodiments, X¹ is -CR^{13b}, and the compound is represented by formula (la): 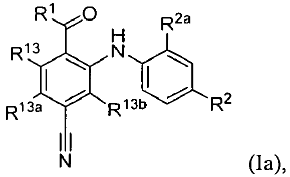 wherein R¹, R², R^{2a}, R¹³, R^{13a}, and R^{13b} are as defined herein in any aspect or embodiment described herein.

In some embodiments of formula (Ia), R¹³, R^{13a}, and R^{13b} are each independently hydrogen, halo, or C₁-C₆ alkyl.

In some embodiments of formula (Ia), R¹³ is hydrogen, halo, or C₁-C₆ alkyl. In some embodiments, R¹³ is hydrogen. In some embodiments, R¹³ is halo. In some embodiments, R¹³ is fluoro, chloro, bromo, or iodo. In some embodiments, R¹³ is fluoro. In some embodiments, R¹³ is C₁-C₆ alkyl. In some embodiments, R¹³ is methyl, ethyl, propyl, isopropyl, butyl, isobutyl, sec-butyl, tert-butyl, pentyl, isopentyl, or hexyl. In some embodiments, R¹³ is methyl.

In some embodiments of formula (Ia), R^{13a} is hydrogen, halo, or C₁-C₆ alkyl. In some embodiments, R^{13a} is hydrogen. In some embodiments, R^{13a} is halo. In some embodiments, R^{13a} is fluoro, chloro, bromo, or iodo. In some embodiments, R^{13a} is fluoro. In some embodiments, R^{13a} is C₁-C₆ alkyl. In some embodiments, R^{13a} is methyl, ethyl, propyl, isopropyl, butyl, isobutyl, sec-butyl, tert-butyl, pentyl, isopentyl, or hexyl. In some embodiments, R^{13a} is methyl.

In some embodiments of formula (Ia), R^{13b} is hydrogen, halo, or C₁-C₆ alkyl. In some embodiments, R^{13b} is hydrogen. In some embodiments, R^{13b} is halo. In some embodiments, R^{13b} is fluoro, chloro, bromo, or iodo. In some embodiments, R^{13b} is fluoro. In some embodiments, R^{13b} is C₁-C₆ alkyl. In some embodiments, R^{13b} is methyl, ethyl, propyl, isopropyl, butyl, isobutyl, sec-butyl, tert-butyl, pentyl, isopentyl, or hexyl. In some embodiments, R^{13b} is methyl.

In some embodiments of formula (Ia), R¹³, R^{13a}, and R^{13b} are each hydrogen. In some embodiments of formula (Ia), R¹³ and R^{13a} are each hydrogen and R^{13b} is halo. In some embodiments of formula (Ia), R¹³ and R^{13a} are each hydrogen and R^{13b} is fluoro.

Returning to formula (I), in some embodiments, X¹ is N, and the compound is represented by formula (Ib): 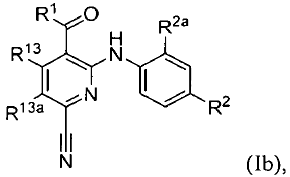 wherein R¹, R², R^{2a}, R^{13a} and R^{13a} are as defined herein in any aspect or embodiment described herein.

In some embodiments of formula (Ib), R¹³ and R^{13a} are each independently hydrogen, halo, or C₁-C₆ alkyl.

In some embodiments of formula (Ib), R¹³ is hydrogen, halo, or C₁-C₆ alkyl. In some embodiments, R¹³ is hydrogen. In some embodiments, R¹³ is halo. In some embodiments, R¹³ is fluoro, chloro, bromo, or iodo. In some embodiments, R¹³ is fluoro. In some embodiments, R¹³ is C₁-C₆ alkyl. In some embodiments, R¹³ is methyl.

In some embodiments of formula (Ib), R^{13a} is hydrogen, halo, or C₁-C₆ alkyl. In some embodiments, R^{13a} is hydrogen. In some embodiments, R^{13a} is halo. In some embodiments, R^{13a} is fluoro, chloro, bromo, or iodo. In some embodiments, R^{13a} is fluoro. In some embodiments, R^{13a} is C₁-C₆ alkyl. In some embodiments, R^{13a} is methyl.

In some embodiments of formula (Ib), R¹³ and R^{13a} are each independently hydrogen or halo. In some embodiments, R¹³ and R^{13a} are each hydrogen. In some embodiments, one of R¹³ and R^{13a} is hydrogen and the other is halo. In some embodiments, one of R¹³ and R^{13a} is hydrogen and the other is fluoro.

With reference to any one of formulae (I), (Ia), and (Ib), in some embodiments, R¹ is -OR⁴, -NR⁵R^{5a}, or -N(OR^{5b})R^{5a}.

In some embodiments of any one of formulae (I), (Ia), and (Ib), R¹ is -OR⁴. In some embodiments, R⁴ is hydrogen. In some embodiments, R⁴ is C₁-C₆ alkyl. In some embodiments, R⁴ is C₃-C₈ cycloalkyl unsubstituted or substituted with one to six R⁶ and R⁶ is as defined and described herein. In some embodiments, R⁴ is C₃-C₈ cycloalkyl unsubstituted or substituted with one to six R⁶ and each R⁶ is independently hydroxy or C₁-C₆ alkyl. In some embodiments, R⁴ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁴ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁴ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁴ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁴ is C₁-C₆ hydroxyalkyl. In some embodiments, R⁴ is C₁-C₆ alkoxy-C₁-C₆ alkyl. In some embodiments, R⁴ is amino-C₁-C₆ alkyl. In some embodiments, R⁴ is C₁-C₆ alkylamino-C₁-C₆ alkyl. In some embodiments, R⁴ is di-(C₁-C₆ alkyl)amino-C₁-C₆ alkyl. In some embodiments, R⁴ is heterocycloalkyl unsubstituted or substituted with one to six R⁶ and R⁶ is as defined and described herein. In some embodiments, R⁴ is heterocycloalkyl unsubstituted or substituted with one to six R⁶ and each R⁶ is independently hydroxy or C₁-C₆ alkyl. In some embodiments, R⁴ is heterocycloalkyl-C₁-C₆ alkyl, wherein the heterocycloalkyl group is unsubstituted or substituted with one to six R⁶ and R⁶ is as defined and described herein. In some embodiments, R⁴ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁴ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁴ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁴ is R⁷-C(O)-C₁-C₆ alkyl; and R⁷ is hydroxy, C₁-C₆ alkoxy, amino, or hydroxyamino.

In some embodiments of any one of formulae (1), (1a), and (Ib), R¹ is selected from the group consisting of -OH,

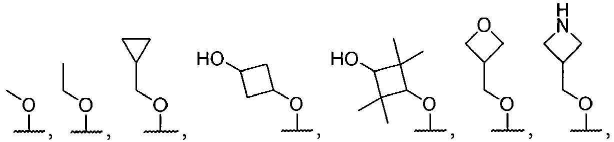

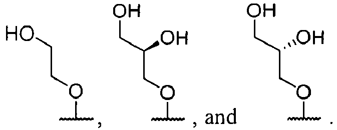

In some embodiments of any one of formulae (I), (Ia), and (Ib), R¹ is -NR⁵R^{5a}. In some embodiments, R⁵ is hydrogen. In some embodiments, R⁵ is C₁-C₆ alkyl. In some embodiments, R⁵ is C₃-C₈ cycloalkyl unsubstituted or substituted with one to six R⁶ and R⁶ is as defined and described herein. In some embodiments, R⁵ is C₃-C₈ cycloalkyl unsubstituted or substituted with one to six R⁶ and each R⁶ is independently hydroxy or C₁-C₆ alkyl. In some embodiments, R⁵ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁵ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁵ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁵ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁵ is C₁-C₆ hydroxyalkyl. In some embodiments, R⁵ is C₁-C₆ alkoxy-C₁-C₆ alkyl. In some embodiments, R⁵ is amino-C₁-C₆ alkyl. In some embodiments, R⁵ is C₁-C₆ alkylamino-C₁-C₆ alkyl. In some embodiments, R⁵ is di-(C₁-C₆ alkyl)amino-C₁-C₆ alkyl. In some embodiments, R⁵ is heterocycloalkyl unsubstituted or substituted with one to six R⁶ and R⁶ is as defined and described herein. In some embodiments, R⁵ is heterocycloalkyl unsubstituted or substituted with one to six R⁶ and each R⁶ is independently hydroxy or C₁-C₆ alkyl. In some embodiments, R⁵ is heterocycloalkyl-C₁-C₆ alkyl, wherein the heterocycloalkyl group is unsubstituted or substituted with one to six R⁶ and R⁶ is as defined and described herein. In some embodiments, R⁵ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁵ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁵ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁵ is R⁷-C(O)-C₁-C₆ alkyl; and R⁷ is hydroxy, C₁-C₆ alkoxy, amino, or hydroxyamino.

In some embodiments of any one of formulae (1), (1a), and (1b), R¹ is -NR⁵R^{5a} and R⁵ is selected from the group consisting of hydrogen,

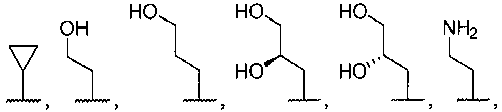

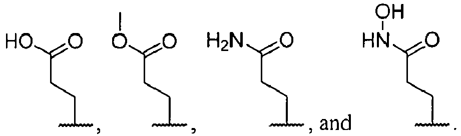

In some embodiments of any one of formulae (1), (1a), and (Ib), R¹ is -N(OR^{5b})R^{5a}. In some embodiments, R^{5b} is hydrogen. In some embodiments, R^{5b} is C₁-C₆ alkyl. In some embodiments, R^{5b} is C₃-C₈ cycloalkyl unsubstituted or substituted with one to six R⁶ and R⁶ is as defined and described herein. In some embodiments, R^{5b} is C₃-C₈ cycloalkyl unsubstituted or substituted with one to six R⁶ and each R⁶ is independently hydroxy or C₁-C₆ alkyl. In some embodiments, R^{5b} is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R^{5b} is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R^{5b} is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R^{5b} is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R^{5b} is C₁-C₆ hydroxyalkyl. In some embodiments, R^{5b} is C₁-C₆ alkoxy-C₁-C₆ alkyl. In some embodiments, R^{5b} is amino-C₁-C₆ alkyl. In some embodiments, R^{5b} is C₁-C₆ alkylamino-C₁-C₆ alkyl. In some embodiments, R^{5b} is di-(C₁-C₆ alkyl)amino-C₁-C₆ alkyl. In some embodiments, R^{5b} is heterocycloalkyl unsubstituted or substituted with one to six R⁶ and R⁶ is as defined and described herein. In some embodiments, R^{5b} is heterocycloalkyl unsubstituted or substituted with one to six R⁶ and each R⁶ is independently hydroxy or C₁-C₆ alkyl. In some embodiments, R^{5b} is heterocycloalkyl-C₁-C₆ alkyl, wherein the heterocycloalkyl group is unsubstituted or substituted with one to six R⁶ and R⁶ is as defined and described herein. In some embodiments, R^{5b} is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R^{5b} is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R^{5b} is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R^{5b} is R⁷-C(O)-C₁-C₆ alkyl; and R⁷ is hydroxy, C₁-C₆ alkoxy, amino, or hydroxyamino.

In some embodiments of any one of formulae (I), (Ia), and (Ib), R¹ is -N(OR^{5b})R^{5a} and -OR^{5b} is selected from the group consisting of -OH,

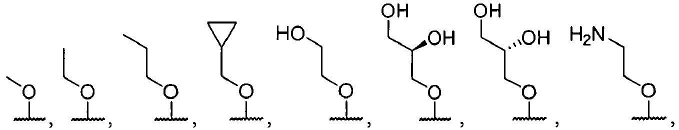

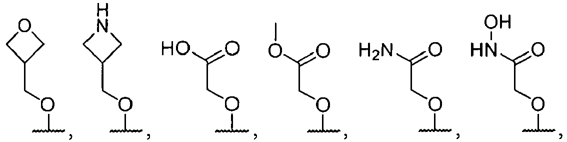

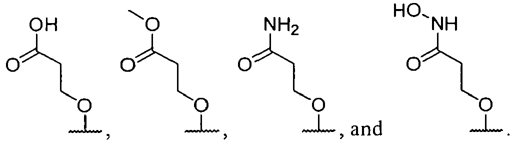

In some embodiments of any one of formulae (1), (1a), and (Ib), R^{5a} is hydrogen. In some embodiments, R^{5a} is C₁-C₆ alkyl. In some embodiments, R^{5a} is C₁-C₄ alkyl. In some embodiments, R^{5a} is methyl, ethyl, propyl, isopropyl, butyl, isobutyl, sec-butyl, or tert-butyl. In some embodiments, R^{5a} is methyl.

With reference to any one of formulae (1), (1a), and (Ib), in some embodiments, R¹ is a N-linked heterocycloalkyl which is unsubstituted or substituted with one or two R⁶, wherein R⁶ is as defined and described herein. In some embodiments, the N-linked heterocycloalkyl is N-linked azetidinyl, N-linked pyrrolidinyl, N-linked isoxazolidinyl, N-linked piperidinyl, or N-linked morpholinyl. In some embodiments, the N-linked heterocycloalkyl is N-linked azetidinyl. In some embodiments, the N-linked heterocycloalkyl is N-linked pyrrolidinyl. In some embodiments, the N-linked heterocycloalkyl is N-linked isoxazolidinyl. In some embodiments, the N-linked heterocycloalkyl is N-linked piperidinyl. In some embodiments, the N-linked heterocycloalkyl is N-linked morpholinyl. In some embodiments, R¹ is N-linked azetidinyl which is unsubstituted or substituted with one or two R⁶, wherein R⁶ is as defined and described herein. In some embodiments, R¹ is N-linked pyrrolidinyl which is unsubstituted or substituted with one or two R⁶, wherein R⁶ is as defined and described herein. In some embodiments, R¹ is N-linked piperidinyl which is unsubstituted or substituted with one or two R⁶, wherein R⁶ is as defined and described herein. In some embodiments, R¹ is N-linked isoxazolidinyl which is unsubstituted or substituted with one or two R⁶, wherein R⁶ is as defined and described herein. In some embodiments, R¹ is N-linked morpholinyl which is unsubstituted or substituted with one or two R⁶, wherein R⁶ is as defined and described herein.

With reference to R⁶ as one or two substituents of the N-linked heterocycloalkyl in any one of formulae (I), (Ia), and (Ib), in some embodiments, each R⁶ is independently hydroxyl, oxo, or amino. In some embodiments, each R⁶ is hydroxy. In some embodiments, each R⁶ is oxo. In some embodiments, each R⁶ is amino. In some embodiments, one of R⁶ is hydroxy and the other R⁶ is amino.

In some embodiments of any one of formulae (I), (Ia), and (Ib), R¹ is a N-linked heterocycloalkyl which is unsubstituted or substituted with hydroxy, oxo, or amino. In some embodiments, R¹ is N-linked azetidinyl which is unsubstituted or substituted with hydroxy, oxo, or amino. In some embodiments, R¹ is N-linked pyrrolidinyl which is unsubstituted or substituted with hydroxy, oxo, or amino. In some embodiments, R¹ is N-linked piperidinyl which is unsubstituted or substituted with hydroxy, oxo, or amino. In some embodiments, R¹ is N-linked isoxazolidinyl which is unsubstituted or substituted with hydroxy, oxo, or amino. In some embodiments, R¹ is N-linked morpholinyl which is unsubstituted or substituted with hydroxy, oxo, or amino.

With reference to any one of formulae (I), (Ia), and (Ib), in some embodiments, R² is halo, C₁-C₆ alkyl, -S-C₁-C₆ alkyl, C₃-C₈ cycloalkyl, C₂-C₆ alkenyl, or C₂-C₆ alkynyl. In some embodiments, R² is halo or C₁-C₆ alkyl. In some embodiments, R² is halo,-CH₃, -SCH₃, C₂-C₃ alkenyl, or C₂-C₃ alkynyl.

In some embodiments of any one of formulae (I), (Ia), and (Ib), R² is halo. In some embodiments, R² is fluoro. In some embodiments, R² is iodo. In some embodiments, R² is chloro. In some embodiments, R² is bromo.

In some embodiments of any one of formulae (I), (Ia), and (Ib), R² is C₁-C₆ alkyl. In some embodiments, R² is C₁-C₃ alkyl. In some embodiments, R² is methyl.

In some embodiments of any one of formulae (I), (Ia), and (Ib), R² is -S-C₁-C₆ alkyl. In some embodiments, R² is -S-C₁-C₃ alkyl. In some embodiments, R² is -SCH₃.

In some embodiments of any one of formulae (I), (Ia), and (Ib), R² is C₃-C₈ cycloalkyl. In some embodiments, R² is cyclopropyl.

In some embodiments of any one of formulae (I), (Ia), and (Ib), R² is C₂-C₆ alkenyl. In some embodiments, R² is C₂-C₄ alkenyl. In some embodiments, R² is vinyl (ethenyl), propenyl, isopropenyl, 1-butenyl, 2-butenyl, isobutenyl, or butadienyl. In some embodiments, R² is vinyl.

In some embodiments of any one of formulae (I), (Ia), and (Ib), R² is C₂-C₆ alkynyl. In some embodiments, R² is C₂-C₃ alkynyl. In some embodiments, R² is acetylenyl or propynyl. In some embodiments, R² is acetylenyl.

With reference to any one of formulae (I), (Ia), and (Ib), in some embodiments, R^{2a} is halo or C₁-C₃ alkyl. In some embodiments, R^{2a} is halo or CH₃. In some embodiments, R^{2a} is fluoro or CH₃. In some embodiments, R^{2a} is iodo or CH₃. In some embodiments, R^{2a} is chloro or CH₃. In some embodiments, R^{2a} is bromo or CH₃.

In some embodiments of any one of formulae (I), (Ia), and (Ib), R^{2a} is halo. In some embodiments, R^{2a} is fluoro. In some embodiments, R^{2a} is iodo. In some embodiments, R^{2a} is chloro. In some embodiments, R^{2a} is bromo.

In some embodiments of any one of formulae (I), (Ia), and (Ib), R^{2a} is C₁-C₆ alkyl. In some embodiments, R^{2a} is C₁-C₃ alkyl. In some embodiments, R^{2a} is CH₃.

With reference to any one of formulae (I), (Ia), and (Ib), in some embodiments, R² and R^{2a} are each halo. In some embodiments, R² is halo and R^{2a} is C₁-C₆ alkyl. In some embodiments, R² is C₁-C₆ alkyl and R^{2a} is halo. In some embodiments, R² is -S-C₁-C₆ alkyl and R^{2a} is halo. In some embodiments, R² is -SCH₃ and R^{2a} is halo. In some embodiments, R² is C₃-C₈ cycloalkyl and R^{2a} is halo. In some embodiments, R² is cyclopropyl and R^{2a} is halo. In some embodiments, R² is C₂-C₆ alkenyl and R^{2a} is halo. In some embodiments, R² is C₂-C₆ alkynyl and R^{2a} is halo. In some embodiments, R² is acetylenyl and R^{2a} is halo. In some embodiments, R² and R^{2a} are each independently fluoro, chloro, bromo, or iodo. In some embodiments, R² is iodo and R^{2a} is fluoro. In some embodiments, R² is halo and R^{2a} is -CH₃. In some embodiments, R² is bromo and R^{2a} is -CH₃. In some embodiments, R² is iodo and R^{2a} is -CH₃. In some embodiments, R² is -SCH₃ and R^{2a} is fluoro. In some embodiments, R² is acetylenyl and R^{2a} is fluoro.

In some embodiments, the compound of formula (1) is represented by any one of the following formulae:

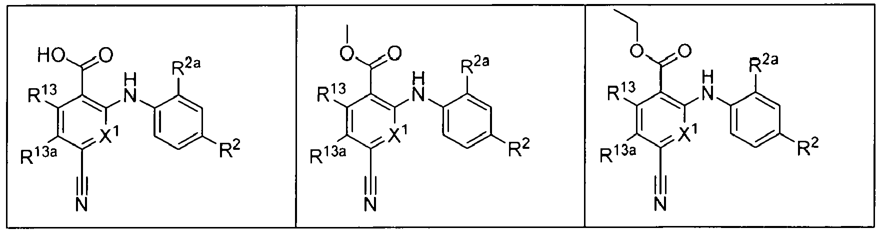

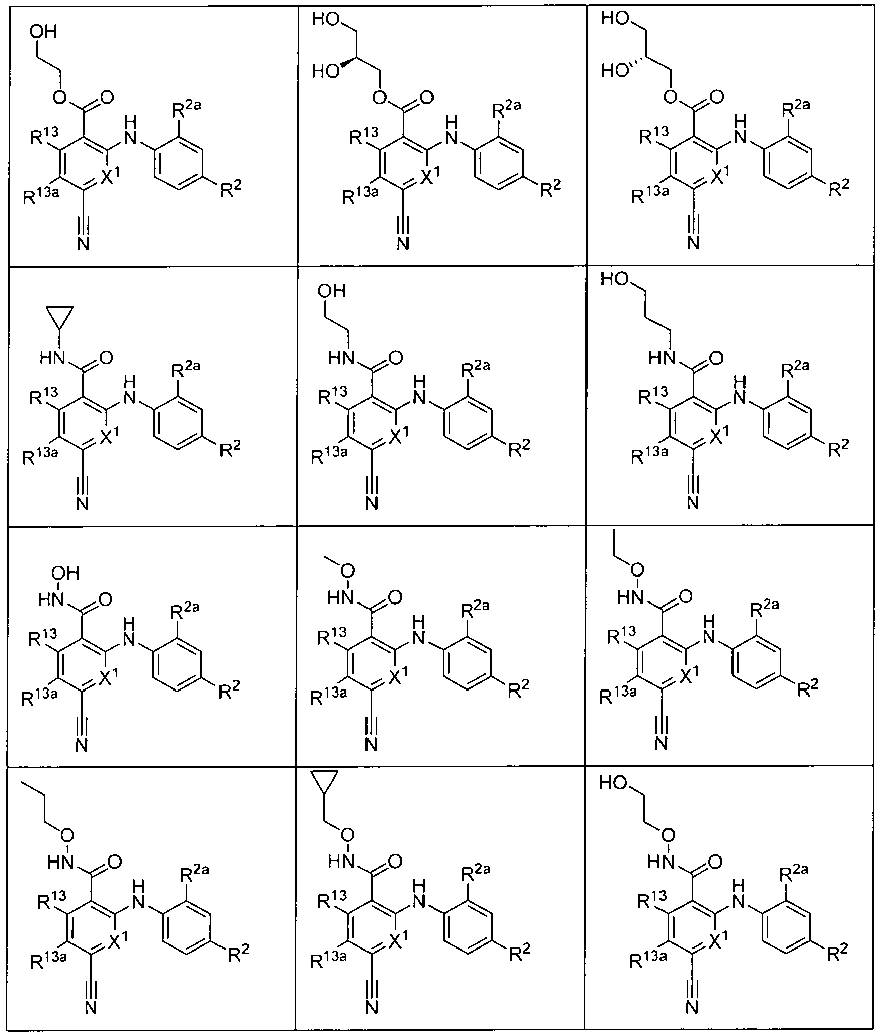

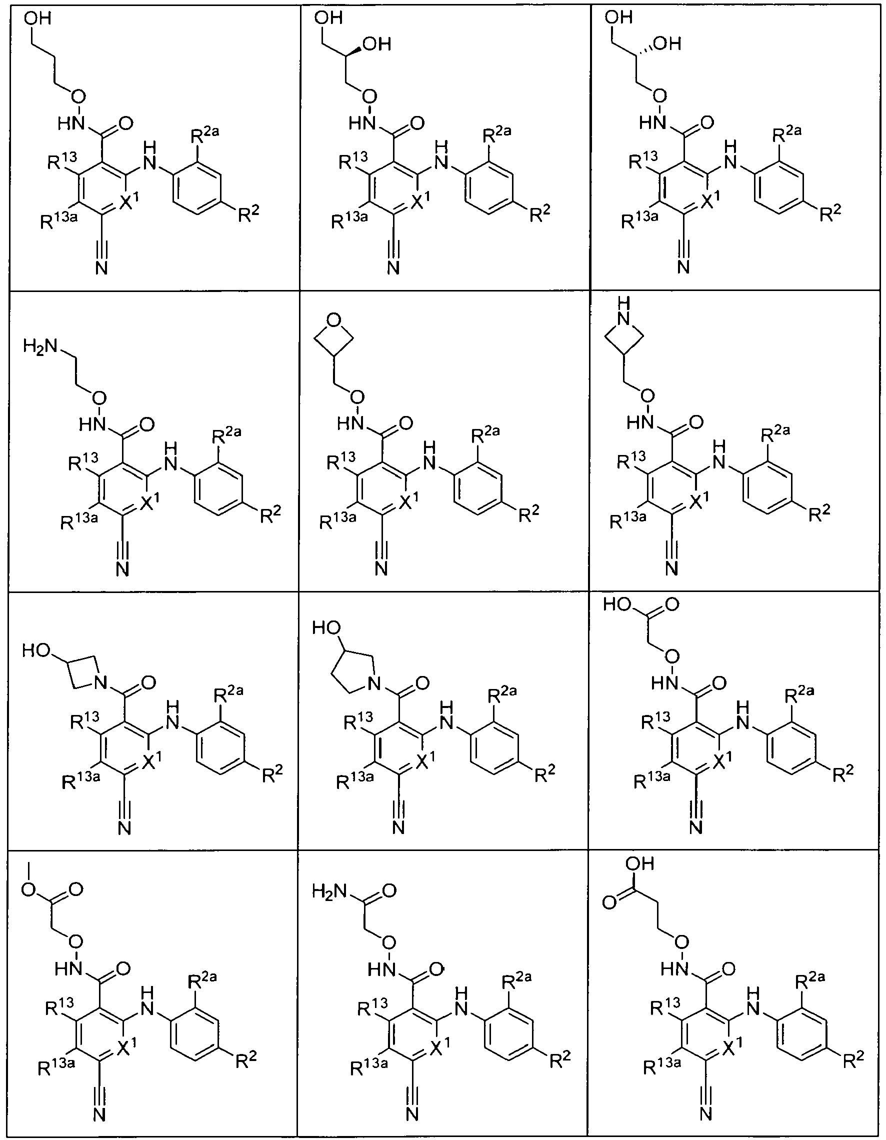

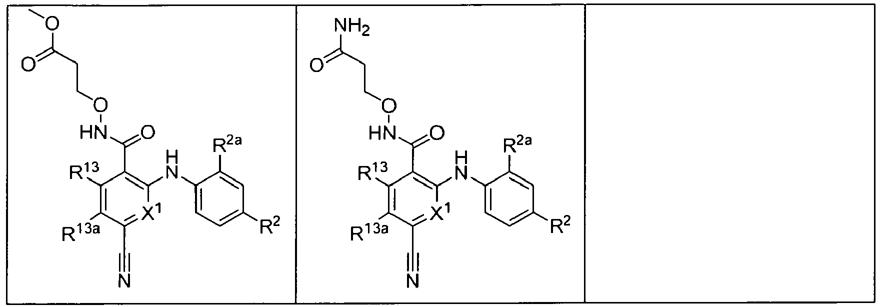

wherein X¹, R², R^{2a}, R¹³, and R^{13a} are as defined herein in any aspect or embodiment described herein.

In some embodiments of the above structures having formula (1), X¹ is -CR^{13b}, and R^{13b} is as defined herein in any aspect or embodiment described herein. In some embodiments of the above structures having formula (1), X¹ is N.

In some embodiments of the above structures having formula (1), R² is iodo and R^{2a} is fluoro. In some embodiments of the above structures, R² is iodo and R^{2a} is methyl. In some embodiments of the above structures, R² is acetylenyl and R^{2a} is fluoro. In some embodiments of the above structures, R² is acetylenyl and R^{2a} is methyl. In some embodiments of the above structures, R² is -SCH₃ and R^{2a} is fluoro. In some embodiments of the above structures, R² is -SCH₃ and R^{2a} is methyl.

In some embodiments of the above structures having formula (1), X¹ is -CR^{13b}; and R¹³, R^{13a}, and R^{13b} are each hydrogen. In some embodiments of the above structures having formula (1), X¹ is -CR^{13b}; R¹³ and R^{13a} are each hydrogen and R^{13b} is halo. In some embodiments of the above structures having formula (1), X¹ is -CR^{13b}; R¹³ and R^{13a} are each hydrogen and R^{13b} is fluoro.

In some embodiments of the above structures having formula (1), X' is N; R¹³ and R^{13a} are each hydrogen.

In some embodiments, the compounds useful in the present methods are compounds of formula (11).

With reference to formula (II), in some embodiments, X² is C₁-C₃ alkyl. In some embodiments, X² is methyl, ethyl, propyl, or isopropyl. In some embodiments, X² is methyl and the compound is represented by formula (IIa): 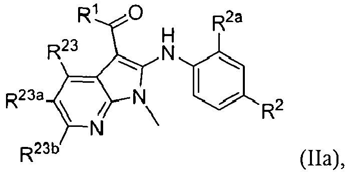 wherein R¹, R², R^{2a}, R²³, R^{23a}, and R^{23b} are as defined herein in any aspect or embodiment described herein.

In some embodiments of formula (II) or (IIa), R²³, R^{23a}, and R^{23b} are each independently hydrogen, halo, C₁-C₆ alkyl, or C₁-C₆ alkoxy.

In some embodiments of formula (II) or (IIa), R²³ is hydrogen, halo, or C₁-C₆ alkyl. In some embodiments, R²³ is hydrogen. In some embodiments, R²³ is halo. In some embodiments, R²³ is fluoro, chloro, bromo, or iodo. In some embodiments, R²³ is fluoro. In some embodiments, R²³ is C₁-C₆ alkyl. In some embodiments, R²³ is methyl, ethyl, propyl, isopropyl, butyl, isobutyl, sec-butyl, tert-butyl, pentyl, isopentyl, or hexyl. In some embodiments, R²³ is methyl.

In some embodiments of formula (II) or (IIa), R^{23a} is hydrogen, halo, C₁-C₆ alkyl, or C₁-C₆ alkoxy. In some embodiments, R^{23a} is hydrogen. In some embodiments, R^{23a} is halo. In some embodiments, R^{23a} is fluoro, chloro, bromo, or iodo. In some embodiments, R^{23a} is fluoro. In some embodiments, R^{23a} is C₁-C₆ alkyl. In some embodiments, R^{23a} is methyl, ethyl, propyl, isopropyl, butyl, isobutyl, sec-butyl, tert-butyl, pentyl, isopentyl, or hexyl. In some embodiments, R^{23a} is methyl. In some embodiments, R^{23a} is C₁-C₆ alkoxy. In some embodiments, R^{23a} is methoxy, ethoxy, propoxy, iso-propoxy, butoxy, 2-butoxy, iso-butoxy, sec-butoxy, tert-butoxy, pentoxy, or hexoxy. In some embodiments, R^{23a} is methoxy.

In some embodiments of formula (II) or (IIa), R^{23b} is hydrogen, halo, or C₁-C₆ alkyl. In some embodiments, R^{23b} is hydrogen. In some embodiments, R^{23b} is halo. In some embodiments, R^{23b} is fluoro, chloro, bromo, or iodo. In some embodiments, R^{23b} is fluoro. In some embodiments, R^{23b} is C₁-C₆ alkyl. In some embodiments, R^{23b} is methyl, ethyl, propyl, isopropyl, butyl, isobutyl, sec-butyl, tert-butyl, pentyl, isopentyl, or hexyl. In some embodiments, R^{23b} is methyl.

In some embodiments of formula (11) or (IIa), R²³, R^{23a}, and R^{23b} are each hydrogen. In some embodiments of formula (II) or (IIa), R²³ and R^{23b} are each hydrogen and R^{23a} is halo, C₁-C₆ alkyl, or C₁-C₆ alkoxy. In some embodiments of formula (II) or (IIa), R²³ and R^{23b} are each hydrogen and R^{23a} is fluoro, methyl, or methoxy.

With reference to formula (11) or (IIa), in some embodiments, R¹ is -OR⁴, -NR⁵R^{5a}, or -N(OR^{5b})R^{5a}.

In some embodiments of formula (II) or (IIa), R¹ is -OR⁴. In some embodiments, R⁴ is hydrogen. In some embodiments, R⁴ is C₁-C₆ alkyl. In some embodiments, R⁴ is C₃-C₈ cycloalkyl unsubstituted or substituted with one to six R⁶ and R⁶ is as defined and described herein. In some embodiments, R⁴ is C₃-C₈ cycloalkyl unsubstituted or substituted with one to six R⁶ and each R⁶ is independently hydroxy or C₁-C₆ alkyl. In some embodiments, R⁴ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁴ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁴ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁴ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁴ is C₁-C₆ hydroxyalkyl. In some embodiments, R⁴ is C₁-C₆ alkoxy-C₁-C₆ alkyl. In some embodiments, R⁴ is amino-C₁-C₆ alkyl. In some embodiments, R⁴ is C₁-C₆ alkylamino-C₁-C₆ alkyl. In some embodiments, R⁴ is di-(C₁-C₆ alkyl)amino-C₁-C₆ alkyl. In some embodiments, R⁴ is heterocycloalkyl unsubstituted or substituted with one to six R⁶ and R⁶ is as defined and described herein. In some embodiments, R⁴ is heterocycloalkyl unsubstituted or substituted with one to six R⁶ and each R⁶ is independently hydroxy or C₁-C₆ alkyl. In some embodiments, R⁴ is heterocycloalkyl-C₁-C₆ alkyl, wherein the heterocycloalkyl group is unsubstituted or substituted with one to six R⁶ and R⁶ is as defined and described herein. In some embodiments, R⁴ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁴ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁴ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁴ is R⁷-C(O)-C₁-C₆ alkyl; and R⁷ is hydroxy, C₁-C₆ alkoxy, amino, or hydroxyamino.

In some embodiments of formula (II) or (IIa), R¹ is selected from the group consisting of -OH,

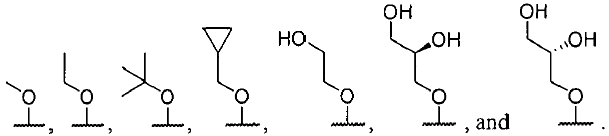

In some embodiments of formula (II) or (IIa), R¹ is -NR⁵R^{5a}. In some embodiments, R⁵ is hydrogen. In some embodiments, R⁵ is C₁-C₆ alkyl. In some embodiments, R⁵ is C₃-C₈ cycloalkyl unsubstituted or substituted with one to six R⁶ and R⁶ is as defined and described herein. In some embodiments, R⁵ is C₃-C₈ cycloalkyl unsubstituted or substituted with one to six R⁶ and each R⁶ is independently hydroxy or C₁-C₆ alkyl. In some embodiments, R⁵ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁵ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁵ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁵ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁵ is C₁-C₆ hydroxyalkyl. In some embodiments, R⁵ is C₁-C₆ alkoxy-C₁-C₆ alkyl. In some embodiments, R⁵ is amino-C₁-C₆ alkyl. In some embodiments, R⁵ is C₁-C₆ alkylamino-C₁-C₆ alkyl. In some embodiments, R⁵ is di-(C₁-C₆ alkyl)amino-C₁-C₆ alkyl. In some embodiments, R⁵ is heterocycloalkyl unsubstituted or substituted with one to six R⁶ and R⁶ is as defined and described herein. In some embodiments, R⁵ is heterocycloalkyl unsubstituted or substituted with one to six R⁶ and each R⁶ is independently hydroxy or C₁-C₆ alkyl. In some embodiments, R⁵ is heterocycloalkyl-C₁-C₆ alkyl, wherein the heterocycloalkyl group is unsubstituted or substituted with one to six R⁶ and R⁶ is as defined and described herein. In some embodiments, R⁵ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁵ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁵ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁵ is R⁷-C(O)-C₁-C₆ alkyl; and R⁷ is hydroxy, C₁-C₆ alkoxy, amino, or hydroxyamino.

In some embodiments of formula (II) or (IIa), R⁵ is selected from the group consisting of hydrogen,

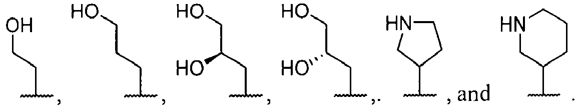

In some embodiments of formula (II) or (IIa), R¹ is -N(OR^{5b})R^{5a}. In some embodiments, R^{5b} is hydrogen. In some embodiments, R^{5b} is C₁-C₆ alkyl. In some embodiments, R^{5b} is C₃-C₈ cycloalkyl unsubstituted or substituted with one to six R⁶ and R⁶ is as defined and described herein. In some embodiments, R^{5b} is C₃-C₈ cycloalkyl unsubstituted or substituted with one to six R⁶ and each R⁶ is independently hydroxy or C₁-C₆ alkyl. In some embodiments, R^{5b} is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R^{5b} is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R^{5b} is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R^{5b} is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R^{5b} is C₁-C₆ hydroxyalkyl. In some embodiments, R^{5b} is C₁-C₆ alkoxy-C₁-C₆ alkyl. In some embodiments, R^{5b} is amino-C₁-C₆ alkyl. In some embodiments, R^{5b} is C₁-C₆ alkylamino-C₁-C₆ alkyl. In some embodiments, R^{5b} is di-(C₁-C₆ alkyl)amino-C₁-C₆ alkyl. In some embodiments, R^{5b} is heterocycloalkyl unsubstituted or substituted with one to six R⁶ and R⁶ is as defined and described herein. In some embodiments, R^{5b} is heterocycloalkyl unsubstituted or substituted with one to six R⁶ and each R⁶ is independently hydroxy or C₁-C₆ alkyl. In some embodiments, R^{5b} is heterocycloalkyl-C₁-C₆ alkyl, wherein the heterocycloalkyl group is unsubstituted or substituted with one to six R⁶ and R⁶ is as defined and described herein. In some embodiments, R^{5b} is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R^{5b} is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R^{5b} is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R^{5b} is R⁷-C(O)-C₁-C₆ alkyl; and R⁷ is hydroxy, C₁-C₆ alkoxy, amino, or hydroxyamino.

In some embodiments of formula (II) or (IIa), R¹ is -N(OR^{5b})R^{5a} and -OR^{5b} is selected from the group consisting of -OH,

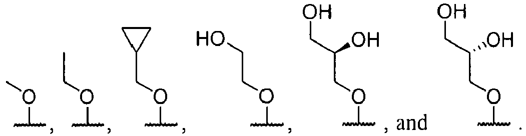

In some embodiments of formula (11) or (IIa), R^{5a} is hydrogen. In some embodiments, R^{5a} is C₁-C₆ alkyl. In some embodiments, R^{5a} is C₁-C₄ alkyl. In some embodiments, R^{5a} can be methyl, ethyl, propyl, isopropyl, butyl, isobutyl, sec-butyl, or tert-butyl. In some embodiments, R^{5a} is methyl.

With reference to formula (II) or (IIa), in some embodiments, R¹ is a N-linked heterocycloalkyl which is unsubstituted or substituted with one or two R⁶, wherein R⁶ is as defined and described herein. In some embodiments, the N-linked heterocycloalkyl is N-linked azetidinyl, N-linked pyrrolidinyl, N-linked isoxazolidinyl, N-linked piperidinyl, or N-linked morpholinyl. In some embodiments, the N-linked heterocycloalkyl is N-linked azetidinyl. In some embodiments, the N-linked heterocycloalkyl is N-linked pyrrolidinyl. In some embodiments, the N-linked heterocycloalkyl is N-linked isoxazolidinyl. In some embodiments, the N-linked heterocycloalkyl is N-linked piperidinyl. In some embodiments, the N-linked heterocycloalkyl is N-linked morpholinyl. In some embodiments, R¹ is N-linked azetidinyl which is unsubstituted or substituted with one or two R⁶, wherein R⁶ is as defined and described herein. In some embodiments, R¹ is N-linked pyrrolidinyl which is unsubstituted or substituted with one or two R⁶, wherein R⁶ is as defined and described herein. In some embodiments, R¹ is N-linked piperidinyl which is unsubstituted or substituted with one or two R⁶, wherein R⁶ is as defined and described herein. In some embodiments, R¹ is N-linked isoxazolidinyl which is unsubstituted or substituted with one or two R⁶, wherein R⁶ is as defined and described herein. In some embodiments, R¹ is N-linked morpholinyl which is unsubstituted or substituted with one or two R⁶, wherein R⁶ is as defined and described herein.

With reference to R⁶ as one or two substituents of the N-linked heterocycloalkyl in formula (II) or (IIa), in some embodiments, each R⁶ is independently hydroxyl, oxo, or amino. In some embodiments, each R⁶ is hydroxy. In some embodiments, each R⁶ is oxo. In some embodiments, each R⁶ is amino. In some embodiments, one of R⁶ is hydroxy and the other R⁶ is amino.

In some embodiments of formula (II) or (IIa), R¹ is a N-linked heterocycloalkyl which is unsubstituted or substituted with hydroxyl, oxo, or amino. In some embodiments, R¹ is N-linked azetidinyl which is unsubstituted or substituted with hydroxy, oxo, or amino. In some embodiments, R¹ is N-linked pyrrolidinyl which is unsubstituted or substituted with hydroxy, oxo, or amino. In some embodiments, R¹ is N-linked piperidinyl which is unsubstituted or substituted with hydroxy, oxo, or amino. In some embodiments, R¹ is N-linked isoxazolidinyl which is unsubstituted or substituted with hydroxy, oxo, or amino. In some embodiments, R' is N-linked morpholinyl which is unsubstituted or substituted with hydroxy, oxo, or amino.

With reference to formulae (11) or (11a), in some embodiments, R² is halo, C₁-C₆ alkyl, -S-C₁-C₆ alkyl, C₃-C₈ cycloalkyl, C₂-C₆ alkenyl, or C₂-C₆ alkynyl. In some embodiments, R² is halo or C₁-C₆ alkyl. In some embodiments, R² is halo,-CH₃, -SCH₃, C₂-C₃ alkenyl, or C₂-C₃ alkynyl.

In some embodiments of formulae (II) or (IIa), R² is halo. In some embodiments, R² is fluoro. In some embodiments, R² is iodo. In some embodiments, R² is chloro. In some embodiments, R² is bromo.

In some embodiments of formulae (II) or (IIa), R² is C₁-C₆ alkyl. In some embodiments, R² is C₁-C₃ alkyl. In some embodiments, R² is methyl.

In some embodiments of formulae (II) or (IIa), R² is -S-C₁-C₆ alkyl. In some embodiments, R² is -S-C₁-C₃ alkyl. In some embodiments, R² is -SCH₃.

In some embodiments of formulae (II) or (IIa), R² is C₃-C₈ cycloalkyl. In some embodiments, R² is cyclopropyl.

In some embodiments of formulae (II) or (IIa), R² is C₂-C₆ alkenyl. In some embodiments, R² is C₂-C₄ alkenyl. In some embodiments, R² is vinyl (ethenyl), propenyl, isopropenyl, 1-butenyl, 2-butenyl, isobutenyl, or butadienyl. In some embodiments, R² is vinyl.

In some embodiments of formulae (II) or (IIa), R² is C₂-C₆ alkynyl. In some embodiments, R² is C₂-C₃ alkynyl. In some embodiments, R² is acetylenyl or propynyl. In some embodiments, R² is acetylenyl.

With reference to formulae (II) or (11a), in some embodiments, R^{2a} is halo or C₁-C₃ alkyl. In some embodiments, R^{2a} is halo or CH₃. In some embodiments, R^{2a} is fluoro or CH₃. In some embodiments, R^{2a} is iodo or CH₃. In some embodiments, R^{2a} is chloro or CH₃. In some embodiments, R^{2a} is bromo or CH₃.

In some embodiments of formulae (II) or (IIa), R^{2a} is halo. In some embodiments, R^{2a} is fluoro. In some embodiments, R^{2a} is iodo. In some embodiments, R^{2a} is chloro. In some embodiments, R^{2a} is bromo.

In some embodiments of formulae (II) or (IIa), R^{2a} is C₁-C₆ alkyl. In some embodiments, R^{2a} is C₁-C₃ alkyl. In some embodiments, R^{2a} is CH₃.

With reference to formulae (II) or (IIa), in some embodiments, R² and R^{2a} are each halo. In some embodiments, R² is halo and R^{2a} is C₁-C₆ alkyl. In some embodiments, R² is C₁-C₆ alkyl and R^{2a} is halo. In some embodiments, R² is -S-C₁-C₆ alkyl and R^{2a} is halo. In some embodiments, R² is -SCH₃ and R^{2a} is halo. In some embodiments, R² is C₃-C₈ cycloalkyl and R^{2a} is halo. In some embodiments, R² is cyclopropyl and R^{2a} is halo. In some embodiments, R² is C₂-C₆ alkenyl and R^{2a} is halo. In some embodiments, R² is C₂-C₆ alkynyl and R^{2a} is halo. In some embodiments, R² is acetylenyl and R^{2a} is halo. In some embodiments, R² and R^{2a} are each independently fluoro, chloro, bromo, or iodo. In some embodiments, R² is iodo and R^{2a} is fluoro. In some embodiments, R² is halo and R^{2a} is -CH₃. In some embodiments, R² is bromo and R^{2a} is -CH₃. In some embodiments, R² is iodo and R^{2a} is -CH₃. In some embodiments, R² is -SCH₃ and R^{2a} is fluoro. In some embodiments, R² is acetylenyl and R^{2a} is fluoro.

In some embodiments, the compound of formula (II) or formula (IIa) is represented by any one of the following formulae:

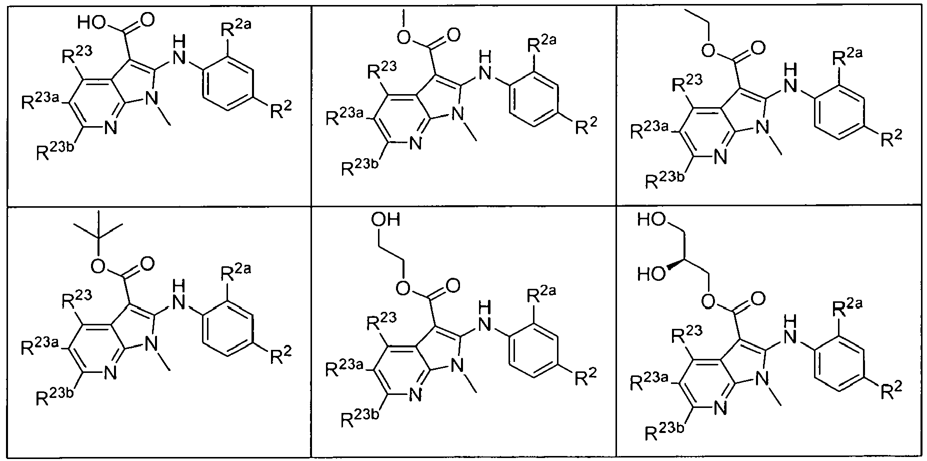

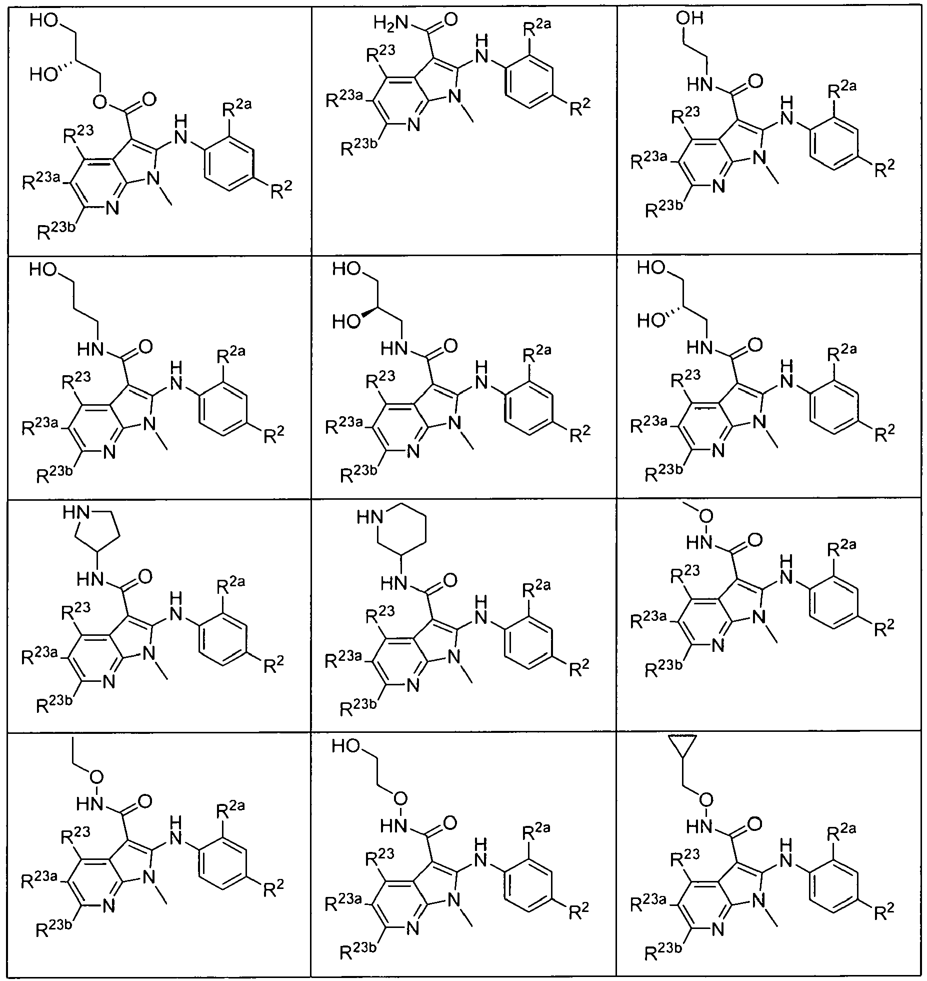

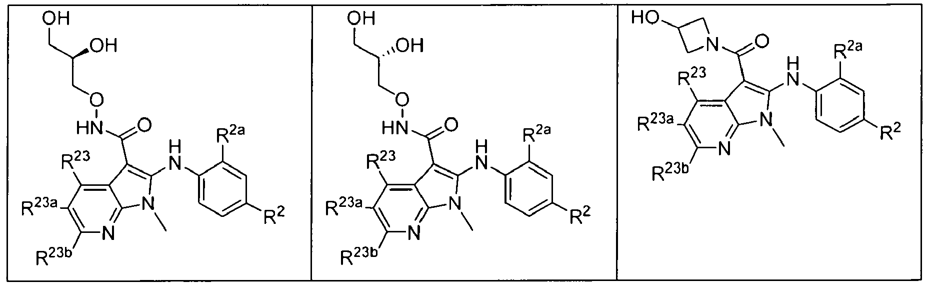

wherein R², R^{2a}, R²³, R^{23a}, and R^{23b} are as defined herein in any aspect or embodiment described herein.

In some embodiments of the above structures having formula (II) or (IIa), R² is iodo and R^{2a} is fluoro. In some embodiments of the above structures, R² is iodo and R^{2a} is methyl. In some embodiments of the above structures, R² is acetylenyl and R^{2a} is fluoro. In some embodiments of the above structures, R² is acetylenyl and R^{2a} is methyl. In some embodiments of the above structures, R² is -SCH₃ and R^{2a} is fluoro. In some embodiments of the above structures, R² is -SCH₃ and R^{2a} is methyl.

In some embodiments of the above structures having formula (II) or (IIa), R²³, R^{23a}, and R^{23b} are each hydrogen. In some embodiments of the above structures, R²³ and R^{23b} are each hydrogen and R^{23a} is fluoro. In some embodiments of the above structures, R²³ and R^{23b} are each hydrogen and R^{23a} is methoxy.

In some embodiments, the compounds useful in the present methods are compounds of formula (111).

With reference to formula (III), in some embodiments, X³ is S and the compound is represented by formula (IIIa): 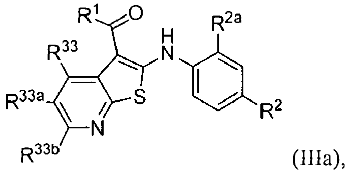 wherein R², R^{2a}, R³³, R^{33a}, and R^{33b} are as defined herein in any aspect or embodiment described herein.

In some embodiments of formula (111), X³ is O and the compound is represented by formula (111b): 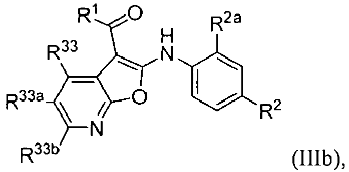 wherein R², R^{2a}, R³³, R^{33a}, and R^{33b} are as defined herein in any aspect or embodiment described herein.

In some embodiments of any one of formulae (III), (IIIa), and (111b), R³³, R^{33a}, and R^{33b} are each independently hydrogen, halo, C₁-C₆ alkyl, or C₁-C₆ alkoxy.

In some embodiments of any one of formulae (III), (IIIa), and (IIIb), R³³ is hydrogen, halo, C₁-C₆ alkyl, or C₁-C₆ alkoxy. In some embodiments, R³³ is hydrogen. In some embodiments, R³³ is halo. In some embodiments, R³³ is fluoro, chloro, bromo, or iodo. In some embodiments, R³³ is fluoro. In some embodiments, R³³ is C₁-C₆ alkyl. In some embodiments, R³³ is methyl, ethyl, propyl, isopropyl, butyl, isobutyl, sec-butyl, tert-butyl, pentyl, isopentyl, or hexyl. In some embodiments, R³³ is methyl. In some embodiments, R³³ is C₁-C₆ alkoxy. In some embodiments, R³³ is methoxy, ethoxy, propoxy, iso-propoxy, butoxy, 2-butoxy, iso-butoxy, sec-butoxy, tert-butoxy, pentoxy, or hexoxy. In some embodiments, R³³ is methoxy.

In some embodiments of any one of formulae (III), (IIIa), and (IIIb), R^{33a} is hydrogen, halo, C₁-C₆ alkyl, or C₁-C₆ alkoxy. In some embodiments, R^{33a} is hydrogen. In some embodiments, R^{33a} is halo. In some embodiments, R^{33a} is fluoro, chloro, bromo, or iodo. In some embodiments, R^{33a} is fluoro. In some embodiments, R^{33a} is C₁-C₆ alkyl. In some embodiments, R^{33a} is methyl, ethyl, propyl, isopropyl, butyl, isobutyl, sec-butyl, tert-butyl, pentyl, isopentyl, or hexyl. In some embodiments, R^{33a} is methyl. In some embodiments, R^{33a} is C₁-C₆ alkoxy. In some embodiments, R^{33a} is methoxy, ethoxy, propoxy, iso-propoxy, butoxy, 2-butoxy, iso-butoxy, sec-butoxy, tert-butoxy, pentoxy, or hexoxy. In some embodiments, R^{33a} is methoxy.

In some embodiments of any one of formulae (III), (IIIa), and (IIIb), R^{33b} is hydrogen, halo, C₁-C₆ alkyl, or C₁-C₆ alkoxy. In some embodiments, R^{33b} is hydrogen. In some embodiments, R^{33b} is halo. In some embodiments, R^{33b} is fluoro, chloro, bromo, or iodo. In some embodiments, R^{33b} is fluoro. In some embodiments, R^{33b} is C₁-C₆ alkyl. In some embodiments, R^{33b} is methyl, ethyl, propyl, isopropyl, butyl, isobutyl, sec-butyl, tert-butyl, pentyl, isopentyl, or hexyl. In some embodiments, R^{33b} is methyl. In some embodiments, R^{33b} is C₁-C₆ alkoxy. In some embodiments, R^{33b} is methoxy, ethoxy, propoxy, iso-propoxy, butoxy, 2-butoxy, iso-butoxy, sec-butoxy, tert-butoxy, pentoxy, or hexoxy. In some embodiments, R^{33b} is methoxy.

In some embodiments of any one of formulae (III), (IIIa), and (IIIb), R³³, R^{33a}, and R^{33b} are each hydrogen. In some embodiments of any one of formulae (III), (IIIa), and (IIIb), R^{33a} and R^{33b} are each hydrogen and R³³ is halo, C₁-C₆ alkyl, or C₁-C₆ alkoxy. In some embodiments of any one of formulae (III), (IIIa), and (IIIb), R³³ and R^{33b} are each hydrogen and R^{33a} is fluoro, methyl, or methoxy. In some embodiments of any one of formulae (III), (IIIa), and (IIIb), R³³ and R^{33a} are each hydrogen and R^{33b} is halo, C₁-C₆ alkyl, or C₁-C₆ alkoxy. In some embodiments of any one of formulae (III), (IIIa), and (IIIb), R^{33a} and R^{33b} are each hydrogen and R³³ is fluoro, methyl, or methoxy. In some embodiments of any one of formulae (III), (IIIa), and (IIIb), R³³ and R^{33b} are each hydrogen and R^{33a} is fluoro, methyl, or methoxy. In some embodiments of any one of formulae (III), (IIIa), and (IIIb), R³³ and R^{33a} are each hydrogen and R^{33b} is fluoro, methyl, or methoxy.

With reference to any one of formulae (III), (IIIa), and (IIIb), in some embodiments, R¹ is -OR⁴, -NR⁵R^{5a}, or -N(OR^{5b})R^{5a}.

In some embodiments of any one of formulae (III), (IIIa), and (IIIb), R¹ is -OR⁴. In some embodiments, R⁴ is hydrogen. In some embodiments, R⁴ is C₁-C₆ alkyl. In some embodiments, R⁴ is C₃-C₈ cycloalkyl unsubstituted or substituted with one to six R⁶ and R⁶ is as defined and described herein. In some embodiments, R⁴ is C₃-C₈ cycloalkyl unsubstituted or substituted with one to six R⁶ and each R⁶ is independently hydroxy or C₁-C₆ alkyl. In some embodiments, R⁴ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁴ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁴ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁴ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁴ is C₁-C₆ hydroxyalkyl. In some embodiments, R⁴ is C₁-C₆ alkoxy-C₁-C₆ alkyl. In some embodiments, R⁴ is amino-C₁-C₆ alkyl. In some embodiments, R⁴ is C₁-C₆ alkylamino-C₁-C₆ alkyl. In some embodiments, R⁴ is di-(C₁-C₆ alkyl)amino-C₁-C₆ alkyl. In some embodiments, R⁴ is heterocycloalkyl unsubstituted or substituted with one to six R⁶ and R⁶ is as defined and described herein. In some embodiments, R⁴ is heterocycloalkyl unsubstituted or substituted with one to six R⁶ and each R⁶ is independently hydroxy or C₁-C₆ alkyl. In some embodiments, R⁴ is heterocycloalkyl-C₁-C₆ alkyl, wherein the heterocycloalkyl group is unsubstituted or substituted with one to six R⁶ and R⁶ is as defined and described herein. In some embodiments, R⁴ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁴ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁴ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁴ is R⁷-C(O)-C₁-C₆ alkyl; and R⁷ is hydroxy, C₁-C₆ alkoxy, amino, or hydroxyamino.

In some embodiments of any one of formulae (III), (IIIa), and (IIIb), R¹ is selected from the group consisting of -OH,

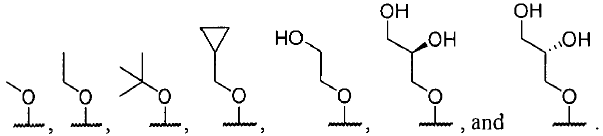

In some embodiments of any one of formulae (III), (IIIa), and (IIIb), R¹ is -NR⁵R^{5a}. In some embodiments, R⁵ is hydrogen. In some embodiments, R⁵ is C₁-C₆ alkyl. In some embodiments, R⁵ is C₃-C₈ cycloalkyl unsubstituted or substituted with one to six R⁶ and R⁶ is as defined and described herein. In some embodiments, R⁵ is C₃-C₈ cycloalkyl unsubstituted or substituted with one to six R⁶ and each R⁶ is independently hydroxy or Ci-Ce alkyl. In some embodiments, R⁵ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁵ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁵ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁵ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁵ is C₁-C₆ hydroxyalkyl. In some embodiments, R⁵ is C₁-C₆ alkoxy-C₁-C₆ alkyl. In some embodiments, R⁵ is amino-C₁-C₆ alkyl. In some embodiments, R⁵ is C₁-C₆ alkylamino-C₁-C₆ alkyl. In some embodiments, R⁵ is di-(C₁-C₆ alkyl)amino-C₁-C₆ alkyl. In some embodiments, R⁵ is heterocycloalkyl unsubstituted or substituted with one to six R⁶ and R⁶ is as defined and described herein. In some embodiments, R⁵ is heterocycloalkyl unsubstituted or substituted with one to six R⁶ and each R⁶ is independently hydroxy or C₁-C₆ alkyl. In some embodiments, R⁵ is heterocycloalkyl-C₁-C₆ alkyl, wherein the heterocycloalkyl group is unsubstituted or substituted with one to six R⁶ and R⁶ is as defined and described herein. In some embodiments, R⁵ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁵ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁵ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁵ is R⁷-C(O)-C₁-C₆ alkyl; and R⁷ is hydroxy, C₁-C₆ alkoxy, amino, or hydroxyamino.

In some embodiments of any one of formulae (III), (IIIa), and (IIIb), R⁵ is selected from the group consisting of hydrogen,

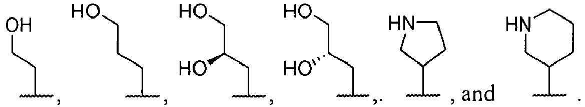

In some embodiments of any one of formulae (III), (IIIa), and (IIIb), R¹ is -N(OR^{5b})R^{5a}. In some embodiments, R^{5b} is hydrogen. In some embodiments, R^{5b} is C₁-C₆ alkyl. In some embodiments, R^{5b} is C₃-C₈ cycloalkyl unsubstituted or substituted with one to six R⁶ and R⁶ is as defined and described herein. In some embodiments, R^{5b} is C₃-C₈ cycloalkyl unsubstituted or substituted with one to six R⁶ and each R⁶ is independently hydroxy or C₁-C₆ alkyl. In some embodiments, R^{5b} is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R^{5b} is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R^{5b} is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R^{5b} is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R^{5b} is C₁-C₆ hydroxyalkyl. In some embodiments, R^{5b} is C₁-C₆ alkoxy-C₁-C₆ alkyl. In some embodiments, R^{5b} is amino-C₁-C₆ alkyl. In some embodiments, R^{5b} is C₁-C₆ alkylamino-C₁-C₆ alkyl. In some embodiments, R^{5b} is di-(C₁-C₆ alkyl)amino-C₁-C₆ alkyl. In some embodiments, R^{5b} is heterocycloalkyl unsubstituted or substituted with one to six R⁶ and R⁶ is as defined and described herein. In some embodiments, R^{5b} is heterocycloalkyl unsubstituted or substituted with one to six R⁶ and each R⁶ is independently hydroxy or C₁-C₆ alkyl. In some embodiments, R^{5b} is heterocycloalkyl-C₁-C₆ alkyl, wherein the heterocycloalkyl group is unsubstituted or substituted with one to six R⁶ and R⁶ is as defined and described herein. In some embodiments, R^{5b} is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R^{5b} is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-Ci-Cc, alkyl, or 2,2-dimethyl-1,3-dioxolan-4-yl-C₁-C₆ alkyl. In some embodiments, R^{5b} is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R^{5b} is R⁷-C(O)-C₁-C₆ alkyl; and R⁷ is hydroxy, C₁-C₆ alkoxy, amino, or hydroxyamino.

In some embodiments of any one of formulae (III), (IIIa), and (IIIb), R¹ is -N(OR^{5b})R^{5a} and -OR^{5b} is selected from the group consisting of -OH,

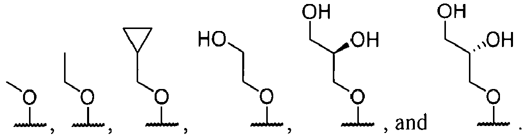

In some embodiments of any one of formulae (III), (IIIa), and (IIIb), R^{5a} is hydrogen. In some embodiments, R^{5a} is C₁-C₆ alkyl. In some embodiments, R^{5a} is C₁-C₄ alkyl. In some embodiments, R^{5a} is methyl, ethyl, propyl, isopropyl, butyl, isobutyl, sec-butyl, or tert-butyl. In some embodiments, R^{5a} is methyl.

With reference to any one of formulae (III), (IIIa), and (IIIb), in some embodiments, R¹ is a N-linked heterocycloalkyl which is unsubstituted or substituted with one or two R⁶, wherein R⁶ is as defined and described herein. In some embodiments, the N-linked heterocycloalkyl is N-linked azetidinyl, N-linked pyrrolidinyl, N-linked isoxazolidinyl, N-linked piperidinyl, or N-linked morpholinyl. In some embodiments, the N-linked heterocycloalkyl is N-linked azetidinyl. In some embodiments, the N-linked heterocycloalkyl is N-linked pyrrolidinyl. In some embodiments, the N-linked heterocycloalkyl is N-linked isoxazolidinyl. In some embodiments, the N-linked heterocycloalkyl is N-linked piperidinyl. In some embodiments, the N-linked heterocycloalkyl is N-linked morpholinyl. In some embodiments, R¹ is N-linked azetidinyl which is unsubstituted or substituted with one or two R⁶, wherein R⁶ is as defined and described herein. In some embodiments, R¹ is N-linked pyrrolidinyl which is unsubstituted or substituted with one or two R⁶, wherein R⁶ is as defined and described herein. In some embodiments, R¹ is N-linked piperidinyl which is unsubstituted or substituted with one or two R⁶, wherein R⁶ is as defined and described herein. In some embodiments, R¹ is N-linked isoxazolidinyl which is unsubstituted or substituted with one or two R⁶, wherein R⁶ is as defined and described herein. In some embodiments, R¹ is N-linked morpholinyl which is unsubstituted or substituted with one or two R⁶, wherein R⁶ is as defined and described herein.

With reference to R⁶ as one or two substituents of the N-linked heterocycloalkyl in any one of formulae (III), (IIIa), and (IIIb), in some embodiments, each R⁶ is independently hydroxy, oxo, or amino. In some embodiments, each R⁶ is hydroxy. In some embodiments, each R⁶ is oxo. In some embodiments, each R⁶ is amino. In some embodiments, one of R⁶ is hydroxy and the other R⁶ is amino.

In some embodiments of any one of formulae (III), (IIIa), and (IIIb), R¹ is a N-linked heterocycloalkyl which is unsubstituted or substituted with hydroxy, oxo, or amino. In some embodiments, R¹ is N-linked azetidinyl which is unsubstituted or substituted with hydroxy, oxo, or amino. In some embodiments, R¹ is N-linked pyrrolidinyl which is unsubstituted or substituted with hydroxy, oxo, or amino. In some embodiments, R¹ is N-linked piperidinyl which is unsubstituted or substituted with hydroxy, oxo, or amino. In some embodiments, R¹ is N-linked isoxazolidinyl which is unsubstituted or substituted with hydroxy, oxo, or amino. In some embodiments, R¹ is N-linked morpholinyl which is unsubstituted or substituted with hydroxy, oxo, or amino.

With reference to any one of formulae (III), (IIIa), and (IIIb), in some embodiments, R² is halo, C₁-C₆ alkyl, -S-C₁-C₆ alkyl, C₃-C₈ cycloalkyl, C₂-C₆ alkenyl, or C₂-C₆ alkynyl. In some embodiments, R² is halo or C₁-C₆ alkyl. In some embodiments, R² is halo,-CH₃, -SCH₃, C₂-C₃ alkenyl, or C₂-C₃ alkynyl.

In some embodiments of any one of formulae (III), (IIIa), and (IIIb), R² is halo. In some embodiments, R² is fluoro. In some embodiments, R² is iodo. In some embodiments, R² is chloro. In some embodiments, R² is bromo.

In some embodiments of any one of formulae (III), (IIIa), and (IIIb), R² is C₁-C₆ alkyl. In some embodiments, R² is C₁-C₃ alkyl. In some embodiments, R² is methyl.

In some embodiments of any one of formulae (III), (IIIa), and (IIIb), R² is -S-C₁-C₆ alkyl. In some embodiments, R² is -S-C₁-C₃ alkyl. In some embodiments, R² is -SCH₃.

In some embodiments of any one of formulae (III), (IIIa), and (IIIb), R² is C₃-C₈ cycloalkyl. In some embodiments, R² is cyclopropyl.

In some embodiments of any one of formulae (III), (IIIa), and (IIIb), R² is C₂-C₆ alkenyl. In some embodiments, R² is C₂-C₄ alkenyl. In some embodiments, R² is vinyl (ethenyl), propenyl, isopropenyl, 1-butenyl, 2-butenyl, isobutenyl, or butadienyl. In some embodiments, R² is vinyl.

In some embodiments of any one of formulae (III), (IIIa), and (IIIb), R² is C₂-C₆ alkynyl. In some embodiments, R² is C₂-C₃ alkynyl. In some embodiments, R² is acetylenyl or propynyl. In some embodiments, R² is acetylenyl.

With reference to any one of formulae (III), (IIIa), and (IIIb), in some embodiments, R^{2a} is halo or C₁-C₃ alkyl. In some embodiments, R^{2a} is halo or CH₃. In some embodiments, R^{2a} is fluoro or CH₃. In some embodiments, R^{2a} is iodo or CH₃. In some embodiments, R^{2a} is chloro or CH₃. In some embodiments, R^{2a} is bromo or CH₃.

In some embodiments of any one of formulae (III), (IIIa), and (IIIb), R^{2a} is halo. In some embodiments, R^{2a} is fluoro. In some embodiments, R^{2a} is iodo. In some embodiments, R^{2a} is chloro. In some embodiments, R^{2a} is bromo.

In some embodiments of any one of formulae (III), (IIIa), and (IIIb), R^{2a} is C₁-C₆ alkyl. In some embodiments, R^{2a} is C₁-C₃ alkyl. In some embodiments, R^{2a} is CH₃.

With reference to any one of formulae (III), (IIIa), and (IIIb), in some embodiments, R² and R^{2a} are each halo. In some embodiments, R² is halo and R^{2a} is C₁-C₆ alkyl. In some embodiments, R² is C₁-C₆ alkyl and R^{2a} is halo. In some embodiments, R² is -S-C₁-C₆ alkyl and R^{2a} is halo. In some embodiments, R² is -SCH₃ and R^{2a} is halo. In some embodiments, R² is C₃-C₈ cycloalkyl and R^{2a} is halo. In some embodiments, R² is cyclopropyl and R^{2a} is halo. In some embodiments, R² is C₂-C₆ alkenyl and R^{2a} is halo. In some embodiments, R² is C₂-C₆ alkynyl and R^{2a} is halo. In some embodiments, R² is acetylenyl and R^{2a} is halo. In some embodiments, R² and R^{2a} are each independently fluoro, chloro, bromo, or iodo. In some embodiments, R² is iodo and R^{2a} is fluoro. In some embodiments, R² is halo and R^{2a} is -CH₃. In some embodiments, R² is bromo and R^{2a} is -CH₃. In some embodiments, R² is iodo and R^{2a} is -CH₃. In some embodiments, R² is -SCH₃ and R^{2a} is fluoro. In some embodiments, R² is acetylenyl and R^{2a} is fluoro.

In some embodiments, the compound of formula (III) is represented by any one of the following formulae:

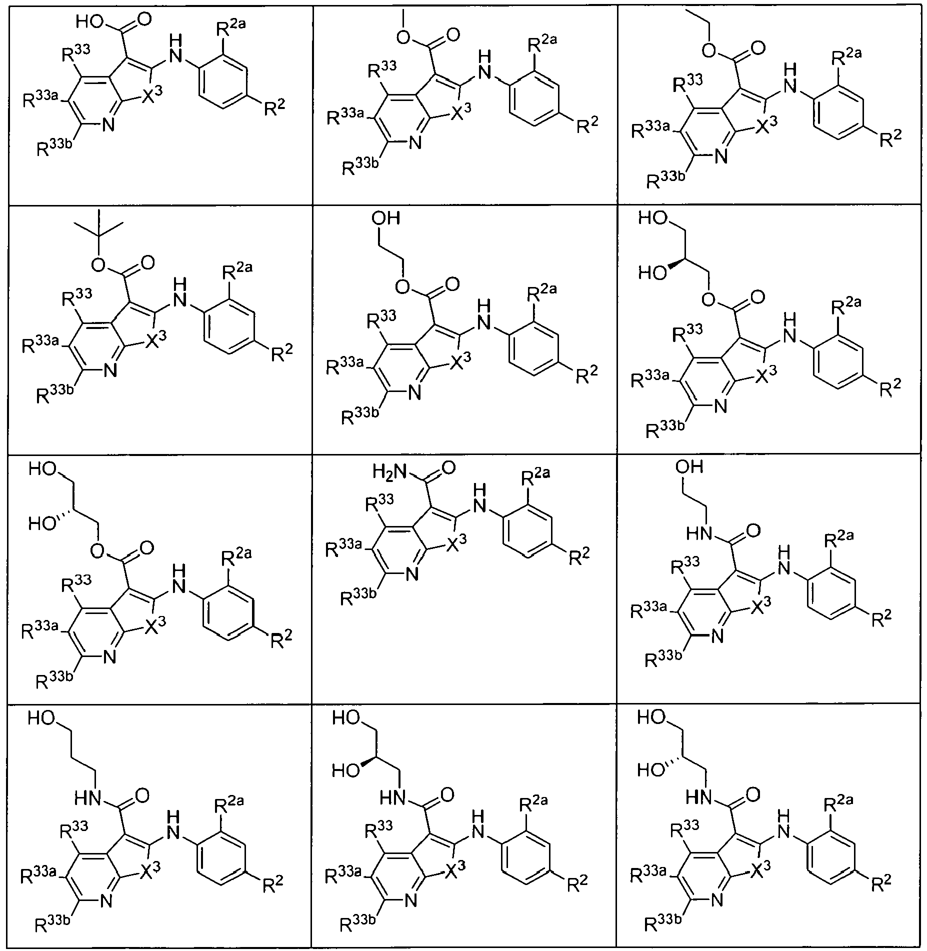

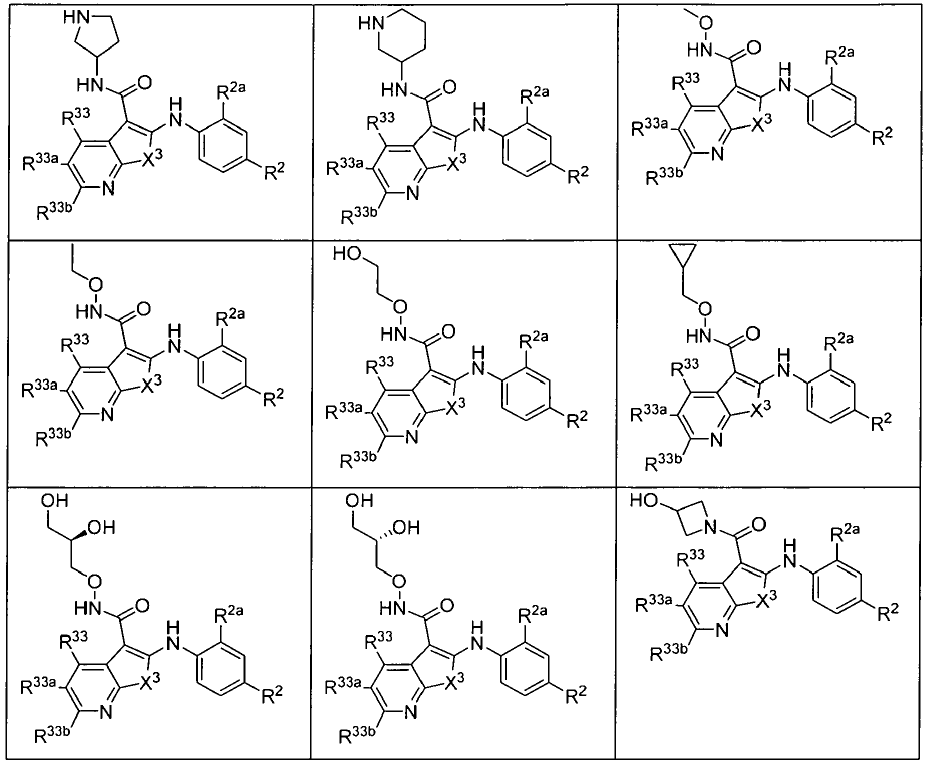

wherein X³, R², R^{2a}, R³³, R^{33a}, and R^{33b} are as defined herein in any aspect or embodiment described herein.

In some embodiments of the above structures having any one of formulae (III), (IIIa), and (IIIb), R² is iodo and R^{2a} is fluoro. In some embodiments of the above structures, R² is iodo and R^{2a} is methyl. In some embodiments of the above structures, R² is acetylenyl and R^{2a} is fluoro. In some embodiments of the above structures, R² is acetylenyl and R^{2a} is methyl. In some embodiments of the above structures, R² is -SCH₃ and R^{2a} is fluoro. In some embodiments of the above structures, R² is -SCH₃ and R^{2a} is methyl.

In some embodiments of the above structures having any one of formulae (III), (IIIa), and (IIIb), R³³, R^{33a}, and R^{33b} are each hydrogen. In some embodiments of the above structures, R³³ and R^{33b} are each hydrogen and R^{33a} is fluoro. In some embodiments of the above structures, R³³ and R^{33b} are each hydrogen and R^{33a} is methyl. In some embodiments of the above structures, R³³ and R^{33b} are each hydrogen and R^{33a} is methoxy. In some embodiments of the above structures, R³³ and R^{33a} are each hydrogen and R^{33b} is methyl. In some embodiments of the above structures, R³³ and R^{33a} are each hydrogen and R^{33b} is methoxy. In some embodiments of the above structures, R^{33a} and R^{33b} are each hydrogen and R³³ is methyl.

In some embodiments, the compounds useful in the present methods are compounds of formula (IV).

With reference to formula (IV), in some embodiments, R⁴³ is cyano, -C(O)NR⁴⁸R^{48a}, or -C(O)R⁴⁶, and R^{43a} is hydrogen, halo, or C₁-C₆ alkyl. In some embodiments, R^{43a} is hydrogen. In some embodiments, R^{43a} is halo. In some embodiments, R^{43a} is fluoro, chloro, bromo, or iodo. In some embodiments, R^{43a} is fluoro. In some embodiments, R^{43a} is C₁-C₆ alkyl. In some embodiments, R^{43a} is methyl, ethyl, propyl, isopropyl, butyl, isobutyl, sec-butyl, tert-butyl, pentyl, isopentyl, or hexyl. In some embodiments, R^{43a} is methyl.

In some embodiments of formula (IV), R⁴³ is cyano and the compound is represented by formula (IVa): 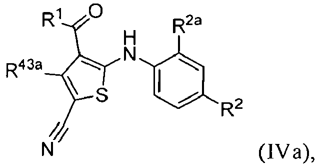 wherein R¹, R², R^{2a}, and R^{43a} are as defined herein in any aspect or embodiment described herein.

In some embodiments of formula (IVa), R^{43a} is hydrogen, halo, or C₁-C₆ alkyl. In some embodiments, R^{43a} is hydrogen, halo, or methyl. In some embodiments, R^{43a} is methyl.

With reference to formula (IV), in some embodiments, R⁴³ is -C(O)NR⁴⁸R^{48a} and the compound is represented by formula (IVb): 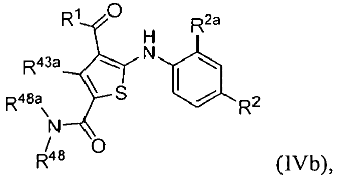 wherein R¹, R², R^{2a}, R^{43a}, R⁴⁸, and R^{48a} are as defined herein in any aspect or embodiment described herein.

In some embodiments of formula (IVb), R^{43a} is hydrogen, halo, or C₁-C₆ alkyl. In some embodiments, R^{43a} is hydrogen, halo, or methyl. In some embodiments, R^{43a} is methyl.

In some embodiments of formula (IVb), R⁴⁸ and R^{48a} are each independently hydrogen or C₁-C₆ alkyl. In some embodiments, R⁴⁸ and R^{48a} are each hydrogen. In some embodiments, R⁴⁸ and R^{48a} are each independently C₁-C₆ alkyl. In some embodiments, one of R⁴⁸ and R^{48a} is hydrogen and the other is C₁-C₆ alkyl.

In some embodiments of formula (IV), R⁴³ is -C(O)NH₂, and R^{43a} is methyl.

With reference to formula (IV), in some embodiments, R⁴³ is -C(O)R⁴⁶, and the compound is represented by formula (1Vc): 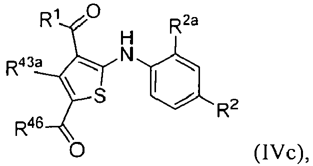 wherein R¹, R², R^{2a}, R^{43a}, and R⁴⁶ are as defined herein in any aspect or embodiment described herein.

In some embodiments of formula (1Vc), R^{43a} is hydrogen, halo, or C₁-C₆ alkyl. In some embodiments, R^{43a} is hydrogen, halo, or methyl. In some embodiments, R^{43a} is methyl.

In some embodiments of formula (IVc), R⁴⁶ is hydrogen, C₁-C₆ alkyl, or C₁-C₆ haloalkyl. In some embodiments, R⁴⁶ is hydrogen, C₁-C₆ alkyl, or C₁-C₆ haloalkyl, wherein the haloalkyl is alkyl substituted with 1, 2, or 3 fluoro.

In some embodiments of formula (IVc), R⁴⁶ is hydrogen. In some embodiments of formula (IVc), R⁴⁶ is C₁-C₆ alkyl. In some embodiments, R⁴⁶ is C₁-C₄ alkyl. In some embodiments, R⁶ is methyl, ethyl, n-propyl, isopropyl, n-butyl, isobutyl, or t-butyl.

In some embodiments of formula (IVc), R⁴⁶ is C₁-C₆ haloalkyl wherein the haloalkyl is alkyl substituted with 1, 2, or 3 fluoro. In some embodiments, R⁴⁶ is C₁-C₄ haloalkyl wherein the C₁-C₄ haloalkyl is C₁-C₄ alkyl substituted with 1, 2, or 3 fluoro. In some embodiments, R⁴⁶ is trifluoromethyl, fluoromethyl, or 2,2,2-trifluoroethyl.

In some embodiments of formula (IVc), R⁴⁶ is C₃-C₈ cycloalkyl or heterocycloalkyl. In some embodiments, R⁴⁶ is C₃-C₈ cycloalkyl. In some embodiments, R⁴⁶ is cyclopropyl or cyclobutyl. In some embodiments, R⁶ is heterocycloalkyl. In some embodiments, R⁴⁶ is a 3 to 8 membered heterocycloalkyl having 1 to 3 heteroatoms of N, O, or S. In some embodiments, R⁴⁶ is a 3 to 6 membered heterocycloalkyl having 1 to 2 heteroatoms of N or O. In some embodiments, R⁴⁶ is aziridinyl, azetidinyl, pyrrolidinyl, piperidinyl, isoxazolidinyl, or morpholinyl.

Returning to formula (IV), in some embodiments, R⁴³ and R^{43a} together form -CH₂CH₂C(O)- or -CH₂CH₂CH₂C(O)-, each of which is unsubstituted or substituted with one or two R⁴⁹. In some embodiments, R⁴³ and R^{43a} together form -CH₂CH₂C(O)- or -CH₂CH₂CH₂C(O)-.

In some embodiments of formula (IV), the compound is represented by formula (1Vd-1) or (1Vd-2): 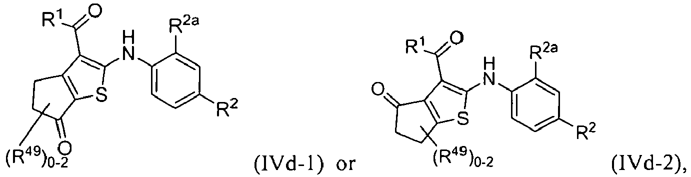 wherein R¹, R², R^{2a}, and R⁴⁹ are as defined herein in any aspect or embodiment described herein.

In some embodiments of formula (IV), the compound is represented by formula (IVe-1) or (IVe-2): 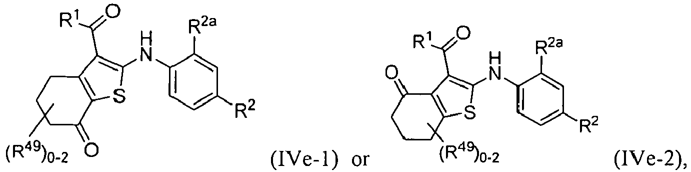 wherein R¹, R², R^{2a}, and R⁴⁹ are as defined herein in any aspect or embodiment described herein.

In some embodiments of any one of formulae (IVd-1), (IVd-2), (IVe-1), and (IVe-2), each R⁴⁹ is independently C₁-C₆ alkyl. In some embodiments of any one of formulae (IVd-1), (IVd-2), (1Ve-1), and (IVe-2), R⁴⁹ is absent.

With reference to any one of formulae (IV), (IVa), (IVb), (IVc), (IVd-1), (IVd-2), (IVe-1) and (IVe-2), in some embodiments, R¹ is -OR⁴, -NR⁵R^{5a}, or -N(OR^{5b})R^{5a}.

In some embodiments of any one of formulae (IV), (1Va), (IVb), (IVc), (IVd-1), (IVd-2), (IVe-I) and (IVe-2), R¹ is -OR⁴. In some embodiments, R⁴ is hydrogen. In some embodiments, R⁴ is C₁-C₆ alkyl. In some embodiments, R⁴ is C₃-C₈ cycloalkyl unsubstituted or substituted with one to six R⁶ and R⁶ is as defined and described herein. In some embodiments, R⁴ is C₃-C₈ cycloalkyl unsubstituted or substituted with one to six R⁶ and each R⁶ is independently hydroxy or C₁-C₆ alkyl. In some embodiments, R⁴ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁴ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁴ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁴ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁴ is C₁-C₆ hydroxyalkyl. In some embodiments, R⁴ is C₁-C₆ alkoxy-C₁-C₆ alkyl. In some embodiments, R⁴ is amino-C₁-C₆ alkyl. In some embodiments, R⁴ is C₁-C₆ alkylamino-C₁-C₆ alkyl. In some embodiments, R⁴ is di-(C₁-C₆ alkyl)amino-C₁-C₆ alkyl. In some embodiments, R⁴ is heterocycloalkyl unsubstituted or substituted with one to six R⁶ and R⁶ is as defined and described herein. In some embodiments, R⁴ is heterocycloalkyl unsubstituted or substituted with one to six R⁶ and each R⁶ is independently hydroxy or C₁-C₆ alkyl. In some embodiments, R⁴ is heterocycloalkyl-C₁-C₆ alkyl, wherein the heterocycloalkyl group is unsubstituted or substituted with one to six R⁶ and R⁶ is as defined and described herein. In some embodiments, R⁴ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁴ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁴ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁴ is R⁷-C(O)-C₁-C₆ alkyl; and R⁷ is hydroxy, C₁-C₆ alkoxy, amino, or hydroxyamino.

In some embodiments of any one of formulae (IV), (IVa), (IVb), (IVc), (IVd-1), (IVd-2), (IVe-1) and (IVe-2), R¹ is selected from the group consisting of -OH,

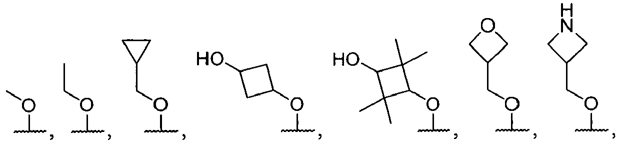

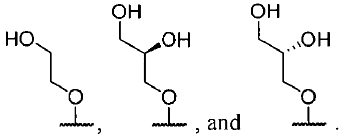

In some embodiments of any one of formulae (IV), (1Va), (IVb), (IVc), (IVd-1), (IVd-2), (IVe-1) and (IVe-2), R¹ is -NR⁵R^{5a}. In some embodiments, R⁵ is hydrogen. In some embodiments, R⁵ is C₁-C₆ alkyl. In some embodiments, R⁵ is C₃-C₈ cycloalkyl unsubstituted or substituted with one to six R⁶ and R⁶ is as defined and described herein. In some embodiments, R⁵ is C₃-C₈ cycloalkyl unsubstituted or substituted with one to six R⁶ and each R⁶ is independently hydroxy or C₁-C₆ alkyl. In some embodiments, R⁵ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁵ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁵ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁵ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁵ is C₁-C₆ hydroxyalkyl. In some embodiments, R⁵ is C₁-C₆ alkoxy-C₁-C₆ alkyl. In some embodiments, R⁵ is amino-C₁-C₆ alkyl. In some embodiments, R⁵ is C₁-C₆ alkylamino-C₁-C₆ alkyl. In some embodiments, R⁵ is di-(C₁-C₆ alkyl)amino-C₁-C₆ alkyl. In some embodiments, R⁵ is heterocycloalkyl unsubstituted or substituted with one to six R⁶ and R⁶ is as defined and described herein. In some embodiments, R⁵ is heterocycloalkyl unsubstituted or substituted with one to six R⁶ and each R⁶ is independently hydroxy or C₁-C₆ alkyl. In some embodiments, R⁵ is heterocycloalkyl-C₁-C₆ alkyl, wherein the heterocycloalkyl group is unsubstituted or substituted with one to six R⁶ and R⁶ is as defined and described herein. In some embodiments, R⁵ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁵ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, 2,2-dimethyl-1,3-dioxolan-4-yl-C₁-C₆ alkyl. In some embodiments, R⁵ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁵ is R⁷-C(O)-C₁-C₆ alkyl; and R⁷ is hydroxy, C₁-C₆ alkoxy, amino, or hydroxyamino.

In some embodiments of any one of formulae (IV), (IVa), (IVb), (IVc), (IVd-1), (IVd-2), (IVe-1) and (IVe-2), R¹ is -NR⁵R^{5a} and R⁵ is selected from the group consisting of hydrogen,

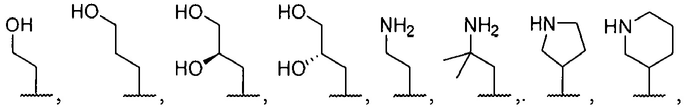

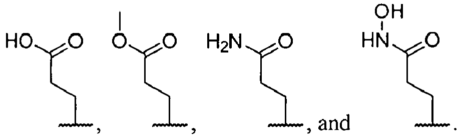

In some embodiments of any one of formulae (IV), (IVa), (IVb), (IVc), (IVd-1), (IVd-2), (IVe-1) and (IVe-2), R¹ is -N(OR^{5b})R^{5a}. In some embodiments, R^{5b} is hydrogen. In some embodiments, R^{5b} is C₁-C₆ alkyl. In some embodiments, R^{5b} is C₃-C₈ cycloalkyl unsubstituted or substituted with one to six R⁶ and R⁶ is as defined and described herein. In some embodiments, R^{5b} is C₃-C₈ cycloalkyl unsubstituted or substituted with one to six R⁶ and each R⁶ is independently hydroxy or C₁-C₆ alkyl. In some embodiments, R^{5b} is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R^{5b} is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R^{5b} is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R^{5b} is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R^{5b} is C₁-C₆ hydroxyalkyl. In some embodiments, R^{5b} is C₁-C₆ alkoxy-C₁-C₆ alkyl. In some embodiments, R^{5b} is amino-C₁-C₆ alkyl. In some embodiments, R^{5b} is C₁-C₆ alkylamino-C₁-C₆ alkyl. In some embodiments, R^{5b} is di-(C₁-C₆ alkyl)amino-C₁-C₆ alkyl. In some embodiments, R^{5b} is heterocycloalkyl unsubstituted or substituted with one to six R⁶ and R⁶ is as defined and described herein. In some embodiments, R^{5b} is heterocycloalkyl unsubstituted or substituted with one to six R⁶ and each R⁶ is independently hydroxy or C₁-C₆ alkyl. In some embodiments, R^{5b} is heterocycloalkyl-C₁-C₆ alkyl, wherein the heterocycloalkyl group is unsubstituted or substituted with one to six R⁶ and R⁶ is as defined and described herein. In some embodiments, R^{5b} is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R^{5b} is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R^{5b} is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R^{5b} is R⁷-C(O)-C₁-C₆ alkyl; and R⁷ is hydroxy, C₁-C₆ alkoxy, amino, or hydroxyamino.

In some embodiments of any one of formulae (IV), (IVa), (IVb), (IVc), (IVd-1), (IVd-2), (IVe-1) and (IVe-2), R¹ is -N(OR^{5b})R^{5a} and -OR^{5b} is selected from the group consisting of-OH,

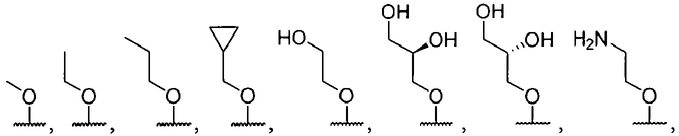

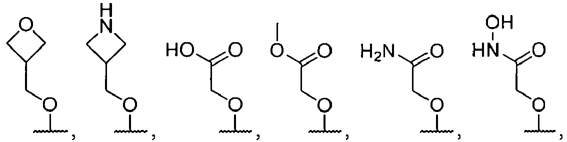

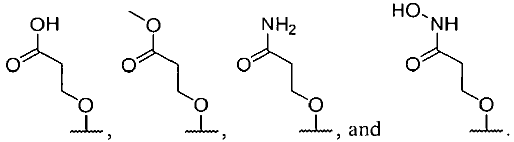

In some embodiments of any one of formulae (IV), (IVa), (IVb), (IVc), (IVd-1), (IVd-2), (IVe-1) and (IVe-2), R^{5a} is hydrogen. In some embodiments, R^{5a} is C₁-C₆ alkyl. In some embodiments, R^{5a} is C₁-C₄ alkyl. In some embodiments, R^{5a} is methyl, ethyl, propyl, isopropyl, butyl, isobutyl, sec-butyl, or tert-butyl. In some embodiments, R^{5a} is methyl.

With reference to any one of formulae (IV), (IVa), (IVb), (IVc), (IVd-1), (1Vd-2), (IVe-1) and (IVe-2), in some embodiments, R¹ is a N-linked heterocycloalkyl which is unsubstituted or substituted with one or two R⁶, wherein R⁶ is as defined and described herein. In some embodiments, the N-linked heterocycloalkyl is N-linked azetidinyl, N-linked pyrrolidinyl, N-linked isoxazolidinyl, N-linked piperidinyl, or N-linked morpholinyl. In some embodiments, the N-linked heterocycloalkyl is N-linked azetidinyl. In some embodiments, the N-linked heterocycloalkyl is N-linked pyrrolidinyl. In some embodiments, the N-linked heterocycloalkyl is N-linked isoxazolidinyl. In some embodiments, the N-linked heterocycloalkyl is N-linked piperidinyl. In some embodiments, the N-linked heterocycloalkyl is N-linked morpholinyl. In some embodiments, R¹ is N-linked azetidinyl which is unsubstituted or substituted with one or two R⁶, wherein R⁶ is as defined and described herein. In some embodiments, R¹ is N-linked pyrrolidinyl which is unsubstituted or substituted with one or two R⁶, wherein R⁶ is as defined and described herein. In some embodiments, R¹ is N-linked piperidinyl which is unsubstituted or substituted with one or two R⁶, wherein R⁶ is as defined and described herein. In some embodiments, R¹ is N-linked isoxazolidinyl which is unsubstituted or substituted with one or two R⁶, wherein R⁶ is as defined and described herein. In some embodiments, R¹ is N-linked morpholinyl which is unsubstituted or substituted with one or two R⁶, wherein R⁶ is as defined and described herein.

With reference to R⁶ as one or two substituents of the N-linked heterocycloalkyl in any one of formulae (IV), (IVa), (IVb), (IVc), (IVd-1), (IVd-2), (IVe-1) and (IVe-2), in some embodiments, each R⁶ is independently hydroxyl, oxo, or amino. In some embodiments, each R⁶ is hydroxy. In some embodiments, each R⁶ is oxo. In some embodiments, each R⁶ is amino. In some embodiments, one of R⁶ is hydroxy and the other R⁶ is amino.

In some embodiments of any one of formulae (IV), (IVa), (IVb), (IVc), (IVd-1), (IVd-2), (IVe-1) and (IVe-2), R¹ is a N-linked heterocycloalkyl which is unsubstituted or substituted with hydroxyl, oxo, or amino. In some embodiments, R¹ is N-linked azetidinyl which is unsubstituted or substituted with hydroxyl, oxo, or amino. In some embodiments, R¹ is N-linked pyrrolidinyl which is unsubstituted or substituted with hydroxyl, oxo, or amino. In some embodiments, R¹ is N-linked piperidinyl which is unsubstituted or substituted with hydroxyl, oxo, or amino. In some embodiments, R¹ is N-linked isoxazolidinyl which is unsubstituted or substituted with hydroxyl, oxo, or amino. In some embodiments, R¹ is N-linked morpholinyl which is unsubstituted or substituted with hydroxyl, oxo, or amino.

With reference to any one of formulae (IV), (IVa), (IVb), (1Vc), (1Vd-1), (1Vd-2), (IVe-1) and (IVe-2), in some embodiments, R² is halo, C₁-C₆ alkyl, -S-C₁-C₆ alkyl, C₃-C₈ cycloalkyl, C₂-C₆ alkenyl, or C₂-C₆ alkynyl. In some embodiments, R² is halo or C₁-C₆ alkyl. In some embodiments, R² is R² is halo,-CH₃, -SCH₃, C₂-C₃ alkenyl, or C₂-C₃ alkynyl.

In some embodiments of any one of formulae (IV), (IVa), (IVb), (IVc), (IVd-1), (IVd-2), (IVe-1) and (IVe-2), R² is halo. In some embodiments, R² is fluoro. In some embodiments, R² is iodo. In some embodiments, R² is chloro. In some embodiments, R² is bromo.

In some embodiments of any one of formulae (IV), (IVa), (IVb), (IVc), (IVd-1), (IVd-2), (IVe-1) and (IVe-2), R² is C₁-C₆ alkyl. In some embodiments, R² is C₁-C₃ alkyl. In some embodiments, R² is methyl.

In some embodiments of any one of formulae (IV), (IVa), (IVb), (IVc), (IVd-1), (IVd-2), (IVe-1) and (IVe-2), R² is -S-C₁-C₆ alkyl. In some embodiments, R² is -S-C₁-C₃ alkyl. In some embodiments, R² is -SCH₃.

In some embodiments of any one of formulae (IV), (IVa), (IVb), (IVc), (IVd-1), (IVd-2), (IVe-1) and (IVe-2), R² is C₃-C₈ cycloalkyl. In some embodiments, R² is cyclopropyl.

In some embodiments of any one of formulae (IV), (IVa), (IVb), (IVc), (IVd-1), (IVd-2), (IVe-1) and (IVe-2), R² is C₂-C₆ alkenyl. In some embodiments, R² is C₂-C₄ alkenyl. In some embodiments, R² is vinyl (ethenyl), propenyl, isopropenyl, 1-butenyl, 2-butenyl, isobutenyl, or butadienyl. In some embodiments, R² is vinyl.

In some embodiments of any one of formulae (IV), (IVa), (IVb), (IVc), (IVd-1), (IVd-2), (IVe-1) and (IVe-2), R² is C₂-C₆ alkynyl. In some embodiments, R² is C₂-C₃ alkynyl. In some embodiments, R² is acetylenyl or propynyl. In some embodiments, R² is acetylenyl.

With reference to any one of formulae (IV), (IVa), (IVb), (IVc), (IVd-1), (IVd-2), (IVe-1) and (IVe-2), in some embodiments, R^{2a} is halo or C₁-C₃ alkyl. In some embodiments, R^{2a} is halo or CH₃. In some embodiments, R^{2a} is fluoro or CH₃. In some embodiments, R^{2a} is iodo or CH₃. In some embodiments, R^{2a} is chloro or CH₃. In some embodiments, R^{2a} is bromo or CH₃.

In some embodiments of any one of formulae (IV), (IVa), (IVb), (IVc), (IVd-1), (IVd-2), (IVe-1) and (IVe-2), R^{2a} is halo. In some embodiments, R^{2a} is fluoro. In some embodiments, R^{2a} is iodo. In some embodiments, R^{2a} is chloro. In some embodiments, R^{2a} is bromo.

In some embodiments of any one of formulae (IV), (IVa), (IVb), (IVc), (IVd-1), (IVd-2), (IVe-1) and (IVe-2), R^{2a} is C₁-C₆ alkyl. In some embodiments, R^{2a} is C₁-C₃ alkyl. In some embodiments, R^{2a} is CH₃.

With reference to any one of formulae (IV), (IVa), (IVb), (IVc), (IVd-1), (IVd-2), (IVe-I) and (IVe-2), in some embodiments, R² and R^{2a} are each halo. In some embodiments, R² is halo and R^{2a} is C₁-C₆ alkyl. In some embodiments, R² is C₁-C₆ alkyl and R^{2a} is halo. In some embodiments, R² is -S-C₁-C₆ alkyl and R^{2a} is halo. In some embodiments, R² is -SCH₃ and R^{2a} is halo. In some embodiments, R² is C₃-C₈ cycloalkyl and R^{2a} is halo. In some embodiments, R² is cyclopropyl and R^{2a} is halo. In some embodiments, R² is C₂-C₆ alkenyl and R^{2a} is halo. In some embodiments, R² is C₂-C₆ alkynyl and R^{2a} is halo. In some embodiments, R² is acetylenyl and R^{2a} is halo. In some embodiments, R² and R^{2a} are each independently fluoro, chloro, bromo, or iodo. In some embodiments, R² is iodo and R^{2a} is fluoro. In some embodiments, R² is halo and R^{2a} is -CH₃. In some embodiments, R² is bromo and R^{2a} is -CH₃. In some embodiments, R² is iodo and R^{2a} is -CH₃. In some embodiments, R² is -SCH₃ and R^{2a} is fluoro. In some embodiments, R² is acetylenyl and R^{2a} is fluoro.

In some embodiments, the compound of formula (IV) is represented by any one of the following formulae:

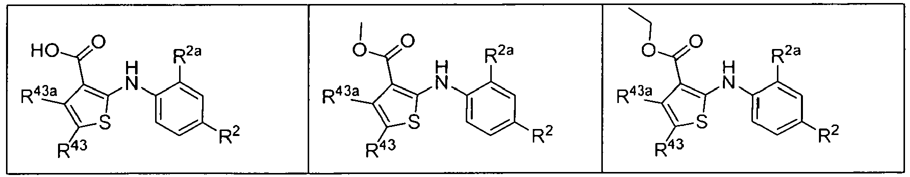

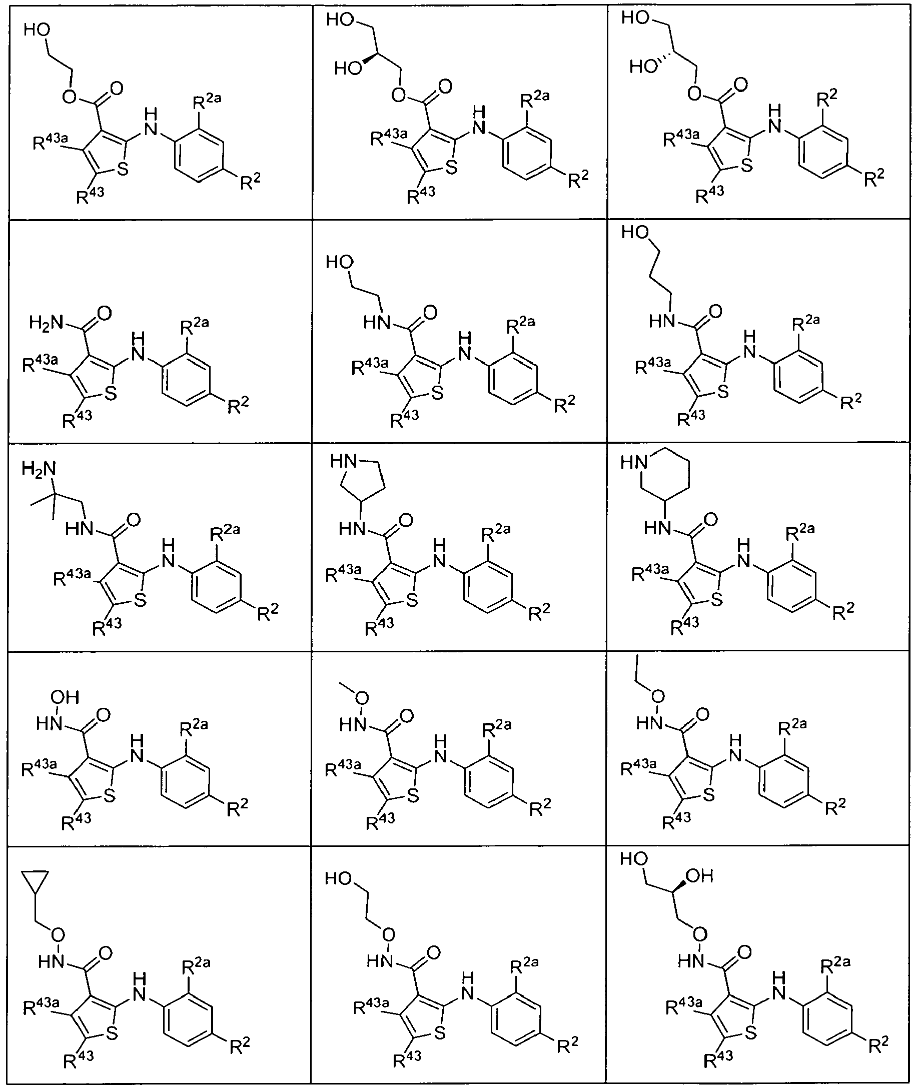

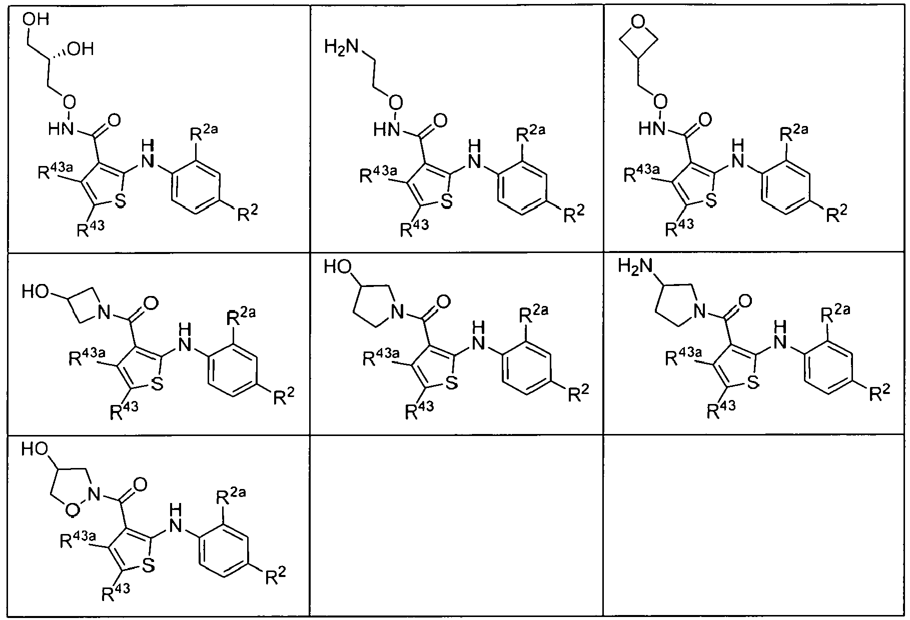

wherein R², R^{2a}, R⁴³, and R^{43a} are as defined herein in any aspect or embodiment described herein.

In some embodiments of the above structures having formula (IV), R² is iodo and R^{2a} is fluoro. In some embodiments of the above structures, R² is iodo and R^{2a} is methyl. In some embodiments of the above structures, R² is acetylenyl and R^{2a} is fluoro. In some embodiments of the above structures, R² is acetylenyl and R^{2a} is methyl. In some embodiments of the above structures, R² is -SCH₃ and R^{2a} is fluoro. In some embodiments of the above structures, R² is -SCH₃ and R^{2a} is methyl.

In some embodiments of the above structures having formula (IV), R⁴³ is cyano, -C(O)NR⁴⁸R^{48a}, or -C(O)R⁴⁶, and R^{43a} is C₁-C₆ alkyl. In some embodiments of the above structures, R⁴³ is cyano and R^{43a} is methyl. In some embodiments of the above structures, R⁴³ is -C(O)NR⁴⁸R^{48a}; R⁴⁸ and R^{48a} are each hydrogen; and R^{43a} is methyl. In some embodiments of the above structures, R⁴³ is -C(O)R⁴⁶; R⁴⁶ is C₁-C₄ alkyl; and R^{43a} is C₁-C₄ alkyl. In some embodiments of the above structures, R⁴³ is -C(O)R⁴⁶; R⁴⁶ is C₁-C₄ alkyl; and R^{43a} is methyl. In some embodiments of the above structures, R⁴³ and R^{43a} together form -CH₂CH₂C(O)- or -CH₂CH₂CH₂C(O)-.

In some embodiments, the compounds useful in the present methods are compounds of formula (V).

With reference to formula (V), in some embodiments, bond "a" is a double bond and the compound is represented by formula (Va): 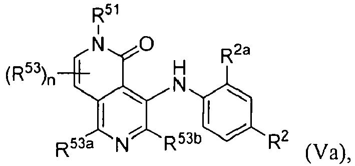 wherein subscript n, R², R^{2a}, R⁵¹, R⁵³, R^{53a}, and R^{53b} are as defined herein in any aspect or embodiment described herein.

In some embodiments of formula (V), bond "a" is a single bond and the compound is represented by formula (Vb): 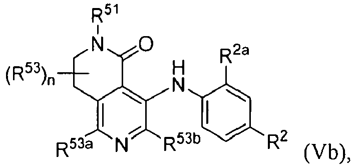 wherein subscript n, R², R^{2a}, R⁵¹, R⁵³, R^{53a}, and R^{53b}, are as defined herein in any aspect or embodiment described herein.

In some embodiments of any one of formulae (V), (Va), and (Vb), each R⁵³ is independently halo or C₁-C₃ alkyl. In some embodiments, each R⁵³ is independently fluoro, chloro, bromo, iodo, methyl, ethyl, propyl, or isopropyl. In some embodiments, each R⁵³ is methyl.

In some embodiments of any one of formulae (V), (Va), and (Vb), subscript n is 0 or I. In some embodiments, subscript n is 0 and the compound is represented by formula (Va-1) or (Vb-1): 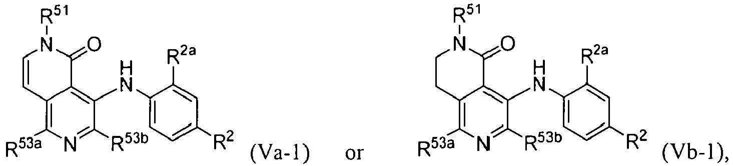 wherein R², R^{2a}, R⁵¹, R^{53a}, and R^{53b} are as defined herein in any aspect or embodiment described herein.

With reference to any one of formulae (V), (Va), (Vb), (Va-1), and (Vb-1), R^{53a} and R^{53b} are each independently hydrogen, halo, or C₁-C₆ alkyl.

In some embodiments of any one of formulae (V), (Va), (Vb), (Va-1), and (Vb-1), R^{53a} is hydrogen, halo, or C₁-C₆ alkyl. In some embodiments, R^{53a} is hydrogen. In some embodiments, R^{53a} is halo. In some embodiments, R^{53a} is fluoro, chloro, bromo, or iodo. In some embodiments, R^{53a} is fluoro. In some embodiments, R^{53a} is C₁-C₆ alkyl. In some embodiments, R^{53a} is methyl, ethyl, propyl, isopropyl, butyl, isobutyl, sec-butyl, tert-butyl, pentyl, isopentyl, or hexyl. In some embodiments, R^{53a} is methyl.

In some embodiments of any one of formulae (V), (Va), (Vb), (Va-1), and (Vb-1), R^{53b} is hydrogen, halo, or C₁-C₆ alkyl. In some embodiments, R^{53b} is hydrogen. In some embodiments, R^{53b} is halo. In some embodiments, R^{53b} is fluoro, chloro, bromo, or iodo. In some embodiments, R^{53b} is fluoro. In some embodiments, R^{53b} is C₁-C₆ alkyl. In some embodiments, R^{53b} can be methyl, ethyl, propyl, isopropyl, butyl, isobutyl, sec-butyl, tert-butyl, pentyl, isopentyl, or hexyl. In some embodiments, R^{53b} is methyl.

In some embodiments of any one of formulae (V), (Va), (Vb), (Va-1), and (Vb-1), R^{53a} and R^{53b} are each hydrogen. In some embodiments, R^{53a} is hydrogen and R^{53b} is halo. In some embodiments, R^{53a} is hydrogen and R^{53b} is fluoro.

with reference to any one of formulae (V), (Va), (Vb), (Va-1), and (Vb-1), in some embodiments, R⁵¹ is C₁-C₆ alkyl. In some embodiments, R⁵ is C₃-C₈ cycloalkyl unsubstituted or substituted with one to six R⁶ and R⁶ is as defined and described herein. In some embodiments, R⁵¹ is C₃-C₈ cycloalkyl unsubstituted or substituted with one to six R⁶ and each R⁶ is independently hydroxy or C₁-C₆ alkyl. In some embodiments, R⁵¹ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁵¹ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁵¹ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁵¹ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁵¹ is C₁-C₆ hydroxyalkyl. In some embodiments, R⁵¹ is C₁-C₆ alkoxy-C₁-C₆ alkyl. In some embodiments, R⁵¹ is amino-C₁-C₆ alkyl. In some embodiments, R⁵¹ is C₁-C₆ alkylamino-C₁-C₆ alkyl. In some embodiments, R⁵¹ is di-(C₁-C₆ alkyl)amino-C₁-C₆ alkyl. In some embodiments, R⁵¹ is heterocycloalkyl unsubstituted or substituted with one to six R⁶ and R⁶ is as defined and described herein. In some embodiments, R⁵¹ is heterocycloalkyl unsubstituted or substituted with one to six R⁶ and each R⁶ is independently hydroxy or C₁-C₆ alkyl. In some embodiments, R⁵ is heterocycloalkyl-C₁-C₆ alkyl, wherein the heterocycloalkyl group is unsubstituted or substituted with one to six R⁶ and R⁶ is as defined and described herein. In some embodiments, R⁵¹ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁵¹ is oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁵¹ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁵¹ is R⁷-C(O)-C₁-C₆ alkyl; and R⁷ is hydroxy, C₁-C₆ alkoxy, amino, or hydroxyamino.

In some embodiments of any one of formulae (V), (Va), (Vb), (Va-1), and (Vb-1), R⁵¹ is selected from the group consisting of hydrogen,

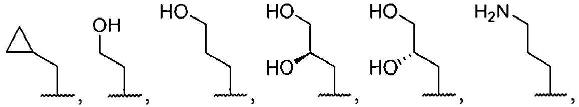

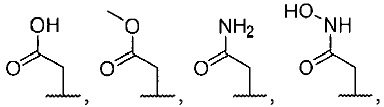

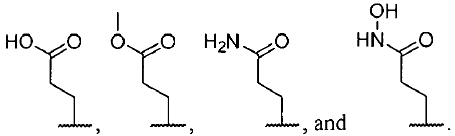

In some embodiments of any one of formulae (V), (Va), (Vb), (Va-1), and (Vb-1), R⁵¹ is OR⁵⁴. In some embodiments, R⁵⁴ is hydrogen. In some embodiments, R⁵⁴ is C₁-C₆ alkyl. In some embodiments, R⁵⁴ is C₃-C₈ cycloalkyl unsubstituted or substituted with one to six R⁶ and R⁶ is as defined and described herein. In some embodiments, R⁵⁴ is C₃-C₈ cycloalkyl unsubstituted or substituted with one to six R⁶ and each R⁶ is independently hydroxy or C₁-C₆ alkyl. In some embodiments, R⁵⁴ is C₃-C₈ cycloalkyl-C₁-C₆ alkyl, wherein the C₃-C₈ cycloalkyl group is unsubstituted or substituted with one to six R⁶ and R⁶ is as defined and described herein. In some embodiments, R⁵⁴ is C₃-C₈ cycloalkyl-C₁-C₆ alkyl, wherein the C₃-C₈ cycloalkyl group is unsubstituted or substituted with one to six R⁶ and each R⁶ is independently hydroxy or C₁-C₆ alkyl. In some embodiments, R⁵⁴ is cyclopropyl, cyclobutyl, cyclopropyl-C₁-C₆ alkyl, or cyclobutyl-C₁-C₆ alkyl; and each of the cyclopropyl and cyclobutyl groups is unsubstituted or substituted with one to six R⁶ and R⁶ is as defined and described herein. In some embodiments, R⁵ is cyclopropyl, cyclobutyl, cyclopropyl-C₁-C₆ alkyl, or cyclobutyl-C₁-C₆ alkyl, each of the cyclopropyl and cyclobutyl groups is unsubstituted or substituted with one to six R⁶ and each R⁶ is independently hydroxy or C₁-C₆ alkyl. In some embodiments, R⁵⁴ is C₁-C₆ hydroxyalkyl. In some embodiments, R⁵⁴ is C₁-C₆ alkoxy-C₁-C₆ alkyl. In some embodiments, R⁵⁴ is amino-C₁-C₆ alkyl. In some embodiments, R⁵⁴ is C₁-C₆ alkylamino-C₁-C₆ alkyl. In some embodiments, R⁵⁴ is di-(C₁-C₆ alkyl)amino-C₁-C₆ alkyl. In some embodiments, R⁵⁴ is heterocycloalkyl unsubstituted or substituted with one to six R⁶ and R⁶ is as defined and described herein. In some embodiments, R⁵⁴ is heterocycloalkyl unsubstituted or substituted with one to six R⁶ and each R⁶ is independently hydroxy or C₁-C₆ alkyl. In some embodiments, R⁵⁴ is heterocycloalkyl-C₁-C₆ alkyl, wherein the heterocycloalkyl group is unsubstituted or substituted with one to six R⁶ and R⁶ is as defined and described herein. In some embodiments, R⁵⁴ is heterocycloalkyl-C₁-C₆ alkyl, wherein the heterocycloalkyl group is unsubstituted or substituted with one to six R⁶ and each R⁶ is independently hydroxy or C₁-C₆ alkyl. In some embodiments, R⁵⁴ is oxetanyl, azetidinyl, pyrrolidinyl, piperidinyl, oxetanyl-C₁-C₆ alkyl, azetidinyl-C₁-C₆ alkyl, pyrrolidinyl-C₁-C₆ alkyl, piperidinyl-C₁-C₆ alkyl, or 2,2-dimethyl-1,3-dioxolan-4-yl-C₁-C₆ alkyl. In some embodiments, R⁵⁴ is R⁷-C(O)-C₁-C₆ alkyl; and R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino. In some embodiments, R⁵⁴ is R⁷-C(O)-C₁-C₆ alkyl; and R⁷ is hydroxy, C₁-C₆ alkoxy, amino, or hydroxyamino.

In some embodiments of any one of formulae (V), (Va), (Vb), (Va-1), and (Vb-1), R⁵¹ is selected from the group consisting of -OH,

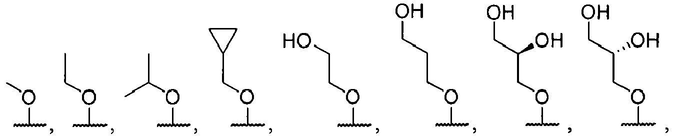

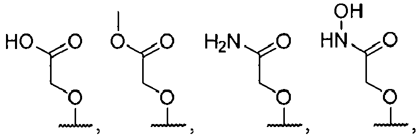

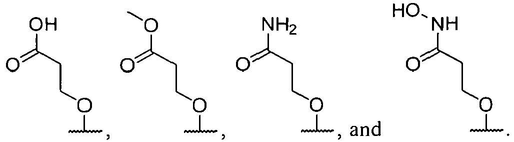

With reference to any one of formulae (V), (Va), (Vb), (Va-1), and (Vb-1), in some embodiments, R² is halo, C₁-C₆ alkyl, -S-C₁-C₆ alkyl, C₃-C₈ cycloalkyl, C₂-C₆ alkenyl, or C₂-C₆ alkynyl. In some embodiments, R² is halo or C₁-C₆ alkyl. In some embodiments, R² is halo, -SCH₃, -CH₃, C₂-C₃ alkenyl, or C₂-C₃ alkynyl.

In some embodiments of any one of formulae (V), (Va), (Vb), (Va-1), and (Vb-1), R² is halo. In some embodiments, R² is fluoro. In some embodiments, R² is iodo. In some embodiments, R² is chloro. In some embodiments, R² is bromo.

In some embodiments of any one of formulae (V), (Va), (Vb), (Va-1), and (Vb-1), R² is C₁-C₆ alkyl. In some embodiments, R² is C₁-C₃ alkyl. In some embodiments, R² is methyl.

In some embodiments of any one of formulae (V), (Va), (Vb), (Va-1), and (Vb-1), R² is -S-C₁-C₆ alkyl. In some embodiments, R² is -S-C₁-C₃ alkyl. In some embodiments, R² is -SCH₃.

In some embodiments of any one of formulae (V), (Va), (Vb), (Va-1), and (Vb-I), R² is C₃-C₈ cycloalkyl. In some embodiments, R² is cyclopropyl.

In some embodiments of any one of formulae (V), (Va), (Vb), (Va-1), and (Vb-1), R² is C₂-C₆ alkenyl. In some embodiments, R² is C₂-C₄ alkenyl. In some embodiments, R² is vinyl (ethenyl), propenyl, isopropenyl, I-butenyl, 2-butenyl, isobutenyl, or butadienyl. In some embodiments, R² is vinyl.

In some embodiments of any one of formulae (V), (Va), (Vb), (Va-1), and (Vb-1), R² is C₂-C₆ alkynyl. In some embodiments, R² is C₂-C₃ alkynyl. In some embodiments, R² is acetylenyl or propynyl. In some embodiments, R² is acetylenyl.

With reference to any one of formulae (V), (Va), (Vb), (Va-1), and (Vb-1), in some embodiments, R^{2a} is halo or C₁-C₃ alkyl. In some embodiments, R^{2a} is halo or CH₃. In some embodiments, R^{2a} is fluoro or CH₃. In some embodiments, R^{2a} is iodo or CH₃. In some embodiments, R^{2a} is chloro or CH₃. In some embodiments, R^{2a} is bromo or CH₃.

In some embodiments of any one of formulae (V), (Va), (Vb), (Va-1), and (Vb-1), R^{2a} is halo. In some embodiments, R^{2a} is fluoro. In some embodiments, R^{2a} is iodo. In some embodiments, R^{2a} is chloro. In some embodiments, R^{2a} is bromo.

In some embodiments of any one of formulae (V), (Va), (Vb), (Va-1), and (Vb-1), R^{2a} is C₁-C₆ alkyl. In some embodiments, R^{2a} is C₁-C₃ alkyl. In some embodiments, R^{2a} is CH₃.

With reference to any one of formulae (V), (Va), (Vb), (Va-1), and (Vb-1), in some embodiments, R² and R^{2a} are each halo. In some embodiments, R² is halo and R^{2a} is C₁-C₆ alkyl. In some embodiments, R² is C₁-C₆ alkyl and R^{2a} is halo. In some embodiments, R² is -S-C₁-C₆ alkyl and R^{2a} is halo. In some embodiments, R² is -SCH₃ and R^{2a} is halo. In some embodiments, R² is C₃-C₈ cycloalkyl and R^{2a} is halo. In some embodiments, R² is cyclopropyl and R^{2a} is halo. In some embodiments, R² is C₂-C₆ alkenyl and R^{2a} is halo. In some embodiments, R² is C₂-C₆ alkynyl and R^{2a} is halo. In some embodiments, R² is acetylenyl and R^{2a} is halo. In some embodiments, R² and R^{2a} are each independently fluoro, chloro, bromo, or iodo. In some embodiments, R² is iodo and R^{2a} is fluoro. In some embodiments, R² is halo and R^{2a} is -CH₃. In some embodiments, R² is bromo and R^{2a} is -CH₃. In some embodiments, R² is iodo and R^{2a} is -CH₃. In some embodiments, R² is -SCH₃ and R^{2a} is fluoro. In some embodiments, R² is acetylenyl and R^{2a} is fluoro.

In some embodiments, the compound of formula (V) is represented by any one of the following formulae:

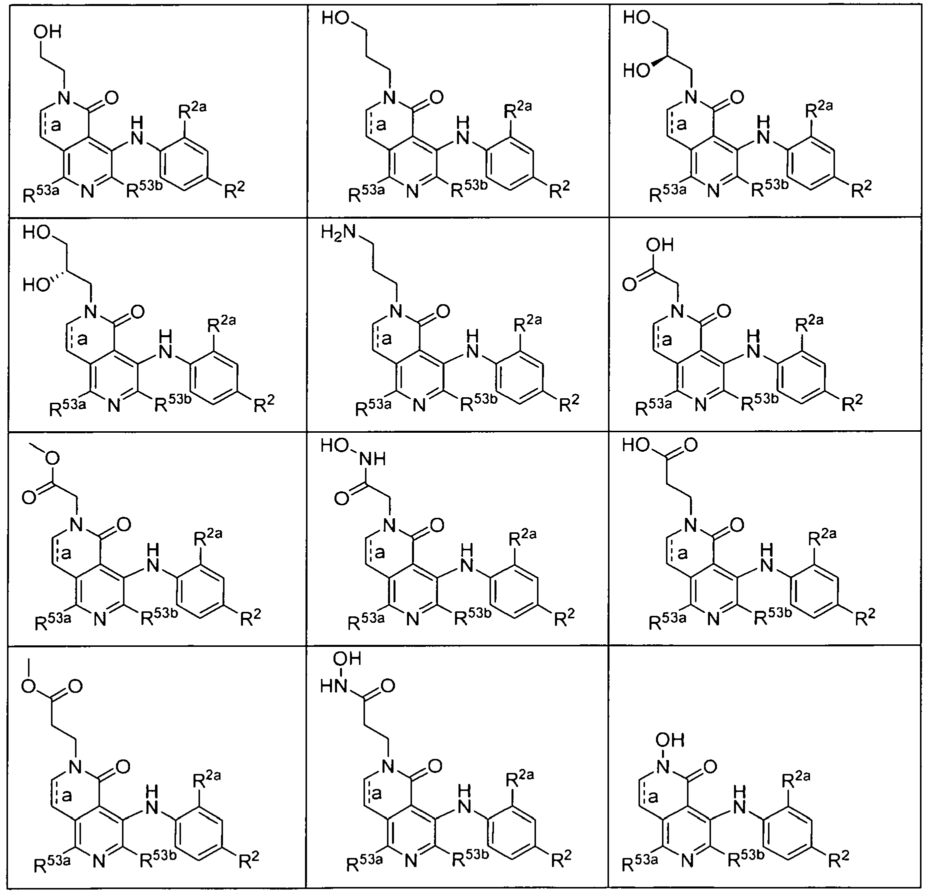

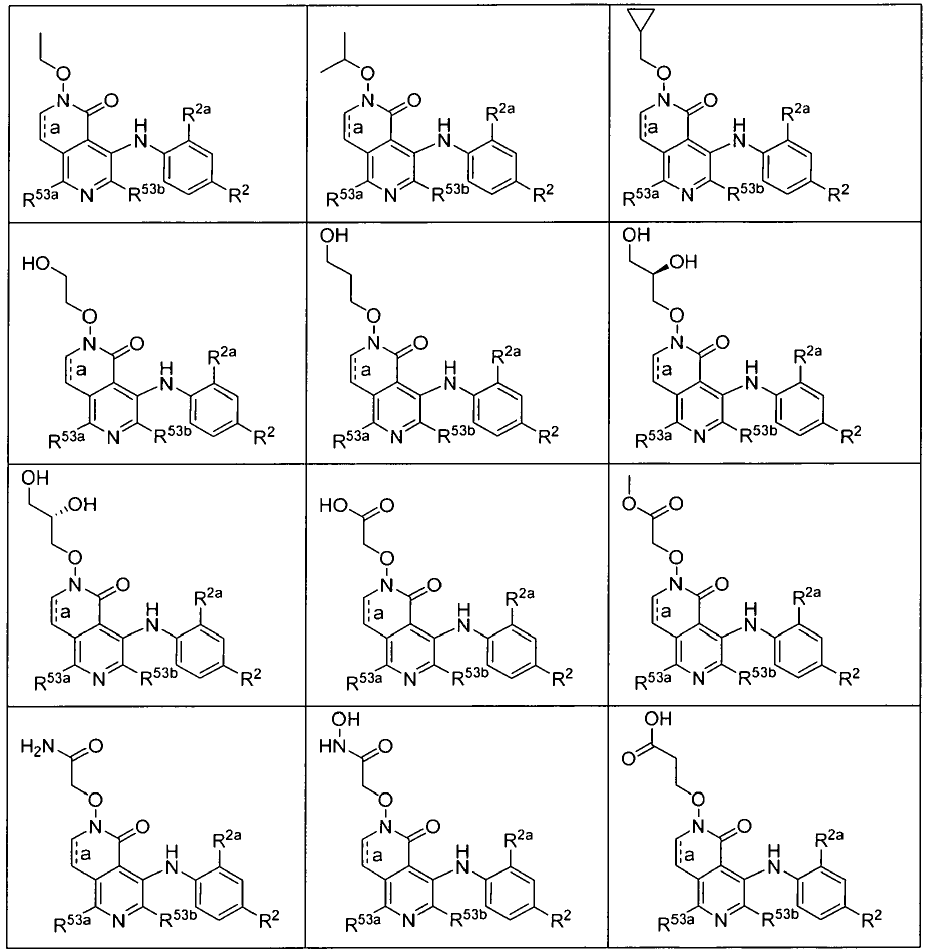

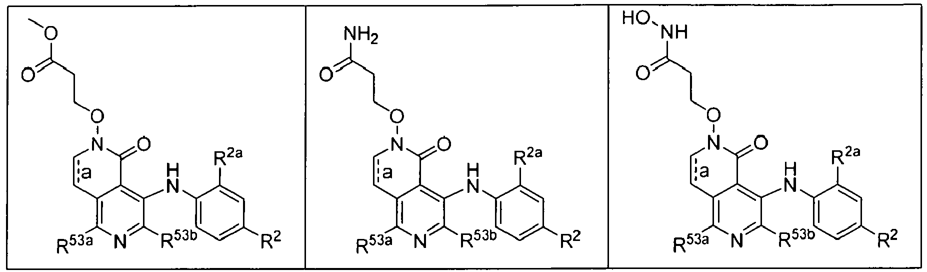

wherein bond "a", R², R^{2a}, R^{53a}, and R^{53b} are as defined herein in any aspect or embodiment described herein.

In some embodiments of the above structures having formula (V), bond "a" is a double bond. In some embodiments of the above structures having formula (V), bond "a" is a single bond.

In some embodiments of the above structures having formula (V), R² is iodo and R^{2a} is fluoro. In some embodiments of the above structures, R² is iodo and R^{2a} is methyl. In some embodiments of the above structures, R² is acetylenyl and R^{2a} is fluoro. In some embodiments of the above structures, R² is acetylenyl and R^{2a} is methyl. In some embodiments of the above structures, R² is -SCH₃ and R^{2a} is fluoro. In some embodiments of the above structures, R² is -SCH₃ and R^{2a} is methyl.

In some embodiments of the above structures having formula (V), R^{53a} and R^{53b} are each hydrogen. In some embodiments of the above structures, R^{53a} is hydrogen and R^{53b} is halo. In some embodiments of the above structures, R^{53a} is hydrogen and R^{53b} is fluoro.

Exemplified compounds having any one of formulae (1), (II), (III), (IV), and (V) are listed in Tables 1-5.

**Table 1: Compounds of formula (1)**

| **No.** | **Structure** | **No.** | **Structure** |
|---|---|---|---|
| **1.001** | 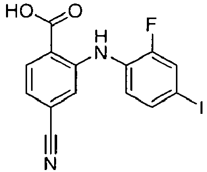 | **1.002** | 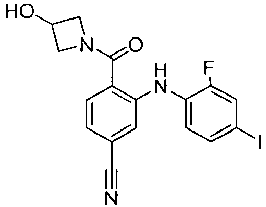 |
| **1.003** | 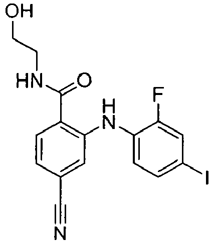 | **1.004** | 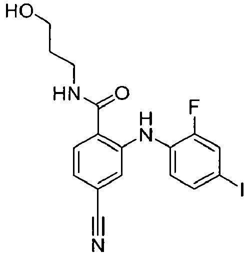 |
| **1.005** | 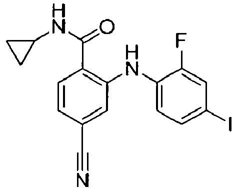 | **1.006** | 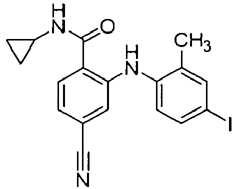 |
| **1.007** | 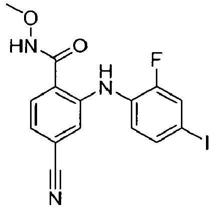 | **1.008** | 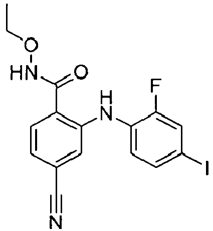 |
| **1.009** | 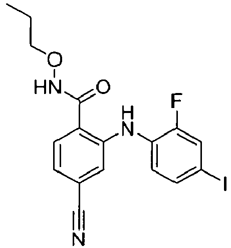 | **1.010** | 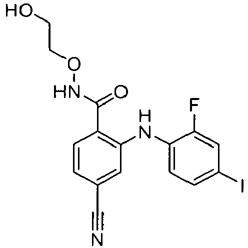 |
| **1.011** | 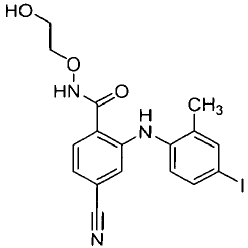 | **1.012** | 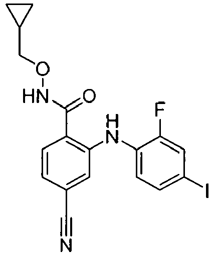 |
| **1.013** | 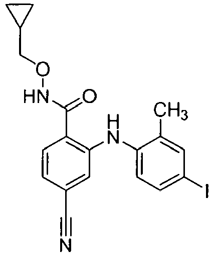 | **1.014** | 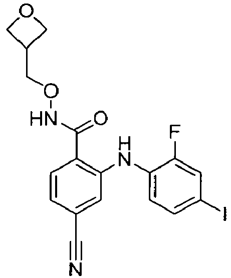 |
| **1.015** | 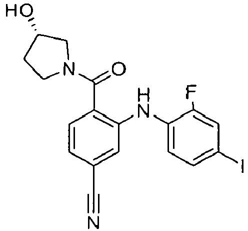 | **1.016** | 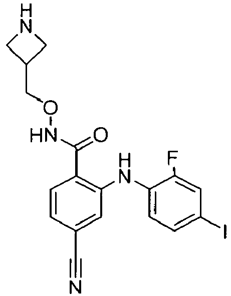 |
| **1.017** | 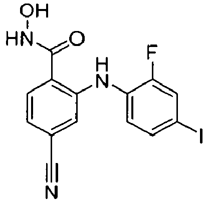 | **1.018** | 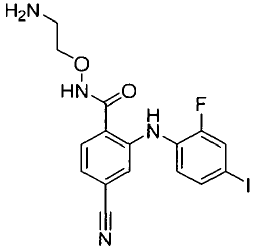 |
| **1.019** | 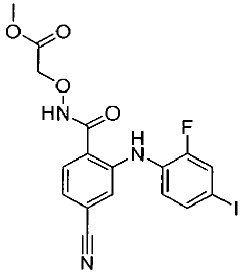 | **1.020** | 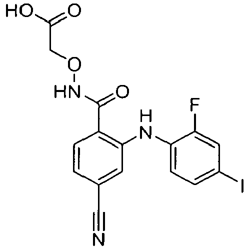 |
| **1.021** | 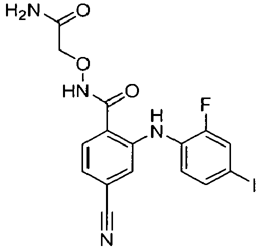 | **1.022** | 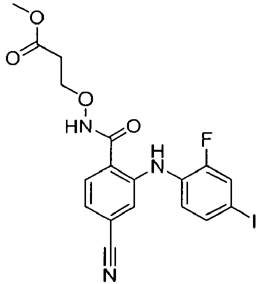 |
| **1.023** | 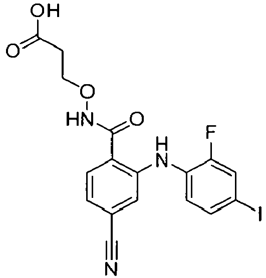 | **1.024** | 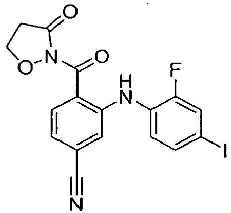 |
| **1.025** | 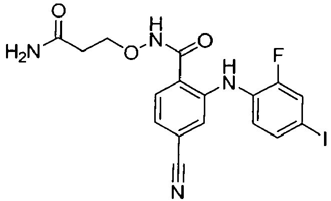 | **1.026** | 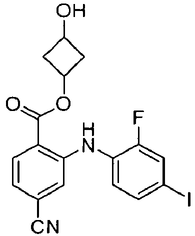 |
| **1.027** | 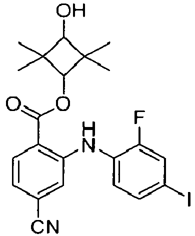 | **1.028** | 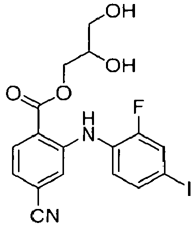 |
| **1.029** | 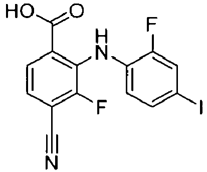 | **1.030** | 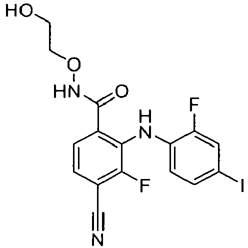 |
| **1.031** | 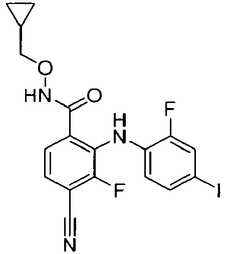 | **1.032** | 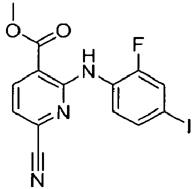 |
| **1.033** | 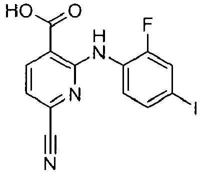 | **1.034** | 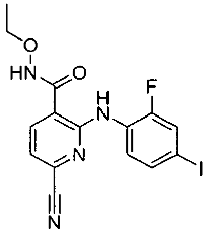 |
| **1.035** | 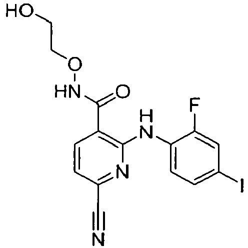 | **1.036** | 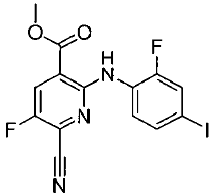 |
| **1.037** | 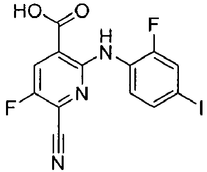 | **1.038** | 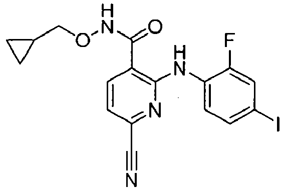 |
| **1.039** | 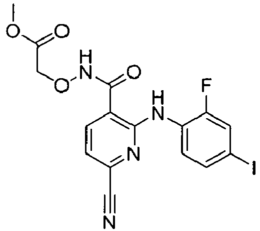 | **1.040** | 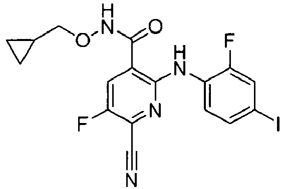 |
| **1.041** | 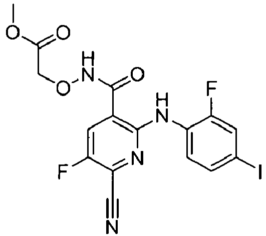 | | |

**Table 2: Compounds of formula (II)**

| **No.** | **Structure** | **No.** | **Structure** |
|---|---|---|---|
| **2.001** | 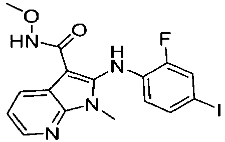 | **2.002** | 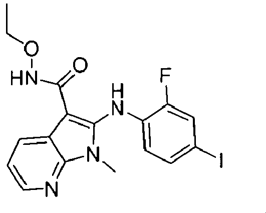 |
| **2.003** | 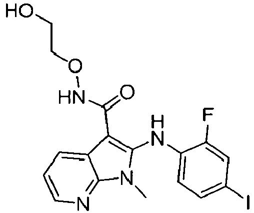 | **2.004** | 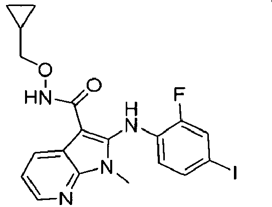 |
| **2.005** | 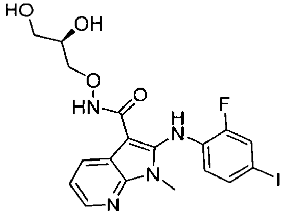 | **2.006** | 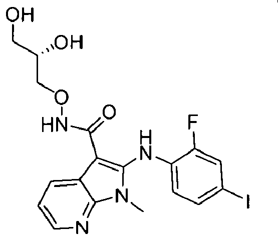 |
| **2.007** | 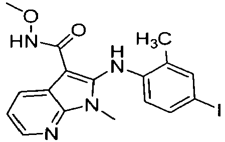 | **2.008** | 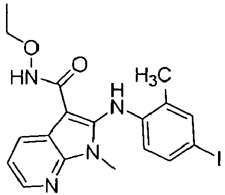 |
| **2.009** | 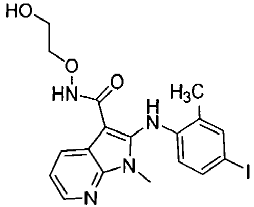 | **2.010** | 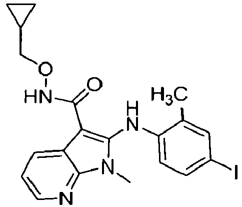 |
| **2.011** | 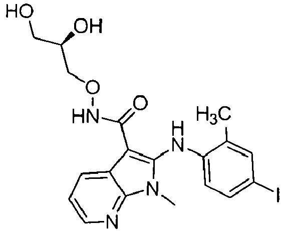 | **2.012** | 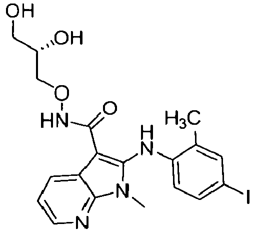 |
| **2.013** | 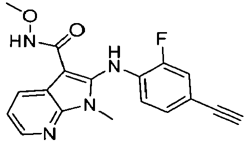 | **2.014** | 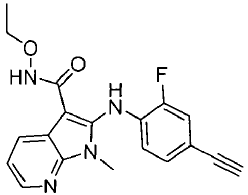 |
| **2.015** | 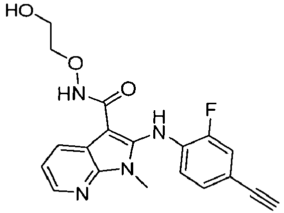 | **2.016** | 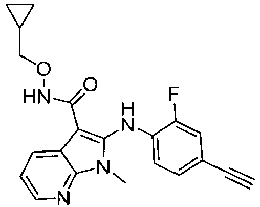 |
| **2.017** | 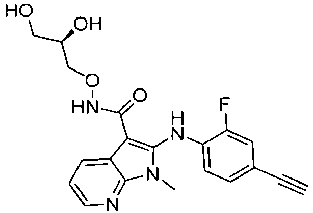 | **2.018** | 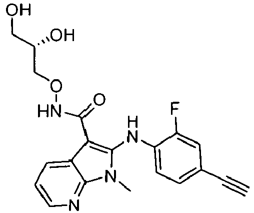 |
| **2.019** | 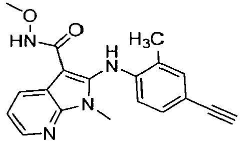 | **2.020** | 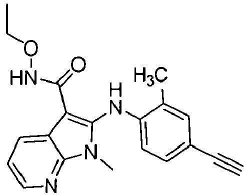 |
| **2.021** | 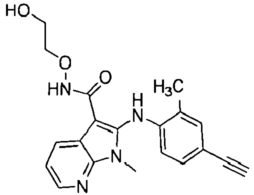 | **2.022** | 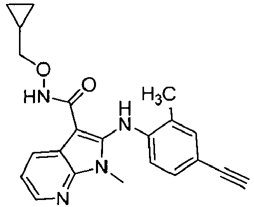 |
| **2.023** | 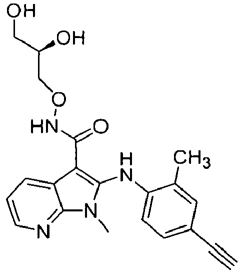 | **2.024** | 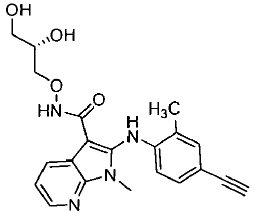 |
| **2.025** | 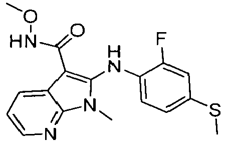 | **2.026** | 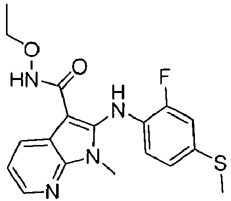 |
| **2.027** | 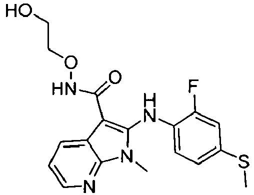 | **2.028** | 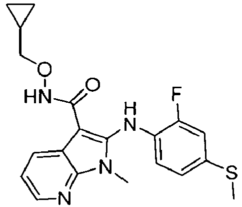 |
| **2.029** | 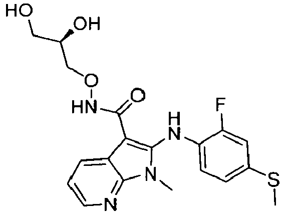 | **2.030** | 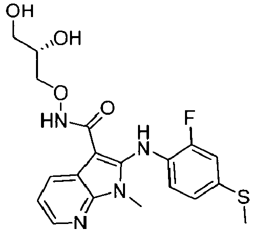 |
| **2.031** | 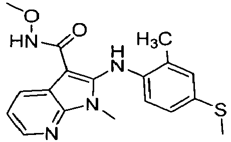 | **2.032** | 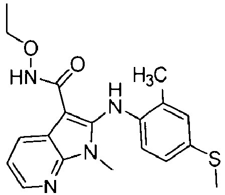 |
| **2.033** | 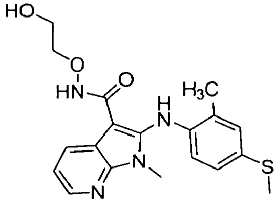 | **2.034** | 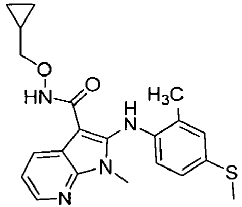 |
| **2.035** | 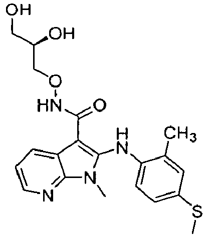 | **2.036** | 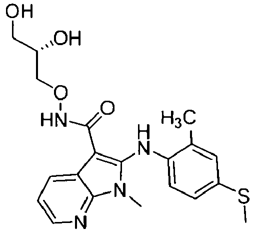 |
| **2.037** | 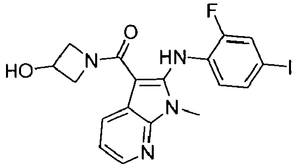 | **2.038** | 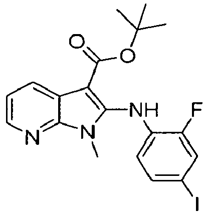 |
| **2.039** | 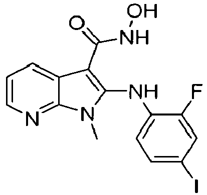 | **2.040** | 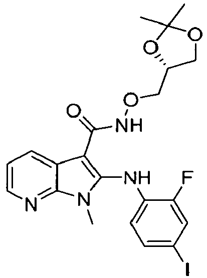 |
| **2.041** | 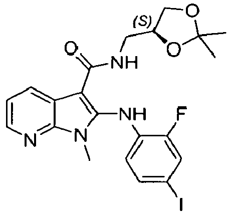 | **2.042** | 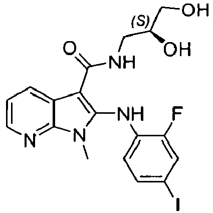 |
| **2.043** | 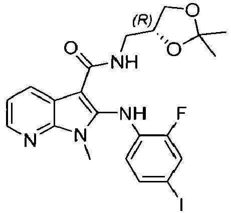 | **2.044** | 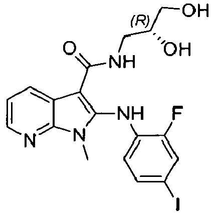 |
| **2.045** | 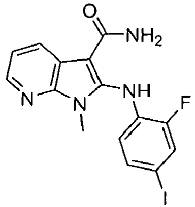 | **2.046** | 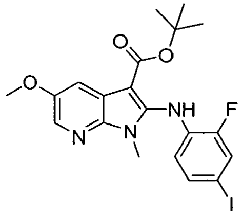 |
| **2.047** | 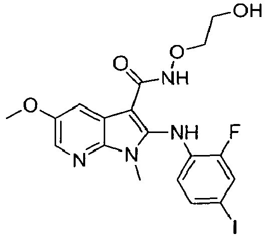 | **2.048** | 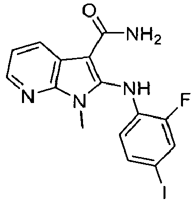 |
| **2.049** | 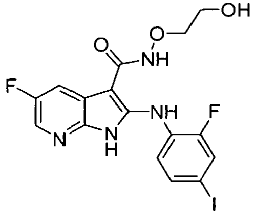 | | |

**Table 3: Compounds of formula (II)**

| **No.** | **Structure** | **No.** | **Structure** |
|---|---|---|---|
| **3.001** | 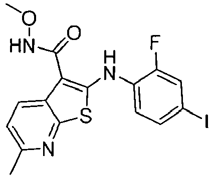 | **3.002** | 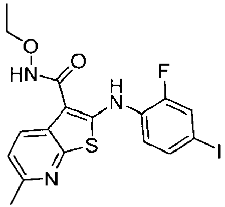 |
| **3.003** | 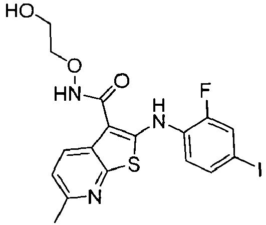 | **3.004** | 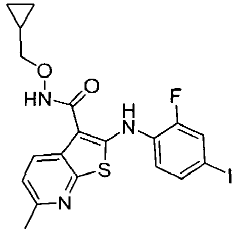 |
| **3.005** | 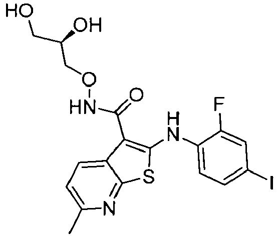 | **3.006** | 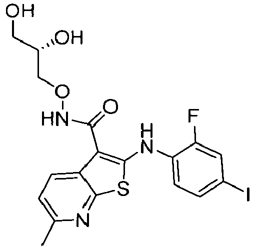 |
| **3.007** | 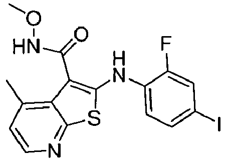 | **3.008** | 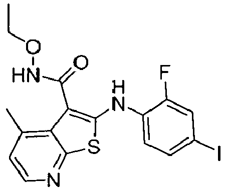 |
| **3.009** | 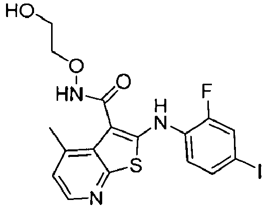 | **3.010** | 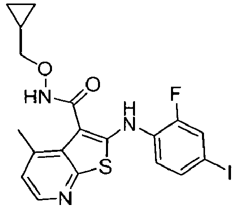 |
| **3.011** | 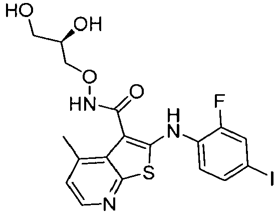 | **3.012** | 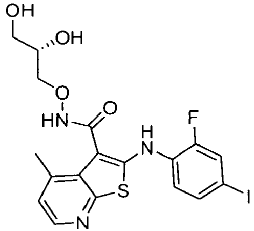 |
| **3.013** | 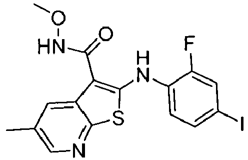 | **3.014** | 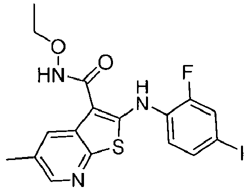 |
| **3.015** | 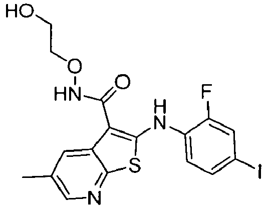 | **3.016** | 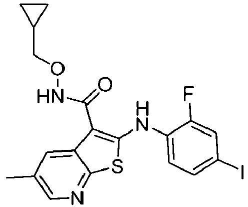 |
| **3.017** | 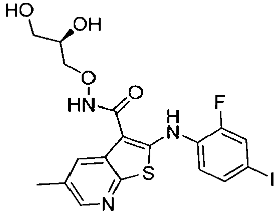 | **3.018** | 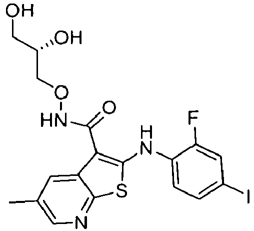 |
| **3.019** | 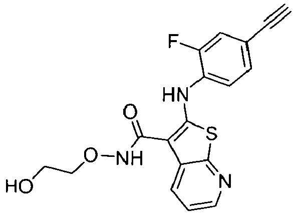 | **3.020** | 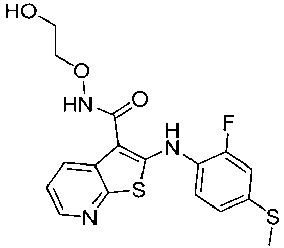 |
| **3.021** | 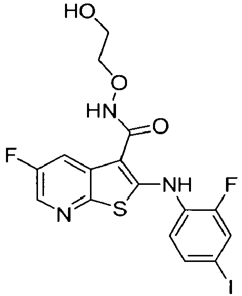 | **3.022** | 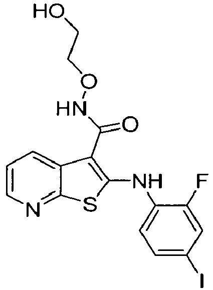 |
| **3.023** | 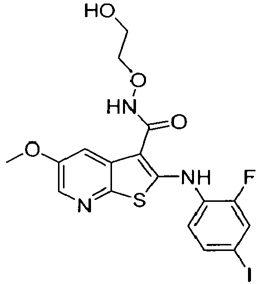 | **3.024** | 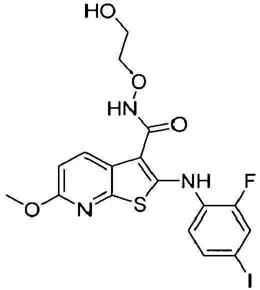 |
| **3.025** | 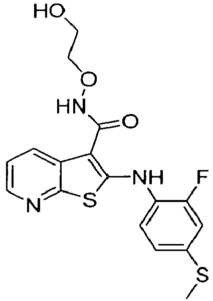 | **3.026** | 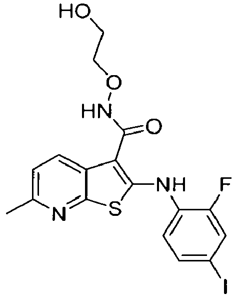 |

**Table 4: Compounds of formula (IV)**

| **No.** | **Structure** | **No.** | **Structure** |
|---|---|---|---|
| **4.001** | 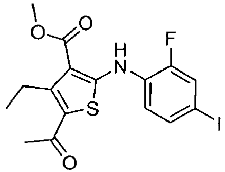 | **4.002** | 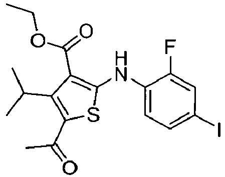 |
| **4.003** | 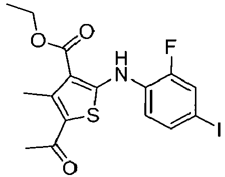 | **4.004** | 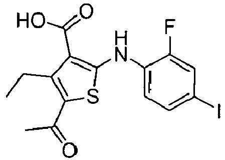 |
| **4.005** | 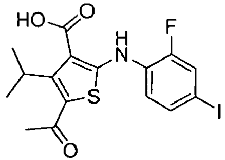 | **4.006** | 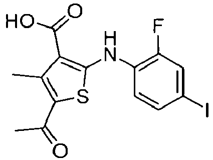 |
| **4.007** | 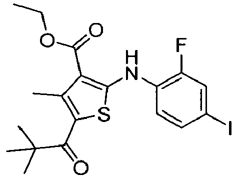 | **4.008** | 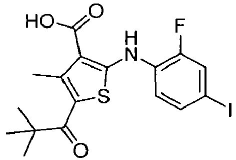 |
| **4.009** | 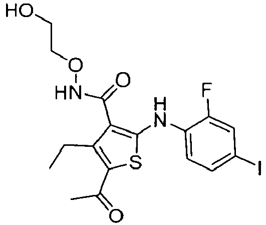 | **4.010** | 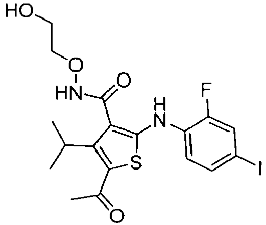 |
| **4.011** | 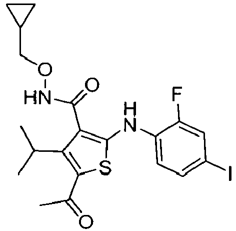 | **4.012** | 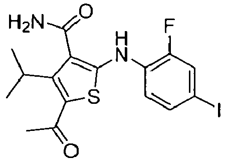 |
| **4.013** | 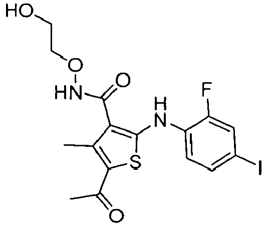 | **4.014** | 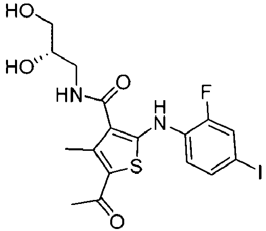 |
| **4.015** | 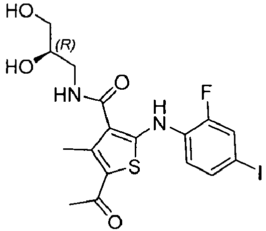 | **4.016** | 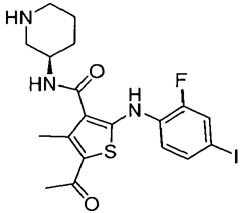 |
| **4.017** | 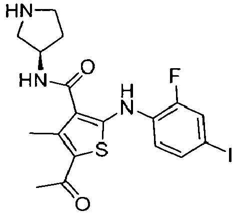 | **4.018** | 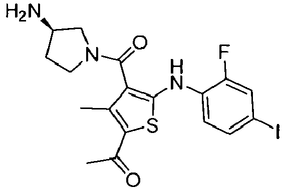 |
| **4.019** | 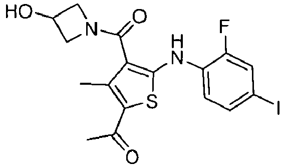 | **4.020** | 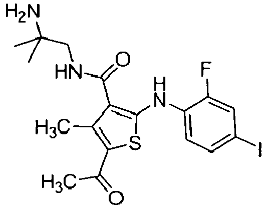 |
| **4.021** | 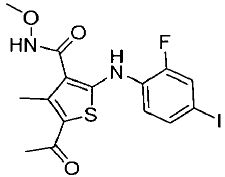 | **4.022** | 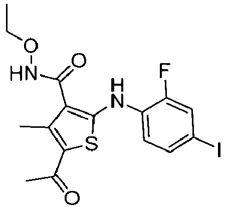 |
| **4.023** | 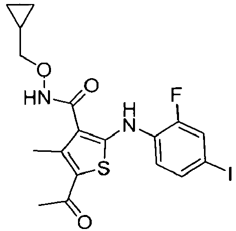 | **4.024** | 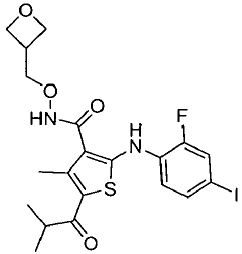 |
| **4.025** | 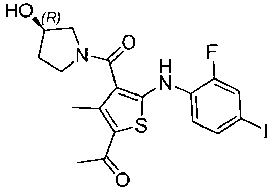 | **4.026** | 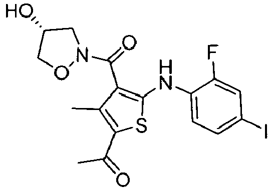 |
| **4.027** | 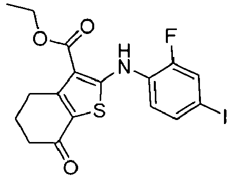 | **4.028** | 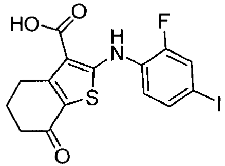 |
| **4.029** | 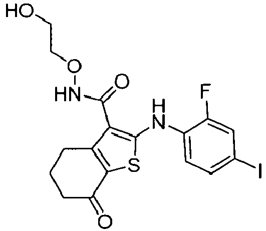 | **4.030** | 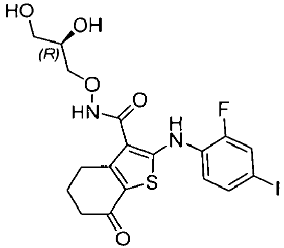 |
| **4.031** | 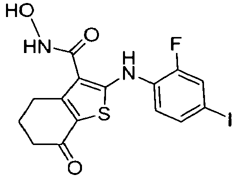 | **4.032** | 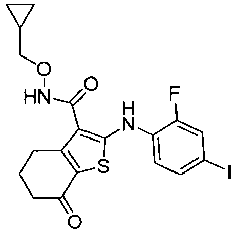 |
| **4.033** | 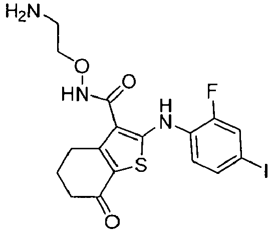 | **4.034** | 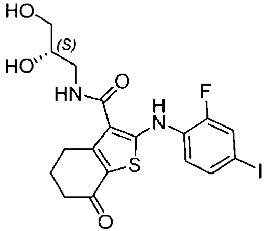 |
| **4.035** | 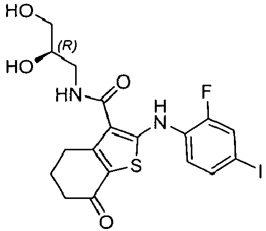 | **4.036** | 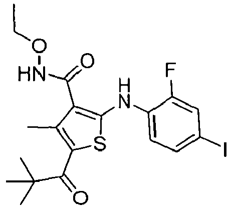 |
| **4.037** | 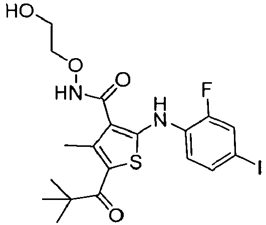 | **4.038** | 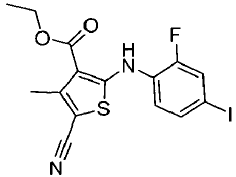 |
| **4.039** | 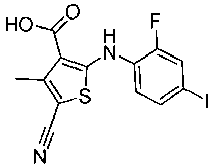 | **4.040** | 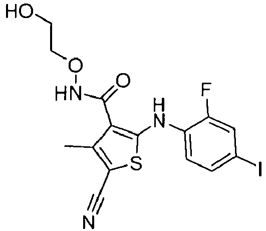 |

**Table 5: Compounds of formula (V)**

| **No.** | **Structure** | **No.** | **Structure** |
|---|---|---|---|
| **5.001** | 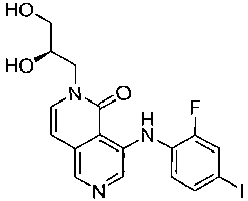 | **5.002** | 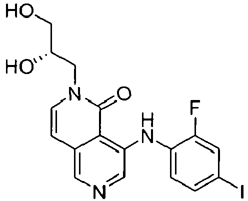 |
| **5.003** | 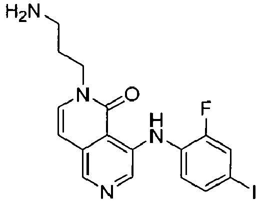 | **5.004** | 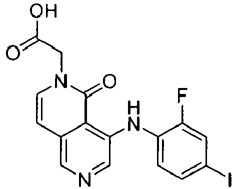 |
| **5.005** | 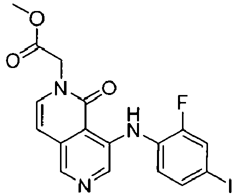 | **5.006** | 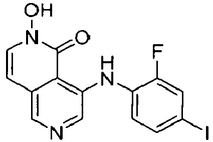 |
| **5.007** | 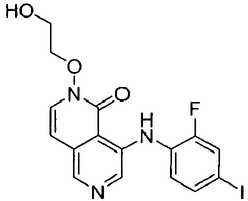 | **5.008** | 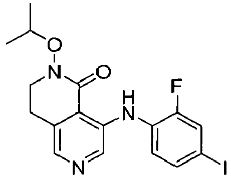 |
| **5.009** | 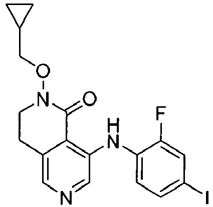 | **5.010** | 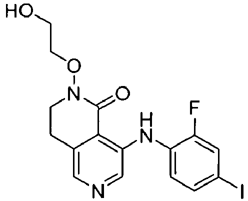 |
| **5.011** | 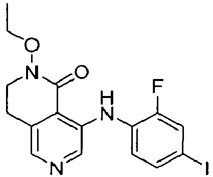 | **5.012** | 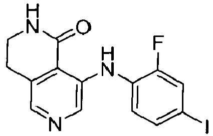 |

The compounds of the present invention may exist as salts. The present invention includes such salts. Examples of applicable salt forms include hydrochlorides, hydrobromides, sulfates, methanesulfonates, nitrates, maleates, acetates, citrates, fumarates, tartrates (eg (+)-tartrates, (-)-tartrates or mixtures thereof including racemic mixtures, succinates, benzoates and salts with amino acids such as glutamic acid. These salts may be prepared by methods known to those skilled in art. Also included are base addition salts such as sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds of the present invention contain both basic and acidic functionalities that allow the compounds to be converted into either base or acid addition salts.

Other salts include acid or base salts of the compounds used in the methods of the present invention. Illustrative examples of pharmaceutically acceptable salts are mineral acid (hydrochloric acid, hydrobromic acid, phosphoric acid, and the like) salts, organic acid (acetic acid, propionic acid, glutamic acid, citric acid and the like) salts, and quaternary ammonium (methyl iodide, ethyl iodide, and the like) salts. It is understood that the pharmaceutically acceptable salts are non-toxic. Additional information on suitable pharmaceutically acceptable salts can be found in Remington's Pharmaceutical Sciences, 17th ed., Mack Publishing Company, Easton, Pa., 1985, which is incorporated herein by reference.

Pharmaceutically acceptable salts includes salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (*see,* for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the enantiomers, racemates, diastereomers, tautomers, geometric isomers, stereoisometric forms that may be defined, in terms of absolute stereochemistry, as (R)- or (S)- or, as (D)- or (L)- for amino acids, and individual isomers are encompassed within the scope of the present invention. The compounds of the present invention do not include those which are known in art to be too unstable to synthesize and/or isolate. The present invention is meant to include compounds in racemic and optically pure forms. Optically active (R)- and (S)-, or (D)- and (L)-isomers may be prepared using chiral synthons or chiral reagents, or resolved using conventional techniques.

Isomers include compounds having the same number and kind of atoms, and hence the same molecular weight, but differing in respect to the structural arrangement or configuration of the atoms.

It will be apparent to one skilled in the art that certain compounds of this invention may exist in tautomeric forms, all such tautomeric forms of the compounds being within the scope of the invention. Tautomer refers to one of two or more structural isomers which exist in equilibrium and which are readily converted from one isomeric form to another.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the invention.

Unless otherwise stated, the compounds of the present invention may also contain unnatural proportions of atomic isotopes at one or more of the atoms that constitute such compounds. For example, the compounds of the present invention may be labeled with radioactive or stable isotopes, such as for example deuterium (²H), tritium (³H), iodine-125 (¹²⁵I), fluorine-18 (¹⁸F), nitrogen-15 (¹⁵N), oxygen-17 (¹⁷O), oxygen-18 (¹⁸O), carbon-13 (¹³C), or carbon-14 (¹⁴C). All isotopic variations of the compounds of the present invention, whether radioactive or not, are encompassed within the scope of the present invention.

In addition to salt forms, the present invention provides compounds, which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

The compounds of the present application are designed for topical, subcutaneous, intradermal, or intralesional application, resulting in inhibition of MEK activity in the dermal and epidermal layers (or in the birthmark) for treatment of a birthmark. After acting to treat the birthmark, in some embodiments, the compound is designed to be metabolically labile in order to limit systemic toxicity after topical, subcutaneous, transdermal, intradermal, of intralesional application by limiting the amount of time the compound remains in the peripheral circulation. The present application provides a solution for the treatment of a birthmark with compounds which demonstrate the ability to penetrate the skin and suppress phospho-ERK.

### V. COMPOSITION

The present invention provides a pharmaceutical composition including the compound having any one of formulae (1) to (V) and a pharmaceutically acceptable carrier for use in the method for treating a birthmark, as defined and described herein.

The compounds provided herein can be formulated into pharmaceutical compositions using methods available in the art and those disclosed herein. Any of the compounds disclosed herein can be provided in the appropriate pharmaceutical composition and be administered by a suitable route of administration.

Administration of the compound described herein to a subject may be local or non-systemic, *e.g.*, topical, subcutaneous, intradermal, or intralesional. In some embodiments, the compound can be administered by topical administration. In some embodiments, the compound can be administered by intradermal administration. In some embodiments, the compound can be administered by intralesional administration, e.g., by intralesional injection.

The methods provided herein encompass administering pharmaceutical compositions containing at least one compound as described herein, including a compound having any one of formulae (1) to (V) if appropriate in a salt form, either used alone or in the form of a combination with one or more compatible and pharmaceutically acceptable carriers, such as diluents or adjuvants, or with another agent for the treatment of a birthmark (e.g., a MEK-inhibitor responsive or MEK-mediated birthmark) where the subject is in need thereof.

In some embodiments, the second agent can be formulated or packaged with the compound provided herein. Of course, the second agent will only be formulated with the compound provided herein when, according to the judgment of those of skill in the art, such co-formulation should not interfere with the activity of either agent or the method of administration. In some embodiments, the compound provided herein and the second agent are formulated separately. They can be packaged together, or packaged separately, for the convenience of the practitioner of skill in the art.

In clinical practice the active agents provided herein may be administered by any conventional route, in particular topically, subcutaneously, intradermally, or intralesionally. In some embodiments, the compound provided herein is administered topically. In some embodiments, the compound provided herein is administered subcutaneously. In some embodiments, the compound provided herein is administered intradermally. In some embodiments, the compound provided herein is administered intralesionally.

Use may be made, as compositions for topical administration, of pastes, lotions, tinctures, emulsions, sprays, ointments, creams or gels. In alternative embodiments, topical administration may be achieved in the form of patches comprising the active agent, where the patch is in contact with the affected area on the skin. In further embodiments, topical administration may be achieved in form of a liquid paint which dries on the skin after application to the affected area. In some of such embodiments, the composition may comprise a skin penetrating agent such that the active agent penetrates the epidermis and is delivered transdermally. In further embodiments, the compositions are formulated for intradermal injection. In alternate embodiments, the compositions are formulated for intralesional injections.

Use may be made, of compositions for topical administration as pastes, lotions, tinctures, emulsions, sprays, ointments, creams or gels. In these compositions, the active product is mixed with one or more inert excipients including water, acetone, ethanol, ethylene glycol, propylene glycol, butane 1,3 diol, isopropyl myristate, isopropyl palmitate, mineral oil, and mixtures thereof. Topical compositions (e.g., gels, creams, ointments, lotions) may comprise pharmaceutically acceptable polymers. Creams and ointments may comprise a paraffin base. For transdermally delivered active agents, the topical compositions may further comprise a skin penetration enhancer (e.g., DMSO, pyrrolidinone). Skin emollients (e.g., glycerine, cocoa butter) may be present in compositions comprising the compounds having any one of formulae (1) to (V) described herein.

The compositions for topical, subcutaneous, intradermal or intralesional, administration can be emulsions or sterile solutions. Use may be made, as solvent or vehicle, of propylene glycol, a polyethylene glycol, vegetable oils, in particular olive oil, or injectable organic esters, In some embodiments, ethyl oleate. These compositions can also contain adjuvants, in particular wetting, isotonizing, emulsifying, dispersing and stabilizing agents. Sterilization can be carried out in several ways, in some embodiments, using a bacteriological filter, by radiation or by heating. They can also be prepared in the form of sterile solid compositions which can be dissolved at the time of use in sterile water or any other injectable sterile medium.

The compositions for rectal administration are suppositories or rectal capsules which contain, in addition to the active principle, excipients such as cocoa butter, semi-synthetic glycerides or polyethylene glycols.

The compositions can also be aerosols and can be sprayed on the affected area. For use in the form of liquid aerosols, the compositions can be stable sterile solutions or solid compositions dissolved at the time of use in apyrogenic sterile water, in saline or any other pharmaceutically acceptable vehicle. For use in the form of dry aerosols intended to be directly inhaled, the active principle is finely divided and combined with a water-soluble solid diluent or vehicle, in some embodiments, dextran, mannitol or lactose.

In some embodiments, a composition provided herein is a pharmaceutical composition or a single unit dosage form. Pharmaceutical compositions and single unit dosage forms provided herein comprise an effective amount, a prophylactically or therapeutically effective amount of one or more prophylactic or therapeutic agents (*e.g.,* a compound provided herein, or other prophylactic or therapeutic agent), and a typically one or more pharmaceutically acceptable carriers or excipients. In some embodiments, the term "pharmaceutically acceptable" means approved by a regulatory agency of the Federal or a state government or listed in the U.S. Pharmacopeia or other generally recognized pharmacopeia for use in animals, and more particularly in humans. The term "carrier" includes a diluent, adjuvant (*e.g.,* Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water can be used as a carrier when the pharmaceutical composition is administered intravenously. Saline solutions and aqueous dextrose and glycerol solutions can also be employed as liquid carriers, particularly for injectable solutions. Examples of suitable pharmaceutical carriers are described in Remington: The Science and Practice of Pharmacy; Pharmaceutical Press; 22 edition (September 15, 2012).

Typical pharmaceutical compositions and dosage forms comprise one or more excipients. Suitable excipients are well-known to those skilled in the art of pharmacy, and in some embodiments, suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a subject and the specific active ingredients in the dosage form. The composition or single unit dosage form, if desired, can also contain minor amounts of wetting or emulsifying agents, or pH buffering agents.

Lactose free compositions provided herein can comprise excipients that are well known in the art and are listed, in some embodiments, in the U.S. Pharmacopeia (USP 36-NF 31 S2). In general, lactose free compositions comprise an active ingredient, a binder/filler, and a lubricant in pharmaceutically compatible and pharmaceutically acceptable amounts. Exemplary lactose free dosage forms comprise an active ingredient, microcrystalline cellulose, pre gelatinized starch, and magnesium stearate.

Further encompassed herein are anhydrous pharmaceutical compositions and dosage forms comprising active ingredients, since water can facilitate the degradation of some compounds. For example, the addition of water (*e.g.,* 5%) is widely accepted in the pharmaceutical arts as a means of simulating long term storage in order to determine characteristics such as shelf life or the stability of formulations over time. See, *e.g.,* Jens T. Carstensen, Drug Stability: Principles & Practice, 2d. Ed., Marcel Dekker, New York, 1995, pp. 379 80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms provided herein can be prepared using anhydrous or low moisture containing ingredients and low moisture or low humidity conditions. Pharmaceutical compositions and dosage forms that comprise lactose and at least one active ingredient that comprises a primary or secondary amine can be anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions can be packaged using materials known to prevent exposure to water such that they can be included in suitable formulary kits. In some embodiments, suitable packaging include, but are not limited to, hermetically sealed foils, plastics, unit dose containers (*e.g.,* vials), blister packs, and strip packs.

Further provided are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions can take the form of solutions, suspensions, emulsion, gels, creams, ointments, or aerosol formulations. In some embodiments, the compositions are impregnated in a suitable matrix to form patches. In some embodiments, the compositions are sustained-release formulations. The formulation should suit the mode of administration (e.g., topical, subcutaneously, intradermal or intralesional administration). In some embodiments, the pharmaceutical compositions or single unit dosage forms are sterile and in suitable form for administration to a subject, in some embodiments, an animal subject, such as a mammalian subject, in some embodiments, a human subject.

A pharmaceutical composition is formulated to be compatible with its intended route of administration. In some embodiments, routes of administration include, but are not limited to, parenteral, *e.g.,* intradermal, subcutaneous, intramuscular, inhalation, intranasal, intrasynovial, rectal, topical, intralesional, and transmucosal administration. In some embodiments, the route of administration is subcutaneous, intradermal, topical, or intralesional administration. In some embodiments, the route of administration is non-systemic administration. In some embodiments, the composition is formulated in accordance with routine procedures as a pharmaceutical composition adapted for subcutaneous or topical administration to human beings. In some embodiments, a pharmaceutical composition is formulated in accordance with routine procedures for intradermal or intralesional administration to human beings. Typically, compositions for injectable administration (e.g., intradermal or subcutaneous injection) are solutions, emulsions, or suspensions in sterile isotonic aqueous buffer. Where necessary, the composition may also include a solubilizing agent and a local anesthetic such as lignocamne to ease pain at the site of the injection.

In some embodiments, dosage forms include, but are not limited to: ointments; cataplasms (poultices); pastes; dressings; creams; plasters; solutions; patches; aerosols (*e.g.,* nasal spray, inhaler, skin spray); gels; liquid dosage forms suitable for dermal administration to a subject, including suspensions (*e.g.,* aqueous or non-aqueous liquid suspensions, oil in water emulsions, or a water in oil liquid emulsions), solutions, and lotions; liquid dosage forms suitable for mucosal administration to a subject, including suspensions (*e.g.,* aqueous or non-aqueous liquid suspensions, oil in water emulsions, or a water in oil liquid emulsions), solutions, and elixirs; liquid dosage forms suitable for parenteral (e.g., subcutaneous or intradermal) administration to a subject; and sterile solids (*e.g.,* crystalline or amorphous solids) that can be reconstituted to provide liquid dosage forms suitable for parenteral administration to a subject.

The composition, shape, and type of dosage forms provided herein will typically vary depending on their use. In some embodiments, a dosage form used in the initial treatment of a birthmark (e.g., a MEK-inhibitor responsive or MEK-mediated birthmark) may contain larger amounts of one or more of the active ingredients it comprises than a dosage form used in the maintenance treatment of the same disorder or disease. Similarly, a parenteral (intradermal) dosage form may contain smaller amounts of one or more of the active ingredients it comprises than a topical form used to treat the same disease or disorder. These and other ways in which specific dosage forms encompassed herein will vary from one another will be readily apparent to those skilled in the art. See, *e.g.,* Remington: The Science and Practice of Pharmacy; Pharmaceutical Press; 22 edition (September 15, 2012).

Generally, the ingredients of compositions are supplied either separately or mixed together in unit dosage form, in some embodiments, as a dry lyophilized powder or water free concentrate in a hermetically sealed container such as an ampoule or sachet indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Typical dosage forms comprise a compound provided herein, or a pharmaceutically acceptable salt, solvate or hydrate thereof lie within the range of from about 0.1 mg to about 1000 mg per day, given as a single once-a-day dose in the morning or as divided doses throughout the day taken with food. Particular dosage forms can have about 0.1, 0.2, 0.3, 0.4, 0.5, 1.0, 2.0, 2.5, 5.0, 10.0, 15.0, 20.0, 25.0, 50.0, 100, 200, 250, 500 or 1000 mg of the active compound.

In some instances the compositions described herein comprise lubricants. Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, in some embodiments, a syloid silica gel (AEROSIL 200, manufactured by W.R. Grace Co. of Baltimore, MD), a coagulated aerosol of synthetic silica (marketed by Degussa Co. of Plano, TX), CAB O SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### Delayed Release Dosage Forms

Active ingredients such as the compounds provided herein can be administered by controlled release means or by delivery devices that are well known to those of ordinary skill in the art. In some embodiments, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500; each of which is incorporated herein by reference in its entirety. Such dosage forms can be used to provide slow or controlled release of one or more active ingredients using, in some embodiments, hydroxypropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the active ingredients provided herein and can be incorporated for example in bandages or patches. Thus encompassed herein are single unit dosage forms suitable for dermal administration such as, but not limited to, gels, ointments, patches, creams, lesion dressings, and the like, that are adapted for controlled release.

All controlled release pharmaceutical products have a common goal of improving drug therapy over that achieved by their non-controlled counterparts. Ideally, the use of an optimally designed controlled release preparation in medical treatment is characterized by a minimum of drug substance being employed to cure or control the disease or disorder in a minimum amount of time. Advantages of controlled release formulations include extended activity of the drug, reduced dosage frequency, and increased subject compliance. In addition, controlled release formulations can be used to affect the time of onset of action or other characteristics, such as blood levels of the drug, and can thus affect the occurrence of one or more side (*e.g.,* adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various physiological factors including, but not limited to, pH, temperature, enzymes, water, or other physiological factors associated with birthmarks or compounds.

In some embodiments, the drug may be administered using a transdermal patch, liposomes, or other modes of administration. In some embodiments, polymeric materials can be used. In some embodiments, a controlled release system can be placed in a subject at an appropriate site determined by a practitioner of skill, *i.e.,* thus requiring only a fraction of the systemic dose (*see, e.g.,* Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, *e.g.,* polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinyl alcohol and cross-linked partially hydrolyzed polyvinyl acetate, that is surrounded by an outer polymeric membrane, *e.g.,* polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral (e.g., intradermal) compositions is highly dependent on the specific nature thereof, as well as the needs of the subject.

### Parenteral (Intramuscular, Subcuraneous, Intralesional, Intradermal) Dosage Forms

In some embodiments, provided are parenteral dosage forms. In some embodiments, parenteral dosage forms can be administered to subjects by various routes including, but not limited to, subcutaneous, intramuscular, and intra-dermal. In some embodiments, parenteral dosage forms can be administered to subjects by various routes including, but not limited to, subcutaneous, intramuscular, intradermal, or intralesional. Because their administration typically bypasses subjects' natural defenses against contaminants, parenteral dosage forms are typically, sterile or capable of being sterilized prior to administration to a subject. In some embodiments, parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. In some embodiments, suitable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms.

### Topical & Mucosal Dosage Forms

Also provided are topical, and mucosal dosage forms. Transdermal, mucosal, and topical dosage forms include, but are not limited to, pastes, sprays, aerosols, creams, lotions, ointments, gels, solutions, emulsions, suspensions, or other forms known to one of skill in the art. See, *e.g.,* Remington: The Science and Practice of Pharmacy; Pharmaceutical Press; 22 edition (September 15, 2012); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

The term "pharmaceutically acceptable carrier" refers to a pharmaceutically-acceptable material, composition or vehicle, such as a liquid or solid filler, diluent, excipient, solvent or encapsulating material, involved in carrying or transporting any subject composition or component thereof. Each carrier must be "acceptable" in the sense of being compatible with the subject composition and its components and not injurious to the patient. Suitable carriers (*e.g.,* excipients and diluents) and other materials that can be used to provide transdermal, topical, and, in some embodiments, mucosal dosage forms encompassed herein are well known to those skilled in the pharmaceutical arts, and depend on the particular tissue to which a given pharmaceutical composition or dosage form will be applied. With that fact in mind, typical carriers include, but are not limited to, water, acetone, ethanol, ethylene glycol, propylene glycol, butane 1,3 diol, isopropyl myristate, isopropyl palmitate, mineral oil, and mixtures thereof to form lotions, tinctures, creams, emulsions, gels or ointments, which are nontoxic and pharmaceutically acceptable. In some embodiments, materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. Moisturizers or humectants can also be added to pharmaceutical compositions and dosage forms if desired. Examples of such additional ingredients are well known in the art. See, *e.g.,* Remington: The Science and Practice of Pharmacy; Pharmaceutical Press; 22 edition (September 15, 2012).

Depending on the specific tissue to be treated, additional components may be used prior to, in conjunction with, or subsequent to treatment with active ingredients provided. In some embodiments, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery enhancing or penetration enhancing agent. Different salts, hydrates or solvates of the active ingredients can be used to further adjust the properties of the resulting composition.

### Dosage and Unit Dosage Forms

In human therapeutics, a doctor will determine the posology which he considers most appropriate according to a preventive or curative treatment and according to the age, weight, stage of the disorder or disease and other factors specific to the subject to be treated. In some embodiments, doses are from about 1 to about 1000 mg per day for an adult, or from about 5 to about 250 mg per day or from about 10 to 50 mg per day for an adult. In some embodiments, doses are from about 5 to about 400 mg per day or 25 to 200 mg per day per adult. In some embodiments, dose rates of from about 50 to about 500 mg per day are also contemplated.

In further aspects, provided are methods of treating birthmarks (e.g., MEK-inhibitor responsive or MEK-mediated birthmarks) in a subject by administering, to a subject in need thereof, a therapeutically effective amount or an effective amount of a compound provided herein, or a pharmaceutically acceptable salt thereof. The amount of the compound or composition which will be therapeutically effective or effective in the treatment of a birthmark or one or more symptoms thereof will vary with the nature and severity of the disease or condition, and the route by which the active ingredient is administered. The frequency and dosage will also vary according to factors specific for each subject depending on the specific therapy (*e.g.,* therapeutic or prophylactic agents) administered, the severity of the disorder, disease, or condition, the route of administration, as well as age, body, weight, response, and the past medical history of the subject. Effective doses may be extrapolated from dose-response curves derived from *in vitro* or animal model test systems.

In some embodiments, exemplary doses of a composition include milligram or microgram amounts of the active compound per kilogram of subject or sample weight (*e.g.,* about 10 micrograms per kilogram to about 50 milligrams per kilogram, about 100 micrograms per kilogram to about 25 milligrams per kilogram, or about 100 microgram per kilogram to about 10 milligrams per kilogram). For compositions provided herein, in some embodiments, the dosage administered to a subject is 0.140 mg/kg to 3 mg/kg of the subject's body weight, based on weight of the active compound. In some embodiments, the dosage administered to a subject is between 0.20 mg/kg and 2.00 mg/kg, between 0.30 mg/kg and 1.50 mg/kg between 1 mg/kg and 100 mg/kg, between 5 mg/kg and 50 mg/kg, between 10 mg/kg and 50 mg/kg, between 20 mg/kg and 50 mg/kg, between 15 mg/kg and 40 mg/kg, between 15 mg/kg and 35 mg/kg, between 15 mg/kg and 30 mg/kg, between 25 mg/kg and 35 mg/kg, between 10 mg/kg and 30 mg/kg, between 10 mg/kg and 20 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, or about 50 mg/kg of the subject's body weight.

In some embodiments, the recommended daily dose range of a composition provided herein for the diseases or disorders described herein lie within the range of from about 0.1 mg to about 1000 mg per day, given as a single once-a-day dose or as divided doses throughout a day. In some embodiments, the daily dose is administered twice daily in equally divided doses. In some embodiments, a daily dose range should be from about 10 mg to about 200 mg per day, in some embodiments, between about 10 mg and about 150 mg per day, in further embodiments, between about 25 and about 100 mg per day. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with subject response.

Different therapeutically effective amounts or an effective amounts may be applicable for different diseases and conditions, as will be readily known by those of ordinary skill in the art. Similarly, amounts sufficient to prevent, manage, treat or ameliorate such disorders, but insufficient to cause, or sufficient to reduce, adverse effects associated with the composition provided herein are also encompassed by the herein described dosage amounts and dose frequency schedules. Further, when a subject is administered multiple dosages of a composition provided herein, not all of the dosages need be the same. In some embodiments, the dosage administered to the subject may be increased to improve the prophylactic or therapeutic effect of the composition or it may be decreased to reduce one or more side effects that a particular subject is experiencing.

In some embodiments, the dosage of the composition provided herein, based on weight of the active compound, administered to prevent, treat, manage, or ameliorate a disorder, or one or more symptoms thereof in a subject is 0.1 mg/kg, 1 mg/kg, 2 mg/kg, 3 mg/kg, 4 mg/kg, 5 mg/kg, 6 mg/kg, 10 mg/kg, or 15 mg/kg or more of a subject's body weight. In some embodiments, the dosage of the composition or a composition provided herein administered to prevent, treat, manage, or ameliorate a disorder, or one or more symptoms thereof in a subject is a unit dose of 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

In some embodiments, treatment or prevention can be initiated with one or more loading doses of a compound or composition provided herein followed by one or more maintenance doses. In such embodiments, the loading dose can be, for instance, about 60 to about 400 mg per day, or about 100 to about 200 mg per day for one day to five weeks. The loading dose can be followed by one or more maintenance doses. In some embodiments, each maintenance does is, independently, about from about 10 mg to about 200 mg per day, between about 25 mg and about 150 mg per day, or between about 25 and about 80 mg per day. Maintenance doses can be administered daily and can be administered as single doses, or as divided doses.

In some embodiments, a dose of a compound or composition provided herein can be administered to achieve a steady-state concentration of the active ingredient in blood or serum of the subject. The steady-state concentration can be determined by measurement according to techniques available to those of skill or can be based on the physical characteristics of the subject such as height, weight and age. In some embodiments, a sufficient amount of a compound or composition provided herein is administered to achieve a steady-state concentration in blood or serum of the subject of from about 300 to about 4000 ng/mL, from about 400 to about 1600 ng/mL, or from about 600 to about 1200 ng/mL. In some embodiments, loading doses can be administered to achieve steady-state blood or serum concentrations of about 1200 to about 8000 ng/mL, or about 2000 to about 4000 ng/mL for one to five days. In some embodiments, maintenance doses can be administered to achieve a steady-state concentration in blood or serum of the subject of from about 300 to about 4000 ng/mL, from about 400 to about 1600 ng/mL, or from about 600 to about 1200 ng/mL.

In some embodiments, administration of the same composition may be repeated and the administrations may be separated by at least 1 day, 2 days, 3 days, 5 days, 10 days, 15 days, 30 days, 45 days, 2 months, 75 days, 3 months, or 6 months. In some embodiments, administration of the same prophylactic or therapeutic agent may be repeated and the administration may be separated by at least at least 1 day, 2 days, 3 days, 5 days, 10 days, 15 days, 30 days, 45 days, 2 months, 75 days, 3 months, or 6 months.

In certain aspects, provided herein are unit dosages comprising a compound, or a pharmaceutically acceptable salt thereof, in a form suitable for administration. Such forms are described in detail herein. In some embodiments, the unit dosage comprises 1 to 1000 mg, 5 to 250 mg or 10 to 50 mg active ingredient. In particular embodiments, the unit dosages comprise about 1, 5, 10, 25, 50, 100, 125, 250, 500 or 1000 mg active ingredient. Such unit dosages can be prepared according to techniques familiar to those of skill in the art.

The dosage may vary within a range depending upon the dosage form employed and the route of administration utilized. For any compound, the therapeutically effective dose or an effective dose can be estimated initially from cell culture assays. A dose may be formulated in animal models to achieve a level in the skin with the birthmark, *e.g.,* any birthmark described herein, that includes the IC₅₀ (i.e., the concentration of the test compound that achieves a half-maximal inhibition of symptoms) as determined in cell culture. Such information can be used to more accurately determine useful doses in humans. In addition, levels in plasma may be measured, for example, by high performance liquid chromatography, in order to ascertain systemic exposure.

It should also be understood that a specific dosage and treatment regimen for any particular patient will depend upon a variety of factors, including the activity of the specific compound employed, the age, the size of the lesion, number of lesions, general health, sex, diet, time of administration, drug combination, and the judgment of the treating physician and the severity of the particular disease being treated. The amount of a soft MEK inhibitor, e.g., a soft MEK inhibitor described herein, in the composition will also depend upon the particular soft MEK inhibitor in the composition.

In some embodiments, the topical, subcutaneous, intradermal, or intralesional dose is about 0.01 µg/cm², about 0.05 µg/cm², about 0.1 µg/cm², about 0.15 µg/cm², about 0.2 µg/cm², about 0.3 µg/cm², about 0.4 µg/ cm², about 0.5 µg/cm², about 0.6 µg/cm², about 0.7 µg/cm², about 0.8 µg/cm², or about 0.9 µg/cm²; or is within about 0.01-0.03 µg/cm², about 0.03-0.05 µg/cm², about 0.05-0.1 µg/cm², about 0.1-0.3 µg/cm², about 0.3-0.5 µg/cm², about 0.5-0.8 µg/cm², about 0.8-1.0 µg/cm², about 1-10 µg/cm², about 10-20 µg/cm², about 20-30 µg/cm², about 30-40 µg/cm², about 40-50 µg/cm², about 50-60 µg/cm², about 60-70 µg/cm², about 70-80 µg/cm², about 80-90 µg/cm², about 90-100 µg/cm², about 100-125 µg/cm², about 125-150 µg/cm², about 150-175 µg/cm², about 175-200 µg/cm², about 200-250 µg/cm², about 250-300 µg/cm², about 300-350 µg/cm², about 350-400 µg/cm², about 400-450 µg/cm², about 450-500 µg/cm², about 500-550 µg/cm², about 550-600 µg/cm², about 600-650 µg/cm², about 650-700 µg/cm², about 700-750 µg/cm², about 750-800 µg/cm², about 800-850 µg/cm², about 850-900 µg/cm², about 900-950 µg/cm², or about 950-1000 µg/cm².

In some embodiments, the topical, subcutaneous, intradermal, or intralesional dose is within about 0.5-1.0 mg/cm², 1.0-1.5 mg/cm², 1.5-2.0 mg/cm², 2.5-2.5 mg/cm², 3.0-3.5 mg/cm², 3.5-5.0 mg/cm², 5.0-7.5 mg/cm², 7.5-10 mg/cm², 1-10 mg/cm², about 10-20 mg/cm², about 20-30 mg/cm², about 30-40 mg/cm², about 40-50 mg/cm², about 50-60 mg/cm², about 60-70 mg/cm², about 70-80 mg/cm², about 80-90 mg/cm², about 90-100 mg/cm², about 100-125 mg/cm², about 125-150 mg/cm², about 150-175 mg/cm², about 175-200 mg/cm², about 200-250 mg/cm², about 250-300 mg/cm², about 300-350 mg/cm², about 350-400 mg/cm², about 400-450 mg/cm², about 450-500 mg/cm², about 500-550 mg/cm², about 550-600 mg/cm², about 600-650 mg/cm², about 650-700 mg/cm², about 700-750 mg/cm², about 750-800 mg/cm², about 800-850 mg/cm², about 850-900 mg/cm², about 900-950 mg/cm², or about 950-1000 mg/cm².

### VI. KITS

Also provided are kits for use in methods of treatment of a birthmark (e.g., a MEK-inhibitor responsive or MEK-mediated birthmark), where the subject is in need thereof. The kits can include a compound or composition provided herein, a second agent or composition, and instructions providing information to a health care provider regarding usage for treating a birthmark (e.g., a MEK-inhibitor responsive or MEK-mediated birthmark). Instructions may be provided in printed form or in the form of an electronic medium such as a floppy disc, CD, or DVD, or in the form of a website address where such instructions may be obtained. A unit dose of a compound or composition provided herein, or a second agent or composition, can include a dosage such that when administered to a subject, a therapeutically effective plasma level of the compound or composition can be maintained in the subject for at least 1 day. In some embodiments, a compound or composition can be included as a sterile aqueous pharmaceutical composition or dry powder (*e.g.,* lyophilized) composition.

In some embodiments, suitable packaging is provided. As used herein, "packaging" includes a solid matrix or material customarily used in a system and capable of holding within fixed limits a compound provided herein and/or a second agent suitable for administration to a subject. Such materials include glass and plastic (*e.g.,* polyethylene, polypropylene, and polycarbonate) bottles, vials, paper, plastic, and plastic-foil laminated envelopes and the like. If e-beam sterilization techniques are employed, the packaging should have sufficiently low density to permit sterilization of the contents.

### VII. COMBINATION THERAPIES

In some embodiments, the compounds and compositions provided herein are useful in methods of treatment of a birthmark (e.g., a MEK-inhibitor responsive or MEK-mediated birthmark) where the subject is in need thereof, that comprise further administration of a second agent effective for the treatment of a birthmark. The second agent can be any agent known to those of skill in the art to be effective for the treatment of dermal disorders or diseases, including those currently approved by the United States Food and Drug Administration, or other similar body of a country foreign to the United States.

In some embodiments, a compound provided herein is administered in combination with one second agent. In further embodiments, a compound provided herein is administered in combination with two second agents. In still further embodiments, a compound provided herein is administered in combination with two or more second agents.

In some embodiments, the methods encompass the step of administering (e.g., topically, subcutaneously, intradermally or intralesionally) to the subject in need thereof an amount of a compound effective for the treatment of a birthmark (e.g., a MEK-inhibitor responsive or MEK-mediated birthmark) where the subject is in need thereof in combination with a second agent effective for the treatment or prevention of birthmarks (e.g., MEK-inhibitor responsive or MEK-mediated birthmarks) where the subject is in need thereof. The compound can be any compound as described herein, and the second agent can be any second agent described in the art or herein. In some embodiments, the compound is in the form of a pharmaceutical composition or dosage form, as described elsewhere herein.

As used herein, the term "in combination" includes the use of more than one therapy (e.*g*., one or more prophylactic and/or therapeutic agents). The use of the term "in combination" does not restrict the order in which therapies (*e.g.,* prophylactic and/or therapeutic agents) are administered to a subject with a disorder. A first therapy (*e.g.,* a prophylactic or therapeutic agent such as a compound provided herein) can be administered prior to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g.,* a prophylactic or therapeutic agent) to a subject with a disorder.

As used herein, the term "synergistic" includes a combination of a compound provided herein and another therapy (*e.g.,* a prophylactic or therapeutic agent) which has been or is currently being used to prevent, manage or treat a disorder, which is more effective than the additive effects of the therapies. A synergistic effect of a combination of therapies (*e.g.,* a combination of prophylactic or therapeutic agents) permits the use of lower dosages of one or more of the therapies and/or less frequent administration of said therapies to a subject with a disorder. The ability to utilize lower dosages of a therapy (*e.g.,* a prophylactic or therapeutic agent) and/or to administer said therapy less frequently reduces the toxicity associated with the administration of said therapy to a subject without reducing the efficacy of said therapy in the prevention or treatment of a disorder). In addition, a synergistic effect can result in improved efficacy of agents in the prevention or treatment of a disorder. Finally, a synergistic effect of a combination of therapies (*e.g.,* a combination of prophylactic or therapeutic agents) may avoid or reduce one or more adverse or unwanted side effects associated with the use of either therapy alone.

The active compounds provided herein can be administered in combination or alternation with another therapeutic agent, in particular an agent effective in the treatment of a birthmark (e.g., a MEK-inhibitor responsive or MEK-mediated birthmark) where the subject is in need thereof. In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the birthmark to be alleviated. It is to be further understood that for any particular subject, specific dosage regimens and schedules should be adjusted over time according to the individual need and the professional judgment of the person administering or supervising the administration of the compositions.

In some embodiments, dosages of the second agents to be used in a combination therapy are provided herein. In some embodiments, dosages lower than those which have been or are currently being used to treat MEK-inhibitor responsive or MEK-mediated skin conditions are used in the combination therapies provided herein. The recommended dosages of second agents can be obtained from the knowledge of those of skill in the art. For those second agents that are approved for clinical use, recommended dosages are described in, for example, Hardman et al., eds., 1996, Goodman & Gilman's The Pharmacological Basis Of Therapeutics 9th Ed, McGraw-Hill, New York; Physician's Desk Reference (PDR) 57th Ed., 2003, Medical Economics Co., Inc., Montvale, NJ; which are incorporated herein by reference in their entirety.

The disclosure provides combination treatments by administration of a MEK inhibitor, e.g. a soft MEK inhibitor, described herein with one or more additional agent(s). In some embodiments, the one or more additional agent(s) is selected from:
agents that treat acne (*e.g.,* Accutane, Azelaic acid, Benzoyl Peroxide, Salicylic acid);
analgesics (*e.g.,* Acetaminophen, Capsaicin), *e.g.,* a Cox2 Inhibitor, *e.g.* Celecoxib);
anesthetics (*e.g.,* Benzocaine, Benzocaine/Menthol, Dibucaine, Diperodon, Lidocaine, Lidocaine/ Prilocaine, Pramoxine);
anti-infectives (*e.g.,* Crotamiton);
anti-prurittus (*e.g.,* Ammonium lactate, Benzocaine, an ascomycin macrolactam, *e.g.,* Pimecrolimus);
anti-prurittus/SHT3 receptor antagonists (*e.g*., Ondansetron);
antibiotics (*e.g.*, clindamycin, doxycycline, erythromycin, tetracycline);
anticholinergic antiemetics (*e.g.*, diphenhydramine);
antifibrotics (*e.g*., Collagenase, Pirfenidone);
antihistamines (*e.g*., Triprolidine (Actifed^{®}), Fexofenadine (Allergra^{®}, Allegra^{®} D-12, Allegra^{®}-24), Astepro/Astelin Nasal Spray (Azalastine) (Dymista^{®}), Hydroxyzine hydrochloride (Atarax^{®}), Diphenhydramine Hydrochloride (Benadryl^{®}), Brompheniramine (Dimetapp^{®} Cold and Allergy Elixir), Zyrtec^{®} (Cetirizine), Chlor-Trimeton^{®} (Chlorpheniramine), Descoratadine (Clarinex^{®}, Clarinex^{®} D-12, and Clarinex^{®} D-24), Loratadine (Claritin^{®}, Claritin^{®} D-12, Claritin^{®} D-24, and Alavert^{®}), Dimenhydrinate (Dramamine^{®}), Diphenhydramine (Benadryl^{®} Allergy, Nytol^{®}, Sominex^{®}), Doxylamine (Vicks^{®} NyQuil^{®}, Alka-Seltzer^{®} Plus NightTime Cold Medicine), Cyproheptadine (Periactin^{®}), Promethazine (Phenergan^{®}), Acrivastine (Semprex^{®}, Semprex^{®}-D), Clemastine (Tavist^{®}), doxylamine (Unisom^{®}), Levoceterizine (Xyzal^{®});
mast cell stabalizers (*e.g.* Beta2-adrenergic agonists, Cromoglicic acid, cromolyn sodium, Gastrocrom^{®}, Ketotifen, Methylxanthines, Omalizumab, Pemirolast, Quercetin, Ketotifen (Zaditen^{®}));
anti-inflammatory agents (*e.g*., NSA1D (*e.g.* Aspirin, Choline and magnesium salicylates, Diclofenac potassium (Cataflam^{®}), Diclofenac sodium (Voltaren^{®}, Voltaren^{®} XR), Diclofenac sodium with misoprostol (Arthrotec^{®}), Diflunisal (Dolobid^{®}), Etodolac (Lodine^{®}, Lodine^{®} XL), Fenoprofen calcium (Nalfon^{®}), Flurbiprofen (Ansaid^{®}), Ibuprofen (Advil^{®}, Motrin^{®}, Motrin@ IB, Nuprin^{®}), Indomethacin (Indocin^{®}, Indocin^{®} SR), Ketoprofen (Actron^{®}, Orudis^{®}, Orudis^{®} KT, Oruvail^{®}), Magnesium salicylate (Arthritab, Bayer^{®} Select, Doan's Pills, Magan, Mobidin, Mobogesic) Meclofenamate sodium (Meclomen^{®}), Mefenamic acid (Ponstel^{®}), Meloxicam (Mobic^{®}), Nabumetone (Relafen^{®}), Naproxen (Naprosyn^{®}, Naprelan^{®}), Naproxen sodium (Aleve^{®}, Anaprox^{®}), Oxaprozin (Daypro^{®}), Piroxicam (Feldene^{®}), Rofecoxib (Vioxx^{®}), Salsalate (Amigesic, Anaflex 750, Disalcid, Marthritic, Mono-Gesic, Salflex, Salsitab), Sodium salicylate, Sulindac (Clinoril^{®}), Tolmetin sodium (Tolectin^{®}), Valdecoxib (Bextra^{®}));
Receptor Tyrosine Kinase Inhibitor (*e.g*. Sunitinib);
Alkylating Agents (*e.g*., Dacarbazine, Carboplatin);
CDK 4/6 Inhibitors (*e.g*., LEE01 1);
PKC Inhibitors (*e.g*., AEB071);
MAPK inhibitors (*e.g.,* RAS Inhibitors/Farnesyltransferase inhibitor (*e.g.* Tipifamib), Raf Kinase Inhibitor (*e.g.* Sorafenib (BAY 43-9006, Nexavar), Vemurafenib, Dabrafenib, LGX818, TAK-632, MLN2480, PLX-4720), ERK Inhibitors (*e.g*., SCH772984, VTX11e);
BRAF inhibitors (e.g., vemurafenib, dabrafenib)
P13K Inhibitor (*e.g*., LY294002);
AKT Inhibitor (*e.g*., MK 2206);
PI3K/AKT Inhibitor (*e.g.* buparlisib, Cixutumumab);
mTOR Inhibitors (*e.g.* Topical Rapamycin, RAD001 (Everolimus/Rapamycin), Temsirolimus, Sirolimus);
Tyrosine Kinase Inhibitors (*e.g.* Imatinib (Gleevec^{®}), Cabozantinib (inhibitor of tyrosine kinases c-Met and VEGFR2), Nilotinib (Tasigna^{®});
VEGF Inhibitor (*e.g*. Ranibizumab (Lucentis^{®}), Cediranib);
Immune Response Modifier (*e.g*. Topical Imiquimod, Interferon, PEG Interferon);
Calcium Channel Blocker (*e.g*. Avocil (Medenna)/15% Verapamil, vitamin D separately, Doxycyline Injections);
Statin (*e.g*. Lovastatin, Methotrexate, Vinblastine, Pregabalin, Temozolomide, PLX3397);
HDAC Inhibitor (*e.g*. AR-42);
HSP- 90 Inhibitors (*e.g*. Ganetespib);
retinoids (*e.g*. adapalene, Isotretinoin, tazarotene, tretinoin);
steroids (*e.g*. Alclometasone, Amcinonide, Betamethasone, Betamethasone dipropionate, Betamethasone dipropionate, augmented, Budesonide, Clobetasol propionate, Cortisone, Desonide, Dexamethasone, Diflorasone diacetate, Fluocinolone acetonide, Fluocinonide, Flurandrenolide, Fluticasone propionate, Halobetasol propionate, Halocinonide, Hydrocortisone, Hydrocortisone butyrate, Hydrocortisone valerate, Methylprednisolone, Mometasone, Mometasone furoate, Prednicarbate, Prednisolone, Prednisone, Triamcinolone, Triamcinolone acetonide);
topical calcineurin inhibitors (*e.g*., pimecrolimus (Elidel^{®} Cream 1%, Novartis, tacrolimus (Protopic^{®} Ointment, Astellas)); and
Non-pharmaceutical Interventions (*e.g*. photodynamic Therapy (Levulan Kerastick Topical + light), Electrodesication (ED), YAG Laser).

In various embodiments, the therapies (*e.g*., a compound provided herein and the second agent) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart. In various embodiments, the therapies are administered no more than 24 hours apart or no more than 48 hours apart. In some embodiments, two or more therapies are administered within the same patient visit. In some embodiments, the compound provided herein and the second agent are administered concurrently.

In some embodiments, the compound provided herein and the second agent are administered at about 2 to 4 days apart, at about 4 to 6 days apart, at about 1 week part, at about 1 to 2 weeks apart, or more than 2 weeks apart.

In some embodiments, administration of the same agent may be repeated and the administrations may be separated by at least 1 day, 2 days, 3 days, 5 days, 10 days, 15 days, 30 days, 45 days, 2 months, 75 days, 3 months, or 6 months. In some embodiments, administration of the same agent may be repeated and the administration may be separated by at least at least 1 day, 2 days, 3 days, 5 days, 10 days, 15 days, 30 days, 45 days, 2 months, 75 days, 3 months, or 6 months.

In some embodiments, a compound provided herein and a second agent are administered to a patient, In some embodiments, a mammal, such as a human, in a sequence and within a time interval such that the compound provided herein can act together with the other agent to provide an increased benefit than if they were administered otherwise. In some embodiments, the second active agent can be administered at the same time or sequentially in any order at different points in time; however, if not administered at the same time, they should be administered sufficiently close in time so as to provide the desired therapeutic or prophylactic effect. In some embodiments, the compound provided herein and the second active agent exert their effect at times which overlap. Each second active agent can be administered separately, in any appropriate form and by any suitable route. In some embodiments, the compound provided herein is administered before, concurrently or after administration of the second active agent.

In some embodiments, the compound provided herein and the second agent are cyclically administered to a patient. Cycling therapy involves the administration of a first agent (*e.g.,* a first prophylactic or therapeutic agent) for a period of time, followed by the administration of a second agent and/or third agent (*e.g.,* a second and/or third prophylactic or therapeutic agent) for a period of time and repeating this sequential administration. Cycling therapy can reduce the development of resistance to one or more of the therapies, avoid or reduce one or more of the side effects of one of the therapies, and/or improve the efficacy of the treatment.

In some embodiments, the compound provided herein and the second active agent are administered in a cycle of less than about 3 weeks, about once every two weeks, about once every 10 days or about once every week. One cycle can comprise the administration of a compound provided herein and the second agent by infusion over about 90 minutes every cycle, about 1 hour every cycle, about 45 minutes every cycle. Each cycle can comprise at least 1 week of rest, at least 2 weeks of rest, at least 3 weeks of rest. The number of cycles administered is from about 1 to about 12 cycles, more typically from about 2 to about 10 cycles, and more typically from about 2 to about 8 cycles.

In some embodiments, courses of treatment are administered concurrently to a patient, *i.e.,* individual doses of the second agent are administered separately yet within a time interval such that the compound provided herein can work together with the second active agent. In some embodiments, one component can be administered once per week in combination with the other components that can be administered once every two weeks or once every three weeks. In other words, the dosing regimens are carried out concurrently even if the therapeutics are not administered simultaneously or during the same day.

The second agent can act additively or synergistically with the compound provided herein. In some embodiments, the compound provided herein is administered concurrently with one or more second agents in the same pharmaceutical composition. In some embodiments, a compound provided herein is administered concurrently with one or more second agents in separate pharmaceutical compositions. In some embodiments, a compound provided herein is administered prior to or subsequent to administration of a second agent. Also contemplated are administration of a compound provided herein and a second agent by the same or different routes of administration, *e.g.,* oral and parenteral. In some embodiments, when the compound provided herein is administered concurrently with a second agent that potentially produces one or more adverse side effects including, but not limited to, toxicity, the second active agent can advantageously be administered at a dose that falls below the threshold that the adverse side effect is elicited.

### VIII. EXAMPLES

### General Synthetic Methods

The compounds provided herein can be prepared, isolated or obtained by any method apparent to those of skill in the art. Compounds provided herein can be prepared according to the Exemplary Preparation Schemes provided below. Reaction conditions, steps and reactants not provided in the Exemplary Preparation Schemes would be apparent to, and known by, those skilled in the art. As used herein, the symbols and conventions used in these processes, schemes and examples, regardless of whether a particular abbreviation is specifically defined, are consistent with those used in the contemporary scientific literature, for example, the Journal of the American Chemical Society or the Journal of Biological Chemistry. Specifically, but without limitation, the following abbreviations may be used in the examples and throughout the specification: g (grams); mg (milligrams); mL (milliliters); µL (microliters); mM (millimolar); µM (micromolar); Hz (Hertz); MHz (megahertz); mmol (millimoles); hr or hrs (hours); min (minutes); MS (mass spectrometry); ESI (electrospray ionization); TLC (thin layer chromatography); HPLC (high pressure liquid chromatography); THF (tetrahydrofuran); CDCl₃ (deuterated chloroform); AcOH (acetic acid); DCM (dichloromethane); DMSO (dimethylsulfoxide); DMSO-*d₆* (deuterated dimethylsulfoxide); EtOAc (ethyl acetate); MeOH (methanol); Tees (2,2,2-trichloroethoxysulfonyl); -Si(*tert*-Bu)(Ph)₂ and -Si^{t}BuPh₂ (tert-butyldiphenylsilyl); and BOC (*t*-butyloxycarbonyl).

For all of the following examples, standard work-up and purification methods known to those skilled in the art can be utilized. Unless otherwise indicated, all temperatures are expressed in °C (degrees Celsius). All reactions are conducted at room temperature unless otherwise noted. Synthetic methodologies illustrated herein are intended to exemplify the applicable chemistry through the use of specific examples and are not indicative of the scope of the disclosure.

### Compounds of formula (I)

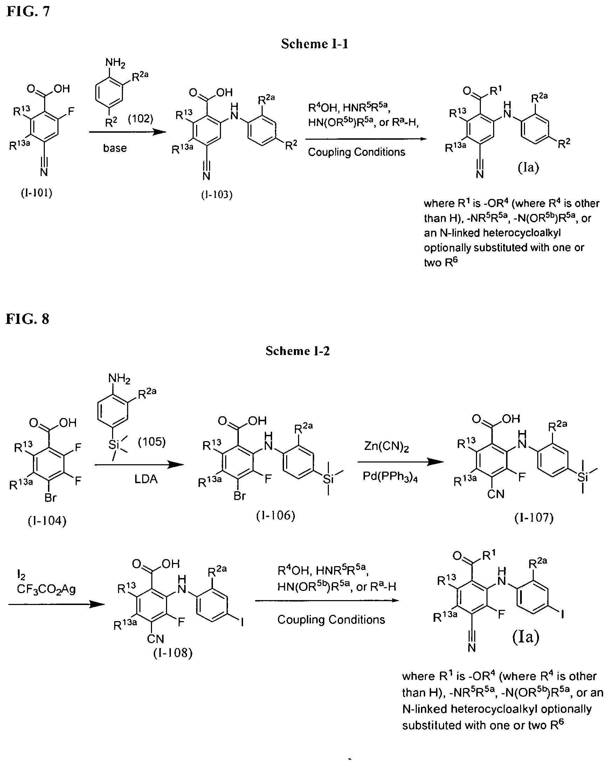

Compounds of formula (Ia) can be prepared according to Scheme 1-1, as shown in FIG. 7, in which R¹ is -OR⁴, -NR⁵R^{5a}, -N(OR^{5b})R^{5a}, or a N-linked heterocycloalkyl which is unsubstituted or substituted with one or two R⁶; and X¹, R², R^{2a}, R¹³, R^{13a}, R⁴, R⁵, R^{5a}, R^{5b}, and R⁶ are as defined in any aspect, embodiment, or claim as described herein. In some embodiments, R¹³ and R^{13a} are each hydrogen.

Starting from commercially-available or routinely-accessible acids of formula (1-101) and commercially-available or routinely-accessible anilines of formula (1-102), compounds of formula (1-103) can be prepared by methods apparent to those of skill in the art. The acid-containing compound of formula (1-103) can be activated with numerous reagents apparent to those of skill in the art to produce compounds with a suitable leaving group attached to the carbonyl of the C(O)OH acid group, for example an acid chloride produced from the reaction of (I-103) with thionyl chloride or an active ester produced from the reaction of (1-103) with reagents such as EDCI or HOBt. The acid chlorides or active esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (1a).

Compounds of formula (Ia) can be prepared according to Scheme 1-2, as shown in FIG. 8, in which R¹ is -OR⁴, -NR⁵R^{5a}, -N(OR^{5b})R^{5a}, or a N-linked heterocycloalkyl unsubstituted or substituted with one or two R⁶; R² is iodo; R^{13b} is fluoro; and R^{2a}, R¹³, R^{13a} R⁴, R⁵, R^{5a}, R^{5b}, and R⁶ are as defined in any aspect, embodiment, or claim as described herein. In some embodiments, R¹³ and R^{13a} are each hydrogen.

Starting from commercially-available or routinely-accessible acids of formula (1-104) and commercially-available or routinely-accessible anilines of formula (I-105), compounds of formula (I-106) can be prepared by methods apparent to those of skill in the art. A compound of formula (1-106) is then treated with Zn(CN)₂ in the presence of a catalyst such as Pd(PPh₃)₄. The compound of formula (1-108) is prepared by treating (1-107) with iodine in the presence of siver trifluoroacetate or alternatively with iodine monochloride. The acid-containing compound of formula (1-108) can be activated with numerous reagents apparent to those of skill in the art to produce compounds with a suitable leaving group attached to the carbonyl of the C(O)OH acid group, for example an acid chloride produced from the reaction of (1-108) with thionyl chloride or an active ester produced from the reaction of (1-108) with reagents such as EDC1 or HOBt. The acid chlorides or active esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (1a), in which R² is iodo and R^{13b} is fluoro.

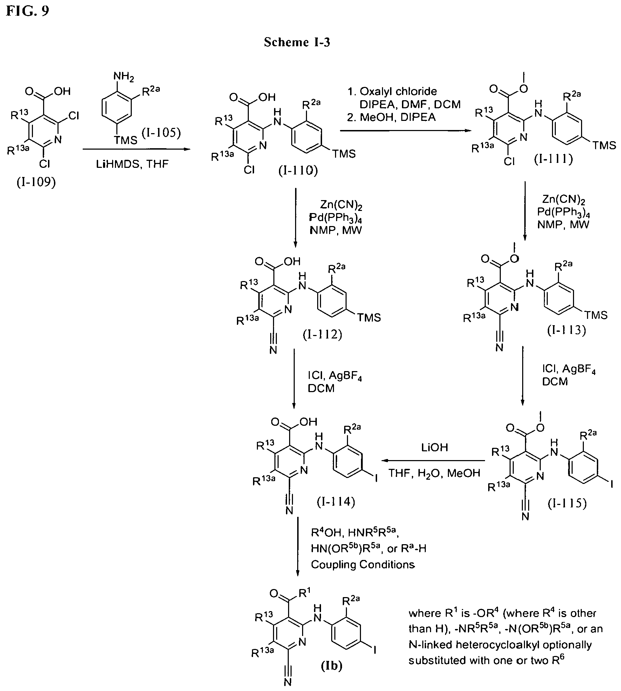

Compounds of formula (Ib) can be prepared according to Scheme 1-3, as shown in FIG. 9, in which R¹ is -OR⁴, -NR⁵R^{5a}, -N(OR^{5b})R^{5a}, or a N-linked heterocycloalkyl unsubstituted or substituted with one or two R⁶; R² is iodo; R^{3b} is fluoro; and R^{2a}, R¹³, R^{13a}, R⁴, R⁵, R^{5a}, R^{5b}, and R⁶ are as defined in any aspect, embodiment, or claim as described herein. In some embodiments, R¹³ and R^{13a} are each hydrogen. In some embodiments, R³ is hydrogen and R^{13a} is fluoro.

Starting from commercially-available or routinely-accessible acids of formula (1-109) and commercially-available or routinely-accessible anilines of formula (1-105), compounds of formula (I-110) can be prepared by methods apparent to those of skill in the art. Alternatively, compounds of formula (I-110) can be converted to methyl ester of formula (1-111) by methods known in the art. A compound of formula (1-110) or formula (1-11 1) is then treated with Zn(CN)₂ in the presence of a catalyst such as Pd(PPh₃)₄. The compound of formula (1-114) or formula (1-115) is prepared by treating the compound of formula (1-110) or formula (1-11 1) with iodine in the presence of siver trifluoroacetate or alternatively with iodine monochloride. In the case of the compound of formula (1-115), the methyl ester is then hydrolyzed to the corresponding acid. The acid-containing compound of formula (1-114) can be activated with numerous reagents apparent to those of skill in the art to produce compounds with a suitable leaving group attached to the carbonyl of the C(O)OH acid group, for example an acid chloride produced from the reaction of (I-114) with thionyl chloride or an active ester produced from the reaction of (1-114) with reagents such as EDC1 or HOBt. The acid chlorides or active esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (1b).

### Compounds of formula (11)

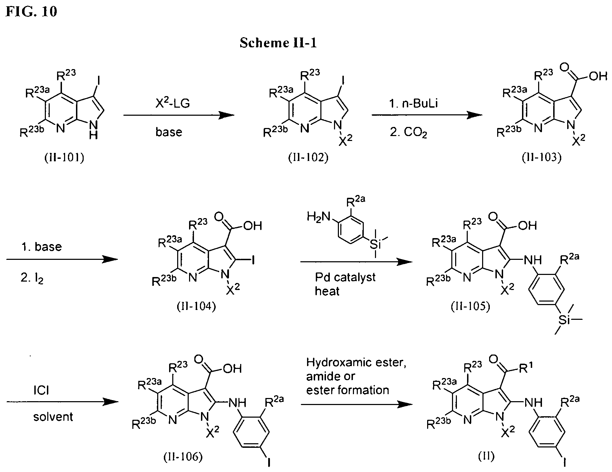

Compounds of formula (11) can be prepared according to any one of Schemes 11-1, 11-2, 11-3, 11-4, and 11-5, as shown in FIGs. 10-14, respectively. As shown in Schemes 11-1, 11-2, 11-3, 11-4, and 11-5, R¹ is -OR⁴, -NR⁵R^{5a}, -N(OR^{5b})R^{5a}, or a N-linked heterocycloalkyl which is unsubstituted or substituted with one or two R⁶; and X², R², R^{2a}, R²³, R^{23a}, R^{23b}, R⁴, R⁵, R^{5a}, R^{5b}, and R⁶ are as defined in any aspect, embodiment, or claim as described herein. In some embodiments, X² is methyl. In some embodiments, X² is methyl; R²³ and R^{23b} are each hydrogen; and R^{23a} is fluoro, methyl, or methoxy. In some embodiments, X² is methyl and R²³, R^{23a}, and R^{23b} are each hydrogen.

Compounds of formula (11) may be prepared according to Scheme 11-1, as shown in FIG. 10, starting from the commercially available intermediate (11-101). Compounds of formula (11-102) are prepared by alkylation of the intermediates (11-101) with X²-LG, wherein LG is a suitable leaving group. Compounds of formula (11-103) can be prepared by methods apparent to those of skill in the art and are converted to compounds of formula (11-104) by iodination. A compound of formula (11-104) is then reacted with an aniline in the presence of a Pd catalyst to provide a compound of formula (11-105). The compound of formula (11-106) is prepared by treating (11-106) with iodine in the presence of siver trifluoroacetate or alternatively with iodine monochloride. The acid-containing compound of formula (11-106) can be activated with numerous reagents apparent to those of skill in the art to produce compounds with a suitable leaving group attached to the carbonyl of the C(O)OH acid group, for example an acid chloride produced from the reaction of (11-106) with thionyl chloride. The acid chlorides can then be reacted with compounds of formula R⁴OH, HNR⁵R^{5a}, HN(OR^{5b})R^{5a}, R^{a}-H (where R^{a} is an N-linked heterocycloalkyl unsubstituted or substituted with one or two R⁶), or suitable protected forms thereof to produce compounds of formula (II), in which R² is iodo.

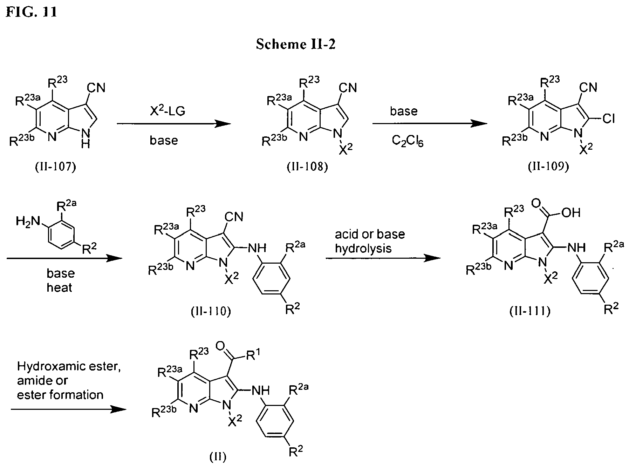

Compounds of formula (11) may be prepared according to Scheme 11-2, as shown in FIG. 11, starting from the commercially available or readily accessible intermediate of formula (11-107). Compounds of formula (11-108) are prepared by alkylation of the intermediates (11-107) with X²-LG, wherein LG is a suitable leaving group. Compounds of formula (11-109) can be prepared by methods apparent to those of skill in the art by chlorination. A compound of formula (11-109) is then reacted with an aniline to provide a compound of formula (II-110), which is further converted to an acid of formula (11-1 1 1). The acid-containing compound of formula (II-111) can be activated with numerous reagents apparent to those of skill in the art to produce compounds with a suitable leaving group attached to the carbonyl of the C(O)OH acid group, for example an acid chloride produced from the reaction of (II-111) with thionyl chloride. The acid chlorides can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (II).

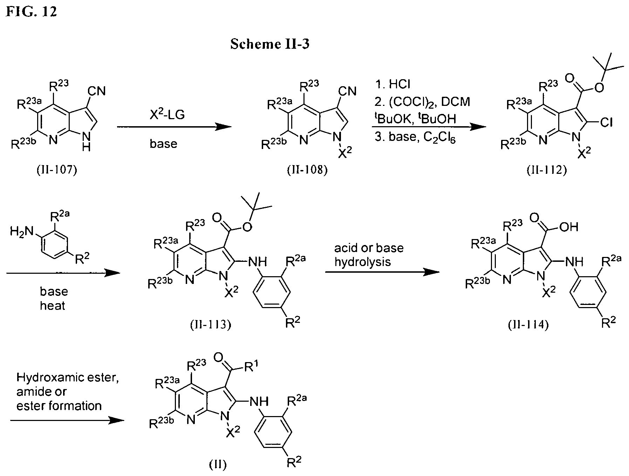

Compounds of formula (II) may be prepared according to Scheme 11-3, as shown in FIG. 12, starting from the commercially available or readily accessible intermediate of formula (11-107). Compounds of formula (11-108) are prepared by alkylation of the intermediates (11-107) with X²-LG, wherein LG is a suitable leaving group. Compounds of formula (11-112) can be prepared by methods apparent to those of skill in the art by hydrolysis of the nitrile group to an acid, protection of the acid group, and chlorination. A compound of formula (II-112) is then reacted with an aniline to provide a compound of formula (II-113), which is further hydrolyzed to an acid of formula (11-114). The acid-containing compound of formula (II-114) can be activated with numerous reagents apparent to those of skill in the art to produce compounds with a suitable leaving group attached to the carbonyl of the C(O)OH acid group, for example an acid chloride produced from the reaction of (II-114) with thionyl chloride. Alternatively, compounds of formula (11-113) can be directly converted to acid chlorides or activated esters as described in Scheme 11-4 or 11-5. The acid chlorides or activated esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (11).

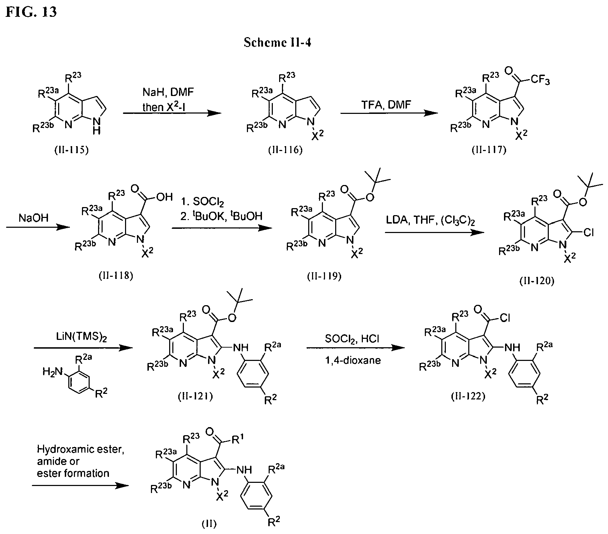

Compounds of formula (II) may be prepared according to Scheme 11-4, as shown in FIG. 13, starting from the commercially available or readily accessible intermediate of formula (11-1 15). Intermediate (11-116) which is prepared by N-alkylation on the pyrrole nitrogen of (II-115) is readily converted to the trifluoromethyl ketone intermediate (11-117), hydrolyzed to the corresponding acid (11-118), and converted to the tert-butyl ester (II-119) by esterification by methods apparent to those of skill in the art. Chlorination in the 2-position of intermediate (II-119) by treating (11-1 19) with a base such as LDA followed by treatment with a chlorinating agent such as perchloroethane affords the chlorinated intermediate (11-120). Displacement of the chlorine of intermediate (11-120) with a suitable aniline in the presence of a base gives intermediate (11-121) which can be readily converted to the corresponding acid chloride (11-122) by treatment with thionyl chloride, with or without the addition of 4 N HCl in dioxane (J. Org. Chem., 2017, 82 (6), pp 3245-3251). Alternatively, Intermediate (11-122) can be converted to an activated ester by treatment with an alcohol such as pentafluorophenol. The acid chlorides or activated esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of Formula (II).

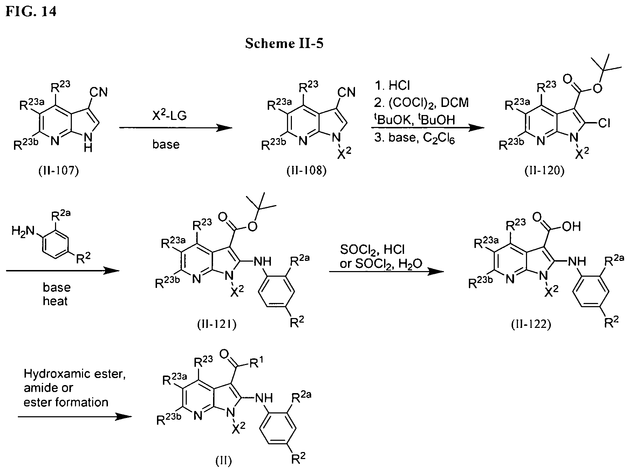

In addition, compounds of formula (11) may be prepared by the synthetic route given in Scheme 11-5, as shown in FIG. 14. Nitriles (11-108) which are prepared by N-alkylation on the pyrrole nitrogen of (11-107) can be hydrolyzed to the corresponding acids and esterified to form t-butyl esters (II-120). Then, following the sequence of Scheme 11-4, compounds of formula (II) may be prepared.

### Compounds of formula (111)

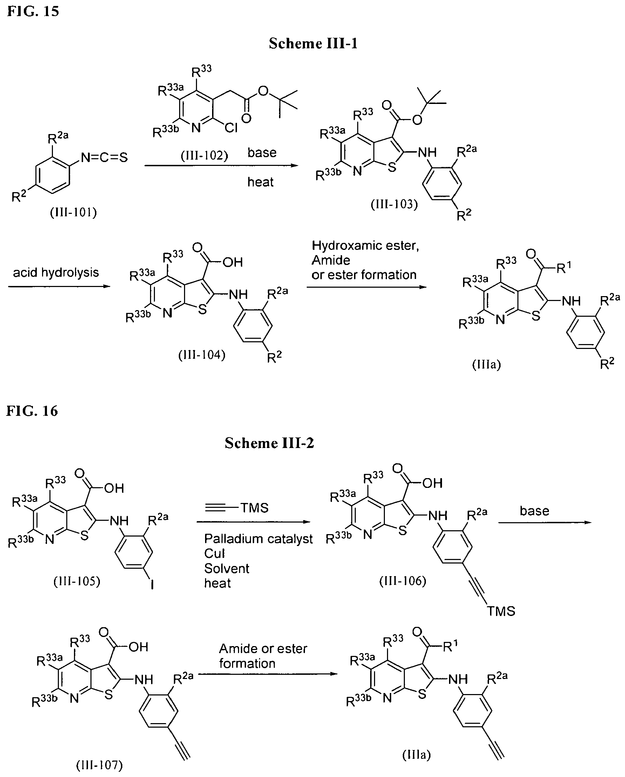

Compounds of formula (IIIa) can be prepared according to Scheme III-1 or III-2, as shown in FIGs. 15-16, respectively. As shown in Scheme 111-1 or 111-2, R¹ is -OR⁴, -NR⁵R^{5a}, -N(OR^{5b})R^{5a}, or a N-linked heterocycloalkyl which is unsubstituted or substituted with one or two R⁶; and R², R^{2a}, R³³, R^{33a}, R^{33b}, R⁴, R⁵, R^{5a}, R^{5b}, and R⁶ are as defined in any aspect, embodiment, or claim as described herein. In some embodiments, R^{33a} and R^{33b} are each hydrogen and R³³ is fluoro, methyl, or methoxy. In some embodiments, R³³ and R^{33b} are each hydrogen and R^{33a} is fluoro, methyl, or methoxy. In some embodiments, R³³ and R^{33a} are each hydrogen and R^{33b} is fluoro, methyl, or methoxy. In some embodiments, X² is methyl and R³³, R^{33a}, and R^{33b} are each hydrogen.

Compounds of formula (IIIa) can be prepared according to Scheme 111-1, as shown in FIG. 15. Starting from commercially-available or routinely-accessible substituted phenyl isothiocyanate (111-101) and intermediate (111-102), intermediate (111-103) can be prepared by methods apparent to those of skill in the art. Intermediate (111-103) can be readily hydrolyzed by contact with an appropriate acid such as hydrochloric acid or trifluoroacetic acid in an appropriate solvent such as dichloromethane or dioxane to give intermediate (111-104). The acid intermediate (111-104), can be activated with numerous reagents apparent to those of skill in the art to produce compounds with a suitable leaving group attached to the carbonyl, for example an acid chloride produced from the reaction of (111-104) with thionyl chloride or an active ester produced from the reaction of (111-104) with reagents such as EDCl or HOBt. The acid chlorides or active esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (IIIa).

Alternatively, compounds of formula (IIIa) wherein R² is C₂-C₆ alkynyl group can be prepared according to Scheme 111-2, as shown in FIG. 16, from compounds of formula (IIIa) wherein R² is initially iodo (i.e., formula 111-105). Reactions conditions similar to those published in the Journal of Medicinal Chemistry 2007, 50, 5090-5102 can be employed to afford the transformation from an aromatic iodo group to alkyne. Intermediate (IIIa-107) can be converted to a compound of formula (IIIa) using procedures described herein and above for Scheme 111-1.

### Compounds of formula (IV)

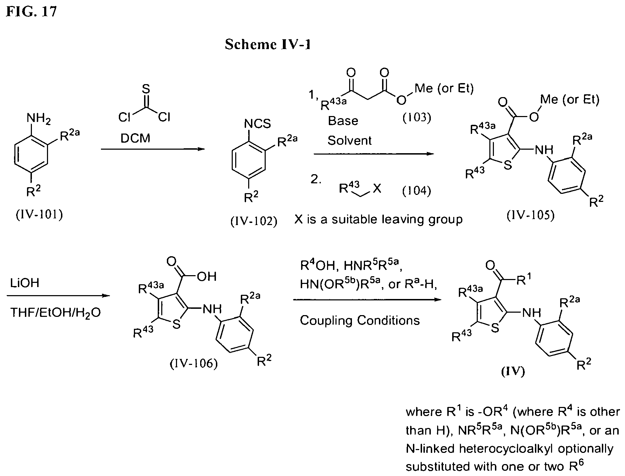

Compounds of any one of formulae (IVa), (1Vb), and (1Vc) can be prepared according to Scheme 1V-1, as shown in FIG. 17, in which R¹ is -OR⁴, -NR⁵R^{5a}, -N(OR^{5b})R^{5a}, or a N-linked heterocycloalkyl which is unsubstituted or substituted with one or two R⁶; and R², R^{2a}, R^{43a} , R⁴, R⁵, R^{5a}, R^{5b}, R⁶, R⁴⁶, R⁴⁸, and R^{48a} are as defined in any aspect, embodiment, or claim as described herein.

Starting from commercially-available or routinely-accessible anilines of formula (IV-101) and thiophosgene, intermediate (1V-102) can be prepared by methods apparent to those of skill in the art. Intermediate (IV-102) can be readily be condensed with an intermediate of formula (IV-103) in the presence of a base and solvent, followed by treatment with an intermediate of formula (IV-104) to yield the intermediate of formula (IV-105). The intermediate (IV-105) is then hydrolyzed by contact with an appropriate base such as LiOH in an appropriate solvent such as THF and/or ethanol and/or water to give intermediate (1V-106). The acid intermediate (IV-106) can be activated with numerous reagents apparent to those of skill in the art to produce compounds with a suitable leaving group attached to the carbonyl, for example an acid chloride produced from the reaction of intermediate (IV-106) with thionyl chloride or an active ester produced from the reaction of intermediate (IV-106) with reagents such as EDCI or HOBt. The acid chlorides or active esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce any one of formulae (IVa), (IVb), and (IVc) wherein R⁴³ is cyano, -C(O)NR⁴⁸R^{48a}, or -C(O)R⁴⁶.

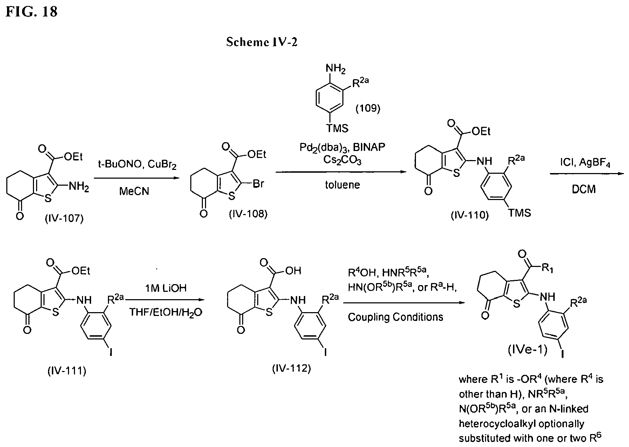

Compounds of formula (IV) wherein R^{3a} and R³ together form -CH₂CH₂C(O)- or -CH₂CH₂CH₂C(O)-, for example compounds of formula (IVe-1) can be prepared using procedures described in WO2008/020206 or by Scheme IV-2 as showun in FIG. 18. As shown in Scheme IV-2, R¹ is -OR⁴, -NR⁵R^{5a}, -N(OR^{5b})R^{5a}, or a N-linked heterocycloalkyl which is unsubstituted or substituted with one or two R⁶; and R^{2a}, R⁴, R⁵, R^{5a}, R^{5b}, and R⁶ are as defined in any aspect, embodiment, or claim as described herein. In some embodiments, R^{2a} is fluoro.

Starting from a commercially-available or routinely-accessible compound of formula (IV-108) and commercially-available or routinely-accessible anilines of formula (1V-109), compounds of formula (1V-1 10) can be prepared by methods apparent to those of skill in the art. The compound of formula (1V-1 11) is prepared by treating (IV-110) with iodine in the presence of siver trifluoroacetate or alternatively with iodine monochloride. Compounds of formula (IV-111) are then subjected to ester hydrolysis to provide the acid-containing compound of formula (IV-112). The acid group of the compound (1V-112) can be activated with numerous reagents apparent to those of skill in the art to produce compounds with a suitable leaving group attached to the carbonyl of the C(O)OH acid group, for example an acid chloride produced from the reaction of (1V-1 12) with thionyl chloride or an active ester produced from the reaction of (IV-112) with reagents such as EDC1 or HOBt. The acid chlorides or active esters can then be reacted with compounds of formula R⁴OH, HNR⁵R^{5a}, HN(OR^{5b})R^{5a}, R^{a}-H (where R^{a} is an N-linked heterocycloalkyl unsubstituted or substituted with one or two R⁶), or suitable protected forms thereof to produce compounds of formula (IVe-1), in which R² is iodo. Similarily, compounds of any one of formulae (IVd-1), (1Vd-2), and (IVe-2) wherein R² is iodo can be prepared according to Scheme 1V-2 starting from the corresponding starting material.

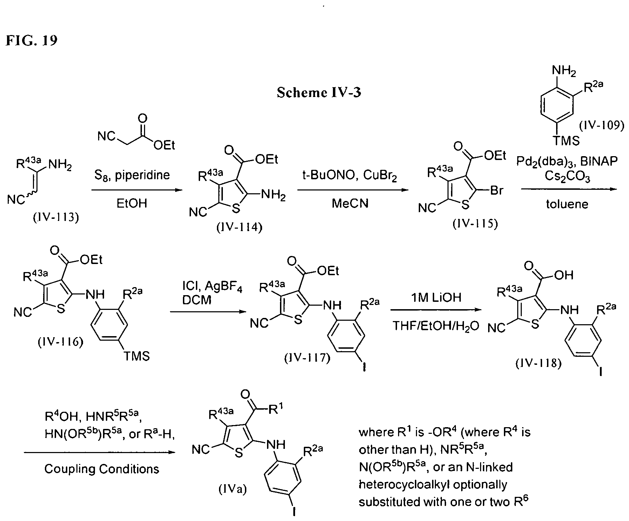

Compounds of formula (1Va) can be prepared according to Scheme 1V-3, as shown in FIG. 19, in which R¹ is -OR⁴, -NR⁵R^{5a}, -N(OR^{5b})R^{5a}, or a N-linked heterocycloalkyl which is unsubstituted or substituted with one or two R⁶; and R^{2a}, R^{43a}, R⁴, R⁵, R^{5a}, R^{5b}, and R⁶ are as defined in any aspect, embodiment, or claim as described herein. In some embodiments, R^{2a} is fluoro. In some embodiments, R^{43a} is methyl.

Starting from commercially-available or routinely-accessible compounds of formula (1V-1 13), compounds of formula (1V-115) can be prepared by methods known in the art. From compounds of formula (1V-115) and commercially-available or routinely-accessible anilines of formula (1V-109), compounds of formula (1V-116) can be prepared by methods apparent to those of skill in the art. The compound of formula (IV-117) is prepared by treating (1V-1 16) with iodine in the presence of siver trifluoroacetate or alternatively with iodine monochloride. Compounds of formula (1V-1 17) are then subjected to ester hydrolysis to provide the acid-containing compound of formula (1V-1 18). The acid group of the compound (1V-1 18) can be activated with numerous reagents apparent to those of skill in the art to produce compounds with a suitable leaving group attached to the carbonyl of the C(O)OH acid group, for example an acid chloride produced from the reaction of (IV-118) with thionyl chloride or an active ester produced from the reaction of (1V-1 18) with reagents such as EDC1 or HOBt. The acid chlorides or active esters can then be reacted with compounds of formula R⁴OH, HNR⁵R^{5a}, HN(OR^{5b})R^{5a}, R^{a}-H (where R^{a} is a N-linked heterocycloalkyl unsubstituted or substituted with one or two R⁶), or suitable protected forms thereof to produce compounds of formula (1Va), in which R² is iodo.

### Compounds of formula (V)

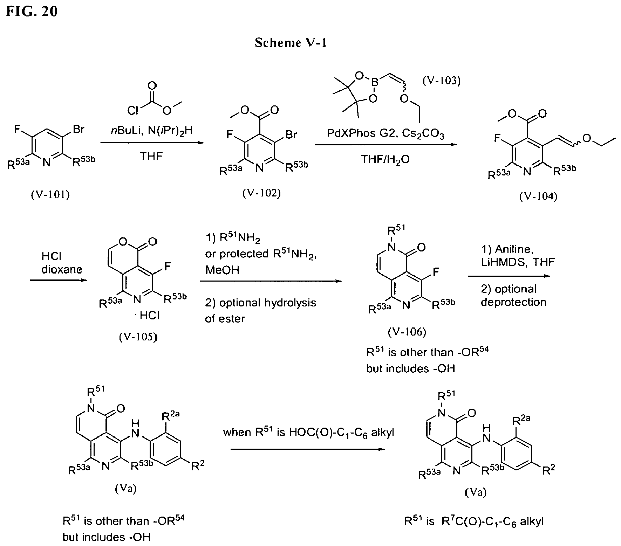

Compounds of formula (Va) can be prepared according to Scheme V-1, as shown in FIG. 20, in which subscript n is 0; R⁵¹ is other than -OR⁵⁴, but includes -OH; and R², R^{2a}, R⁵¹, R^{53a}, and R^{53b} are as defined in any aspect, embodiment, or claim as described herein. In some embodiments, R^{53a} and R^{53b} are each hydrogen.

Starting from commercially-available or routinely-accessible pyridines of formula (V-101), and commercially-available methyl chloroformate, compounds of formula (V-102) can be prepared by methods apparent to those of skill in the art. A compound of formula (V-102) is then reacted with a compound of formula (V-103) in the presence of Cs₂CO₃ and a catalyst, for example PdXPhos G2, wherein the compound of formul (V-103) is prepared from pinacolborane and ethyl vinyl ether. Cyclization of the compound of formula (V-104) under an acidic conditions provides the intermediate of formula (V-105). Various commercially-available or routinely-accessible amines or protected amines of R⁵¹NH₂ can react with the intermediate of formula (V-105) to provide compounds of formula (V-106). Compounds of formula (Va) can be prepaed by a coupling reaction of the compounds of formula (V-106) with commercially-available or routinely-accessible anilines by methods apparent to those of skill in the art, followed by optional deprotection. In some embodiments of compounds of formula (Va) wherein R⁵¹ is HOC(O)-C₁-C₆ alkyl, after further activation of the -C(O)OH group by methods known in the art, these compounds are converted to compounds of formula (Va), in which R⁵¹ is R⁷C(O)-C₁-C₆ alkyl.

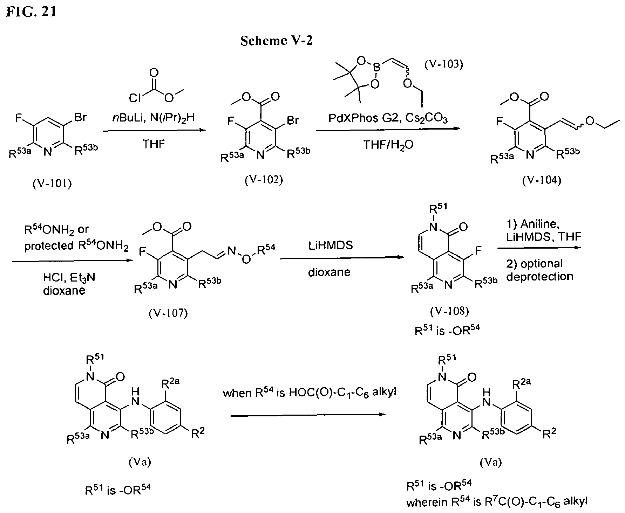

Compounds of formula (Va) can be prepared according to Scheme V-2, as shown in FIG. 21, in which subscript n is 0; R⁵¹ is -OR⁵⁴; and R², R^{2a}, R^{53a}, R^{53b}, and R⁵⁴ are as defined in any aspect, embodiment, or claim as described herein. In some embodiments, R^{53a} and R^{53b} are each hydrogen.

Starting from the common intermediate of formula (V-104) as shown in Scheme V-1, various commercially-available or routinely-accessible hydroxyamines or pretected hydroxyamines of R⁵⁴ONH₂ can react with the compound of formula (V-104) to provide compounds of formula (V-107). Cyclization of the compound of formula (V-107) under a base provides a compound of formula (V-108) (note any ester group in R⁵¹ is also hydrolyzed to a corresponding acid). Compounds of formula (Va) can be prepaed by a coupling reaction of the compounds of formula (V-108) with commercially-available or routinely-accessible anilines by methods apparent to those of skill in the art, followed by optional deprotection. In some embodiments of compounds of formula (Va) wherein R⁵¹ is -OR⁵⁴ and R⁵⁴ is HOC(O)-C₁-C₆ alkyl, after further activation of the C(O)OH group by methods known in the art, these compounds are converted to compounds of formula (1a), in which R⁵¹ is -OR⁵⁴ and R⁵⁴ is R⁷C(O)-C₁-C₆ alkyl. In some ebodiments, when R⁵¹ is -OH, the corresponding compound of formula (Va) is prepared according to Scheme V-1.

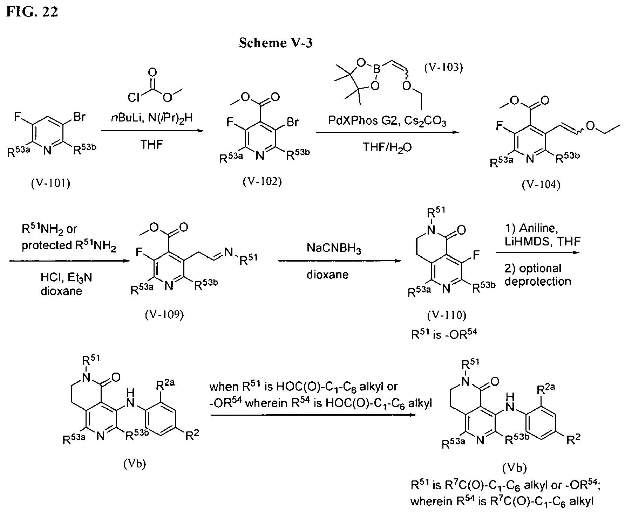

Compounds of formula (Vb) can be prepared according to Scheme V-3, as shown in FIG. 22, in which subscript n is 0 and R⁵¹, R², R^{2a}, R^{53a}, and R^{53b} are as defined in any aspect, embodiment, or claim as described herein. In some embodiments, R^{53a} and R^{53b} are each hydrogen.

Starting from the common intermediate of formula (V-1 04) as shown in Scheme V-1, various commercially-available or routinely-accessible amines, hydroxyamines, pretected amines, or protected hydroxyamines of R⁵¹NH₂ can react with the compound of formula (V-104) to provide compounds of formula (V-109). Reductive cyclization of the compound of formula (V-109) under a reducing agent (e.g., NaCNBH₃) provides a compound of formula (V-110) (note that an ester group in R⁵¹ is also hydrolyzed to a corresponding acid). Compounds of formula (Va) can be prepaed by a coupling reaction of the compounds of formula (V-110) with commercially-available or routinely-accessible anilines by methods apparent to those of skill in the art, followed by optional deprotection. In some embodiments of compounds of formula (Va) wherein R⁵¹ is HOC(O)-C₁-C₆ allcyl or-OR⁵⁴ and R⁵⁴ is HOC(O)-C₁-C₆ alkyl, after further activation of the C(O)OH group by methods known in the art, these compounds are converted to compounds of formula (1a), in which R⁵¹ is R⁷C(O)-C₁-C₆ alkyl or -OR⁵⁴ and R⁵⁴ is R⁷C(O)-C₁-C₆ alkyl.

Utilizing the Exemplary Preparation Schemes provided herein and procedures known to one of ordinary skill in the art, the compounds in Tables 1-5 can be prepared.

### Example 1: Human Birthmark Explant Protocol

This study establishes an *in vitro* birthmark explant model.

**Study Objectives:** The primary objective was to assess the efficacy of a topically-formulated compound described herein in suppressing p-ERK, a downstream biomarker of RAS/MAPK signaling in epidermal nevus and nevus sebaceous. The secondary objectives was to assess permeability (where the compound was applied topically) in birthmark explants treated with a compound described herein.

**Sample Collection and Eligibility:** Discarded human birthmark (epidermal nevus and nevus sebaceous) samples were obtained from the Stanford Surgery Clinic, using an approved human subjects protocol (Stanford 1RB#18325). Specimens were identified under the direction of the Principal Investigator and placed in cell proliferation media (DMEM/F12 containing penicillin/streptomycin (0.1%); fungizone (40 µg/mL); B27 (without vitamin A).

Patients had the following data to be enrolled in the study:
Patient must be older than 18 years of age.
Samples must be birthmarks of at least 6mm in size
Samples will be excised by a shave, punch biopsy or elliptical excision.
Patient cannot be undergoing chemotherapy treatment at time of biopsy.

### Study procedures

Samples were primary, untreated birthmarks of at least 6 mm in size; samples were excised by a shave, punch biopsy or elliptical excision. Specimens were identified under the direction of the Principal Investigator. Specimens were chopped into 2mm fragments and placed in 24-well plates containing cell proliferation media (DMEM/F12 containing penicillin/streptomycin (0.1%); fungizone (40 µg/mL); B27 (without vitamin A) and submerged in media with drug. For topical gel application, samples were placed in 96 well plates with epidermal surface exposed to air.

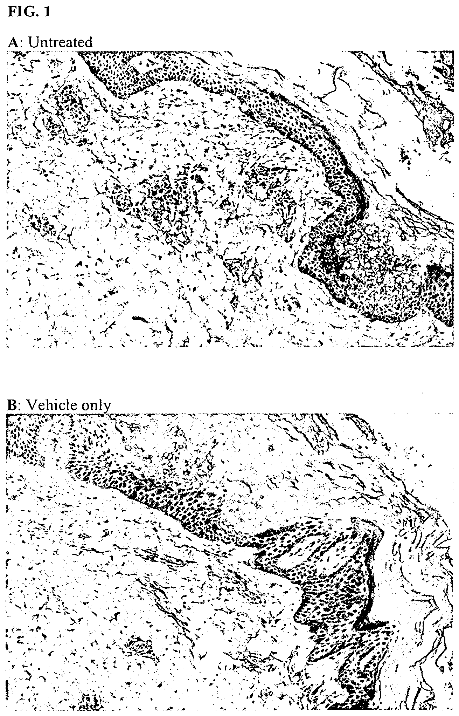

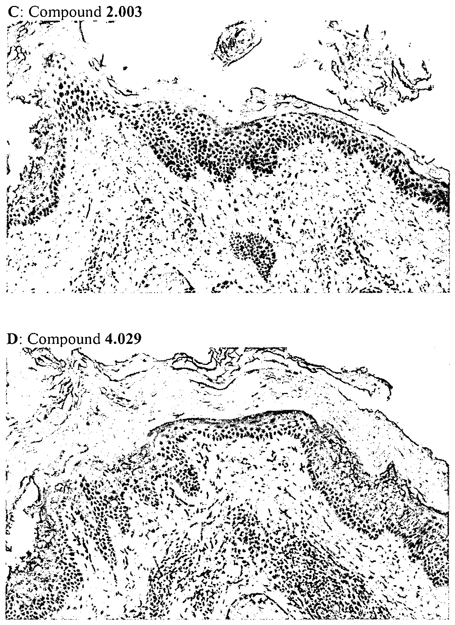

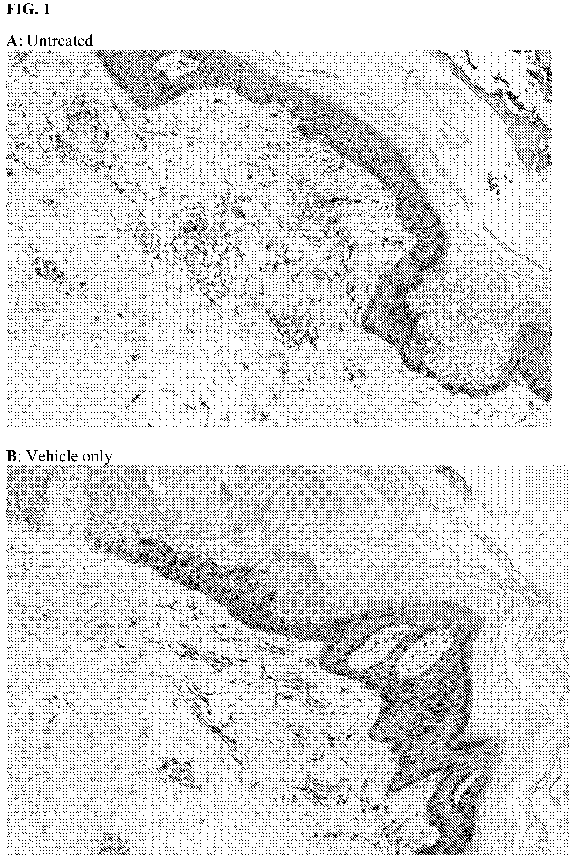

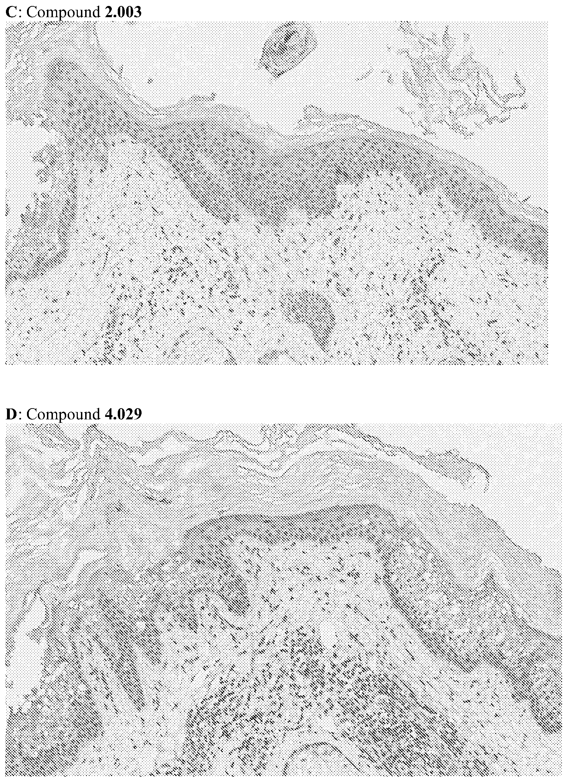

For the data provided in FIG. 1, an explant sample was submerged in media and 5 µL of Compound No. **2.003** in 100% DMSO was added to media to make stated concentrations of 5 µM. Samples were harvested at 4 hours with half snap frozen in liquid nitrogen and half fixed in 10% formalin and paraffin embedded for histologic analysis.

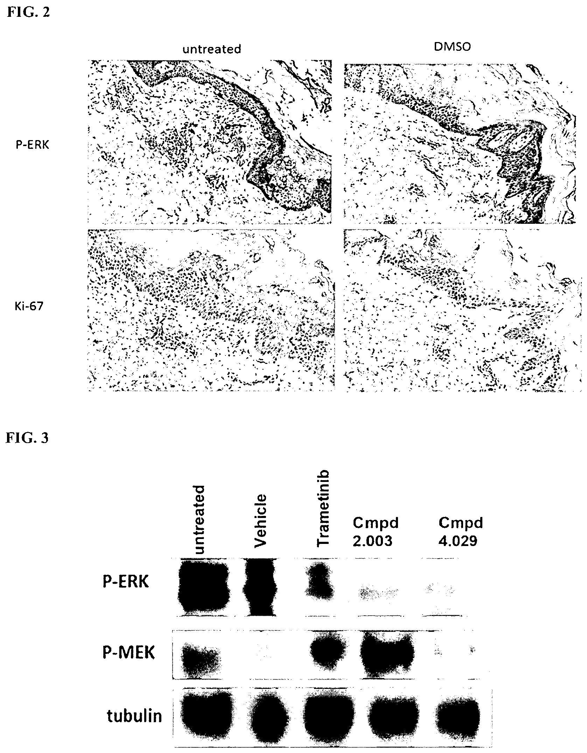

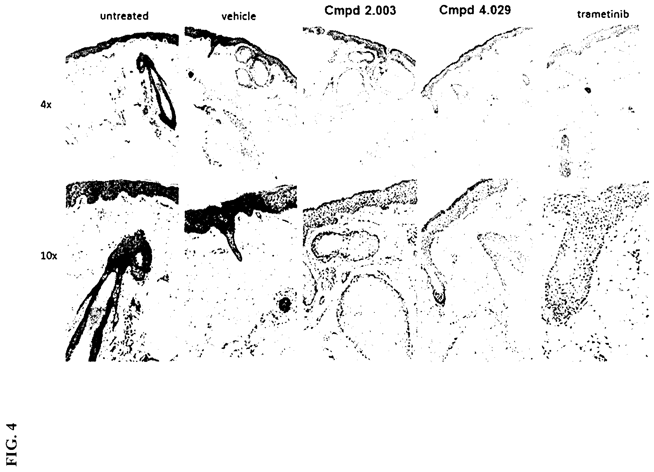

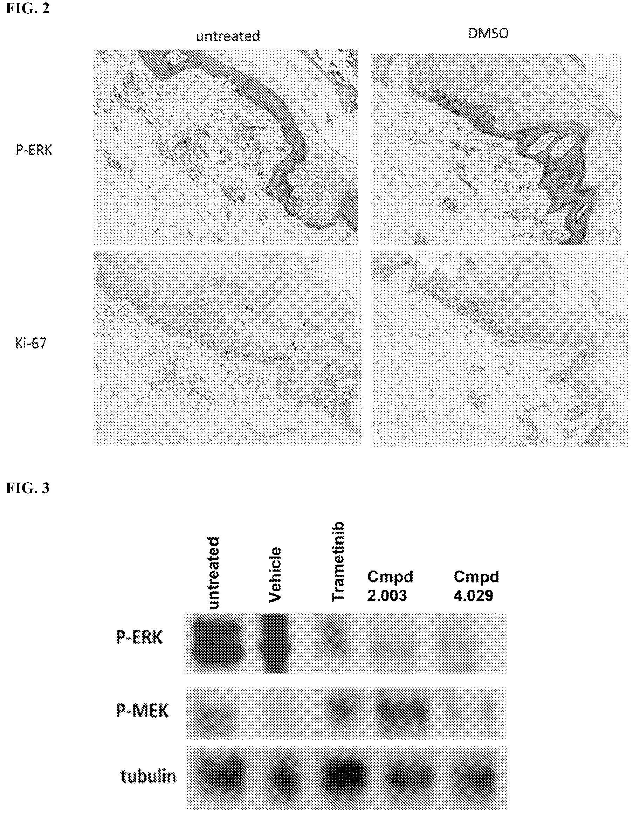

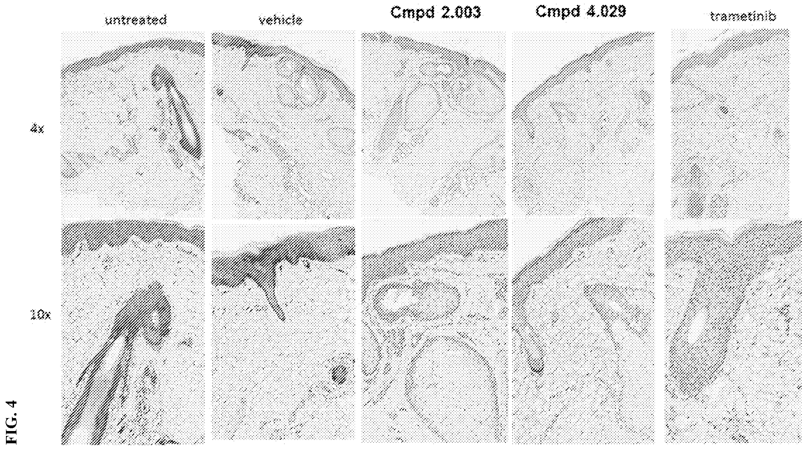

For the data provided in FIGs. 3 and 4, a gel topical gel formulation of Trametinib, Compound No. **2.003,** Compound No. **4.029,** or the gel topical formulation of vehicle only were topically applied to the surface of a birthmark explant at 4 hours for analysis. The gel formulation was 1% hydroxypropyl cellulose in DMSO/PEG 400 1:1 (v/v) and the tested compound was 1% by weight in the gel formulation. Samples were harvested at 4 hours for analysis. Harvested tissue was bisected and with half snap frozen and half fixed in 10% formalin and paraffin embedded for further analysis.

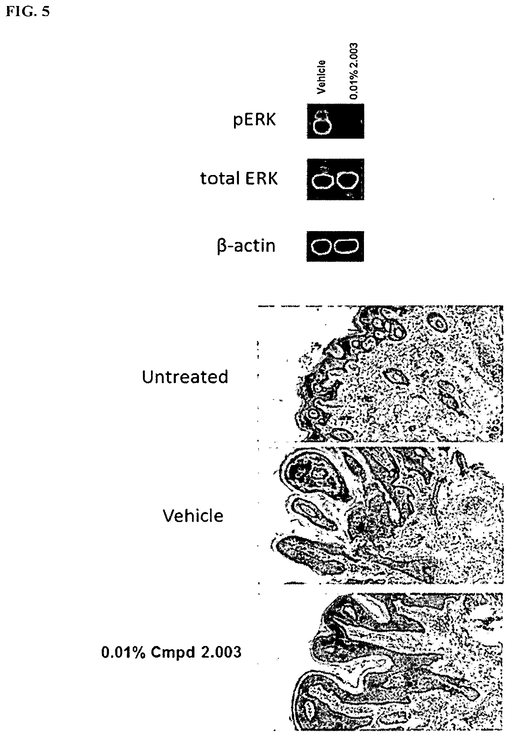

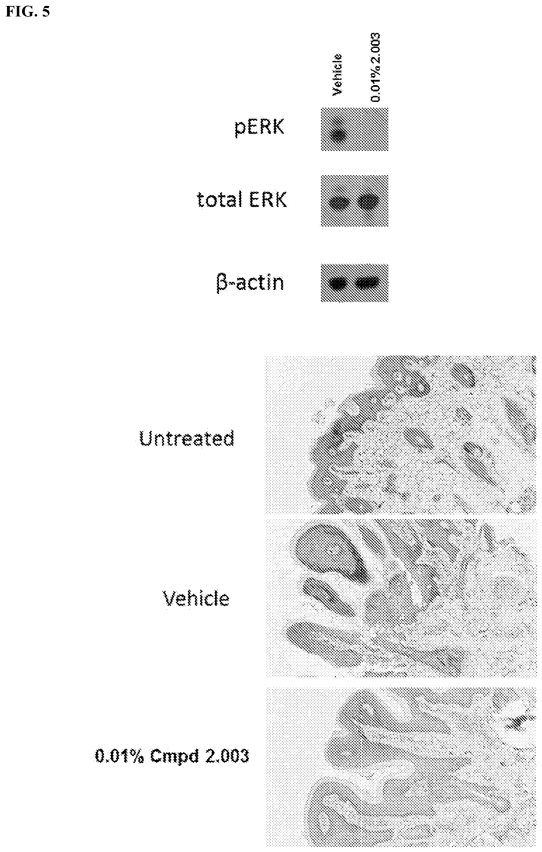

For the data provided in FIG. 5, a gelled topical gel formulation of Compound **2.003** or the gel topical formulation of vehicle only were topically applied to the surface of a birthmark explant at 4 hours for analysis. The gel formulation was 1.5% hydroxypropyl cellulose in the base formulation and the tested compound was 0.01% by weight of the base formulation. The Compositions of the base formulation are listed below. Samples were harvested at 4 hours for analysis. Harvested tissue was bisected and with half snap frozen and half fixed in 10% formalin and paraffin embedded for further analysis.

| **Gel Formulation** | |
|---|---|
| **Ingredient** | **wt/wt%** |
| Ethanol | 46.0 |
| Propylene glycol | 15.0 |
| Capric/caprylic triglyceride | 20.0 |
| Diisopropyl adipate | 10.0 |
| Benzyl alcohol | 2.0 |
| Oleyl alcohol | 5.0 |
| Polysorbate 20 | 2.0 |
| Total weight of the base formulation | 100 |
| wt% Compound No. 2.003 | 0.01 |
| HPC-HY 119 | 1.50 |

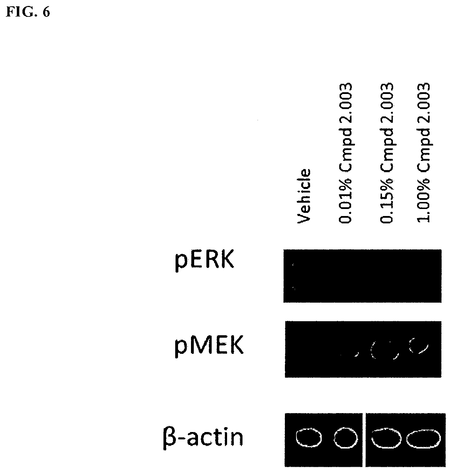

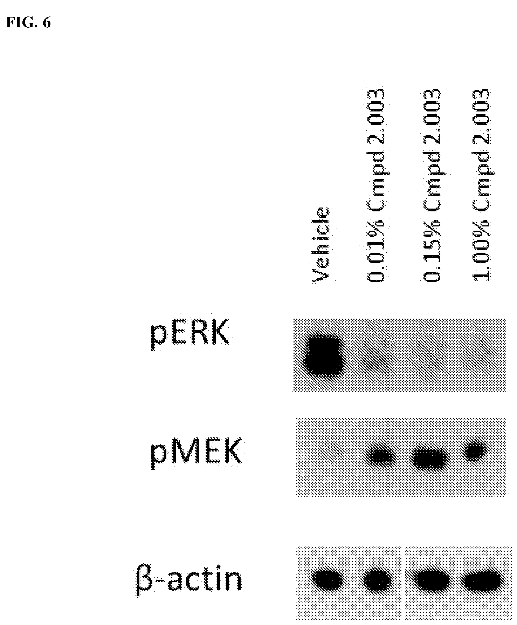

For the data provided in FIG. 6, three gelled topical gel formulations of Compound 2.003 at different concentrations or the gel topical formulation of vehicle only were topically applied to the surface of a birthmark explant at 4 hours for analysis. The gel formulation was 1.5% hydroxypropyl cellulose in the base formulation as shown above and the tested compound was 0.01%. 0.15%, or 1.00% by weight of the base formulation. Samples were harvested at 4 hours for analysis. Harvested tissue was bisected and with half snap frozen and half fixed in 10% formalin and paraffin embedded for further analysis.

**Western Blot analysis**: For immunoblotting, total skin biopies were lysed in lysis buffer and run on Western blots. Antibodies used for immunoblotting included rabbit anti-phospho-p44/42 MAPK (1:3000, Cell Signaling) and rabbit anti-p44/42 MAPK (1:3000, Cell Signaling), rabbit anti-phospho-Mek1/2 (1: 3000, Cell Signaling), mouse anti-actin (1:5000, Sigma-Aldrich), donkey anti-mouse IgG conjugated to horseradish peroxidase (HRP; 1:40,000, Amersham Biosciences) and goat anti-rabbit IgG conjugated HRP (1 :40,000, Jackson ImmunoResearch).

**Immunohistochemistry:** Immunohistochemistry was performed on 5 µm paraffin sections. Antigen retrieval with enzyme treatment (1:1000) using standard protocols. Antibodies used were rabbit p-ERK (Cell Signaling, 4307S, 1:100). Bond Polymer Refine anti-rabbit HRP Detection (Leica Biosystems) was used according to manufacturer's protocol. Sections were then counterstained with hematoxylin, dehydrated and film coverslipped using a TissueTek-Prisma and Coverslipper (Sakura).

**Data Analysis:** Semi-quantative Western blot was used to assess differences in p-ERK in samples treated with a compound described herein compared to vehicle control.

**Study Management:** The study was conducted with oversight from an IRB with patient informed consent and HIPAA authorization.

### Example 2: MEK Inhibition Assay-1

The following procedure can be used to measure biochemical activity. MEK1 inhibitory activity of compounds were tested using the following procedure. *See* Anastassiadis T, et al. Comprehensive assay of kinase catalytic activity reveals features of kinase inhibitor selectivity. Nat Biotechnol. 2011, 29(11), 1039-45.

### Reagents:

Reaction buffer: 20 mM Hepes (pH 7.5), 10 mM MgCl₂, 1 mM EGTA, 0.02% Brij35, 0.02 mg/mL BSA, 0.1 mM Na₃VO₄, 2 mM DTT, 1% DMSO
Enzyme: MEK1, Invitrogen cat# PV3303
N-terminal His-tagged recombinant human full length protein, expressed in insect cells. Activated in vitro by RAF I. MW=49.2 kDa, GenBank Accession No. NP_002746.
Substrate: 5 µM ERK2 (K52R),
Kinase-dead mutant, (GenBank Accession No. NM_0011949), aa2-358 with N-terminal His6 tag, MW=43.63 kDa, expressed in E.coli.

The substrate was prepared in freshly prepared Reaction Buffer. The kinase was delivered into the substrate solution and gently mixed. Test compounds were delivered in 100% DMSO into the kinase reaction mixture by Acoustic technology (Echo550; nanolitter range), and incubated for 20 min at room temperature. ³³P-ATP was delivered into the reaction mixture to initiate the reaction. The reaction mixture was incubated for 2 hours at room temperature. Kinase activity was detected by P81 filter-binding method.

### Example 3: MEK Inhibition Assay-2

MEK1 inhibitory activity of compounds were tested using the following procedure (protocol available at thermofisher.com/content/dam/LifeTech/migration/files/drug-discovery/pdfs.par.60256.file.dat/20130430%20ssbk%20customer%20protocol%20and%20assa y%20conditions.pdf). The Z'-LYTE biochemical assay (ThermoFisher) employs a fluorescence-based, coupled-enzyme format and is based on the differential sensitivity of phosphorylated and non-phosphorylated peptides to proteolytic cleavage.

Test compounds in 100% DMSO were screened in 1% DMSO (final) in the well. For 10 point titrations, 3-fold serial dilutions are conducted from the starting concentration of 30 µM.

The peptide/kinase, MAP2K1 (MEK1) / inactive MAPK1 (ERK2) / Ser/Thr 03, mixture ("Peptide/kinase Mixture") was diluted to a 2X working concentration in the following buffer ("Kinase Buffer"): 50 mM HEPES pH 7.5, 0.01% BR1J-35, 10 mM MgCl2, 1 mM EGTA. The final 10 µL kinase reaction consisted of 0.06 - 0.25 ng MAP2K1 (MEK1), 105 ng inactive MAPK1 (ERK2), and 2 µM Ser/Thr 03 in 50 mM HEPES pH 7.5, 0.01% BRIJ-35, 10 mM MgCl₂, 1 mM EGTA. After the 1 hour incubation, 5 µL of a 1:1024 dilution of Development Reagent A (available from Invitrogen, catalog no. PV3295) was added.

ATP solutions were diluted to a 4X working concentration in Kinase Buffer (50 mM HEPES pH 7.5, 0.01% BRIJ-35, 10 mM MgCl2, 1 mM EGTA). ATP Km apparent was previously determined using a Z'-LYTE assay. The Development Reagent was diluted in Development Buffer (available from Invitrogen, catalog no. P3127).

Assay Protocol: 2.5 µL of 4X test compound or 100 nL of 100X Test Compound plus 2.4 µL Kinase Buffer, 5 µL of the 2X Peptide/Kinase Mixture, 2.5 µL of 4X ATP Solution were added to the plates and placed on a shake plate for 30-seconds. The kinase reaction was allowed to proceed for 60-minute at room temperature, before 5 µL of Development Reagent Solution was added, and the mixture agitated for 30-seconds on a shake plate. The mixture was incubated for 60-minute at room temperature. Fluorescence was measured using a plate reader and the data were analyzed.

The maximum emission ratio was established by the 0% Phosphorylation Control (100% Inhibition Control), which contained no ATP and therefore exhibited no kinase activity. This control yielded 100% cleaved peptide in the Development Reaction. The 100% Phosphorylation Control, which consisted of a synthetically phosphorylated peptide of the same sequence as the peptide substrate, was designed to allow for the calculation of percent phosphorylation. This control yielded a very low percentage of cleaved peptide in the Development Reaction. The 0% Phosphorylation and 100% Phosphorylation Controls allow for the calculation of the percent phosphorylation achieved in a specific reaction well. Control wells did not include any kinase inhibitors.

The minimum emission ratio in a screen was established by the 0% Inhibition Control, which contained active kinase. This control was designed to produce a 10-50% phosphorylated peptide in the Kinase Reaction. Cascade assays may produce up to 70% phosphorylated peptide.

A known inhibitor control standard curve, 10 point titration, was run for each individual kinase on the same plate as the kinase to ensure the kinase was inhibited within an expected IC₅₀ range previously determined.

The following controls are prepared for each concentration of Test Compound assayed. The Development Reaction Interference was established by comparing the Test Compound Control wells that did not contain ATP versus the 0% Phosphorylation Control (which did not contain the Test Compound). The expected value for a non-interfering compound should be 100%. Any value outside of 90% to 110% was flagged. The Test Compound Fluorescence Interference was determined by comparing the Test Compound Control wells that did not contain the Kinase/Peptide Mixture (zero peptide control) versus the 0% Inhibition Control. The expected value for a non-fluorescence compound should be 0%. Any value > 20% was flagged.

The data in Table A was calculated. XL *fit* from IDBS was used. The dose response curve was curve fit to model number 205 (sigmoidal dose-response model). If the bottom of the curve did not fit between -20% & 20% inhibition, it was set to 0% inhibition. If the top of the curve did not fit between 70% and 130% inhibition, it was set to 100% inhibition.

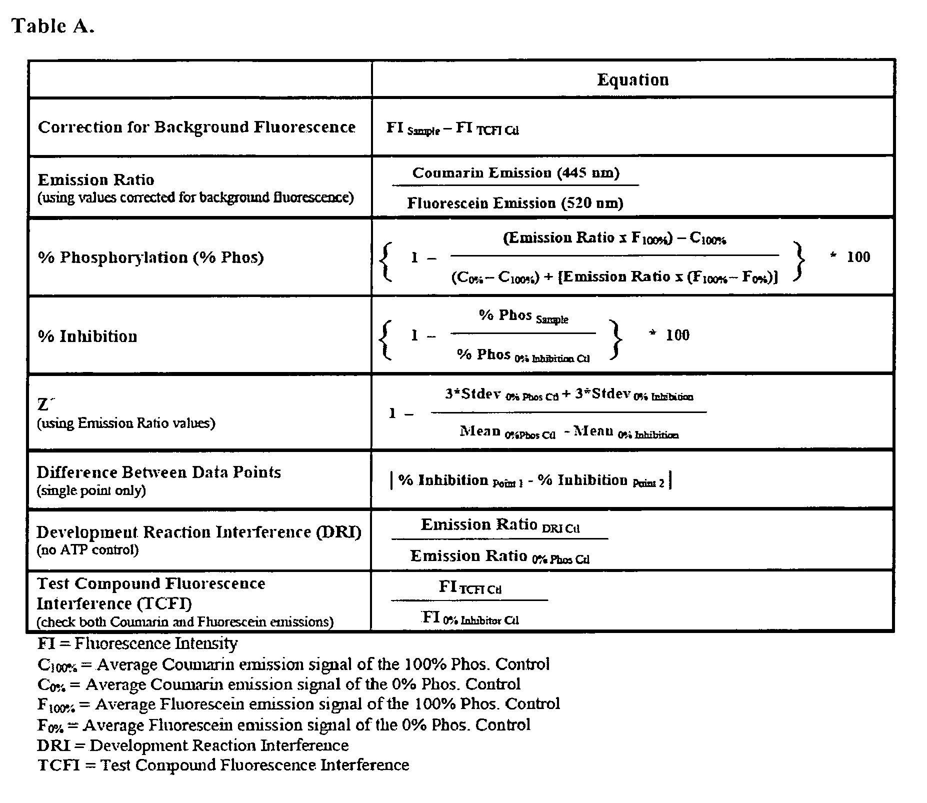

Table 6 lists the MEK1 inhibition assay results of selected compounds according to the above procedure. A indicates an IC₅₀ of less than or equal to 150 nM, B indicates an IC₅₀ of greater than 150 nM and less than or equal to 1.5 µM, and C indicates an IC₅₀ of greater than 1.5 µM.

**Table 6: MEK Inhibition Assay Results**

| **Compound No.** | **IC₅₀ against MEK1** | **Compound No.** | **IC₅₀ against MEK1** | **Compound No.** | **IC₅₀ against MEK1** |
|---|---|---|---|---|---|
| **1.001** | B | **4.013** | B | **5.001** | A |
| **1.002** | C | **4.014** | B | **5.002** | A |
| **1.007** | B | **4.015** | B | **5.003** | B |
| **1.008** | B | **4.029** | A | **5.004** | C |
| **1.009** | B | **4.030** | A | **5.005** | C |
| **1.010** | A | **4.032** | A | **5.006** | B |
| **1.011** | B | **4.033** | A | **5.007** | B |
| **1.012** | B | **4.034** | A | **5.008** | ND |
| **1.013** | B | **4.035** | A | **5.009** | ND |
| **1.017** | A | -- | -- | **5.010** | B |
| **1.018** | B | -- | -- | **5.011** | B |
| **1.022** | C | -- | -- | **5.012** | B |
| **1.030** | A | -- | -- | -- | -- |
| **1.031** | A | -- | -- | -- | -- |

| | | | | | |
|---|---|---|---|---|---|
| ND - not determined | | | | | |

### Example 4: Cell-Based Assay-1

Preparation of cell lines useful for testing the soft MEK inhibitors in NF1 related cell-proliferation assays can be found in Basu et al. Nature 356: 713-715, 1992; and DeClue et al. Cell 69: 265-273, 1992. In addition, exemplary *in vitro* and *in vivo* models to determine efficacy of the soft MEK inhibitors described herein can be found in U.S. Patent Nos. 8,21 1 ,875 and 8,487,004, which are incorporated by reference in their entireties.

### Example 5: Cell-Based Assay-2

Alternatively, the following procedure can be used to measure cell-based activity. Test compounds were dissolved in DMSO in 10 mM stock. Cell Titer-Glo^{®} 2.0 Luminescent cell viability assay reagent was purchased from Promega (Madison, W1). A375 and HCT116 cell lines were purchased from American Type Culture Collection (Manassas, VA). For A375 cells, cell culture media was DMEM + 10%FBS. Cell culture media are listed in the following table. For HCT116 cells, cell culture media was McCoy's 5A + 10%FBS. All media were supplemented with 100 µg/mL of penicillin, and 100 µg/mL of streptomycin. Cultures were maintained at 37 °C in a humidified atmosphere of 5% CO₂ and 95% air.

Test compounds were diluted in DMSO solution with 10-dose and 3-fold dilutions in a source plate starting at 10 mM. 25 nL of each test compound was delivered from the source plate to each well of the 384-well cell culture plates (T = Final) by Echo 550. 25 µL of culture medium containing 2000 of A375 or HCT116 cells was added to each of the wells in duplicates of the cell culture plates (T = 0 and T = Final). 25 µL of Cell Titer Glo 2.0 reagent was added to each well of cell culture plate (T = 0). The contents were mixed on an orbital shaker for 2 min and incubated at room temperature for 15 min to stabilize luminescent signal. Luminescence was recorded by Envision 2104 Multilabel Reader (PerkinElmer, Santa Clara, CA). The number of viable cells in culture was determined based on quantitation of the ATP present in each culture well. The cells in cell culture plate (T = Final) were incubated with the compounds at 37 °C, 5% CO₂ for 72 hours. 25 µL of Cell Titer Glo 2.0 reagent was added to each well. The contents were mixed on an orbital shaker for 2 min and incubated at room temperature for 15 min to stabilize luminescent signal. Luminescence was recorded by Envision 2104 Multilabel Reader (PerkinElmer, Santa Clara, CA). The number of viable cells in culture was determined based on quantitation of the ATP present in each culture well. The GI₅₀ curves were plotted using the GraphPad Prism 4 program based on a sigmoidal dose-response equation Y=Bottom + (Top-Bottom)/(1+10^((LogEC50-X)^{∗}HillSlope)). All parameters in the equation were calculated by GraphPad Prism 4 program. G1₅₀ is the concentration of the compound calculated according to [(Tᵢ - T_{z})/(C - T_{z})] ^{∗} 100 = 50 where Tᵢ is the row data of cells with test compounds at T = Final; T_{z} is the row data of cells without compounds at T = 0 h; C is the row data of cells with control compound staurosporine (Sigma-Aldrich) at T = 72 h. Accordingly, GI₅₀ is the value of 10^{X}, where X was calculated by the Curve Fitting Equation when Y = 50 using Excel.

### Example 6: Assay Results of Tested Compounds

### Tested Compounds of formula (1)

The following applies to Table 7 below for tested compounds of formula (1).

Assay 1 is the biochemical MEK IC₅₀ (nM) assay as described in Example 2. A1 indicates an IC₅₀ of less than or equal to 150 nM, and B1 indicates an IC₅₀ of greater than 150 nM and less than or equal to 1.5 µM.

Assay 2 is the A375 (BRAF) GI₅₀ (nM) cell-based assay as described in Example 5. A2 indicates an IC₅₀ of less than or equal to 500 nM, and B2 indicates an IC₅₀ of greater than 500 nM and less than or equal to 1.5 µM.

Assay 3 is the HCT116 (Kras) GI₅₀ (nM) assay as described in **Example 5.** A3 indicates an IC₅₀ of less than or equal to 750 nM, and B3 indicates an IC₅₀ of greater than 750 nM and less than or equal to 2 µM.

**Table 7: Assay Results of Tested Compounds of formula (1)**

| **Comp. No.** | **Assay** | | |
|---|---|---|---|
| | **1** | **2** | **3** |
| **1.007** | B1 | B2 | B3 |
| **1.008** | A1 | B2 | B3 |
| **1.009** | B1 | -- | -- |
| **1.010** | A1 | A2 | A3 |
| **1.030** | A1 | A2 | A3 |
| **1.031** | A1 | A2 | B3 |

### Tested Compounds of formula (II)

The following applies to the Table 8 below for tested compounds of formula (II). NT indicates that the compound was not tested in a particular assay. Assay 1 is the biochemical MEK IC₅₀ (nM) assay as described in **Example 2** and as used for all tested compounds except compounds 2.042, 2.044, 2.047, and 2.048 which were tested using **Example 3.** Assay 2 is the A375 (BRAF) G1₅₀ (nM) cell-based assay as described in **Example 5.** Assay 3 is the HCT116 (Kras) GI₅₀ (nM) assay as described in **Example 5.**

**Table 8: Assay Results of Tested Compounds of formula (II)**

| **Comp. No.** | **Assay** | | |
|---|---|---|---|
| | **1** | **2** | **3** |
| **2.003** | 246 | 63 | 196 |
| **2.004** | 260 | 346 | 346 |
| **2.006** | 371 | 81 | 448 |
| **2.015** | 2450 | NT | NT |
| **2.039** | 671 | 809 | 5006 |
| **2.042** | 1770 | NT | NT |
| **2.044** | 1780 | NT | NT |
| **2.047** | 243 | NT | NT |
| **2.048** | 9610 | NT | NT |

### Tested Compounds of formula (111)

The following applies to the table 9 below for tested compounds of formula (I1I). NT indicates that the compound was not tested in a particular assay. Assay 1 is the biochemical MEK IC₅₀ (nM) assay as described in **Example 2** and as used for compounds 2 and 6; compounds 1, 3, 5, and 7-8 were tested using **Example 3.** Assay 2 is the A375 (BRAF) GI₅₀ (nM) cell-based assay as described in **Example 5.** Assay 3 is the HCT116 (Kras) GI₅₀ (nM) assay as described in **Example 5.**

**Table 9: Assay Results of Tested Compounds of formula (I1I)**

| **Comp. No.** | **Assay** | | |
|---|---|---|---|
| | **1** | **2** | **3** |
| **3.019** | 1030 | 141 | 348 |
| **3.020** | 915 | 145 | 257 |
| **3.021** | 256 | NT | NT |
| **3.022** | 74 | NT | NT |
| **3.023** | 150 | NT | NT |
| **3.024** | 2910 | NT | NT |
| **3.026** | 256 | NT | NT |

### Tested Compounds of formula (IV)

The following applies to Table 10 below for tested compounds of formula (IV).

Assay 1 is the biochemical MEK IC₅₀ (nM) assay as described in **Example 2.** A1 indicates an IC₅₀ of less than or equal to 500 nM; B1 indicates an IC₅₀ of greater than 500 nM and less than or equal to 1 µM; C1 indicates an IC₅₀ of greater than 1 µM and less than or equal to 5 µM; and Dl indicates an IC₅₀ of greater than 5 µM and less than or equal to 10 µM.

Assay 2 is the A375 (BRAF) GI₅₀ (nM) cell-based assay as described in **Example 5.** A2 indicates an IC₅₀ of less than or equal to 1 µM, and B2 indicates an IC₅₀ of greater than 1 µM and less than or equal to 2 µM.

Assay 3 is the HCT116 (Kras) GI₅₀ (nM) assay as described in **Example 5.** A3 indicates an IC₅₀ of less than or equal to 5 µM, and B3 indicates an IC₅₀ of greater than 5 µM and less than or equal to 10 µM.

**Table 10: Assay Results of Tested Compounds**

| **Comp. No.** | **Assay** | | |
|---|---|---|---|
| | **1** | **2** | **3** |
| **4.013** | A1 | A2 | A3 |
| **4.016** | B1 | B2 | A3 |
| **4.017** | B1 | B2 | A3 |
| **4.018** | D1 | -- | -- |
| **4.019** | ND | -- | -- |
| **4.020** | C1 | A2 | B3 |
| **4.021** | B1 | -- | -- |
| **4.022** | A1 | -- | -- |
| **4.023** | A1 | A2 | B3 |
| **4.024** | C1 | -- | -- |
| **4.029** | A1 | A2 | A3 |
| **4.032** | A1 | A2 | A3 |

### Example 7: In Vivo Model

Study Procedures: A topical formulation of a compound described herein along with a topical formulation of vehicle are applied to the skin of nude mice in duplicate. Skin is biopsied at discrete time intervals and bisected with half snap frozen in liquid nitrogen and half formalin fixed and paraffin embedded. Protein is isolated for Western blot analysis for p-ERK levels. p-ERK immunostaining is performed of FFPE sections for cell-specific analysis of p-ERK levels. Additional analysis includes H&E staining to investigate skin integrity.

A compound is assessed in suppressing p-ERK, a downstream biomarker of RAS/MAPK signaling in murine skin. In addition, proliferation of murine skin, apoptosis in murine skin, and histologic integrity of murine skin are also assessed.

Mice: 8 week old 129 mice obtained from Jackson laboratories are shaved prior to start of study. Approximately 21 mice were used for study. A compound is applied to the hairless dorsal skin of the mouse and at 12 hour intervals and skin biopsies are obtained prior to treatment, 24 hours, 72 hours and at 96 hours using 6mm punch biopsies.

Western Blot analysis: For immunoblotting, epidermal skin is snap frozen in liquid nitrogen immediately afterbiopsy. The epidermis is lysed in lysis buffer and run on Western blots. Antibodies used for immunoblotting include rabbit anti-phospho-p44/42 MAPK (1:3000, Cell Signaling) and rabbit anti-p44/42 MAPK (1:3000, Cell Signaling), mouse anti-actin (1:5,000, Sigma-Aldrich), donkey anti-mouse IgG conjugated to horseradish peroxidase (HRP; 1:40,000, Amersham Biosciences) and goat anti-rabbit 1gG conjugated HRP (1:40,000, Jackson ImmunoResearch).

Immunohistochemistry: Immunohistochemistry is performed on 5 µm paraffin sections. Antigen retrival is accomplished with enzyme treatment (1:1000) using standard protocols. Antibodies used are rabbit p-ERK (Cell Signaling, 4307S, 1:100). Bond Polymer Refine anti-rabbit HRP Detection (Leica Biosystems) is used according to manufacturer's protocol. Sections are then counterstained with hematoxylin, dehydrated and film coverslipped using a TissueTek-Prisma and Coverslipper (Sakura).

Histologic analysis: H&E is performed on 5 µM paraffin sections and tissue is examined to assess for cellular toxicity, inflammation or other changes in the integrity of murine skin.

Exogenous RAS activation in murine skin: The experiments are to be conducted in untreated murine skin. Alternatively, skin is pre-treated with TPA to enhance p-ERK levels. TPA-induced RAS/MAPK activation is performed with 96 hours of 12.5uG TPA in 100 µL acetone to the skin of nude mice. Studies are performed 48 hours after TPA exposure.

T-test is used to assess differences in p-ERK and Ki-67 in samples treated with topical MEK1 inhibitors compared to vehicle control.

### Example 8: 4-cyano-2-((2-fluoro-4-iodophenyl)amino)benzoic acid

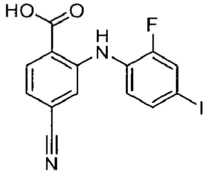

A solution of 4-cyano-2-fluorobenzoic acid (3.0 g, 18.1 mmol) in THF (30 mL) stirred under N₂ at -78 C was treated with LDA (2.0 M in THF, 27.2 mL, 54.5 mmol) added dropwise. After 20 min a solution of 2-fluoro-4-iodoaniline (12.9 g, 54.5 mmol) in dry THF (15 mL) was added dropwise and the reaction mixture was further stirred allowing it to warm up to room temperature. After 16 h the reaction mixture was concentrated *in vacuo,* acidified with 1M HCl and extracted twice with Et₂O. The combined organic phase was washed with brine, dried over Na₂SO₄, filtered, and concentrated *in vacuo.* The crude residue was purified by trituration in boiling DCM to give the product (1.57 g, 22.7%) as a yellow solid. *m*/*z* 381.1 [M-H]⁻. ¹H NMR (400 MHz, DMSO-*d*₆) δ ppm 13.91 (s, 1H), 9.74 (s, 1H), 8.04 (d, *J*=8.2 Hz, 1H), 7.77 (dd, *J*=10.3, 2.0 Hz, 1H), 7.58 (dt, *J*=8.5, 1.3 Hz, 1H), 7.42 - 7.33 (m, 2H), 7.24 (dd, *J*=8.2, 1.6 Hz, 1H)

### Example 9: 4-cyano-2-((2-fluoro-4-iodophenyl)amino)benzoic acid

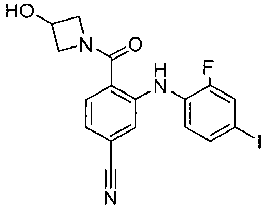

A microwave vial was charged with 4-cyano-2-((2-fluoro-4-iodophenyl)amino)benzoic acid (80% pure) (0.19 g, 0.4 mmol), 3-hydroxyazetidine hydrochloride (0.07 g, 0.6 mmol), HATU (0.25 g, 0.6 mmol) and diisopropyl ethyl amine (140 µL, 0.8 mmol) and *N,N-*dimethylformamide (6 mL). The reaction mixture was stirred at room temperature overnight. The reaction was quenched with water and extracted with ethyl acetate. The organics were washed with water, brine and dried over sodium sulfate. The solvents were evaporated. The residue was purified by flash chromatography (12 g silica, 0-5% methanol in dichloromethane) and again by reverse phase HPLC (30-95% Acetonitrile/water) to give the product as a light yellow solid (71 mg, 41%). *mlz* 438.1 [M+1]⁺. ¹H NMR (300 MHz, DMSO-d6): δ 8.97 (s, 1H), 7.69-7.65 (dd, J=1.2 Hz and 0.9Hz, 1H), 7.55-7.47 (m, 2H), 7.40 (s, 1H), 7.28-7.18 (m, 2H), 5.80 (s, 1H), 4.45 (s, 1H), 4.35 (t, J=7.6 Hz, 1H), 4.21 (t, J=8.9 Hz, 1H), 4.00-3.98 (m, 1H), 3.76-3.73 (m, 1H).

The following compounds were prepared as described in Example 9, replacing the 3-hydroxyazetidine hydrochloride with an appropriate amine which is commercially available or prepared using conditions known to one of ordinary skill in the art.

| **Ex. No.** | **Comp. No.** | **Structure** | **1H NMR (400 MHz, DMSO-d6)** | ***m*/*z*** |
|---|---|---|---|---|
| **10** | 1.003 | 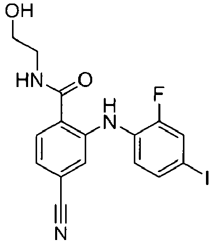 | δ 7.75 (d, J=8.1 Hz,1H), 7.59-7.50 (m, 2H), 7.37 (s, 1H), 7.23-7.17 (m, 2H), 3.70 (t, J=5.9 Hz, 2H), 3.48 (t, J=5.6 Hz, 2H). (CD3OD) | 424.2 [M-1]⁻ |
| **11** | 1.004 | 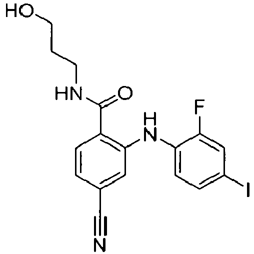 | δ 7.69 (d, J=8.1 Hz, 1H), 7.59-7.50 (m, 2H), 7.38 (s, 1H), 7.23-7.17 (m, 2H), 3.64 (t, J=6.2 Hz, 2H), 3.46 (t, J=7.1 Hz, 2H), 1.84-1.79 (m, 2H). (CD3OD) | 438.2 [M-1]⁻ |
| **12** | 1.005 | 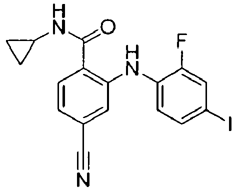 | δ 9.78 (s, 1H), 8.82 (d, J=4.2 Hz, 1H), 7.74-7.67 (m, 2H), 7.50 (d, J=8.1 Hz, 1H), 7.41 (s, 1H), 7.30-7.25 (m, 2H), 2.85-2.81 (m, 1H), 0.73-0.66 (m, 2H), 0.59-0.56 (m, 2H). | 420.2 [M-1]⁻ |
| **13** | 1.006 | 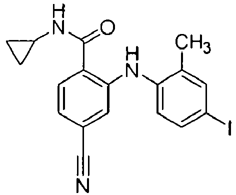 | δ 9.65 (s, 1H), 8.78 (s, 1H), 7.72 (d, J=8.4 Hz, 1H), 7.64 (s, 1H), 7.51 (d, J=8.4 Hz, 1H), 7.25 (s, 1H), 7.21-7.18 (m, 1H), 7.11 (d, J=8.1 Hz, 1H), 2.84-2.82 (m, 1H), 2.16 (s, 3H), 0.71-0.67 (m, 2H), 0.59-0.56 (m, 2H). | 416.2 [M-1]⁻ |
| **14** | 1.007 | 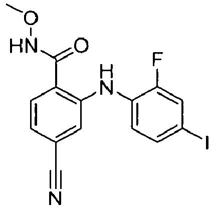 | δ 12.05 (s, 1H), 9.26 (s, 1H), 7.69 (d, J=10.5 Hz, 1H), 7.62 (d, J=7.5 Hz, 1H), 7.50 (d, J=8.7 Hz, 1H), 7.40 (s, 1H), 7.31-7.26 (m, 2H), 3.69 (s, 3H). | 410.2 [M-1]⁻ |
| **15** | 1.008 | 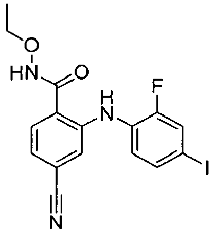 | δ 11.94 (s, 1H), 9.25 (s, 1H), 7.71-7.67 (m, 2H), 7.49 (d, J=8.1 Hz,1H), 7.40 (s, 1H), 7.32-7.23 (m, 2H), 3.94-3.86 (q, 2H), 1.18 (t, J=7.1 Hz, 3H). | 424.2 [M-1]⁻ |
| **16** | 1.009 | 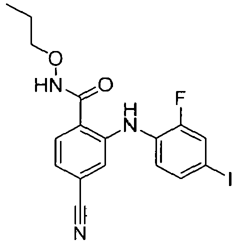 | δ 11.93 (s, 1H), 9.23 (s, 1H), 7.69 (d, J=10.5 Hz, 1H), 7.62 (d, J=7.5 Hz, 1H), 7.49 (d, J=8.7 Hz, 1H), 7.39 (s, 1H), 7.32-7.25 (m, 2H), 3.81 (t, J=6.5 Hz, 2H), 1.62-1.55 (m, 2H), 0.92 (t, J=7.2 Hz, 3H). | 438.2 [M-1]⁻ |
| **17** | 1.010 | 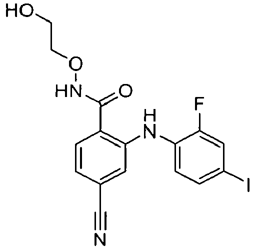 | δ 7.74-7.66 (m, 2H), 7.50-7.47 (m, 1H), 7.39 (s, 1H), 7.27 (t, J=7.4 Hz, 2H), 3.90 (t, J=4.7 Hz, 2H), 3.60 (t, J=4.8 Hz, 2H). | 440.1 [M-1]⁻ |
| **18** | 1.011 | 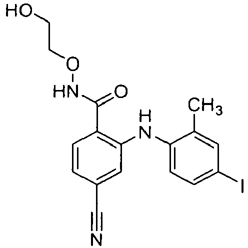 | δ 12.01 (s, 1H), 9.09 (s, 1H), 7.64-7.61 (m, 2H), 7.52 (d, J=8.4 Hz, 1H), 7.22-7.19 (m, 2H), 7.10 (d, J=8.7 Hz, 1H), 3.90 (t, J=4.7 Hz, 2H), 3.61 (t, J=5.2 Hz, 2H), 2.15 (s, 3H) | 436.2 [M-1]⁻ |
| **19** | 1.012 | 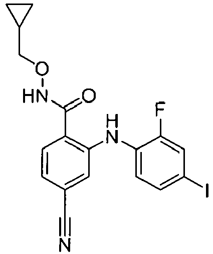 | δ 12.01 (s, 1H), 9.24 (s, 1H), 7.70-7.61 (m, 2H), 7.49 (d, J=8.1 Hz, 1H), 7.38 (s, 1H), 7.3 1-7.22 (m, 2H), 3.68 (d, J=7.2 Hz, 2H), 1.07-1.00 (m, 1H), 0.52-0.49 (m, 2H), 0.25-0.24 (m, 2H) | 450.2 [M-1]⁻ |
| **20** | 1.013 | 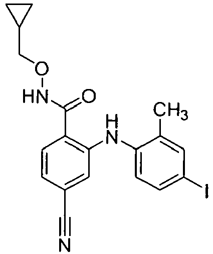 | δ 11.92 (s, 1H), 9.08 (s, 1H), 7.64-7.58 (m, 2H), 7.51 (d, J=8.4 Hz, 1H), 7.21-7.19 (m, 2H), 7.09 (d, J=9.0 Hz, 1H), 3.68 (d, J=8.4 Hz, 2H), 2.14 (s, 3H), 1.08-1.06 (m, 1H), 0.53-0.50 (m, 2H), 0.26-0.23 (m, 2H) | 446.3 [M-1]⁻ |
| **21** | 1.014 | 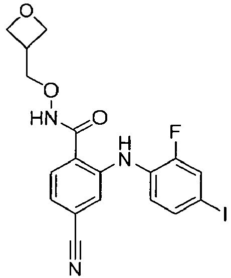 | δ 12.03 (s, 1H), 9.18 (s, 1H), 7.71-7.68 (dd, J=1.8 Hz and 1.8 Hz, 1H), 7.61 (d, J=8.1 Hz, 1H), 7.50 (d, J=8.7 Hz, 1H), 7.39 (s, 1H), 7.32-7.22 (m, 2H), 4.64 (t, J=6.8 Hz, 2H), 4.36 (t, J=6.0 Hz, 2H), 4.10 (d, J=8.4 Hz, 2H) | 466.2 [M-1]⁻ |
| **22** | 1.015 | 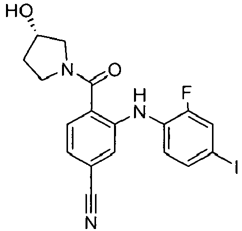 | δ 7.56-7.47 (m, 3H), 7.29-7.26 (m, 2H), 7.09-7.02 (m, 1H), 4.45-4.36 (m, 1H), 3.69-3.45 (m, 4H), 2.07-1.95 (m, 2H). (CD3OD) | 452.1 [M+1]⁺ |

### Example 23: N-(azetidin-3-ylmethoxy)-4-cyano-2-((2-fluoro-4-iodophenyl)amino)benzamide

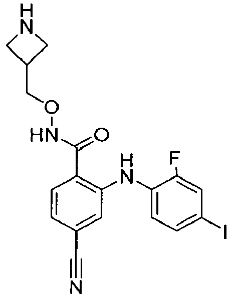

### Step 1: tert-butyl 3-(((4-cyano-2-((2-fluoro-4-iodophenyl)amino)benzamido)oxy)methyl) azetidine-1-carboxylate

A microwave vial was charged with 4-cyano-2-((2-fluoro-4-iodophenyl)amino)benzoic acid (0.100 g, 0.3 mmol), tert-butyl 3-((aminooxy)methyl)azetidine-1-carboxylate, (60% pure) (0.13 g, 0.4 mmol), HATU (0.15 g, 0.4 mmol) and diisopropyl ethyl amine (135 µL, 0.8 mmol) in *N*,*N*-dimethylformamide (3 mL). The reaction mixture was stirred at room temperature overnight. The reaction was quenched with water and extracted with ethyl acetate. The organics were washed with water, brine and dried over sodium sulfate. The solvents were evaporated. The residue was purified by flash chromatography (12 g silica, 0-70% ethyl acetate in hexanes). The product fractions were collected and the solvent was removed to give a yellow oil (77 mg, *52%). m*/*z* 565.2 [M-1]⁻.

### Step 2: N-(azetidin-3-vlmethoxv)-4-cyano-2-((2-fluoro-4-iodophenyl)amino)benzamide

A round bottom flask was charged with tert-butyl 3-(((4-cyano-2-((2-fluoro-4-iodophenyl)amino)benzamido)oxy)methyl)azetidine-1-carboxylate (0.077 g, 0.1 mmol) in dichloromethane (6 mL) and hydrogen chloride (4.0 M in 1,4-dioxane, 0.510 ml, 2.0 mmol) was added dropwise. The reaction mixture was stirred at room temperature overnight. The reaction mixture was concentrated in vacuo. The residue was purified by reverse phase HPLC ((10-95% Acetonitrile/water (0.1%TFA)). The product fractions were collected, washed with sodium bicarbonate and dried in vacuo to give the product as a yellow solid. *m*/*z* 461.7 [M+1]⁺. ¹H NMR (300 MHz,CDCl3): δ 8.41 (s, 1H), 7.67 (d, J=8.1 Hz, 1H), 7.50-7.42 (m, 2H), 7.35 (s, 1H), 7.12-7.03 (m, 1H), 4.16-4.10 (m,1H), 4.06-4.00 (m,1H), 3.94-3.90 (m, 1H), 3.73-3.68 (m, 1H), 2.89-2.79 (m, 3H).

### Example 24: 4-Cyano-2-((2-fluoro-4-iodophenyl)amino)-N-hydroxybenzamide

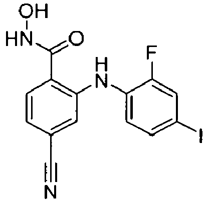

A solution of hydroxylamine hydrochloride (333 mg, 0.52 mmol) in dry DMF (2.4 mL) and dry acetonitrile (2.4 mL) stirred at room temperature was treated with Et₃N (1.33 mL, 9.6 mmol). After 1 h the suspension was diluted with DCM (2.4 mL), cooled down to 0°C and a solution of 4-cyano-2-((2-fluoro-4-iodophenyl)amino)benzoyl chloride (209 mg, 0.52 mmol) in dry THF (2.4 mL) was added. After 1 h the reaction mixture was diluted with EtOAc, quenched with a saturated NH₄Cl aqueous solution, partitioned, and the aqueous phase was extracted with EtOAc. The combined organic phase was washed with brine, dried over Na₂SO₄, filtered, and concentrated *in vacuo.* The crude material was purified by preparative HPLC purification to give the product (37 mg, 17.8%) as a yellow solid. *m*/*z* 396.1 [M-H]⁻. ¹H NMR (400 MHz, DMSO-*d*₆): δ ppm 11.60 (s, 1H), 9.46 (s, 1H), 9.41 (s, 1H), 7.72 (dd, *J*=10.5, 2.0 Hz, 1H), 7.64 (d, *J*=8.0 Hz, 1H), 7.56 - 7.49 (m, 1H), 7.46 (s, 1H), 7.37 - 7.28 (m, 2H).

### Example 25: 2-((4-Cyano-2-((2-fluoro-4-iodophenyl)amino)benzamido)oxy)ethan-1-aminium 2,2,2-trifluoroacetate

### Step 1: tert-Butyl (2-((4-cyano-2-((2-fluoro-4-iodophenyl)amino)benzamido)oxy)ethyl) carbamate

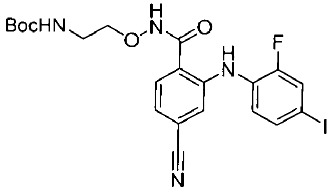

A solution of 4-cyano-2-((2-fluoro-4-iodophenyl)amino)benzoic acid (150 mg, 0.39 mmol) and HATU (298 mg, 0.78 mmol) in dry DMF (3.9 mL) was treated with dry DIPEA (0.13 mL, 0.78 mmol). The reaction mixture was stirred at 50°C for 30 min, cooled down to room temperature and a solution of *tert*-butyl (2-(aminooxy)ethyl)carbamate (103 mg, 0.58 mmol) in dry DMF (0.5 mL) was added. After 16 h the reaction mixture was diluted with EtOAc, quenched with H₂O, partitioned, and the aqueous phase was extracted with EtOAc. The combined organic phase was washed with brine, dried over Na₂SO₄, filtered and concentrated *in vacuo.* The crude residue was purified by a flash column chromatography (Silica, 1-40% EtOAc in hexanes) to give the product (87 mg, 41%) as a yellow oil. UPLC-MS (Acidic Method, 2 min): rt 1.27 min. *m*/*z* 539.1 [M-H]⁻. ¹H NMR (400 MHz, CDCl₃): δ ppm 10.56 (s, 1H), 9.45 (s, 1H), 7.59 (d, *J*=8.1 Hz, 1H), 7.57 - 7.46 (m, 2H), 7.32 (s, 1H), 7.16 - 7.07 (m, 2H), 5.06 (s, 1H), 4.01 (t, *J*=4.9 Hz, 2H), 3.54 - 3.45 (m, 2H), 1.49 (s, 9H).

### Step 2: 2-((4-Cyano-2-((2-fluoro-4-iodophenyl)amino)benzamido)oxy)ethan-1-aminium 2,2,2-trifluoroacetate

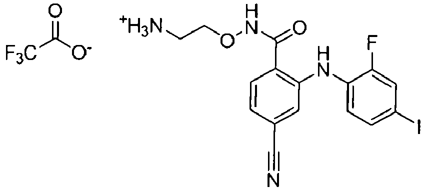

A solution of *tert*-butyl (2-((4-cyano-2-((2-fluoro-4-iodophenyl)amino)benzamido)oxy) ethyl)-carbamate (67 mg, 0.12 mmol) in dioxane (1.0 mL) stirred at room temperature was treated with 4N HCl in dioxane (62 µl, 0.24 mmol). After 1 h an additional portion of 4N HCl in dioxane (62 µl, 0.24 mmol) was added, similarly after 16 h. After 48 h a further portion of 4N HCl in dioxane (0.25 mL, 1 mmol) was added. After 48 h the reaction mixture was concentrated *in vacuo.* The crude material was purified by preparative HPLC purification to give the product (21.1 mg, 32%) as a yellow solid in a form of its trifluoroacetate salt. *m*/*z* 441.1 [M+H]⁺. ¹H NMR (400 MHz, DMSO-*d*₆): δ ppm 7.79 - 7.69 (m, 2H), 7.54 (dd, *J*=8.3, 1.9 Hz, 1H), 7.45 (d, *J*=1.5 Hz, 1H), 7.35 (dd, *J*=8.1, 1.5 Hz, 1H), 7.29 (t, *J*=8.6 Hz, 1H), 4.07 (t, *J*=5.1 Hz, 2H), 3.08 (t, *J*=5.2 Hz, 2H).

### Example 26: Methyl 2-((4-cyano-2-((2-fluoro-4-iodophenyl)amino)benzamido)oxy)acetate

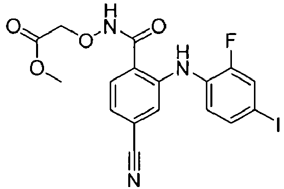

A solution of 4-cyano-2-((2-fluoro-4-iodophenyl)amino)benzoic acid (500 mg, 1.30 mmol) and HATU (995 mg, 2.61 mmol) in dry DMF (13.0 mL) was treated with dry DIPEA (0.45 mL, 2.61 mmol). The reaction mixture was stirred at 50°C for 30 min, cooled down to room temperature and a solution of methyl 2-(aminooxy)acetate hydrochloride (270 mg, 1.96 mmol) in dry DMF (1.0 mL) was added followed by dry DIPEA (0.34 mL, 2.0 mmol). After 48 h the reaction mixture was diluted with EtOAc, quenched with H₂O, partitioned, and the aqueous phase was extracted with EtOAc. The combined organic phase was washed with brine, dried over Na₂SO₄, filtered and concentrated *in vacuo.* The crude residue was purified by a flash column chromatography (Silica, 10-41% EtOAc in hexanes) to give the product (373 mg, 61%) as a yellow solid. *m*/*z* 468.1 [M-H]⁻. ¹H NMR (400 MHz, DMSO-*d*₆): δ ppm 12.29 (s, 1H), 9.16 (s, 1H), 7.76- 7.60 (m, 2H), 7.52 (dt, *J*=8.4, 1.4 Hz, 1H), 7.41 (s, 1H), 7.35 - 7.19 (m, 2H), 4.72 - 4.55 (m, 2H), 3.70 (s, 3H).

### Example 27: 2-((4-Cyano-2-((2-fluoro-4-iodophenyl)amino)benzamido)oxy)acetic acid

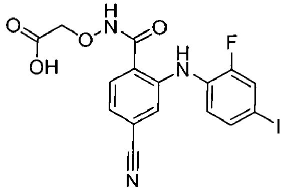

A solution of methyl 2-((4-cyano-2-((2-fluoro-4-iodophenyl)amino)benzamido)oxy) acetate (306 mg, 0.64 mmol) in THF (1.93 mL), MeOH (0.64 mL), and H₂O (1.28 mL) stirred at room temperature was treated with 1M LiOH (0.64 mL, 0.64 mmol). After 24 h an additional portion of 1M LiOH (0.64 mL, 0.64 mmol) was added and stirring continued. After 24 h the reaction mixture was diluted with a saturated NaHCO₃ aqueous solution, partitioned with EtOAc, and the aqueous phase was acidified with 1M HCl and extracted with EtOAc. The organic phase was washed with 1M HCl and brine, dried over NaHCO₃, filtered and concentrated in vacuo to give the product (238 mg, 81%) as a yellow solid. *m*/*z* 454.0 [M-H]⁻. ¹H NMR (400 MHz, DMSO-*d*₆): δ ppm 13.00 (s, 1H), 12.30 (s, 1H), 9.21 (s, 1H), 7.72 (dd, *J*=10.4, 1.9 Hz, 1H), 7.68 (s, 1H), 7.53 (dt, *J*=8.5, 1.4 Hz, 1H), 7.40 (d, *J*=1.6 Hz, 1H), 7.34 - 7.24 (m, 2H), 4.52 (s, 2H).

### Example 28: N-(2-Amino-2-oxoethoxy)-4-cyano-2-((2-fluoro-4-iodophenyl)amino) benzamide

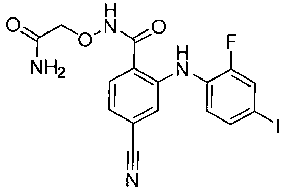

### Step 1: 2-((4-Cyano-2-((2-fluoro-4-iodophenyl)amino)benzamido)oxy)acetyl chloride

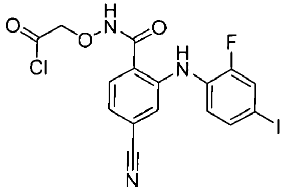

A solution of 2-((4-cyano-2-((2-fluoro-4-iodophenyl)amino)benzamido)oxy)acetic acid (181 mg, 0.39 mmol) in dioxane (1.5 mL) stirred at room temperature under N₂ was treated with SOCl₂ (0.3 mL, 3.9 mmol). After 24 h the reaction mixture was concentrated *in vacuo* and azeotroped with dry toluene (3 × 5 mL). The resultant orange solid was used in the subsequent reaction without further purification. *m*/*z* 468.1 [M+H]⁻ (detected as the corresponding methyl ester after quenching an aliquot of the mixture with 10% pyridine in MeOH).

### Step 2: N-(2-Amino-2-oxoethoxy)-4-cyano-2-((2-fluoro-4-iodophenyl)amino)benzamide

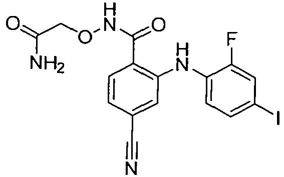

A solution of 2-((4-cyano-2-((2-fluoro-4-iodophenyl)amino)benzamido)oxy)acetyl chloride (188 mg, 0.39 mmol) in dry dioxane (0.9 mL) stirred at 0°C under N₂ was treated with 0.5 M NH₃ in dioxane (0.91 mL). After 48 h the reaction mixture was diluted with EtOAc, quenched with 1M HCl, partitioned and the organic phase was washed with brine, dried over Na₂SO₄, filtered and concentrated *in vacuo.* The crude material was purified by preparative HPLC purification to give the product (49.7 mg, 28%) as a yellow solid. *m*/*z* 453.0 [M-H]⁻. ¹H NMR (400 MHz, DMSO-*d*₆): δ ppm 12.29 (br s, 1H), 9.40 (br s, 1H), 7.74 - 7.69 (m, 2H), 7.54 - 7.48 (m, 1H), 7.40 (s, 1H), 7.34 - 7.24 (m, 2H), 4.34 (s, 2H).

### Example 29: Methyl 3-((4-cyano-2-((2-fluoro-4-iodophenyl)amino)benzamido)oxy) propanoate

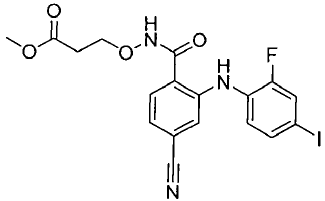

A solution of 4-cyano-2-((2-fluoro-4-iodophenyl)amino)benzoic acid (500 mg, 1.30 mmol) and HATU (995 mg, 2.61 mmol) in dry DMF (13.0 mL) stirred at room temperature was treated with dry DIPEA (0.45 mL, 2.61 mmol). The reaction mixture was stirred at 50°C for 30 min, cooled down to room temperature and a suspension of methyl 3-(aminooxy)propanoate hydrochloride (305 mg, 1.96 mmol) in dry acetonitrile (2.9 mL) and dry THF (2.9 mL) was added, followed by dry DIPEA (0.45 mL, 2.6 mmol). After 16 h the reaction mixture was diluted with EtOAc, quenched with H₂O, partitioned, and the aqueous phase was extracted with EtOAc. The combined organic phase was washed with brine, dried over Na₂SO₄, filtered and concentrated *in vacuo.* The crude residue was purified by a flash column chromatography (Silica, 20-50% EtOAc in hexanes) to give the product (369 mg, 59%) as a yellow solid. *m*/*z* 482.0 [M-H]⁻. ¹H NMR (400 MHz, DMSO-*d*₆): δ ppm 12.05 (s, 1H), 9.29 (s, 1H), 7.72 (dd, *J*=10.5, 1.9 Hz, 1H), 7.66 (s, 1H), 7.53 (d, *J*=8.6 Hz, 1H), 7.42 (d, *J*=1.7 Hz, 1H), 7.36 - 7.25 (m, 2H), 4.14 (m, 2H), 3.61 (s, 3H), 2.70 (t, *J*=6.6 Hz, 2H).

### Example 30: 3-((4-Cyano-2-((2-fluoro-4-iodophenyl)amino)benzamido)oxy)propanoic acid

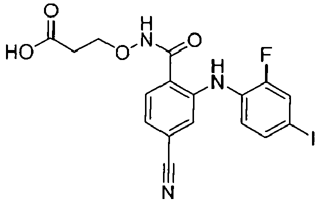

A solution of methyl 3-((4-cyano-2-((2-fluoro-4-iodophenyl)amino)benzamido)oxy) propanoate (321 mg, 0.66 mmol) in THF (4.0 mL), MeOH (1.33 mL), and H₂O (2.65 mL) stirred at room temperature was treated with 1 M LiOH (1.33 mL, 1.33 mmol). After 3 days an additional portion of 1 M LiOH (0.32 mL, 0.32 mmol) was added and stirring continued. After 3 h the reaction mixture was diluted with a saturated NaHCOs aqueous solution, partitioned with EtOAc, and the aqueous phase was acidified and extracted with EtOAc. The first EtOAc extract was washed with 1 M HCl and brine sequentially, dried over NaHCO₃, filtered and concentrated *in vacuo* to give the product (203 mg, 65.5% yield, 79% pure) as a yellow solid. The second EtOAc extract was washed with brine, dried over NaHCO₃, filtered and concentrated in vacuo to give the product (46 mg, 14.8% yield, 100% pure) as a yellow solid. *m*/*z* 468.1 [M-H]⁻. ¹H NMR (400 MHz, DMSO-*d*₆): δ ppm 12.37 (m, 2H), 9.29 (s, 1H), 7.76 - 7.62 (m, 2H), 7.53 (dd, *J* = 8.1, 1.9 Hz, 1H), 7.42 (d, *J* = 1.7 Hz, 1H), 7.35 - 7.24 (m, 2H), 4.12 (s, 2H), 2.61 (t, *J* = 6.1 Hz, 2H).

### Example 31: 3-((2-Fluoro-4-iodophenyl)amino)-4-(3-oxoisoxazolidine-2-carbonyl)benzonitrile

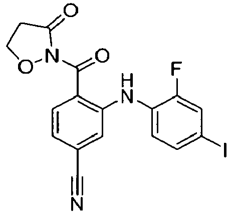

A solution of 3-((4-cyano-2-((2-fluoro-4-iodophenyl)amino)benzamido)oxy)propanoic acid (203 mg, 0.43 mmol) in dioxane (1.7 mL) stirred at room temperature under N₂ was treated with SOCl₂ (0.32 mL, 4.3 mmol). After 24 h the reaction mixture was concentrated *in vacuo* and azeotroped with dry toluene (3 × 5 mL). The resulting orange solid was used in the next step without further purification. *m*/*z* 450.0 [M+H]⁻.

### Example 32: N-(3-Amino-3-oxopropoxy)-4-cyano-2-((2-fluoro-4-iodophenyl)amino) benzamide

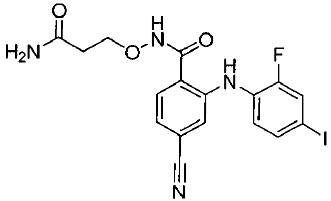

A solution of 3-((2-fluoro-4-iodophenyl)amino)-4-(3-oxoisoxazolidine-2-carbonyl)benzonitrile (195 mg, 0.43 mmol) in dry dioxane (1.03 mL) stirred at 0°C under N₂ was treated with 0.5 M NH₃ in dioxane (0.99 mL, 0.49 mmol). After 30 min the reaction mixture was diluted with EtOAc, quenched with 1M HCl, partitioned and the organic phase was washed with brine, dried over Na₂SO₄, filtered and concentrated *in vacuo.* The crude material was purified by preparative HPLC purification to give the product (22.2 mg, 11%) as a yellow solid. *m*/*z* 467.1 [M-H]⁻. ¹H NMR (400 MHz, DMSO-*d*₆): δ ppm 12.04 (br s, 1H), 9.47 (br s, 1H), 7.71 (dd, .7=10.4, 2.0 Hz, 2H), 7.52 (d, *J*=8.0 Hz, 1H), 7.41 (s, 1H), 7.33 - 7.26 (m, 2H), 6.90 (br s, 2H), 4.09 (t, *J*=5.1 Hz, 2H), 2.44 (t, *J*=6.3 Hz, 2H).

### Example 33: 3-Hydroxycyclobutyl 4-cyano-2-((2-fluoro-4-iodophenyl)amino)benzoate

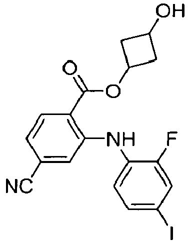

### Step 1: 4-Cyano-2-((2-fluoro-4-iodophenyl)amino)benzoyl chloride (General preparation)

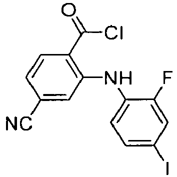

A suspension of 4-cyano-2-((2-fluoro-4-iodophenyl)amino)benzoic acid (200 mg, 0.52 mmol) in dioxane (1.61 mL) stirred at room temperature was treated with SOCl₂ (0.38 mL, 5.23 mmol). The reaction mixture was further stirred at 50°C under N₂. After 48 h the reaction mixture was concentrated *in vacuo* and azeotroped with dry toluene (2 × 5 mL). The resultant crude material in residual toluene was used in the next step without further purification.

### Step 2: 3-Hydroxycyclobutyl 4-cyano-2-((2-fluoro-4-iodophenyl)amino)benzoate

To a solution of cyclobutane-1,3-diol (159 mg, 1.8 mmol) and Et₃N (0.16 mL, 1.1 mmol) in dry THF (1.0 mL) stirred at 0°C under N₂ atmosphere a solution of 4-cyano-2-((2-fluoro-4-iodophenyl)amino)benzoyl chloride (157 mg, 0.39 mmol) in dry THF (1.3 mL) was added. After 1 h the reaction mixture was diluted with EtOAc, quenched with a saturated NH₄Cl aqueous solution, partitioned, and the aqueous phase re-extracted with EtOAc. The combined organic phases were washed with brine, dried over Na₂SO₄, filtered, and concentrated *in vacuo.* The crude residue was purified by a flash column chromatography (Silica, 5-31% EtOAc in hexanes) to give the product (43.7 mg, 24.7%) as a yellow solid. *m*/*z* 451.1 [M-H]⁻. ¹H NMR (400 MHz, DMSO-*d*₆): δ ppm 9.36 (s, 1H), 8.06 (d, *J*=8.2 Hz, 1H), 7.76 (dd, *J*=10.3, 1.9 Hz, 1H), 7.58 (dt, *J*=8.4, 1.3 Hz, 1H), 7.38 - 7.30 (m, 2H), 7.27 (dd, *J*=8.2, 1.6 Hz, 1H), 5.26 (d, *J*=6.5 Hz, 1H), 4.74 (p, *J*=7.3 Hz, 1H), 3.88 (h, *J*=6.9 Hz, 1H), 2.86 - 2.70 (m, 2H), 2.12 - 1.98 (m, 2H).

### Example 34: 3-Hydroxy-2,2,4,4-tetramethylcyclobutyl 4-cyano-2-((2-fluoro-4-iodophenyl)amino)benzoate

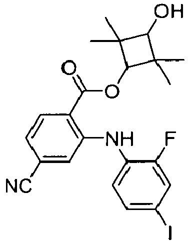

To a solution of 2,2,4,4-tetramethylcyclobutane-1,3-diol (520 mg, 3.6 mmol) and Et₃N (0.32 mL, 2.3 mmol) in dry THF (2.0 mL) stirred at 0°C under N₂ atmosphere a solution of 4-cyano-2-((2-fluoro-4-iodophenyl)amino)benzoyl chloride (314 mg, 0.78 mmol) in dry THF (2.7 mL) was added. After 48 h the reaction mixture was diluted with EtOAc, quenched with a saturated NH₄Cl aqueous solution, partitioned, and the aqueous phase re-extracted with EtOAc. The combined organic phases were washed with brine, dried over Na₂SO₄, filtered, and concentrated *in vacuo.* The crude residue was purified by a flash column chromatography (Silica, 0-50% EtOAc in hexanes) to give the product (26.6 mg, 6.7%) as a yellow solid. *m*/*z* 506.9 [M-H]⁻. ¹H NMR (400 MHz, DMSO-*d*₆): δ ppm (*Note:* a mixture of diastereoisomers 2:1) 9.31 (s, 0.35H), 9.29 (s, 0.67H), 8.10 (d, *J*=8.2 Hz, 0.37H), 8.06 (d, *J*=8.2 Hz, 0.63H), 7.77 (dd, *J*=10.3, 1.9 Hz, 1H), 7.59 (dd, *J*=7.8, 1.9 Hz, 1H), 7.39 - 7.34 (m, 2H), 7.34 - 7.27 (m, 1H), 5.01 (t, *J*=4.8 Hz, 1H), 4.52 (s, 0.35H), 4.41 (d, *J*=0.9 Hz, 0.67H), 3.53 (d, *J*=4.8 Hz, 0.36 H), 3.37 (d, *J*=4.8 Hz, 0.66 H), 1.18 (s, 4H), 1.09 (s, 2H), 1.07 (s, 2H), 1.00 (s, 4H).

### Example 35: 2,3-Dihydroxypropyl 4-cyano-2-((2-fluoro-4-iodophenyl)amino)benzoate

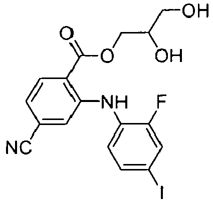

To a solution of 1,2,3-propanetriol (332 mg, 3.6 mmol) and Et₃N (0.32 mL, 2.3 mmol) in dry THF (2.7 mL) stirred at 0°C under N₂ atmosphere a solution of 4-cyano-2-((2-fluoro-4-iodophenyl)amino)benzoyl chloride (314 mg, 0.78 mmol) in dry THF (2.0 mL) was added. After 16 h the reaction mixture was diluted with EtOAc, quenched with a saturated NH₄Cl aqueous solution, partitioned, and the aqueous phase re-extracted with EtOAc. The combined organic phases were washed with brine, dried over Na₂SO₄, filtered, and concentrated *in vacuo.* A portion of the crude material (198 mg) was purified by preparative HPLC purification to give the product (34 mg, 9.5%) as a glassy yellow solid. *m*/*z* 455.0 [M-H]⁻. ¹H NMR (400 MHz, DMSO-*d*₆): δ ppm 9.33 (s, 1H), 8.12 (d, *J*=8.2 Hz, 1H), 7.77 (dd, *J*=10.3, 1.9 Hz, 1H), 7.59 (dt, *J*=8.4, 1.4 Hz, 1H), 7.39 - 7.30 (m, 2H), 7.29 (dd, *J*=8.2, 1.6 Hz, 1H), 5.12 (d, *J*=5.3 Hz, 1H), 4.75 (t, *J*=5.7 Hz, 1H), 4.37 (dd, *J*=11.1, 3.9 Hz, 1H), 4.23 (dd, *J*=11.1, 6.2 Hz, 1H), 3.88 - 3.76 (m, 1H), 3.53 - 3.38 (m, 2H).

### Example 36: 4-cyano-3-fluoro-2-((2-fluoro-4-iodophenyl)amino)benzoic acid

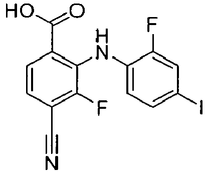

### Step 1: 2-fluoro-4-(trimethylsilyl)aniline

A round bottom flask was charged with 4-bromo-2-fluoroaniline (1.00 g, 5.3 mmol) and anhydrous tetrahydrofuran (8 mL), cooled to-78°C and a 2.5M solution of nBuLi in hexanes (8 ml, 20 mmol) was added dropwise keeping the internal temperature below -60°C. The reaction mixture was treated dropwise with chlorotrimethylsilane (2.26 ml, 17.4 mmol.), keeping the internal temperature below -60°C. The reaction mixture was allowed to warm to 0°C over one hour period. The reaction mixture was poured into ice-cold 2M hydrochloric acid and was vigorously stirred for 10 minutes. The organic layer was separated and the aqueous layer was extracted with ethyl acetate. The combined organics were dried over magnesium sulfate and evaporated to dryness to give the product as colorless oil (0.58g, 54%). *m*/*z* 184.2 [M+1]⁺.

### Step 2: 4-bromo-3-fluoro-2-((2-fluoro-4-(trimethylsilyl)phenyl)amino)benzoic acid

To a stirred solution comprised of 2-fluoro-4-(trimethylsilyl)aniline (1.0 g, 5.1 mmol) in tetrahydrofuran (6 mL) at -78°C was added lithium diisopropylamide (2.0 M in THF/heptane/ethylbenzene, 2.5 ml, 5.1 mmol). The resulting suspension was stirred vigorously for 10 minutes, after which time a solution of 4-bromo-2,3-difluorobenzoic acid (0.400 g, 1.7 mmol) in tetrahydrofuran (5 mL) was added. The cold bath was subsequently removed, and the reaction mixture was stirred at room temperature overnight. The mixture was concentrated and the concentrate was treated with 3M hydrochloric acid (10 mL). The resulting suspension was extracted with ethyl ether. The combined organics were dried over sodium sulfate. The solvents were removed in vacuo. Hexanes was added into the residue. The precipitated beige solid (382 mg) was washed using hexane and a few drop of ethyl acetate and isolated by filtration. *m*/*z* 400.1 [M⁺]. ¹H NMR (300 MHz, DMSO-d6): δ 9.06 (s, 1H), 7.70-7.66 (m, 1H), 7.38-7.33 (m, 1H), 7.27 (d, J=11.7 Hz, 1H), 7.20-7.17 (m, 1H), 6.93-6.86 (m, 1H), 0.22 (s, 9H).

### Step 3: 4-cyano-3-fluoro-2-((2-fluoro-4-(trimethylsilyl)phenyl)amino)benzoic acid

A microwave vial was charged with4-bromo-3-fluoro-2-((2-fluoro-4-(trimethylsilyl)phenyl)amino)benzoic acid (0.180 g, 0.4 mmol), zinc cyanide (0.05 g, 0.4 mmol) and tetrakis(triphenylphosphine)palladium(0) (0.05 g, 0.05 mmol) in *N*,*N*-dimethyl formamide (3 mL) under argon. The reaction mixture was stirred at 90°C overnight. The reaction was quenched with water and extracted with ethyl acetate. The organics were washed with water, brine and dried over sodium sulfate. The solvents were evaporated. The residue was purified by flash chromatography (12 g silica, 0-70% ethyl acetate in hexanes) to give the product as a yellow solid (50 mg, 32%). ¹H NMR (300 MHz, CDCl3): δ 8.95 (s, 1H), 7.93 (d, J=8.4 Hz, 1H), 7.24-7.21 (m, 2H), 7.19-7.08 (m, 1H), 7.00-6.93 (m, 1H), 0.28 (s, 9H).

### Step 4: 4-cyano-3-fluoro-2-((2-fluoro-4-iodophenyl)amino)benzoic acid

A round bottom flask was charged with4-cyano-3-fluoro-2-((2-fluoro-4-(trimethylsilyl)phenyl)amino)benzoic acid (0.49 g, 1.4 mmol) in anhydrous methanol (6 mL) and anhydrous dichloromethane (6 mL). Silver trifluoroacetate (0.66 g, 3.0 mmol) was added. The reaction mixture was cooled to 0°C. Iodine (0.72 g, 2.8 mmol) was then added in one portion. The reaction mixture was stirred at 0°C for 2 hours. The reaction mixture was filtered through celite and the solvents were removed. The residue was treated with saturated sodium thiosulfate and extracted with ethyl acetate. The combined organics were dried over sodium sulfate. The solvents were removed in vacuo. Dichloromethane was added to the residue whereby a solid precipitated out. The solid was collected by filtration and dried to give the product as beige colored solid (200 mg, 35%). ¹H NMR (300 MHz, DMSO-d6): δ 7.83-7.81 (m, 1H), 7.59-7.54 (dd, J=1.8 and 1.8 Hz, 1H), 7.39-7.36 (m, 2H), 6.74-6.66 (m, 1H).

### Example 37: 4-cyano-3-fluoro-2-((2-fluoro-4-iodophenyl)amino)-N-(2-hydroxyethoxy) benzamide

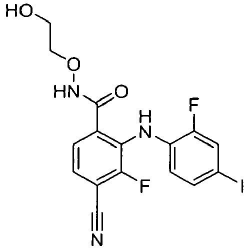

A microwave vial was charged with 4-cyano-3-fluoro-2-((2-fluoro-4-iodophenyl)amino)benzoic acid (0.100 g, 0.2 mmol), 2-(aminooxy)ethanol (0.03 g, 0.4 mmol), HATU (0.14 g, 0.4 mmol) and diisopropyl ethyl amine (86 µL, 0.5 mmol.) and *N,N-*dimethylformamide (3 mL). The reaction mixture was stirred at room temperature overnight. The reaction was quenched with water and extracted with ethyl acetate. The organics were washed with water, brine and dried over sodium sulfate. The solvents were evaporated. The residue was purified by flash chromatography (12 g silica, 0-5% methanol in dichloromethane). The product fractions were collected and the solvent was removed. The residue was purified again by reverse phase HPLC (20-80% Acetonitrile/water) to give the product as a light yellow solid. *m*/*z* 458.0 [M-1]⁻. ¹H NMR (300 MHz, DMSO-d6): δ 11.93 (s, 1H), 8.44 (s, 1H), 7.67-7.62 (m, 1H), 7.56 (d, J=9.6 Hz, 1H), 7.47-7.44 (m, 1H), 7.35 (d, J=8.4 Hz, 1H), 6.70-6.63 (m, 1H), 3.79 (t, J=4.4 Hz, 2H), 3.55-3.53 (m, 2H).

The following compound was prepared as described in Example 37, replacing the 2-(aminooxy)ethanol with an appropriate amine which is commercially available or prepared using conditions known to one of ordinary skill in the art.

| **Ex. No.** | **Comp. No.** | **Structure** | **1H NMR (300 MHz, DMSO-d6)** | ***m*/*z*** |
|---|---|---|---|---|
| **38** | 1.031 | 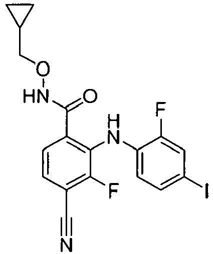 | δ 7.65-7.54 (m, 2H), 7.46-7.43 (m, 1H), 7.35 (d, J=9.0 Hz, 1H), 6.70-6.66 (m, 1H), 3.55 (d, J=6.9Hz, 2H), 1.02-1.01 (m, 1H), 0.50-0.46 (m, 2H), 0.21-0.19 (m, 2H). | 468.2 [M-1]⁻ |

### Example 39: Methyl 6-cyano-2-((2-fluoro-4-iodophenyl)amino)nicotinate

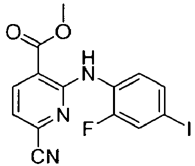

### Step 1: 6-Chloro-2-((2-fluoro-4-(trimethylsilyl)phenyl)amino)nicotinic acid

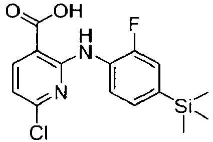

To a solution of 2-fluoro-4-(trimethylsilyl)aniline (9.2 g, 50 mmol) in dry THF (40 mL) stirred at -78°C was added LiHMDS (1M in THF, 100 mL, 100 mmol). The reaction mixture was stirred for 1 h at -78°C followed by an addition of a solution of 2,6-dichloroisonicotinic acid (8 g, 41.7 mmol) in dry THF (31 mL). The reaction mixture was stirred at -78°C for 1 h and then gradually warmed up to room temperature. The reaction mixture was quenched with a saturated NH₄Cl aqueous solution (100 mL) at 0°C, diluted with EtOAc (200 mL), acidified with 1M HCl to pH 3 and partitioned. The aqueous phase was extracted with EtOAc (2×100 mL), the organic phase was washed with brine (100 mL), dried over Na₂SO₄ and concentrated *in vacuo.* The crude material was purified by trituration with methanol to give the product (10.6 g, 75%) as a yellow solid. *m*/*z* 339.1/341.1 [M+H]⁺. ¹H NMR (400 MHz, DMSO-*d*₆): δ ppm 13.94 (s, 1H), 10.79 (d, J=2.9 Hz, 1H), 8.41 (t, J=8.1 Hz, 1H), 8.27 (d, J=8.1 Hz, 1H), 7.37 (m, 2H), 7.00 (d, J=8.1 Hz, 1H), 0.26 (s, 9H).

### Step 2: Methyl 6-chloro-2-((2-fluoro-4-(trimethylsilyl)phenyl)amino)nicotinate

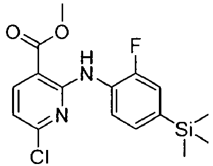

A solution of 6-chloro-2-((2-fluoro-4-(trimethylsilyl)phenyl)amino)nicotinic acid (0.5 g, 1.476 mmol) in DCM (7.4 mL) stirred at room temperature was treated with DIPEA (0.26 mmol, 1.476 mmol). After 10 min the reaction mixture was cooled down to 0°C and was treated with DMF (0.03 mL) and oxalyl chloride (0.12 mL, 1.476 mmol) with subsequent warm up to room temperature. The reaction mixture was stirred for 30 minutes, then slowly added to the solution of DIPEA (0.26 mL, 1.476 mmol) in MeOH (7.4 mL) stirred at 0°C with a subsequent warm up to room temperature. After 15 minutes the reaction mixture was concentrated *in vacuo.* The residue was dissolved in EtOAc (20 mL), washed with a saturated NaHCO₃ aqueous solution (10 mL), H₂O (10 mL), brine (10 mL), dried over Na₂SO₄ and concentrated *in vacuo* to give the product (0.45 g, 87%) as a brown solid. *m*/*z* 353.1/355.1 [M+H]⁺. ¹H NMR (400 MHz, DMSO-*d*₆): δ ppm 10.48 (d, *J*=3.0 Hz, 1H), 8.36 (t, *J*=7.9 Hz, 1H), 8.29 (d, *J*=8.2 Hz, 1H), 7.43 - 7.33 (m, 2H), 7.02 (d, *J*=8.2 Hz, 1H), 3.91 (s, 3H), 0.26 (s, 9H).

### Step 3: Methyl 6-cyano-2-((2-fluoro-4-(trimethylsilyl)phenyl)amino)nicotinate

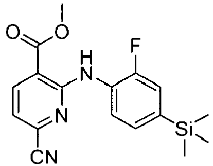

A degassed solution of methyl 6-chloro-2-((2-fluoro-4-(trimethylsilyl)phenyl)amino) nicotinate (0.4 g, 1.13 mmol), zinc cyanide (0.11 g, 0.96 mmol) and tetrakis(triphenylphosphine) palladium(0) (0.13 g, 0.11 mmol) in NMP (3.5 mL) was heated in a microwave oven at 190°C for 15 min. The reaction mixture was filtered, diluted with EtOAc (20 mL), washed with a saturated NaHCO₃ aqueous solution (10 mL), H₂O (10 mL), brine (10 mL), dried over Na₂SO₄ and concentrated *in vacuo.* The crude material was purified by flash column chromatography (Silica, 0-15% EtOAc in hexanes) to give the product (0.133 g, 43%) as a yellow solid. *m*/*z* 344.1 [M+H]⁺. ¹H NMR (400 MHz, DMSO-*d*₆): δ 10.43 (d, *J*=2.8 Hz, 1H), 8.46 (d, *J*=7.8 Hz, 1H), 8.32 (t, *J*=8.0 Hz, 1H), 7.54 (d, *J*=7.8 Hz, 1H), 7.45 - 7.36 (m, 2H), 3.94 (s, 3H), 0.26 (s, 9H).

### Step 4: Methyl 6-cyano-2-((2-fluoro-4-iodophenyl)amino)nicotinate

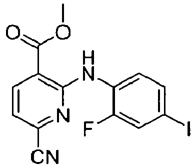

A suspension of silver tetrafluoroborate (85 mg, 0.437 mmol) in DCM (0.5 mL) was stirred at -50°C for 5 min in the dark, then a solution of methyl 6-cyano-2-((2-fluoro-4-(trimethylsilyl)phenyl)amino)-nicotinate (50 mg, 0.146 mmol) in DCM (1 mL) was added dropwise. After 15 min the reaction mixture was treated with iodine monochloride (26 mg, 0.161 mmol) in DCM (0.3 mL). The reaction mixture was stirred for 15 min and an additional portion of iodine monochloride (7 mg, 0.044 mmol) in DCM (0.1 mL) was added. After 15 min the reaction mixture was quenched with a saturated Na₂S₂O₃ aqueous solution (1 mL), extracted with EtOAc (3 × 5 mL), the organic phase was washed with brine (5 mL), dried over Na₂SO₄ and the solvent was removed *in vacuo* to give the product (53 mg, 91%) as a yellow solid. *m*/*z* 398.0 [M+H]⁺. ¹H NMR (400 MHz, DMSO-*d*₆): δ ppm 10.37 (d, *J*=2.8 Hz, 1H), 8.46 (d, *J*=7.8 Hz, 1H), 8.11 (t, *J*=8.6 Hz, 1H), 7.76 (dd, *J*=10.6, 2.0 Hz, 1H), 7.64 (dd, *J*=8.6, 1.7 Hz, 1H), 7.55 (d, *J*=7.8 Hz, 1H), 3.94 (s, 3H).

### Example 40: 6-Cyano-2-((2-fluoro-4-iodophenyl)amino)nicotinic acid

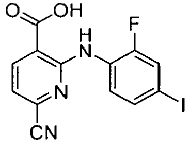

To a suspension of methyl 6-cyano-2-((2-fluoro-4-iodophenyl)amino)nicotinate (0.42 g, 1.06 mmol) in THF (3.2 mL), MeOH (1.05 mL) and H₂O (2.1 mL) stirred at room temperature was treated with 1M LiOH aqueous solution (1.06 mL, 1.06 mmol). After 30 min an additional portion of 1M LiOH aqueous solution (1.06 mL, 1.06 mmol) was added. After 30 min the reaction mixture was concentrated *in vacuo,* the residue was partitioned between EtOAc (10 mL) and H₂O (10 mL). The aqueous phase was acidified to pH 3 with 1M HCl aqueous solution and extracted with EtOAc (3 × 10 mL). The combined organic phase was washed with brine (10 mL), dried over Na₂SO₄ and concentrated *in vacuo* to give the product (0.40 g, quant.) as a yellow solid. *m*/*z* 382.0 [M-H]⁻. ¹H NMR (400 MHz, DMSO-*d*₆): δ ppm 8.42 (d, *J*=7.7 Hz, 1H), 8.21 (t, *J*=8.5 Hz, 1H), 7.73 (dd, *J*=10.8, 1.9 Hz, 1H), 7.62 (d, *J*=8.6 Hz, 1H), 7.52 (d, *J*=7.7 Hz, 1H).

### Example 41: 6-Cyano-N-ethoxy-2-((2-fluoro-4-iodophenyl)amino)nicotinamide

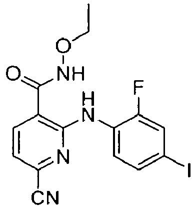

To a suspension of 6-cyano-2-((2-fluoro-4-iodophenyl)amino)nicotinic acid (100 mg, 0.26 mmol) in dry DMF (2.5 mL) were added HATU (200 mg, 0.52 mmol) and DIPEA (0.13 mL, 0.65 mmol) and the reaction mixture was heated at 50°C for 30 min. The reaction mixture was then cooled down to room temperature and a solution of ethoxyamine hydrochloride (38 mg, 0.39 mmol) in dry DMF (0.5 mL) was pre-treated with DIPEA (0.05 mL, 0.39 mmol) for 5 min was added. After 1 h the reaction mixture was concentrated *in vacuo,* the residue was dissolved in EtOAc (5 mL), washed with H₂O (5 mL) and brine (5 mL). The organic phase was dried over Na₂SO₄ and concentrated *in vacuo.* The crude material was purified by flash column chromatography (Silica, 0-37% EtOAc in hexanes) followed by a trituration with diethyl ether to give the product (7 mg, 6%) as a yellow solid. UPLC-MS (Acidic Method, 2 min): rt 1.26 min, *m*/*z* 425.1 [M-H]⁻. ¹H NMR (400 MHz, DMSO-*d*₆): δ ppm 12.18 (s, 1H), 10.89 (s, 1H), 8.16 (d, *J*=7.7 Hz, 2H), 7.71 (dd, *J*=10.7, 2.0 Hz, 1H), 7.60 (dt, *J*=8.6, 1.5 Hz, 1H), 7.55 (d, *J*=7.8 Hz, 1H), 4.00 (d, *J*=7.1 Hz, 2H), 1.24 (t, *J*=7.0 Hz, 3H).

### Example 42: 6-Cyano-2-((2-fluoro-4-iodophenyl)amino)-N-(2-hydroxyethoxy) nicotinamide

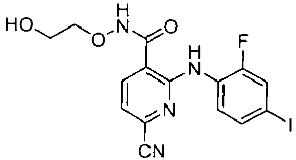

To a solution of 6-cyano-2-((2-fluoro-4-iodophenyl)amino)nicotinic acid (100 mg, 0.261 mmol) and HATU (198.5 mg, 0.522 mmol) in DMF (3 mL) stirred at room temperature DIPEA (91 µl, 0.522 mmol) was added dropwise and the reaction was monitored towards completion of HATU-activation of the acid. After 2 h 2-(aminooxy)ethan-1-ol (30.2 mg, 0.395 mmol) was added to the reaction mixture and it was stirred at room temperature for 45 min. The reaction mixture was quenched with H₂O (30 mL) and extracted with EtOAc (4 × 20 mL). The combined organic phase was washed with brine (2 × 20 mL), dried over Na₂SO₄, and concentrated *in vacuo.* The crude was purified by preparative HPLC to give the product (55.2 mg, 48%) as a yellow solid. *m*/*z* 443.0 [M+H]⁺. ¹H NMR (400 MHz, DMSO-*d*₆): δ ppm 12.29 (br s, 1H), 10.73 (s, 1H), 8.16 (d, *J*=7.8 Hz, 1H), 8.14 (t, *J*=8.5 Hz, 1H), 7.71 (dd, *J*=10.7, 2.0 Hz, 1H), 7.60 (dt, *J*=8.6, 1.3 Hz, 1H), 7.55 (d, *J*=7.8 Hz, 1H), 4.79 (br s, 1H), 4.00 (t, *J*=4.8 Hz, 2H), 3.66 (t, *J*=4.9 Hz, 2H).

### Example 43: Methyl 6-cyano-5-fluoro-2-((2-fluoro-4-iodophenyl)amino)nicotinate

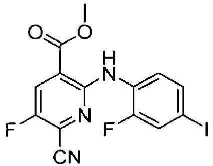

### Step 1: 6-Chloro-5-fluoro-2-((2-fluoro-4-(trimethylsilyl)phenyl)amino)nicotinic acid

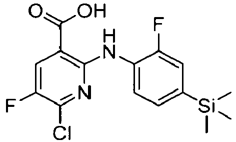

A solution of 2-fluoro-4-(trimethylsilyl)aniline (8.4 g, 45.7 mmol) in dry THF (34 mL) stirred at -78°C was treated with LiHMDS (1M in THF, 91.2 mL, 91.2 mmol). The reaction mixture was stirred for 1 h at -78°C and then a solution of 2,6-dichloro-5-fluoroisonicotinic acid (8 g, 38 mmol) in dry THF (30 mL). The reaction mixture was stirred at -78°C for 1 h, then gradually warmed up to room temperature and stirred for 1 h. The reaction mixture was cooled down to 0°C and was quenched with a saturated NH₄Cl aqueous solution, then diluted with EtOAc (200 mL), acidified with 1M HCl to pH 3, partitioned and the aqueous phase was extracted with EtOAc (2 × 100 mL). The organic phase was washed with brine (100 mL), dried over Na₂SO₄ and concentrated *in vacuo.* The crude material was purified by trituration with methanol to the product (7.5 g, 55%) as a yellow solid. *m*/*z* 357.0/359.0 [M+H]⁺. ¹H NMR (400 MHz, DMSO-*d*₆) δ ppm 10.69 (s, 1H), 8.36 (t, *J*=8.0 Hz, 1H), 8.28 (d, *J*=8.5 Hz, 1H), 7.41 - 7.32 (m, 2H), 0.25 (s, 9H).

### Step 2: Methyl 6-chloro-5-fluoro-2-((2-fluoro-4-(trimethylsilyl)phenyl)amino)nicotinate

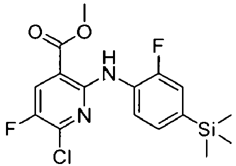

A suspension of 6-chloro-5-fluoro-2-((2-fluoro-4-(trimethylsilyl)phenyl)amino) nicotinic acid (2.4 g, 6.726 mmol) and DMF (0.32 mL) in DCM (32 mL) stirred at 0°C was treated with oxalyl chloride (2.85 mL, 33.63 mmol), then stirred at reflux for 1 h. The reaction mixture was concentrated *in vacuo,* azeotroped with toluene (3 × 25 mL) and then the residue was treated with ice-cold methanol (32 mL). The resulted suspension was stirred at reflux. After 2 h the reaction mixture was cooled down and formed precipitate was filtered under reduced pressure. The collected precipitate was washed with ice-cold methanol (3 × 5 mL) and dried *in vacuo* to give the product (2.18 g, 88%) as a yellow solid. *m*/*z* 371.1/373.1 [M+H]⁺. ¹H NMR (400 MHz, DMSO-*d*₆) δ ppm 10.33 (d, *J*=3.0 Hz, 1H), 8.37 - 8.25 (m, 2H), 7.44 - 7.33 (m, 2H), 3.92 (s, 3H), 0.26 (s, 9H).

### Step 3: Methyl 6-cyano-5-fluoro-2-((2-fluoro-4-(trimethylsilyl)phenyl)amino)nicotinate

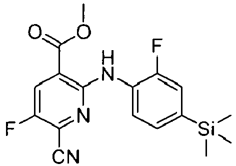

A degassed solution of methyl 6-chloro-5-fluoro-2-((2-fluoro-4-(trimethylsilyl)phenyl)amino)nicotinate (0.5 g, 1.35 mmol), zinc cyanide (0.14 g, 1.215 mmol) and tetrakis(triphenylphosphine)palladium(0) (0.23 g, 0.20 mmol) in NMP (4 mL) was heated in a microwave oven at 150°C for 15 min. The reaction mixture was filtered, diluted with EtOAc (20 mL), washed with a saturated NaHCO₃ aqueous solution (20 mL) and partitioned. The aqueous phase was extracted with EtOAc (3 × 10 mL) and the combined organic phase was washed with H₂O (20 mL), brine (20 mL), dried over Na₂SO₄ and concentrated *in vacuo.* The crude material was purified by flash column chromatography (Silica, 0-15% EtOAc in hexanes) to give the product (0.425 g, 87%) as a yellow solid. *m*/*z* 362.1 [M+H]⁺. ¹H NMR (400 MHz, DMSO-*d*₆) δ ppm 10.29 (d, *J*=2.8 Hz, 1H), 8.51 (d, *J*=8.7 Hz, 1H), 8.28 (t, *J*=8.0 Hz, 1H), 7.46 - 7.37 (m, 2H), 3.96 (s, 3H), 0.27 (s, 9H).

### Step 4: Methyl 6-cyano-5-fluoro-2-((2-fluoro-4-iodophenyl)amino)nicotinate

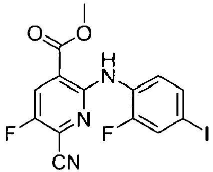

A suspension of silver tetrafluoroborate (0.69 g, 3.531 mmol) in DCM (4.2 mL) was stirred at - 50°C for 10 min in the dark, then a solution of methyl 6-cyano-5-fluoro-2-((2-fluoro-4-(trimethylsilyl)phenyl)-amino)nicotinate (0.425 g, 1.177 mmol) in DCM (8.5 mL) was added dropwise. After 30 min the reaction mixture was treated with iodine monochloride (0.21 g, 1.295 mmol) in DCM (2.5 mL). The reaction mixture was stirred for 30 min and an additional portion of iodine monochloride (0.21 g, 1.295 mmol) in DCM (2.5 mL) was added. After 30 min the reaction mixture was quenched with a saturated Na₂S₂O₃ aqueous solution (10 mL), extracted with EtOAc (3 × 25 mL), the organic phase was washed with brine (25 mL), dried over Na₂SO₄ and the solvent was removed *in vacuo* to give the product (0.36 g, 76%) as a yellow solid. *m*/*z* 416.4 [M+H]⁺. ¹H NMR (400 MHz, DMSO-*d*₆): δ ppm 10.22 (s, 1H), 8.50 (d, *J*=8.8 Hz, 1H), 8.05 (t, *J*=8.6 Hz, 1H), 7.76 (dd, *J*=10.6, 2.0 Hz, 1H), 7.66 - 7.60 (m, 1H), 3.95 (s, 3H).

### Example 44: 6-Cyano-5-fluoro-2-((2-fluoro-4-iodophenyl)amino)nicotinic acid

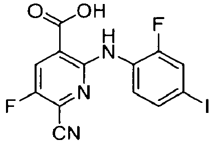

To a suspension of methyl 6-cyano-5-fluoro-2-((2-fluoro-4-iodophenyl)amino)nicotinate (0.36 g, 0.898 mmol) in THF (2.7 mL), MeOH (0.9 mL) and H₂O (1.8 mL) stirred at room temperature was treated with 1M LiOH aqueous solution (0.9 mL, 0.898 mmol). After 30 min the reaction mixture was concentrated *in vacuo,* the residue was partitioned between EtOAc (10 mL) and H₂O (10 mL). The aqueous phase was acidified to pH 3 with 1M HCl aqueous solution and extracted with EtOAc (3 × 10 mL). The combined organic phase was washed with brine (10 mL), dried over Na₂SO₄ and concentrated in *vacuo* to give the product (0.30 g, 86%) as a brown solid. *m*/*z* 400.0 [M-H]⁻. ¹H NMR (400 MHz, DMSO-*d*₆): δ ppm 10.70 (s, 1H), 8.43 (d, *J*=8.7 Hz, 1H), 8.13 (t, *J*=8.7 Hz, 1H), 7.72 (dd, *J*=10.7, 1.9 Hz, 1H), 7.61 (dt, = 8.6, 1.6 Hz, 1H).

### Example 45: tert-Butyl 2-((2-fluoro-4-iodophenyl)amino)-1-methyl-1H-pyrrolo[2,3-b]pyridine-3-carboxylate

### Alternative A for Preparation of 1-Methyl-1H-pyrrolo[2,3-b]pyridine-3-carboxylic acid

### Step 1 of Alternative A: 1-Methyl-1H-pyrrolo[2,3-b]pyridine-3-carbonitrile

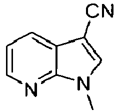

A solution of 7-azaindole-3-carbonitrile (9.0 g, 62.8 mmol) in dry DMF (80 mL) was cooled to 0 °C in an ice bath and treated with sodium hydride (5.0 g, 125.7 mmol, 60% in mineral oil) in a portion-wise manner. The resulting mixture was stirred for 45 min at 0 °C, then treated with iodomethane (7.8 mL, 125.7 mmol) and gradually warmed up to a room temperature over 1 h. The mixture was then cautiously poured into H₂O (450 mL) and extracted with EtOAc (3 × 50 mL). The combined organic phases were washed sequentially with water (3 × 50 mL) and brine, dried over Na₂SO₄, filtered, and concentrated *in vacuo.* The crude was purified by flash column chromatography (Silica 120 g, 10-50% EtOAC in hexane) to give the product (7.3 g, 74%) as an off-white solid. UPLC-MS (Acidic Method, 4 min): rt 2.30 min, *m*/*z* 158.2 [M+H]⁺. ¹H NMR (400 MHz, CDCl₃): δ ppm 8.48 (dd, *J* = 4.7, 1.5 Hz, 1H), 8.09 (dd, *J* = 7.9, 1.6 Hz, 1H), 7.75 (s, 1H), 7.25-7.30 (m, 1H), 3.98 (s, 3H).

### Step 2 of Alternative A: 1-Methyl-1H-pyrrolo[2,3-b]pyridine-3-carboxylic acid

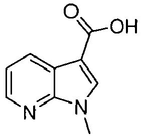

A suspension of 1-methyl-1*H*-pyrrolo[2,3-*b*]pyridine-3-carbonitrile (7.3 g, 46.5 mmol) in concentrated hydrochloric acid (12 M, 73 mL, 876 mmol) was heated at 100°C with stirring for 10 h, resulting in a clear solution. The reaction mixture was then cooled to 5°C (ice bath) and cautiously treated with 40% NaOH aqueous solution until the pH reached 2 leading to a formation of a white precipitate. The resulting mixture was stirred for 1 h, filtered, and the solid was washed with H₂O until the filtrate became pH neutral before being dried *in vacuo* to give the product (6.9 g, 84%) as a white solid. UPLC-MS (Basic Method, 2 min): rt 0.17 min, *m*/*z* 175.2 [M+H]⁺. ¹H NMR (400 MHz, DMSO-*d*₆): δ ppm 8.28 -8.37 (m, 2H), 8.22 (s, 1H), 7.25 (dd, *J* = 7.8, 4.7 Hz, 1H), 3.86 (s, 3H).

### Alternative B for Preparation of 1-Methyl-1H-pyrrolo[2,3-b]pyridine-3-carboxylic acid

### Step 1 of Alternative B: 1-methyl-1H-pyrrolo[2,3-b]pyridine

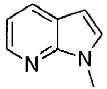

To a suspension of sodium hydride, 60% dispersion in mineral oil (36.5 g, 914 mmol), in anhydrous DMF (300 mL) cooled in an ice bath was added a solution of 7-azaindole (90.0 g, 762 mmol) in DMF (200 mL) *via* addition funnel over 3 h. The reaction mixture was stirred for 30 minutes and cooled in an ice bath before methyl iodide (52 mL, 838 mmol) was added *via* dropping funnel over 30 minutes. After stirring the reaction mixture at r.t. over the weekend, UPLC analysis showed the reaction was incomplete. Additional methyl iodide (5 mL, 80.3 mmol) was added and the reaction monitored by UPLC until completion. The reaction mixture was cooled in an ice bath and quenched with H₂O, extracted into EtOAc (3 × 800 mL) and the combined organic layers were washed with brine, dried over Na₂SO₄, and the solvent removed *in vacuo* to give the desired product (130.7 g (89.4 g, 89% active compound)) as a dark brown biphasic oil. UPLC-MS (Acidic Method, 2 min): rt 0.75 min, *m*/*z* 133.1 [M+H]⁺. ¹H NMR (400 MHz, DMSO-*d*₆) δ ppm 8.25 (dd, *J* = 4.6, 1.5 Hz, 1H), 7.94 (dd, *J* = 7.8, 1.5 Hz, 1H), 7.50 (d, *J* = 3.4 Hz, 1H), 7.07 (dd, *J* = 7.8, 4.7 Hz, 1H), 6.45 (d, *J* = 3.4 Hz, 1H), 3.82 (s, 3H).

### Step 2 of Alternative B: 2,2,2-trifluoro-1-(1-methyl-1H-pyrrolo[2,3-b]pyridin-3-yl)ethanone

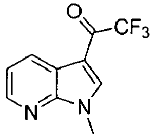

To a solution of 1-methyl-1*H*-pyrrolo[2,3-*b*]pyridine (89.4 g, 676 mmol) in DMF (450 mL) cooled in an ice bath, was added TFAA (141 mL, 1.01 mol) dropwise *via* addition funnel 3 h. The reaction was stirred at r.t. overnight before dilution with H₂O (1 L) over 1 h. Addition of H₂O resulted in precipitate formation, which was stirred for 30 minutes before filtration. The solid was washed with H₂O and dried to give the desired product (121 g, 79%) as a white solid. UPLC-MS (Acidic Method, 2 min): rt 1.05 min, *m*/*z* 229.1 [M+H]⁺. ¹H NMR (400 MHz, DMSO-*d*₆) δ ppm: 8.80 (d, *J* = 1.5 Hz, 1H), 8.52 (q, *J* = 1.5 Hz, 1H), 8.50 (s, 1H), 7.40 - 7.49 (m, 1H), 3.97 (s, 3H); ¹⁹F NMR (376 MHz, DMSO-*d*₆) δ ppm: -71.6 (s, IF).

### Step 3 of Alternative B: 1-methyl-1H-pyrrolo[2,3-b]pyridine-3-carboxylic acid

To a flask containing solid 2,2,2-trifluoro-1-(1-methyl-1*H*-pyrrolo[2,3-*b*]pyridine-3-yl)ethenone (89.9 g, 394 mmol) was added 5 M NaOH (788 mL, 3.94 mol), the resultant mixture was heated to 50 °C overnight. The reaction mixture was diluted by 50% with H₂O and washed with TBME (800 mL). The resultant aqueous layer was acidified to pH 1 with concentrated HCl (330 mL), resulting in formation of a white precipitate. The precipitate was filtered, washed with H₂O (1.2 L) and dried under vacuum at 40 °C, to a constant weight giving the desired product (69.9 g, 99%) as a white solid. UPLC-MS (Basic Method, 2 min): rt 0.17 min, *m*/*z* 175.2 [M+H]⁺. ¹H NMR (400 MHz, DMSO-*d*₆) δ ppm 8.28 -8.37 (m, 2H), 8.22 (s, 1H), 7.25 (dd, *J* = 7.8, 4.7 Hz, 1H), 3.86 (s, 3H

### Alternative 1 for the Preparation of tert-Butyl 1-methyl-1H-pyrrolo[2,3-b]pyridine-3-carboxylate

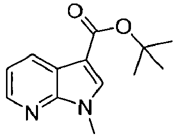

To a suspension of 1-methyl-1*H*-pyrrolo[2,3-*b*]pyridine-3-carboxylic acid (10.0 g, 56.8 mmol), prepared as described above in Alternative A or B, in anhydrous DCM (390 mL) cooled on ice, oxalyl chloride (14.4 mL, 170.4 mmol) was added dropwise over 15 min and the mixture was stirred at room temperature for 2 h. The mixture was then concentrated *in vacuo* to give a yellow solid, which was treated with tert-butanol (300 mL, 3.14 mol), followed by an addition of potassium *tert*-butoxide (10.2 g, 91 mmol). The resulting mixture was stirred at room temperature for 16 h and then concentrated *in vacuo.* The crude was purified by flash column chromatography (Silica 120 g, 0-10% MeOH in DCM) to give the product (12.6 g, 86%) as a light brown solid. UPLC-MS (Acidic Method, 2 min): rt 1.10 min, *m*/*z* 233.1 [M+H]⁺. ¹H NMR (400 MHz, DMSO-*d*₆) δ ppm 8.35 (dd, *J* = 4.6, 1.6 Hz, 1H), 8.27 (dd, *J* = 7.8, 1.6 Hz, 1H), 8.20 (s, 1H), 7.27 (dd, *J* = 7.9, 4.6 Hz, 1H), 3.86 (s, 3H), 1.56 (s, 9H).

### Alternative 2 for the Preparation of tert-Butyl 1-methyl-1H-pyrrolo[2,3-b]pyridine-3-carboxylate

1-Methyl-1*H*-pyrrolo[2,3-*b*]pyridine-3-carboxylic acid (68.7 g, 390 mmol), prepared as described above in Alternative A or B, was added to thionyl chloride (700 mL, 9.67 mol) under stirring at room temperature, and the resulting mixture was stirred overnight. The thionyl chloride was then removed under vacuum to give a thick suspension, which was co-distilled from toluene (3 × 200 mL) to give an off-white solid. This material was subsequently suspended in *tert*-butanol (500 mL). Solid potassium *tert*-butoxide (70 g, 624 mmol) was added to the suspension in a portion-wise manner, and the resulting mixture was stirred overnight. The solvent was removed under vacuum to give a thick solid, which was partitioned between EtOAc (1.5 L) and a saturated solution of aqueous NaHCO₃ (1 L). The organic phase was collected and washed with a saturated solution of aqueous NaHCO₃ (1 L), before being dried over Na₂SO₄, filtered and evaporated to dryness to give the desired product (66.7 g, 74%) as a green solid.

### tert-Butyl 2-chloro-1-methyl-1H-pyrrolo[2,3-b]pyridine-3-carboxylate

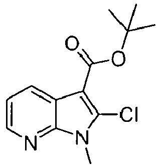

A solution of *tert*-butyl 1-methyl-1*H*-pyrrolo[2,3-*b*]pyridine-3-carboxylate (4.8 g, 21.0 mmol) in dry THF (90 mL), prepared as described in Alternative 1 or Alternative 2 above, was flushed with N₂, cooled to -78°C and then treated with a solution of LDA (2 M in THF, 21 mL, 42 mmol). The mixture was stirred at -78 °C for 0.5 h. A solution of hexachloroethane (9.9 g, 42.0 mmol) in dry THF (30 mL) was added and the mixture was gradually warmed up to a room temperature and stirred for 1.5 h. The mixture was treated with saturated NH₄Cl aqueous solution and extracted with EtOAc (3 × 50 mL). The combined organic phases were dried over Na₂SO₄, filtered and concentrated *in vacuo.* The crude was purified by flash column chromatography (Silica 80 g, 0-12% EtOAc in hexanes) to give the product (4.5 g, 81%) as a pale-yellow solid. UPLC-MS (Acidic Method, 2 min): rt 1.21 min, *m*/*z* 267.1/269.0 [M+H]⁺. ¹H NMR (400 MHz, DMSO-*d*₆) δ ppm 8.37 (dd, *J* = 4.8, 1.6 Hz, 1H), 8.27 (dd, *J* = 7.9, 1.6 Hz, 1H), 7.32 (dd, *J* = 7.9, 4.7 Hz, 1H), 3.83 (s, 3H), 1.58 (s, 9H)

### tert-Butyl 2-((2-fluoro-4-iodophenyl)amino)-1-methyl-1H-pyrrolo[2,3-b]pyridine-3-carboxylate

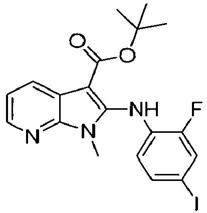

A suspension of *tert*-butyl 2-chloro-1-methyl-1*H*-pyrrolo[2,3-*b*]pyridine-3-carboxylate (1.0 g, 3.8 mmol) and 2-fluoro-4-iodoaniline (0.8 g, 3.6 mmol) in dry THF (20 mL) was flushed with N₂, cooled to -78 °C and treated with a solution of LiHMDS (1 M in THF, 7.5 mL, 7.5 mmol). The mixture was gradually warmed up to room temperature and stirred for 3 h. The mixture was quenched with saturated NH₄Cl aqueous solution and then extracted with EtOAc (3 × 50 mL). The combined organic phases were dried over Na₂SO₄, filtered, and concentrated *in vacuo.* The crude was purified by flash column chromatography (Silica 40 g, 0-12% EtOAc in hexanes) to give the product (1.5 g, 87%) as a yellow solid. UPLC-MS (Acidic Method, 2 min): rt 1.42 min, *m*/*z* 468.1 [M+H]⁺. ¹H NMR (400 MHz, DMSO-*d*₆) δ ppm 8.57 (s, 1H), 8.24 (dd, *J* = 4.8, 1.6 Hz, 1H), 8.16 (dd, *J* = 7.8, 1.6 Hz, 1H), 7.63 (dd, *J* = 10.8, 2.0 Hz, 1H), 7.37 (dt, *J* = 8.5, 0.9 Hz, 1H), 7.23 (dd, *J* = 7.8, 4.8 Hz, 1H), 6.68 (t, *J* = 8.8 Hz, 1H), 3.55 (s, 3H), 1.41 (s, 9 H)

### Example 46: 2-((2-Fluoro-4-iodophenyl)amino)-N-(2-hydroxyethoxy)-1-methyl-1H-pyrrolo[2,3-b]pyridine-3-carboxamide

### 2-((2-Fluoro-4-iodophenyl)amino)-1-methyl-1H-pyrrolo[2,3-b]pyridine-3-carbonyl chloride

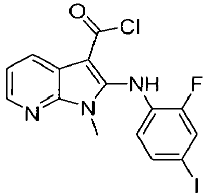

To tert-butyl 2-((2-fluoro-4-iodophenyl)amino)-1-methyl-1*H*-pyrrolo[2,3-*b*]pyridine-3-carboxylate (0.6 g, 1.3 mmol), thionyl chloride (0.9 mL, 12.8 mmol) was added followed by H₂O (23 µL). The flask was sealed with a rubber septum and the mixture was stirred at room temperature for 18 h. The mixture was concentrated to dryness *in vacuo* to give the product (0.5 g, 94%) as a beige solid. UPLC-MS (Acidic Method, 2 min): rt 1.28 min, *m*/*z* 426.0 [M+H]⁺ (detected as the corresponding methyl ester after quenching an aliquot of the mixture with MeOH).

Alternative preparation: A stirred solution of *tert*-butyl 2-((2-fluoro-4-iodophenyl)amino)-1-methyl-1*H*-pyrrolo[2,3-*b*]pyridine-3-carboxylate (5.00 g, 10.7 mmol) in anhydrous 1,4-dioxane (28 mL) was treated with thionyl chloride (7.7 mL, 107 mmol) at ambient temperature, followed by a 4 N solution of hydrogen chloride in 1,4-dioxane (14 mL, 5.35 mmol), and the resulting mixture was heated to 50 °C for 48 h. The reaction mixture was cooled to 40 °C and subjected to a continuous distillation process under vacuum from anhydrous toluene (maintaining the total volume of the batch around 30 mL) to remove the thionyl chloride and 1,4-dioxane. The resulting dark grey suspension of 2-((2-fluoro-4-iodophenyl)amino)-1-methyl-1*H-*pyrrolo[2,3-*b*]pyridine-3-carbonyl chloride was used in subsequent steps without further purification. UPLC-MS (Acidic Method, 2 min): rt 1.29 min, m/z 426.0 [M+H]⁺ (following the quenching of an aliquot of the batch into methanol to give the corresponding methyl ester).

### Alterntaive 1 for the preparation of 2-((2-Fluoro-4-iodophenyl)amino)-N-(2-hydroxyethoxy)-1-methyl-1H-pyrrolo[2,3-b]pyridine-3-carboxamide

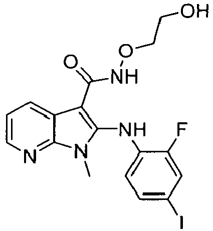

A solution of 2-((2-fluoro-4-iodophenyl)amino)-1-methyl-1*H*-pyrrolo[2,3-*b*]pyridine-3-carbonyl chloride (460 mg, 1.07 mmol) in dry DCM (27 mL) was cooled to 0°C in an ice bath and then treated with dry pyridine (970 µL, 11.98 mmol) and the mixture was stirred for 15 min followed by an addition of (2-aminooxy)ethanol (124 mg, 1.61 mmol) in dry DCM (2 mL). The mixture was stirred for 15 min, then diluted with DCM and acidified with 1 M citric acid aqueous solution to pH 3. The organic phase was washed with H₂O, brine, dried over Na₂SO₄ and concentrated *in vacuo.* The crude was purified by preparative HPLC to give the product (181 mg, 36%) as a white solid. UPLC-MS (Acidic Method, 4 min): rt 2.67 min, *m*/*z* 471.2 [M+H]⁺ . ¹H NMR (400 MHz, DMSO-*d*₆): δ ppm 10.84 (br s, 1H), 8.69 (br s, 1H), 8.25 (dd, *J* = 4.8, 1.4 Hz, 1H), 8.13 (dd, *J* = 7.9, 1.5 Hz, 1H), 7.63 (dd, *J* = 10.8, 1.9 Hz, 1H), 7.33 (dd, *J* = 8.5, 1.1 Hz, 1H), 7.21 (dd, *J* = 7.8, 4.8 Hz, 1H), 6.52 (t, *J* = 8.8 Hz, 1H), 4.74 (br s, 1H), 3.79 (t, *J* = 4.9 Hz, 2H), 3.48 -3.54 (m, 5 H).

### Alterntaive 2 for the preparation of 2-((2-Fluoro-4-iodophenyl)amino)-N-(2-hydroxyethoxy)-1-methyl-1H-pyrrolo[2,3-b]pyridine-3-carboxamide

To a solution of 2-(aminooxy)ethanol (8.41 g, 109 mmol) in anhydrous THF (20 mL) at 0 °C was added a suspension of 2-((2-fluoro-4-iodophenyl)amino)-1-methyl-1*H*-pyrrolo[2,3-*b*]pyridine-3-carbonyl chloride (9.37 g, 21.8 mmol) in anhydrous THF (80 mL) and residual toluene *via* syringe. After 40 minutes UPLC analysis showed complete conversion. The reaction mixture was partitioned between EtOAc (300 mL) and H₂O (300 mL), the biphasic mixture was filtered and the organic layer separated. The aqueous layer was extracted with EtOAc (200 mL) and the organics combined, washed with brine, dried over Na₂SO₄ and the solvent removed *in vacuo.* The crude solid was suspended in EtOAc (40 mL, 4 volumes), stirred over the weekend and filtered to give the desired product (7.45g, 73%) as a dark beige solid which can be recrystallized from anisole. UPLC-MS (Acidic Method, 2 min): rt 1.01 min, *m*/*z* 471.2 [M+H]⁺. ¹H NMR (400 MHz, DMSO-*d*₆) δ ppm 10.84 (br s, 1H), 8.69 (br s, 1H), 8.25 (dd, *J* = 4.8, 1.4 Hz, 1H), 8.13 (dd, *J* = 7.9, 1.5 Hz, I H), 7.63 (dd, *J* = 10.8, 1.9 Hz, 1H), 7.33 (dd, *J* = 8.5, 1.1 Hz, 1H), 7.21 (dd, *J* = 7.8, 4.8 Hz, 1H), 6.52 (t, *J* = 8.8 Hz, 1H), 4.74 (br s, 1H), 3.79 (t, *J* = 4.9 Hz, 2H), 3.48 -3.54 (m, 5 H).

### Example 47: 2-((4-Ethynyl-2-fluorophenyl)amino)-N-(2-hydroxyethoxy)-1-methyl-1H-pyrrolo[2,3-b]pyridine-3-carboxamide

### 2-((2-Fluoro-4-((trimethylsilyl)ethynyl)phenyl)amino)-N-(2-hydroxyethoxy)-1-methyl-1H-pyrrolo[2,3-b]pyridine-3-carboxamide

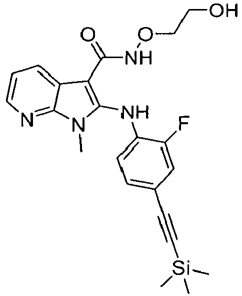

A solution of 2-((2-fluoro-4-iodophenyl)amino)-*N*-(2-hydroxyethoxy)-1-methyl-1*H-*pyrrolo[2,3-*b*]pyridine-3-carboxamide (100 mg, 0.21 mmol, 71% pure by UPLC-MS), copper(I) iodide (1 mg, 0.004 mmol), PdCl₂(PPh₃)₂ (3 mg, 0.004 mmol) in dry THF (0.5 mL) flushed with N₂, trimethylsilylacetylene (32 µL, 0.23 mmol) in Et₃N (21 µL, 1.49 mmol) was added. The mixture was stirred at room temperature for 3 h. The mixture was diluted with Et₂O, filtered through a pad of Celite^{®} and the filtrate was concentrated *in vacuo.* The crude was purified by flash column chromatography (Silica 4 g, 20-70% EtOAc in hexanes) to give the product (25 mg, 38%) as a colorless oil. UPLC-MS (Acidic Method, 2 min): rt 1.20 min, *m*/*z* 441.1 [M+H]⁺. ¹H NMR (400 MHz, DMSO-*d*₆) δ ppm 10.83 (s, 1H), 8.75 (s, 1H), 8.27 (dd, *J* = 4.7, 1.6 Hz, 1H), 8.15 (dd, *J* = 7.9, 1.5 Hz, 1H), 7.51-7.63 (m, 1H), 7.33 (dd, *J* = 12.1, 1.8 Hz, 1H), 7.22 (dd, *J* = 7.9, 4.8 Hz, 1H), 7.09 (dd, *J* = 8.3, 1.6 Hz, 1H), 6.60 (t, *J* = 8.7 Hz, 1H), 4.70 (t, *J* = 5.8 Hz, 1H), 3.78 (t, *J* = 4.9 Hz, 2H), 3.47-3.57 (m, 5 H), 0.19 (s, 9H)

### 2-((4-Ethynyl-2-fluorophenyl)amino)-N-(2-hydroxyethoxy)-1-methyl-1H-pyrrolor2.3-b]pyridine-3-carboxamide

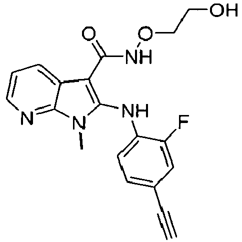

A solution of 2-((2-fluoro-4-((trimethylsilyl)ethynyl)phenyl)amino)-*N*-(2-hydroxyethoxy)-1-methyl-1*H*-pyrrolo[2,3-*b*]pyridine-3-carboxamide (100 mg, 0.23 mmol, 80% pure by UPLC-MS) in MeOH (2.1 mL) was treated with K₂CO₃ (35 mg, 0.25 mmol). The mixture was stirred for 18 h at room temperature. The mixture was purified by preparative HPLC to give the product (15 mg, 22%) as a white solid. UPLC-MS (Acidic Method, 2 min): rt 0.93 min, *m*/*z* 369.1 [M+H]⁺. ¹H NMR (400 MHz, DMSO-*d*₆) δ ppm 8.25 (br d, *J* = 3.5 Hz, 1H), 8.14 (d, *J* = 7.8 Hz, 1H), 7.35 (br d, J= 11.8 Hz, 1H), 7.21 (dd, *J* = 7.7, 4.8 Hz, 1H), 7.11 (dd, *J* = 8.3, 1.4 Hz, 1H), 6.63 (t, *J* = 8.7 Hz, 1H), 4.09 (s, 1H), 3.78 (t, *J* = 4.7 Hz, 2H), 3.37-3.55 (m, 5 H).

### Example 48: 2-((2-Fluoro-4-iodophenyl)amino)-N-hydroxy-1-methyl-1H-pyrrolo[2,3-b]pyridine-3-carboxamide

### Perfluorophenyl 2-((2-fluoro-4-iodophenyl)amino)-1-methyl-1H-pyrrolo[2,3-b]pyridine-3-carboxylate

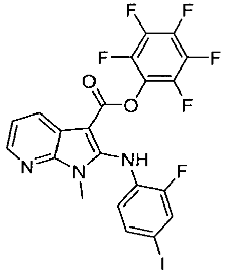

A solution of 2-((2-fluoro-4-iodophenyl)amino)-1-methyl-1*H*-pyrrolo[2,3-*b*]pyridine-3-carbonyl chloride (0.9 g, 2.1 mmol) in dry DCM (50 mL) was cooled to 0 °C in an ice bath and then treated with Et₃N (0.8 mL, 5.4 mmol) and pentafluorophenol (0.6 g, 3.2 mmol) and stirred for 1 h. The mixture was diluted with 1:1 DCM/H₂O solution. The organic phase was washed with brine, dried over Na₂SO₄, and concentrated *in vacuo* to give the product (1.56 g, quantitative) that was used in the next step without further purification. UPLC-MS (Acidic Method, 2 min): rt 1.43 min, *m*/*z* 577.8 [M+H]⁺. ¹H NMR (400 MHz, DMSO-*d*₆) δ ppm 9.13 (s, 1H), 8.29 (dd, *J* = 4.8, 1.6 Hz, 1H), 8.13-8.17 (m, 1H), 7.59-7.64 (m, 1H), 7.42 (dt, *J* = 8.4, 1.0 Hz, 1H), 7.20-7.31 (m, 1H), 6.95 (t, *J* = 8.7 Hz, 1H), 3.65 (s, 3H).

### 2-((2-Fluoro-4-iodophenyl)amino)-N-hydroxy-1-methyl-1H-pyrrolo[2,3-b]pyridine-3-carboxamide

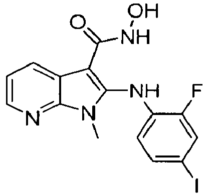

A suspension of perfluorophenyl 2-((2-fluoro-4-iodophenyl)amino)-1-methyl-1*H-*pyrrolo[2,3-*b*]-pyridine-3-carboxylate (400 mg, 0.69 mmol, 85% pure by UPLC-MS) in dry DMF (2.4 mL) was treated with hydroxylamine hydrochloride (58 mg, 0.83 mmol) and DIPEA (43 µL, 2.43 mmol). The mixture was stirred for 1h at room temperature. The mixture was concentrated *in vacuo,* then the residue was diluted with 1:1 EtOAc/H₂O solution. The organic phase was washed with water, brine, dried over Na₂SO₄, and concentrated *in vacuo.* The crude was purified by preparative HPLC to give the product (104 mg, 39%) as an off-white solid. UPLC-MS (Acidic Method, 2 min): rt 1.03 min, *m*/*z* 427.0 [M+H]⁺. ¹H NMR (400 MHz, DMSO-*d*₆) δ ppm 10.34 (br s, 1H), 8.83 (br s, 1H), 8.72 (br s, 1H), 8.26 (dd, *J* = 4.8, 1.5 Hz, 1H), 8.18 (dd, *J* = 7.8, 1.6 Hz, 1H), 7.64 (dd, *J* = 10.8, 2.0 Hz, 1H), 7.34 (dt, *J* = 8.4, 0.9 Hz, 1H), 7.21 (dd, *J* = 7.9, 4.8 Hz, 1H), 6.45 (t, *J* = 8.9 Hz, 1H), 3.52 (s, 3H).

### Example 49: (R)-N-(2,3-Dihydroxypropoxy)-2-((2-fluoro-4-iodophenyl)amino)-1-methyl-1H-pyrrolo[2,3-b]pyridine-3-carboxamide

### (R)-2-((2,2-Dimethyl-1,3-dioxolan-4-yl)methoxy)isoindoline-1,3-dione

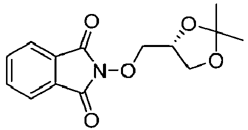

To a suspension of *N*-hydroxyphthalimide (6.6 g, 40.5 mmol) in THF (135 mL) at 0 °C was added triphenylphosphine (10.6 g, 40.5 mmol) and (*S*)-(2,2-dimethyl-[1,3]dioxolan-4-yl)-methanol (5 mL, 40.5 mmol). Diisopropyl azodicarboxylate (10.3 mL, 52.7 mmol) was added dropwise whilst keeping the internal temperature below 15 °C. Upon completion of the addition, the mixture was warmed to room temperature and stirred under N₂ for 2 h. The solvent was removed *in vacuo* and the residue was diluted with DCM (50 mL). The resulting precipitate was filtered and the filtrate was concentrated *in vacuo.* The crude was purified by flash column chromatography (Silica 340 g, 10-100% EtOAc in hexane) to give the product (11.1 g, 99%) as a white solid. UPLC-MS (Acidic Method, 2 min): rt 1.06 min, *m*/*z* 278.1 [M+H]⁺. ¹H NMR (400 MHz, CDCl₃) δ ppm 7.85-7.82 (m, 2H), 7.76-7.75 (m, 2H), 4.52-4.46 (m, 1H), 4.31 (dd, *J* = 10.0, 5.5 Hz, 1H), 4.17 (dd, *J* = 8.5, 6.0 Hz, 1H), 4.13 (dd, *J* = 10.0, 6.0 Hz, 1H), 3.96 (dd, *J* = 8.5, 5.5 Hz, 1H), 1.39 (s, 3H), 1.33 (s, 3H).

### (R)-O-((2,2-Dimethyl-1,3-dioxolan-4-yl)methyl)hydroxylamine

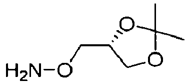

To a suspension of (*R*)-2-((2,2-dimethyl-1,3-dioxolan-4-yl)methoxy)isoindoline-1,3-dione (3.0 g, 10.8 mmol) in DCM (22 mL) at 0°C was added methyl hydrazine (0.62 mL, 11.9 mmol) dropwise. The resultant mixture was warmed to room temperature and stirred under N₂ for 1h. The solvent was removed *in vacuo* and the residue was diluted with diethyl ether (20 mL). The mixture was stirred for 0.5 h before filtering and washing with diethyl ether (2 × 20 mL). The filtrate was concentrated to dryness *in vacuo* to give the product (0.85 g, 46%) as a pale-yellow oil. ¹H NMR (400 MHz, CDCl₃) δ ppm 5.00-4.94 (m, 1H), 4.38-4.32 (m, 1H), 4.06 (dd, *J* = 8.5, 6.5 Hz, 1H), 3.74 (dd, *J* = 6.0, 5.0 Hz, 1H), 3.69 (dd, *J* = 8.5, 6.5 Hz, 1H), 1.43 (s, 3H), 1.37 (s, 3H).

### (R)-N-((2,2-Dimethyl-1,3-dioxolan-4-yl)methoxy)-2-((2-fluoro-4-iodophenyl)amino)-1-methyl-1H-pyrrolo[2,3-b]pyridine-3-carboxamide

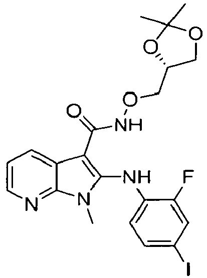

To a solution of perfluorophenyl 2-((2-fluoro-4-iodophenyl)amino)-1-methyl-1*H-*pyrrolo[2,3--*b*]pyridine-3-carboxylate, prepared as described in Example 4, (390 mg, 0.676 mmol) in DMF (2 mL) was added a solution of (*R*)-O-((2,2-dimethyl-1,3-dioxolan-4-yl)methyl)hydroxylamine (149 mg, 1.010 mmol) in DMF (0.5 mL) and DIPEA (24 µL, 1.350 mmol). The resultant mixture was stirred at room temperature under N₂ for 18 h. The reaction mixture was diluted with ice-cold H₂O (50 mL) and then extracted with EtOAc (2 × 25 mL). The combined organic phases were washed with brine (2 × 100 mL), dried over Na₂SO₄, and concentrated *in vacuo* to give the product (300 mg, 82%) as a dark red solid that was used in the next step without further purification. UPLC-MS (Acidic Method, 2 min): rt 1.15 min, *m*/*z* 541.1 [M+H]⁺. ¹H NMR (400 MHz, CDCl₃) δ ppm 8.82 (s, 1H), 8.75 (s, 1H), 8.26 (dd, *J* = 5.0, 1.5 Hz, 1H), 7.87 (d, *J* = 7.5 Hz, 1H), 7.50 (dd, *J* = 10.0, 1.5 Hz, 1H), 7.40-7.37 (m, 1H), 7.20 (dd, *J* = 7.5, 5.0 Hz, 1H), 6.66 (app t, *J* = 8.5 Hz, 1H), 5.00-4.95 (m, 1H), 4.51-4.45 (m, 1H), 4.18-4.05 (m, 2H), 3.85 (dd, *J* = 9.0, 6.5 Hz, 1H), 3.52 (s, 3H), 1.46 (s, 3H), 1.40 (s, 3H)

### Example 50: (R)-N-(2,3-Dihydroxypropoxy)-2-((2-fluoro-4-iodophenyl)amino)-1-methyl-1H-pyrrolo[2,3-b]pyridine-3-carboxamide

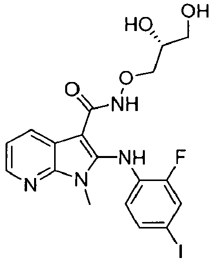

To a solution of (*R*)-*N*-(2,2-dimethyl-1,3-dioxolan-4-yl)methoxy)-2-((2-fluoro-4-iodophenyl)amino)-1-methyl-1*H*-pyrrolo[2,3-*b*]pyridine-3-carboxamide (220 mg, 0.40 mmol) in MeOH (5 mL) was added *p*-toluene sulfonic acid monohydrate (39 mg, 0.20 mmol) and ethylene glycol (13 µL, 2.40 mmol). The resultant mixture was stirred at room temperature under N₂ for 0.5h. A few drops of Et₃N were added to the reaction mixture and the solvent was removed in *vacuo.* The crude product was purified by preparatory HPLC to give the product (47 mg, 24%) as an off-white solid. UPLC-MS (Acidic Method, 2 min): rt 0.96 min, *m*/*z* 501.0 [M+H]⁺.¹H NMR (400 MHz, DMSO-*d*₆) δ ppm 10.89 (s, 1H), 8.71 (s, 1H), 8.27 (dd, *J* = 5.0, 1.5 Hz, 1H), 8.16 (dd, *J* = 7.5, 1.5 Hz, 1H), 7.64 (dd, *J* = 10.5, 1.5 Hz, 1H), 7.37-7.35 (m, 1H), 7.23 (dd, *J* = 7.5, 5.0 Hz, 1H), 6.55 (app t, *J* = 8.5 Hz, 1H), 4.93 (s, 1H), 4.60 (s, 1H), 3.87-3.82 (m, 1H), 3.72-3.66 (m, 2H), 3.54 (s, 3H), 3.39-3.35 (m, 2H).

### Example 51: N-(Cyclopropylmethoxy)-2-((2-fluoro-4-iodophenyl)amino)-1-methyl-1H-pyrrolo[2,3-b]pyridine-3-carboxamide

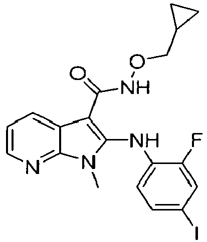

To a solution of perfluorophenyl 2-((2-fluoro-4-iodophenyl)amino)-1-methyl-1*H-*pyrrolo[2,3-*b*]pyridine-3-carboxylate, prepared as described in Example 4, (400 mg, 0.69 mmol) in DMF (2.5 mL) was added a solution of *O*-(cyclopropylmethyl)hydroxylamine hydrochloride (100 mg, 0.83 mmol) in DMF (0.5 mL) and DIPEA (24 µL, 1.35 mmol). The resultant mixture was stirred at room temperature under N₂ for 18 h. The reaction mixture was diluted with ice-cold H₂O (50 mL) and then extracted with EtOAc (2 × 25 mL). The combined organic phases were washed with brine (2 × 100 mL), dried over Na₂SO₄, and concentrated *in vacuo.* The crude product was purified by preparative HPLC (Reach Separations, UK) to give the product (135 mg, 36%) as a white solid. UPLC-MS (Acidic Method, 4 min): rt 1.95 min, *m*/*z* 481.0 [M+H]⁺. ¹H NMR (400 MHz, CDCl₃) δ ppm 11.42 (s, 1H), 10.75 (s, 1H), 8.67 (s, 1H), 8.25 (dd, *J* = 5.0, 1.5 Hz, 1H), 8.14 (dd, *J* = 8.0, 1.5 Hz, 1H), 7.63 (dd, *J* = 10.5, 2.0 Hz, 1H), 7.36-7.33 (m, 1H), 7.22 (dd, *J* = 7.5, 5.0 Hz, 1H), 3.56 (d, *J* = 7.0 Hz, 1H), 3.55 (s, 3H), 1.04-0.97 (m, 1H), 0.50-0.45 (m, 2H), 0.22-0.18 (m, 2H).

### Example 52: 2-((2-Fluoro-4-iodophenyl)amino)-N-(2-hydroxyethoxy)-5-methoxy-1-methyl-1H-pyrrolo[2,3-b]pyridine-3-carboxamide

### 5-Methoxy-1-methyl-1H-pyrrolo[2,3-b]pyridine

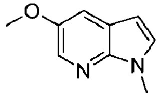

A solution of 5-methoxy-7-azaindole (5.0 g, 33.7 mmol) in dry DMF (25 mL) was cooled to 0 °C in an ice bath and treated with sodium hydride (1.6 g, 40.5 mmol, 60% in mineral oil) in a portion-wise manner. The resulting mixture was stirred for 1 h at 0 °C, then treated with iodomethane (2.3 mL, 37.1 mmol) and was stirred at 0 °C for 1 h. The mixture was then cautiously poured into H₂O (200 mL) and extracted with EtOAc (3 × 30 mL). The combined organic phases were washed sequentially with water (3 × 30 mL) and brine, dried over Na₂SO₄, filtered, and concentrated under vacuum to give the product (5.8 g, quantitative) as a light-brown solid. UPLC-MS (Acidic Method, 4 min): rt 0.91 min, *m*/*z* 161.1 [M+H]⁺. ¹H NMR (400 MHz, CDCl₃) δ ppm 8.12 (d, *J*=2.6 Hz, 1H), 7.42 (d, *J*=2.8 Hz, 1H), 7.27 (s, 1H), 7.16 (d, *J*=3.4 Hz, 1H), 6.37 (d, *J*=3.4 Hz, 1H), 3.88 (s, 3H), 3.87 (s, 3H).

### 5-Methoxy-methyl-1H-pyrrolo[2,3-b]pyridine-3-carboxylic acid

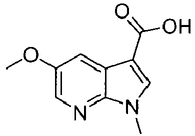

A solution of Sanethoxy-1-methyl-1*H*-pyrrolo[2,3-*b*]pyridine (5.5 g, 46.5 mmol) in dry DMF (7 mL) was cooled to 0 °C in an ice bath and treated with trifluoroacetic anhydride (10.6 g, 50.7 mmol). The reaction mixture was then gradually warmed up to room temperature and left stirring for 1 h. The resulting mixture was cooled to 0 °C in an ice bath and treated with water (200 mL) and extracted with DCM (3 × 30 mL). The combined organic phases were dried over Na₂SO₄, filtered, and concentrated under vacuum. The residue was suspended in 5M NaOH aqueous solution (68 mL) and heated at 50°C for 18 h. The reaction mixture was washed with Et₂O (1 × 30 mL) and cautiously treated with 1M HCl aqueous solution until pH = 1 leading to a formation of a beige precipitate. The solid was collected by filtration, washed with H₂O until the filtrate became pH neutral, and dried to give the product (5.9 g, 85%) as a beige solid. UPLC-MS (Acidic Method, 2 min): rt 0.80 min, *m*/*z* 207.1 [M+H]⁺. ¹H NMR (400 MHz, DMSO-*d*₆) δ ppm 8.18 (s, 1H), 8.10 (d, *J*=2.9 Hz, 1H), 7.81 (d, *J*=2.9 Hz, 1H), 3.86 (s, 3H), 3.84 (s, 3H)*.*

### tert-Butyl 5-methoxy-1-methyl-1H-pyrrolo[2,3-b]pyridine-3-carboxylate

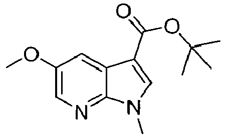

To a suspension of 5-methoxy-1-methyl-1*H*-pyrrolo[2,3-*b*]pyridine-3-carboxylic acid (5.9 g, 28.6 mmol) in anhydrous DCM (200 mL) cooled on ice, oxalyl chloride (7.3 mL, 85.8 mmol) was added dropwise over 15 min and the mixture was stirred at room temperature for 1.5 h. The mixture was then concentrated under vacuum to give a yellow solid, which was cooled on ice, then treated with *tert*-butanol (150 mL, 1.6 mol), followed by an addition of potassium *tert*-butoxide (5.1 g, 45.8 mmol). The resulting mixture was gradually warmed up to room temperature and left stirring for 18 h. The reaction mixture was concentrated under vacuum, distributed between EtOAc (50 mL) and H₂O (50 mL) and extracted with EtOAc (3 × 50 mL). The combined organic phases were dried over Na₂SO₄, filtered, and concentrated under vacuum. The crude material was purified by flash column chromatography (Silica 120 g, 0-3% MeOH in DCM) to give the product (3.9 g, 53%) as a yellow solid. UPLC-MS (Acidic Method, 2 min): rt 1.15 min, *m*/*z* 263.2 [M+H]⁺. ¹H NMR (400 MHz, DMSO-*d*₆) δ ppm 8.08 - 8.14 (m, 2H), 7.76 - 7.81 (m, 1H), 3.86 (s, 3H), 3.83 (s, 3H), 1.57 (s, 9H).

### tert-Butyl 2-chloro-5-methoxy-1-methyl-1H-pyrrolo[2,3-b]pyridine-3-carboxylate

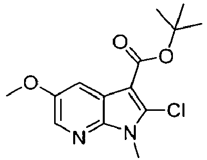

A solution of *tert*-butyl 5-methoxy-1-methyl-1*H*-pyrrolo[2,3-*b*]pyridine-3-carboxylate (2.2 g, 8.6 mmol) in dry THF (36 mL) was flushed with N₂, cooled to -78°C and then treated with a solution of LDA (2M in THF, 8.55 mL, 17.1 mmol). The mixture was stirred at -78 °C for 30 min. A solution of hexachloroethane (4.1 g, 17.1 mmol) in dry THF (12 mL) was added and the mixture was gradually warmed up to a room temperature and stirred for 2 h. The mixture was treated with saturated NH₄Cl aqueous solution and extracted with EtOAc (3 × 30 mL). The combined organic phases were dried over Na₂SO₄, filtered and concentrated under vacuum. The crude was purified by flash column chromatography (Silica 120 g, 0-10% EtOAc in hexanes) to give the product (2.4 g, 95%) as a beige solid. UPLC-MS (Acidic Method, 2 min): rt 1.27 min, *m*/*z* 297.1/299.0 [M+H]⁺. ¹H NMR (400 MHz, DMSO-*d*₆) δ ppm 8.12 (br d, *J*=2.6 Hz, 1H), 7.78 (br d, *J*=2.5 Hz, 1H), 3.86 (s, 3H), 3.79 (s, 3H), 1.59 (s, 9H).

### tert-Butyl 2-((2-fluoro-4-iodophenyl)amino)-5-methoxy-1-methyl-1H-pyrrolo[2,3-b]pyridine-3-carboxylate

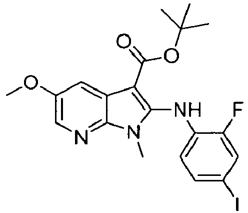

A suspension of *tert*-butyl 2-chloro-5-methoxy-1-methyl-1*H*-pyrrolo[2,3-*b*]pyridine-3-carboxylate (2.4 g, 8.1 mmol) and 2-fluoro-4-iodoaniline (1.8 g, 7.7 mmol) in dry THF (44 mL) was flushed with N₂, cooled to -78 °C and treated with a solution of LiHMDS (1M in THF, 16.2 mL, 16.2 mmol). The mixture was gradually warmed up to room temperature and stirred for 1.5 h. The mixture was quenched with saturated NH₄Cl aqueous solution and then extracted with EtOAc (3 × 25 mL). The combined organic phases were dried over Na₂SO₄, filtered, and concentrated under vacuum. The crude material was purified by flash column chromatography (Silica 120 g, 0-10% EtOAc in hexanes) to give the product (3.5 g, 91%) as a pale yellow solid. UPLC-MS (Acidic Method, 4 min): rt 2.59 min, *m*/*z* 498.0 [M+H]⁺. ¹H NMR (400 MHz, DMSO-*d*₆) δ ppm 8.56 (s, 1H), 7.98 - 8.03 (m, 1H), 7.74 (d, *J*=2.8 Hz, 1H), 7.64 (dd, *J*=10.8, 1.9 Hz, 1H), 7.37 (d, *J*=8.5 Hz, 1H), 6.65 (t, *J*=8.8 Hz, 1H), 3.86 (s, 3H), 3.56 (s, 3H), 1.41 (s, 9H)

### 2-((2-Fluoro-4-iodophenyl)amino)-5-methoxy-1-methyl-1H-pyrrolo[2,3-b]pyridine-3-carbonyl chloride

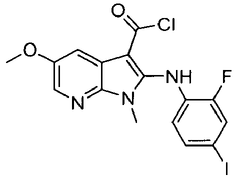

Thionyl chloride (5.1 mL, 69.8 mmol) was added to *tert*-butyl 2-((2-fluoro-4-iodophenyl)amino)-5-ethoxy-1-methyl-1*H*-pyrrolo[2,3-*b*]pyridine-3-carboxylate (3.5 g, 7.0 mmol) followed by H₂O (130 µL, 7.0 mmol). The flask was sealed with a rubber septum and the mixture was stirred at room temperature for 1.5 h. The mixture was concentrated to dryness under vacuum to give the product (3.6 g, 55%) as a beige solid and was used in the next step without further purification. UPLC-MS (Acidic Method, 2 min): rt 1.31 min, *m*/*z* 456.0 [M+H]⁺ (detected as the corresponding methyl ester after quenching an aliquot of the mixture with MeOH).

### 2-((2-Fluoro-4-iodophenyl)amino)-N-(2-hydroxyethoxy)-5-methoxy-1-methyl-1H-pyrrolo[2,3-b]pyridine-3-carboxamide

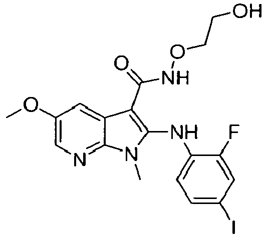

A solution of 2-((2-fluoro-4-iodophenyl)amino)-5-methoxy-1-methyl-1*H*-pyrrolo[2,3-b]pyridine-3-carbonyl chloride (1.0 g, 2.2 mmol) in dry DCM (57 mL) was cooled to 0 °C in an ice bath and then treated with dry pyridine (2 mL, 24.4 mmol) and the mixture was stirred for 5 min followed by an addition of (2-aminooxy)ethanol (0.4 g, 5.4 mmol) in dry DCM (5 mL). The mixture was stirred for 15 min, then concentrated under vacuum. The crude material was purified by preparative HPLC to give the product (85 mg, 13%) as a beige-yellow solid. UPLC-MS (Acidic Method, 2 min): rt 1.07 min, *m*/*z* 501.0 [M+H]⁺. ¹H NMR (400 MHz, DMSO-*d*₆) δ ppm 10.79 (s, 1H), 8.70 (br s, 1H), 8.02 (d, *J*=2.6 Hz, 1H), 7.75 (d, *J*=2.6 Hz, 1H), 7.64 (dd, *J*=10.9, 1.8 Hz, 1H), 7.35 (d, *J*=8.6 Hz, 1H), 6.52 (t, *J*=8.8 Hz, 1H), 4.75 (br t, *J*=5.8 Hz, 1H), 3.80 - 3.89 (m, 5H), 3.48 - 3.56 (m, 5H).

### Example 53: (S)-N-((2,2-dimethyl-1,3-dioxolan-4-yl)methyl)-2-((2-fluoro-4-iodophenyl)amino)1-methyl-1H-pyrrolo[2,3-b]pyridine-3-carboxamide

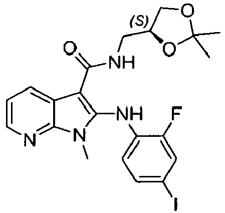

A solution of 2-((2-fluoro-4-iodophenyl)amino)-1-methyl-1*H*-pyrrolo[2,3-*b*]pyridine-3-carbonyl chloride (See Example 2, 937 mg, 2.18 mmol) in dry THF (4 mL) under N₂ was cooled in an ice-water bath while stirring. The reaction mixture was treated with a solution of (*S*)-(+)-(2,2-dimethyl-1,3-dioxolan-4-yl)methanamine (256 mg, 1.95 mmol) and diisopropylethylamine (0.33 mL, 1.95 mmol) in dry THF (5 mL) and stirred for 18 h. Reaction mixture was concentrated *in vacuo* and the crude residue was purified by flash column chromatography (Silica 40 g, 20-80% EtOAc in hexane) to give the product (512 mg, 44.4%) as an off-white solid which was used in subsequent steps without further purification. UPLC-MS (Acidic Method, 2 min): rt 1.10 min, *m*/*z* 525.0 [M+H]⁺. ¹H NMR (400 MHz, DMSO-*d*₆) δ ppm 8.84 (s, 1H), 8.34 (dd, *J*=7.9, 1.6 Hz, 1H), 8.29 (dd, *J*=4.8, 1.6 Hz, 1H), 7.65 (dd, *J*=10.7, 1.9 Hz, 1H), 7.59 (s, 1H), 7.32 - 7.36 (m, 1H), 7.24 (dd, *J*=7.8, 4.8 Hz, 1H), 6.44 (t, *J*=8.8 Hz, 1H), 4.05 - 4.10 (m, 1H), 3.86 (dd, *J*=8.3, 6.27 Hz, 1H), 3.56 - 3.62 (m, 1H), 3.54 (s, 3H), 3.36 (td, *J*=5.8, 2.0 Hz, 2H), 1.26 (s, 3H), 1.22 (s, 3H).

### Example 54: (S)-N-(2,3-dihydroxypropyl)-2-((2-fluoro-4-iodophenyl)amino)-1-methyl-1H-pyrrolo[2,3-b]pyridine-3-carboxamide

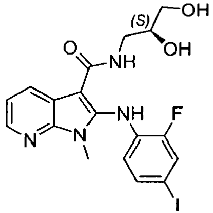

A solution of (*S*)-*N*-((2,2-dimethyl-1,3-dioxolan-4-yl)methyl)-2-((2-fluoro-4-iodophenyl)-amino)-1-methyl-1*H*-pyrrolo[2,3-*b*]pyridine-3-carboxamide (512 mg, 0.97 mmol) in 1,4-dioxane (5.1 mL) was treated with 4 N HCl in 1,4-dioxane (0.61 mL, 2.42 mmol) and stirred at room temperature for 72 h. The reaction mixture was concentrated, the residue was resuspended in 1,4-dioxane (5.1 mL) with addition of 4 N HCl in 1,4-dioxane (0.61 mL, 2.42 mmol) and stirred for 16 h until completion. The reaction mixture was concentrated and the crude residue was purified by preparatory HPLC to give the product (172 mg, 36.6%) as a flocculant white solid. UPLC-MS (Acidic Method, 2 min): rt 0.89 min, *m*/*z* 485.0 [M+H]⁺. ¹H NMR (400 MHz, DMSO-*d*₆) δ ppm 8.89 (s, 1H), 8.33 (dd, *J*=7.9, 1.5 Hz, 1H), 8.29 (dd, *J*=4.8, 1.5 Hz, 1H), 7.65 (dd, *J*=10.7, 1.9 Hz, 1H), 7.52 (t, *J*=5.5 Hz, 1H), 7.35 (d, *J*=8.6 Hz, 1H), 7.24 (dd, *J*=7.9, 4.8 Hz, 1H), 6.46 (t, *J*=8.8 Hz, 1H), 4.79 (d, *J*= 4.8 Hz, 1H), 4.57 (t, *J*= 5.8 Hz, 1H), 3.52 (m, 4H), 3.39 (m, 1H), 3.15 - 3.31 (m, 3H).

### Example 55: ((R)-N-((2,2-dimethyl-1,3-dioxolan-4-yl)methyl)-2-((2-fluoro-4-iodophenyl)amino)-1-methyl-1H-pyrrolo[2,3-b]pyridine-3-carboxamide

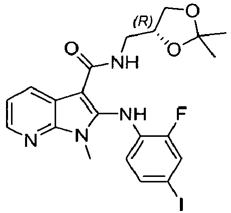

A solution of 2-((2-fluoro-4-iodophenyl)amino)-1-methyl-1*H*-pyrrolo[2,3-*b*]pyridine-3-carbonyl chloride (See Example 2, 937 mg, 2.18 mmol) in dry THF (4 mL) under N₂ was cooled in an ice-water bath while stirring. The reaction mixture was treated with a solution of (R)-(-)-(2,2-dimethyl-1,3-dioxolan-4-yl)methanamine (256 mg, 1.95 mmol) and diisopropylethylamine (0.33 mL, 1.95 mmol) in dry THF (5 mL) and stirred for 18 h. Reaction mixture was concentrated *in vacuo* and the crude residue was purified by flash column chromatography (Silica 40 g, 20-80% EtOAc in hexane) to give the product (442 mg, 38.5%) as an off-white solid which was used in subsequent steps without further purification. UPLC-MS (Acidic Method, 2 min): rt 1.10 min, *m*/*z* 525.0 [M+H]⁺. ¹H NMR (400 MHz, DMSO-*d*₆) δ ppm 8.83 (s, 1H), 8.32 - 8.36 (m, 1H), 8.27 - 8.32 (m, 1H), 7.62 - 7.68 (m, 1H), 7.55 - 7.62 (m, 1H), 7.31 - 7.37 (m, 1H), 7.21 - 7.27 (m, 1H), 6.40 - 6.47 (m, 1H), 4.05 - 4.09 (m, 1H), 3.84 - 3.89 (m, 1H), 3.57 - 3.62 (m, 1H), 3.54 (s, 3H), 3.37 - 3.38 (m, 2H), 1.26 (s, 3H), 1.22 (s, 3H).

### Example 56: (R)-N-(2,3-dihydroxypropyl)-2-((2-fluoro-4-iodophenyl)amino)-1-methyl-1H-pyrrolo[2,3-b]pyridine-3-carboxamide

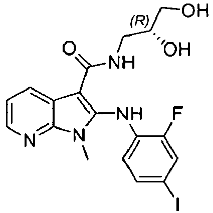

A solution of (*R*)-*N*-((2,2-dimethyl-1,3-dioxolan-4-yl)methyl)-2-((2-fluoro-4-iodophenyl)-amino)-1-methyl-1*H*-pyrrolo[2,3-*b*]pyridine-3-carboxamide (442 mg, 0.84 mmol) in 1,4-dioxane (4.4 mL) was treated with 4 N HCl in 1,4-dioxane (0.52 mL, 2.1 mmol) and stirred at room temperature for 72 h. The reaction mixture was concentrated, the residue was resuspended in 1,4-dioxane (4.4 mL) with addition of 4 N HCl in 1,4-dioxane (0.52 mL, 2.1 mmol) and stirred for 16 h until completion. The reaction mixture was concentrated and the crude residue was purified by preparatory HPLC to give the product (156 mg, 38%) as a flocculant white solid. UPLC-MS (Acidic Method, 2 min): rt 0.89 min, *m*/*z* 485.0 [M+H]⁺. ¹H NMR: (400 MHz, DMSO-*d*₆) δ ppm 8.89 (s, 1H), 8.33 (dd, *J*=7.9, 1.5 Hz, 1H), 8.29 (dd, *J*=4.8, 1.5 Hz, 1H), 7.64 (dd, *J*=10.8, 1.9 Hz, 1H), 7.48 - 7.56 (m, 1H), 7.35 (dd, *J*=8.5, 1.1 Hz, 1H), 7.24 (dd, *J*=7.8, 4.8 Hz, 1H), 6.46 (t, *J*=8.8 Hz, 1H), 4.79 (d, *J*=4.8 Hz, 1H), 4.57 (t, *J*=5.8 Hz, 1H), 3.50 - 3.57 (m, 4H), 3.41 (dt, *J*=13.2, 5.6 Hz, 1H), 3.14 - 3.31 (m, 3H).

### Example 57: 2-(2-Fluoro-4-iodoanilino)-1-methyl-1H-pyrrolo[2,3-b]pyridine-3-carboxamide

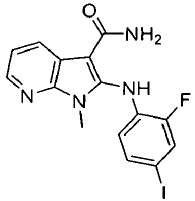

A suspension of 2-((2-fluoro-4-iodophenyl)amino)-1-methyl-1*H*-pyrrolo[2,3-*b*]pyridine-3-carbonyl chloride (0.50 g, 1.16 mmol) in 1,4-dioxane (2.3 mL) was stirred under N₂ on an ice/water bath and 0.5 M NH₃ in 1,4-dioxane (2.7 mL, 1.33 mmol) was added dropwise over 5 min. An additional portion of 1,4-dioxane (2.3 mL) was added and the reaction mixture was stirred for the next 18 h while warming up to room temperature. Then the reaction mixture was concentrated to dryness *in vacuo* and the crude was purified by flash column chromatography (Silica 20 g, 20-100% EtOAC in hexane) to give the product (39.1 mg, 9%) as an off-white solid. UPLC-MS (Acidic Method, 2 min): rt 0.97 min, *m*/*z* 411.0 [M+H]⁺. ¹H NMR (400 MHz, DMSO-*d*₆) δ ppm 9.07 (s, 1H), 8.34 (dd, *J* = 7.9, 1.5 Hz, 1H), 8.27 (dd, *J* = 4.8, 1.5 Hz, 1H), 7.67 (dd, *J* = 10.7, 1.9 Hz, 1H), 7.38 (dd, *J* = 8.4, 1.1 Hz, 1H), 7.22 (dd, *J* = 7.9, 4.8 Hz, 1H), 7.13 (br s, 2H), 6.52 (t, *J* = 8.8 Hz, 1H), 3.50 (s, 3H).

### Example 58: 2-((4-ethynyl-2-fluorophenyl)amino)-N-(2-hydroxyethoxy)thieno[2,3-b]pyridine-3-carboxamide

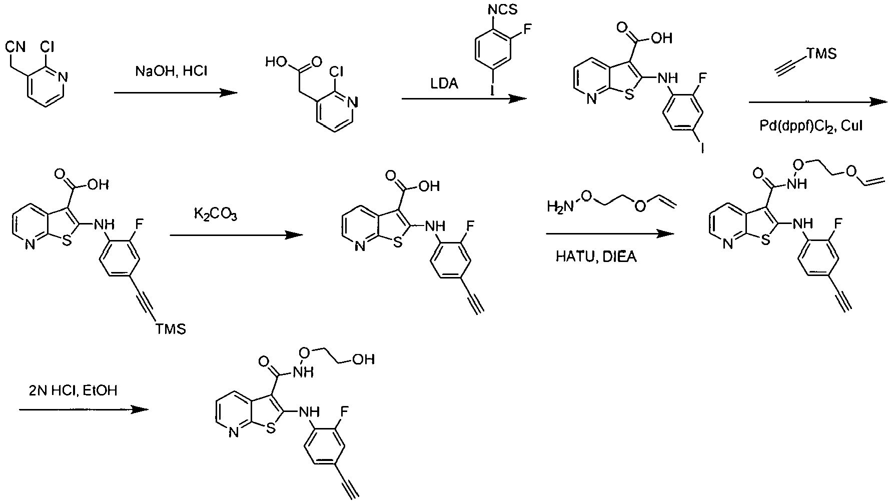

### Step 1: 2-fluoro-4-iodo-1-isothiocyanatobenzene

Thiophosgene (8.0 g, 69.6 mmol) was added to a rapidly stirred mixture of 2-fluoro-4-iodoaniline (15.00 g, 63.3 mmol) in dichloromethane (200 mL) and water (150 mL). The reaction mixture was stirred at room temperature overnight. The organic phase was separated, washed with saturated sodium bicarbonate solution, dried over sodium sulfate and filtered. The solvents were removed under vacuum to give the title compound as a beige colored solid (17.1 g, 96.8%). ¹HNMR (300 MHz, DMSO-d6): δ 7.87 (dd, J=1.8Hz and 1.8Hz, 1H), 7.60 (d, J=8.1Hz, 1H), 7.24 (t, J=8.3Hz, 1H).

### Step 2: 2-(2-chloropyridin-3-yl)acetic acid

A solution of 15% w/w sodium hydroxide (150 mL) was added to 2-(2-chloropyridin-3-yl)acetonitrile (10.0 g, 62.3 mmol). The mixture was heated at reflux for 1 hour then cooled to room temperature. The mixture was further cooled to 0-5 °C and then acidified with con. HCl (~60 mL) to pH 1. The suspension was left to stand for 1 hour in an ice bath. The precipitate formed was collected by filtration and washed with cold water, then cold 2-propanol (100 mL × 2). The solid was dried in a vacuo to get the title compound as an off-white solid (10.6 g, 99%). ¹HNMR (300 MHz, d⁶-DMSO): δ 12.63 (s, 1H), 8.32 (dd, J = 4.8 and 1.9 Hz, 1H), 7.86 (dd, J = 7.6 and 1.9 Hz, 1H), 7.41 (dd, J = 7.5 and 4.5 Hz, 1H), 3.75 (s, 2H).

### Step 3: 2-((2-fluoro-4-iodophenyl)amino)thieno[2,3-b]yridine-3-carboxylic acid

To a stirred solution of diisopropyl amine (0.82 mL, 5.8 mmol) in anhydrous THF (5 mL) cooled to -15°C was added n-butyl lithium (2.5 M in hexanes, 2.3 mL, 5.8 mmol) slowly, maintaining the temperature of the flask between -10°C and 0°C. The resultant mixture was stirred at room temperature for 15 minutes before being cooling to 0°C. The LDA thus formed was added to a rapidly stirred suspension of 2-(2-chloropyridin-3-yl)acetic acid (500 mg, 2.9 mmol) in anhydrous THF (10 mL) at 0°C. The resultant bright yellow suspension was stirred at 0°C for 15 min. A solution of 2-fluoro-4-iodo-1-isothiocyanatobenzene (814 mg, 2.9 mmol) in anhydrous THF (10 mL) was then added to the reaction mixture (brown suspension) and heated to 65°C for 18 hours. The reaction mixture was cooled and the volatiles removed in vacuo. The resultant crude product was redissolved in THF, cooled to 0°C and 10% aqueous acetic acid in water (10 mL) was added slowly. Acetonitrile (5 mL) was added slowly until a brown solid developed, the solid was isolated by filtration and washed with ether and acetonitrile to give the title compound. LC/MS: [M+1]⁺ 415; ¹H NMR (300 MHz, DMSO-d6): δ 10.74 (s, 1H), 9.21 (s, 1H), 8.36-8.25 (m, 2H), 7.79 (d, J = 1.8 Hz, 1H), 7.68-7.61 (m, 1H), 7.51 (t, J=8.5 Hz, 1H), 7.42-7.31 (m, 1H).

### Step 4: 2-((2-fluoro-4-((trimethylsilyl)ethynyl)phenyl)amino)thieno[2,3-b]pyridine-3-carboxylic acid

A round bottom flask was charged with 2-((2-fluoro-4-iodophenyl)amino)thieno[2,3-b]pyridine-3-carboxylic acid (0.150 g, 0.4 mmol), copper iodide (3 mg, 0.02 mmol) bis(triphenyl)palladium(ll)dichloride (12 mg, 0.02 mmol) in dry THF (10 mL) under nitrogen. The flask was degassed and flushed with nitrogen 3 times. Then trimethyl silyl acetylene (0.040 g, 0.4 mmol) dissolved in 0.4 mL of triethylamine was added very slowly over a period of 10 minutes. The suspension was stirred for 15 hours. The reaction mixture was then poured into ethyl acetate (150 mL) and washed with water (3X50 mL), brine (50 mL) and dried over Na₂SO₄. The solvents were removed under reduced pressure and the residue purified by flash chromatography (12 g silica, 0-10% MeOH in DCM) to get the product as a pale brown oil (90 mg, 65%). LC/MS: [M+1] 385.1.

### Step 5: 2-((4-ethynyl-2-fluorophenyl)amino)thieno[2,3-b]pyridine-3-carboxylicacid

A round bottom flask was charged with 2-((2-fluoro-4-((trimethylsilyl)ethynyl)phenyl)amino)thieno[2,3-b]pyridine-3-carboxylic acid (0.090 g, 0.2 mmol), methanol (5 mL) and THF (1 mL) and potassium carbonate (0.065 g, 0.5 mmol) was added to the resulting suspension. The reaction mixture was stirred at room temperature for 3 hours at which point both TLC and LC/MS indicated completion of the reaction. Water was added to the reaction mixture and extracted with ethyl acetate (3X25 mL) and the combined organics were washed with brine and dried over MgSO4. The solvents were evaporated to dryness to give the compound as brown solid (60 mg, 82%). This was used in the next reaction without further purification. LC/MS: 312.3 [M+1].

### Step 6: 2-((4-ethynyl-2-fluorophenyl)amino)-N-(2-(vinyloxy)ethoxy)thieno[2,3-b]pyiridine-3-carboxamide

A microwave vial was charged with 2-((4-ethynyl-2-fluorophenyl)amino)thieno[2,3-b]pyridine-3-carboxylic acid (0.060 g, 0.2 mmol), O-(2-(vinyloxy)ethyl)hydroxylamine (0.03 g, 0.3 mmol), HATU (0.11 g, 0.3 mmol) and diisopropyl ethyl amine (66 ul, 0.4 mmol) in DMF(4 mL). The reaction mixture was stirred at room temperature for 2 hours. The reaction was diluted with water (50 mL) and extracted with ethyl acetate (3X25 mL). The solvents were removed under reduced pressure and the residue purified by flash chromatography (4g silica, 0-5% MeOH in DCM) to get the product as yellow solid (35 mg, 46%). LC/MS: 398.2 [M+1].

### Step 7: 2-((4-ethynyl-2-fluorophenyl)amino)-N-(2-hydroxyethoxy)thieno[2,3-b]pyridine-3-carboxamide

A round bottom flask was charged with 2-((4-ethynyl-2-fluorophenyl)amino)-N-(2-(vinyloxy)ethoxy)thieno[2,3-b]pyridine-3-carboxamide (0.03 g, 0.1 mmol), ethanol (3 mL) and 2N HCl (1 mL) and the reaction mixture stirred at room temperature for 1 hour. The solvents were removed and the aqueous residue was neutralized to pH 7 with 1N NaOH solution and extracted with ethyl acetate (3X25 mL). The combined organics were washed with water, brine and dried over MgSO4. The solvents were removed under reduced pressure and the residue was purified by flash chromatography (4g, 0-10% methanol in dichloromethane) to get the product as pale yellow solid (13 mg, 43%). LC/MS: [M+1]⁺ 372.0; ¹H NMR (300 MHz, CDCl3): δ 8.47 (s, 1H), 8.36 (dd, J=4.5 and 1.2 Hz, 1H), 7.90 (dd, J=8.1 and 1.2 Hz, 1H), 7.1 (t, J=8.4 Hz, 1H), 7.37-7.30 (m, 3H), 4.17-4.15 (m, 2H), 3.85 (t, J=4.5 Hz, 2H).

### Example 59: 2-((2-fluoro-4-(methylthio)phenyl)amino)-N-(2-hydroxyethoxy)thieno[2,3-b]pyridine-3-carboxamide

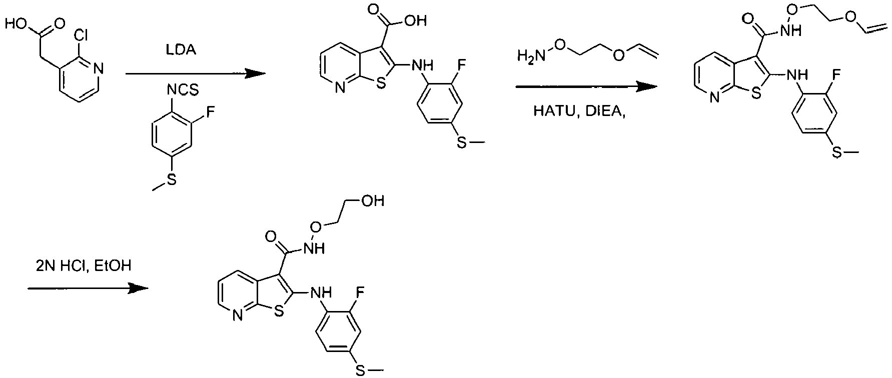

### Step 1: (3-fluoro-4-isothiocyanatophenyl)(methyl)sulfane

Thiophosgene (5.48 g, 47.7 mmol) was added to a rapidly stirred mixture of 2-Fluoro-4-(methylsulphanyl)aniline (5.00 g, 31.8 mmol) in DCM (60 mL) and water(40 mL). The reaction mixture was stirred at room temperature overnight. The organic phase was separated, washed with saturated sodium bicarbonate solution, dried over sodium sulfate and filtered. The solvents were removed in vacuo to give the title compound as a yellow solid (5 g, 78.9%). ¹HNMR (300 MHz, DMSO-d6): δ 7.41-7.31 (m, 2H), 7.09 (d, J=8.4 Hz, 1H), 2.49 (s,3H).

### Step 2: 2-(2-fluoro-4-(methylthio)phenylamino)thieno[2,3-b]pyridine-3-carboxylic acid

To a stirred solution of diisopropyl amine (1.65 mL, 11.7 mmol) in anthydrous THF (10 mL) cooled to -15°C was added n-butyl lithium (2.5 M in hexanes, 4.80 mL, 12.0 mmol) slowly between -10°C and 0°C. The resultant mixture was stirred at room temperature for 15 minutes before being cooled to 0°C. The solution of LDA thus formed was added to a rapidly stirred suspension of 2-(2-chloropyridin-3-yl)acetic acid (1.00 g, 5.8 mmol) in anhydrous THF( 20 mL ) at 0°C. Upon complete addition of the LDA solution the resultant bright yellow suspension was stirred at 0°C for 15 minutes. A solution of (3-fluoro-4-isothiocyanatophenyl)(methyl)sulfane (1.63 g, 8.2 mmol) in anhydrous THF (10 mL) was then added to the reaction mixture and heated to 65°C for 18 hours. The reaction mixture was cooled to room temperature and the volatiles removed in vacuo. The resultant brown gum was redissolved in THF, cooled to 0°C and 10% aq acetic acid 10 mL added slowly. Acetonitrile (5 mL) was added slowly until a yellow solid developed, the solid was isolated by filtration and washed with ether and acetonitrile to give the title compound as a yellow solid (546mg, 20%). LC/MS: [M+1] 335. ¹HNMR ( 300 MHz, DMSO-d6): δ 8.34 ( d, J=8.1 Hz, 1H ), 7.85-8.20 ( m, 1H), 7.61 (t, J = 8.6 Hz, 1H), 7.39-7.30 ( m, 2H), 7.21 (d, J = 9.2 Hz, 1H ), 2.52 (s, 3H).

### Step 3: N-(2-(vinyloxy)ethoxy)-2-(2-fluoro-4-(methylthio)phenylamino)thieno[2,3-b]pyridine-3-carboxamide

A vial was charged with 2-(2-fluoro-4-(methylthio)phenylamino)thieno[2,3-b]pyridine-3-carboxylic acid (0.100 g, 0.1 mmol), O-(2-(vinyloxy)ethyl)hydroxylamine (25 mg, 0.2 mmol), HATU (0.085 g, 0.2 mmol) and diisopropyl ethyl amine (52 ul, 0.3 mmol) and DMF (4 mL). The reaction mixture was stirred at room temperature overnight. The reaction was quenched with water and extracted with ethyl acetate. The organics were washed with water, brine and dried over sodium sulfate. The solvents were evaporated to give a light yellow solid (88 mg) which was used without further purification. LC/MS: [M+1] 420.0.

### Step 4: 2-(2-fluoro-4-(methylthio)phenylamino)-N-(2-hydroxyethoxy)thieno[2,3-b]pyridine-3-carboxamide

A microwave reaction vial was charged with N-(2-(vinyloxy)ethoxy)-2-(2-fluoro-4-(methylthio)phenylamino)thieno[2,3-b]pyridine-3-carboxamide (0.088 g), ethanol (2 mL), and 2N HCl (2mL). The reaction mixture was stirred at room temperature for one hour. The reaction was quenched with water and adjusted to pH to 8 - 10, then extracted with ethyl acetate. The organics were washed with water, brine and dried over sodium sulfate. The solvents were evaporated. The residue was purified by flash chromatography (4 g silica, 0-5% MeOH/DCM). The product fractions were collected and the solvents were removed in vacuo. The residue was purified again by preparative TLC (5% MeOH/DCM) and dried to give a light yellow solid (12 mg, 24%). LC/MS: [M+1]: 394.19. ¹HNMR ( 300 MHz, CDCl3 ): δ10.70 (s, 1H), 8.37 ( s, 1H ), 8.33-7.84 ( m, 1H ), 7.88-7.81 ( m, 1H ), 7.52 (t, J = 8.4Hz, 1H ), 7.32-7.25 ( m, 1H ), 7.12-7.06 ( m, 1H ), 4.15 ( t, J = 4.38Hz, 2H), 3.85 ( m, 2H ), 2.46 ( s, 3H).

### Example 60: Synthesis of 2-((2-Fluoro-4-iodophenyl)amino)-N-(2-hydroxyethoxy)thieno[2,3-b]pyridine-3-carboxamide

### Step 1: (2-Chloropyridin-3-yl)methanol

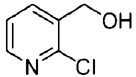

To a stirred solution of methyl 2-chloronicotinate (25 g, 145.71 mmol) in anhydrous THF (250 mL) at 0 °C under an atmosphere of nitrogen was added solid lithium aluminium hydride (11.06 g, 291.41 mmol) in small portions over a period of 20 min, maintaining the internal temperature below 5 °C. The resulting grey suspension was heated to 50 °C for 2 h, before being allowed to cool to room temperature with stirring over 16 h. The reaction mixture was then cooled to 0 °C and quenched by the cautious addition of a saturated solution of aqueous sodium sulphate, resulting in the formation of a suspension. The mixture was passed through a pad of celite, and the pad was subsequently washed with ethyl acetate (2 × 50 mL). The combined filtrates were diluted with ethyl acetate (500 mL) and washed water (250 mL), before being dried over sodium sulphate, filtered and evaporated to dryness to give the crude product as a brown oil. Purification by silica gel chromatography eluting with a gradient of ethyl acetate (10 to 40%) in hexane afforded the desired compound as a pale brown solid (7.13 g, 34.1%). UPLC-MS (Acidic Method, 2 min): rt = 0.60 min, *m*/*z* 144.0 [M+H]⁺. ¹H NMR (400 MHz, CDCl₃) δ ppm 8.28 (dd, *J* = 4.77Hz, 1.76Hz, 1H), 7.93 (dt, *J* = 7.59Hz, 0.97Hz, 0.97Hz, 1H), 7.29 (dd, *J* = 7.40Hz, 5.14Hz, 1H), 4.79 (s, 2H), 3.23 (br, s, 1H).

### Step 2: (2-Chloropyridin-3-yl)methyl methanesulfonate

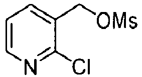

Methanesulfonyl chloride (7.70 mL, 99.380 mmol) was added dropwise to a stirred solution of (2-chloropyridin-3-yl)methanol (7.134 g, 49.960 mmol) and Et₃N (13.85 mL, 99.380 mmol) in dichloromethane (50 mL) at 0 °C, taking care to maintain the internal temperature below 5 °C. Upon completion of the addition, the reaction mixture was warmed to ambient temperature with stirring for 2 h. The reaction mixture was quenched with water (200 mL) and extracted with dichloromethane (2 × 200 mL). The combined organics were dried over Na₂SO₄, filtered and evaporated to dryness to afford the desired product as a brown oil (12.02 g, 100%). This material was taken in to the next step without any further purification. UPLC-MS (Acidic Method, 2 min): rt = 0.79 min, *m*/*z* 222.0 [M+H]⁺. ¹H NMR (400 MHz, CDCl₃) δ ppm 8.43-8.36 (m, 1H), 7.88-7.84 (m, 1H), 7.35-7.28 (m, 1H), 5.33 (s, 2H), 3.69 (s, 1H), 3.11 (s, 3H)

### Step 3: 2-(2-Chloropyridin-3-yl)acetonitrile

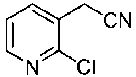

Sodium cyanide (7.98 g, 162.77 mmol) was added in a single portion to a solution of (2-chloropyridin-3-yl)methyl methanesulfonate (12.02 g, 54.24 mmol) in DMF (50 mL) at room temperature, and the resulting mixture was stirred at the same temperature for 2 h. The reaction was then poured into water (200 mL), and the resulting mixture was extracted with ethyl acetate (2 × 300 mL). The combined organics were dried over Na₂SO₄, filtered and evaporated to dryness to give the crude product, which was purified by column chromatography eluting with a gradient of ethyl acetate (10 to 30%) in hexane to afford the desired product as a pale brown solid (5.34 g, 64.5%). UPLC-MS (Acidic Method, 2 min): rt = 0.73 min, *m*/*z* 153.0 [M+H]⁺. ¹H NMR (400 MHz, CDCl₃) δ 8.40 (dd, *J* = 4.77Hz, 1.76Hz, 1H), 7.89 (ddt, *J* = 7.62Hz, 1.79Hz, 0.82Hz, 0.82Hz, 1H), 7.34 (dd, *J* = 7.53Hz, 4.77Hz, 1H), 3.87 (s, 2H).

### Step 4: 2-(2-Chloropyridin-3-yl)acetic acid

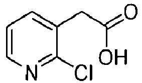

2-(2-Chloropyridin-3-yl)acetonitrile (5.34 g, 34.97 mmol) was dissolved in a 15% (w/w) aqueous NaOH solution (50 mL), and the resulting solution was stirred at ambient temperature for 60 min. The reaction was then acidified to pH = 1 with concentrated hydrochloric acid, forming a beige precipitate, which was collected by filtration, washed with water (2 × 25 mL) and dried in an oven under vacuum at 40 °C to afford the desired product as a beige solid (5.57 g, 92.8%). UPLC-MS (Acidic Method, 2 min): rt = 0.66 min, *m*/*z* 172.0 [M+H]⁺. ¹H NMR (400 MHz, DMSO-d6) δ ppm 12.64 (br, s, 1H), 8.33 (dd, *J* = 4.77Hz, 2.01Hz, 1H), 7.86 (dd, *J* = 7.53Hz, 2.01Hz, 1H), 7.41 (dd, *J* = 7.53Hz, 4.77Hz, 1H), 3.76 (s, 2H).

### Step 5: tert-Butyl 2-(2-chloropyridin-3-yl)acetate

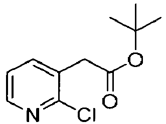

To a cooled solution of *N*,*N'*-dicyclohexylcarbodiimide (7.36 g, 35.69 mmol, ) in dichloromethane (120 mL) was added DMAP (3.17 g, 25.96 mmol) at 0 °C, followed by 2-(2-chloropyridin-3-yl)acetic acid (5.57 g, 32.45 mmol), and the resulting mixture was stirred at 0 °C for 5 min. *tert*-Butanol (9.3 mL, 97.337 mmol) was then added to the reaction, and the resulting mixture was allowed to warm to room temperature with stirring for 12 h. The reaction was then evaporated to dryness to give a residue, which was dissolved in diethyl ether (400 mL). The ether solution was then filtered through a pad of celite, which was washed with diethyl ether (2 × 200 mL). The combined filtrates were washed sequentially with 1 M aqueous NaOH (300 mL), 2 N aqueous HCl (300 mL), water (300 mL) and brine (200 mL). The organic layer was then dried over Na₂SO₄, filtered and evaporated to dryness to give the crude product as a residue. Purification by flash column chromatography eluting with a gradient of ethyl acetate (5-20%) in hexane to afford the desired product as beige solid (5.25 g, 71.0%). UPLC-MS (Acidic Method, 2 min): rt = 1.08 min, *m*/*z* 228.1 [M+H]⁺. ¹H NMR (400 MHz, CDCl₃) δ ppm 8.31 (dd, *J*=4.77Hz, 2.01Hz, 1H), 7.63 (dd, *J*=7.53Hz, 2.01Hz, 1H), 7.22 (dd, *J*=7.53Hz, 4.77Hz, 1H), 3.68 (s, 2H), 1.46 (s, 9H).

### Step 6: tert-Butyl-2-((2-fluoro-4-iodophenyl)amino)thieno[2,3-b]pyridine-3-carboxylate

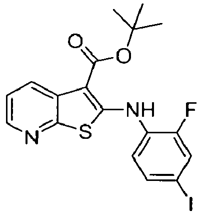

To a solution of tert-butyl 2-(2-chloropyridin-3-yl)acetate (5.05 g, 22.16 mmol, 1 eq) in tetrahydrofuran (200 mL) was added sodium *tert*-butoxide (2.24 g, 23.27 mmol, 1.05 eq), and the resulting mixture was stirred at room temperature for 15 min. 2-Fluoro-4-iodo-1-isothiocyanatobenzene (6.18 g, 22.16 mmol, 1 eq) was then added to the reaction, and the resulting solution was stirred for 30 min. The mixture was then stirred at reflux for 16 h, before being cooled to room temperature and partitioned between ethyl acetate (300 mL) and water (300 mL). The aqueous layer was collected and extracted with ethyl acetate (300 mL), and the combined organics were dried over Na₂SO₄, filtered and concentrated. The crude material was purified by column chromatography eluting with dichloromethane to afford the desired product as a beige solid (8.49 g, 78.3%). UPLC-MS (Acidic Method, 2 min): rt = 1.54 min, *m*/*z* 471.0 [M+H]⁺. ¹H NMR (400 MHz, CDCl₃) δ ppm 10.70 (br, s, 1H), 8.25 (q, *J*=1.67Hz, 1H), 8.23 (s, 1H), 7.46-7.43 (m, 2H), 7.40-7.35 (m, 1H), 7.19 (dd, *J*=4.52Hz, 3.01Hz, 1H), 1.61 (s, 9H).

### Step 7: 2-((2-Fluoro-4-iodophenyl)amino)thieno[2,3-b]pyridine-3-carboxylic acid

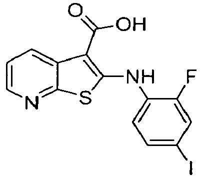

A solution of *tert*-butyl 2-((2-fluoro-4-iodophenyl)amino)thieno[2,3-*b*]pyridine-3-carboxylate (1.00 g, 2.13 mmol) in dichloromethane (20 mL) was treated with 4 N HCl in dioxane (20 mL), and the resulting mixture was stirred at room temperature for 48 h. The solution was then concentrated to dryness to afford an oil, which was co-distilled with dichloromethane (2 × 25 mL) to afford the desired product as a yellow solid (1.04 g, 100%). UPLC-MS (Acidic Method, 2 min): rt = 1.24 min, *m*/*z* 414.9 [M+H]⁺. ¹H NMR (400 MHz, DMSO-*d*₆) δ ppm 10.73 (s, 1H), 8.42 (dd, *J*=8.16Hz, 1.63Hz, 1H), 8.38 (dd, *J* = 4.77Hz, 1.51Hz, 1H), 7.90 (dd, *J* = 10.16Hz, 1.88Hz, 1H), 7.76-7.73 (m, 1H), 7.60 (t, *J* = 8.53Hz, 8.53Hz, 1H), 7.48 (dd *J* = 8.28Hz, 4.77Hz, 1H), 3.63 (s, 1H).

### Step 8: 2-((2-Fluoro-4-iodophenyl)amino)-N-(2-hydroxyethoxy)thieno[2,3-b]pyridine-3-carboxamide

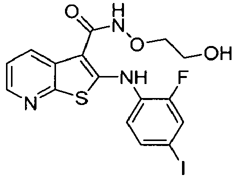

To a stirred solution of 2-((2-fluoro-4-iodophenyl)amino)thieno[2,3-b]pyridine-3-carboxylic acid (1.5 g, 3.62 mmol, 1 eq), HATU (1.9 g, 5.07 mmol, 1.4 eq) and pyridine (0.59 mL, 7.24 mmol, 2 eq) in a mixture of DMF (15 mL) and DMSO (15 mL) was added 2-aminooxyethanol (0.56 g, 7.24 mmol, 2 eq), and the resulting mixture was stirred at room temperature for 16 h. The reaction was poured into water (100 mL), and the resulting mixture was extracted with ethyl acetate (3 × 50 mL). The combined organics were washed with brine (3 × 50 mL), dried over Na₂SO₄, filtered and concentrated to give the crude as a residue. Purification by prep-HPLC to afford the desired product (209 mg, 12%) as a pale yellow solid. UPLC-MS (Acidic Method, 2 min): rt 1.11 min, *m*/*z* 473.9 [M+H]⁺. ¹H NMR (400 MHz, DMSO-*d*₆) δ ppm 11.29 (br s, 1H), 10.36 (br s, 1H), 8.30-8.41 (m, 1H), 8.02-8.19 (m, 1H), 7.70-7.87 (m, 1H), 7.61 (d, *J*=8.7 Hz, 1H), 7.42 (br t, *J*=8.7 Hz, 2H), 4.63-4.86 (m, 1H), 3.94 (br d, *J*=4.4 Hz, 2H), 3.65 (br d, *J*=4.6 Hz, 2H).

### Example 61: Synthesis of (R)-N-(2,3-Dihydroxypropoxy)-2-((2-fluoro-4-iodophenyl)amino)thieno[2,3-b]pyridine-3-carboxamide

### Step 1: (R)-N-((2,2-Dimethyl-1,3-dioxolan-4-yl)methoxy)-2-((2-fluoro-4-iodophenyl)amino)thieno[2,3-b]pyridine-3-carboxamide

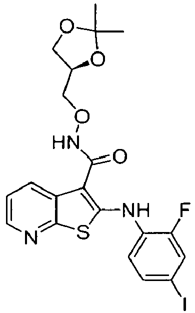

To a magnetically stirred mixture of 2-((2-fluoro-4-iodophenyl)amino)thieno[2,3-b]pyridine-3-carboxylic acid in DMF was added HATU and TEA, and the resulting mixture was agitated for 15 min. (R)-O-((2,2-Dimethyl-1,3-dioxolan-4-yl)methyl)hydroxylamine was then added to the reaction, and the resulting mixture was agitated at room temperature for 15 h. The reaction mixture was poured into water (30 mL), affording a yellow precipitate, which was collected by filtration. The resulting solid was washed with water (2 × 25 mL) and dried under vacuum at 40 °C to give the crude product as a yellow solid (0.27 g), which was used directly in the next step. UPLC-MS (Acidic Method, 2 min): rt 1.17 min, *m*/*z* 544.0 [M+H]⁺

### Step 2: (R)-N-(2,3-Dihydroxypropoxy)-2-((2-fluoro-4-iodophenyl)amino)thieno[2,3-b]pyridine-3-carboxamide

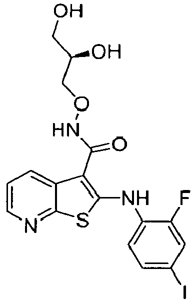

A solution of (*R*)-*N*-((2,2-dimethyl-1,3-dioxolan-4-yl)methoxy)-2-((2-fluoro-4-iodophenyl)amino)thieno[2,3-*b*]pyridine-3-carboxamide (270 mg, 0.50 mmol) in MeOH (10 mL) and ethylene glycol (140 ⊐L) was treated with *p*-toluene sulfonic acid monohydrate (41 mg, 0.21 mmol), and the resulting mixture was agitated for 15 h. The mixture was evaporated to dryness to give the crude product as a gum, which was purified by preparative HPLC, affording the desired product as an off-white solid (70 mg). UPLC-MS (Acidic Method, 2 min): rt 0.96 min, *m*/*z* 503.9 [M+H]⁺. ¹H NMR (400 MHz, DMSO-*d*6) δ ppm 10.94-11.70 (m, 1H), 10.06-10.79 (m, 1 H), 8.29 (br s, 1H), 8.13 (br d, *J*=4.39 Hz, 1H). 7.76 (br d, *J*=10.04 Hz, 1H), 7.55-7.64 (m, 1H) 7.31-7.46 (m, 2H), 4.82-5.12 (m, 1H), 4.51-4.72 (m, 1H), 3.93-4.05 (m, 1H), 3.72-3.86 (m, 2H), 3.42 (br d, *J*=4.77 Hz, 2H).

### Example 62: 2-((2-Fluoro-4-iodophenyl)amino)-N-(2-hydroxyethoxy)-6-methylthieno[2,3-b]pyridine-3-carboxamide

### Step 1: 2-Chloro-6-methylpyridin-3-yl)methanol

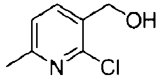

A cooled solution of methyl 2-chloro-6-methylpyridine-3-carboxylate (10 g, 53.88 mmol) in anhydrous tetrahydrofuran (120 mL) was treated with Lithium aluminium hydride (4.09 g, 107.75 mmol), which was added in a portion-wise over 20 min, taking care to maintain the internal temperature below 10 °C. Upon completion of the addition, the reaction mixture was stirred at 50 °C for 12 h. The reaction was then chilled in an ice-water bath and quenched by the addition of a saturated aqueous solution of Na₂SO₄ (100 mL), resulting in the formation of a suspension, which was filtered through a pad of celite, washing with ethyl acetate (2 × 300 mL). The combined filtrate was evaporated to dryness to give a light orange oil, which was purified by column chromatography eluting with a gradient of ethyl acetate (0 to 40%) in hexane to afford the desired product as a pale yellow oil (5.5 g, 65%). UPLC-MS (Acidic Method, 2 min): rt = 0.70 min, *m*/*z* 158.0 [M+H]⁺. ¹H NMR (400 MHz, CDCl₃) δ ppm 7.74 (d, *J*=7.7 Hz, 1H), 7.11 (d, *J*=7.8 Hz, 1H), 4.74 (s, 2H), 2.52 (s, 3H), 2.46 (br s, 1H)

### Step 2: (2-Chloro-6-methylpyridin-3-yl)methyl methanesulfonate

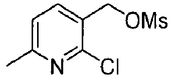

Methanesulfonyl chloride (5.4 mL, 69.8 mmol) was added dropwise to a solution of (2-chloro-6-methylpyridin-3-yl)methanol (5.5 g, 34.9 mmol) and Et₃N (9.7 mL, 69.8 mmol) in dichloromethane (52 mL) at 0 °C, taking care to maintain the internal temperature below 5°C. Upon completion of the addition, the reaction mixture was allowed to warm to room temperature with stirring for 2 h, before being quenched with water (200 mL) and extracted with dichloromethane (2 × 250 mL). The combined organics were dried over Na₂SO₄, filtered and concentrated to afford the desired product as a brown oil (7.9 g, 96%), which was used directly in the next step without any further purification. UPLC-MS (Acidic Method, 2 min): rt = 0.86 min, *m*/*z* 236.0 [M+H]⁺. ¹H NMR (400 MHz, CDCl₃) δ ppm 7.71 (t, *J*=7.5 Hz, 1H), 7.14 (dd, *J*=7.7, 14.62 Hz, 1H), 5.29 (s, 1H), 4.65 (s, 1H), 3.03-3.16 (m, 3H), 2.54 (d, *J*=6.2 Hz, 3H)

### Step 3: 2-(2-Chloro-6-methylpyridin-3-yl)acetonitrile

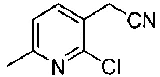

To a solution of (2-chloro-6-methylpyridin-3-yl)methyl methanesulfonate (7.9 g, 33.5 mmol) in DMF (48 mL) was added sodium cyanide (4.9 g, 100.5 mmol), and the resulting mixture was stirred for 2 h at room temperature. The reaction was then poured into water (250 mL), and the resulting mixture was extracted with ethyl acetate (3 × 250 mL). The combined organics were dried over Na₂SO₄, filtered and concentrated to afford the crude as a residue, which was purified by column chromatography eluting with a gradient of ethyl acetate (0 to 30%) in hexane to afford the desired product as a pale yellow solid (3.2 g, 58%). UPLC-MS (Acidic Method, 2 min): rt = 0.84 min, *m*/*z* 167.0 [M+H]⁺. ¹H NMR (400 MHz, CDCl₃) δ ppm 7.76 (d, *J*=7.8 Hz, 1H), 7.17 (d, *J*=7.8 Hz, 1H), 3.82 (s, 2H), 2.56 (s, 3H)

### Step 4: 2-(2-Chloro-6-methylpyridin-3-yl)acetic acid

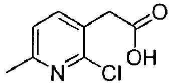

A stirred solution of 2-(2-chloro-6-methylpyridin-3-yl)acetonitrile (3.2 g, 19.2 mmol) in 15% (w/w) aqueous NaOH solution (32 mL) was heated at reflux for 30 min. The mixture was then cooled to room temperature and acidified to pH = 1 with concentrated HCl, affording a beige precipitate, which was collected by filtration, washed with water (2 × 50 mL) and dried under vacuum to afford the desired product as a beige solid (3.2 g, 90%). UPLC-MS (Acidic Method, 2 min): rt = 0.76 min, *m*/*z* 186.1 [M+H]⁺. ¹H NMR (400 MHz, CDCl₃) δ ppm 7.53 (d, *J*=7.7 Hz, 1H), 7.09 (d, *J*=7.7 Hz, 1H), 3.79 (s, 2H), 2.53 (s, 3H)

### Step 5: tert-Butyl 2-(2-chloro-6-methylpyridin-3-yl)acetate

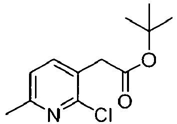

To a stirred solution of *N*,*N'*-dicyclohexylcarbodiimide (3.9 g, 18.92 mmol) in dichloromethane (72 mL) at 0 °C was added DMAP (1.7 g 13.76 mmol), followed by 2-(2-chloro-6-methylpyridin-3-yl)acetic acid (3.2 g, 17.2 mmol), and the resulting mixture was stirred at 0 °C for 5 min. *tert*-Butanol (4.9 mL, 51.6 mmol) was then added to the reaction, and the resulting mixture was allowed to warm to room temperature with stirring over 16 h. The reaction was then evaporated to dryness to give a residue, was dissolved in diethyl ether (400 mL) and passed through a pad of celite, washing with diethyl ether (2 × 200 mL). The combined filtrate was washed sequentially with 1 M aqueous NaOH (300 mL), 2 N aqueous HCl (300 mL), water (300 mL) and brine (200 mL). The organic layer was dried over Na₂SO₄, filtered and concentrated. The crude material was purified by column chromatography eluting with a gradient of ethyl acetate (0 to 30%) in hexane to afford the desired product as a pale yellow oil (3.03 g, 73%). UPLC-MS (Acidic Method, 2 min): rt 1.14 min, *m*/*z* 242.1 [M+H]⁺. ¹H NMR (400 MHz, CDCl₃) δ ppm 7.50 (d, *J*=7.7 Hz, 1H), 7.05 (d, *J*=7.7 Hz, 1H), 3.62 (s, 2H), 2.51 (s, 3H), 1.44 (s, 9H)

### Step 6: tert-Butyl 2-((2-fluoro-4-iodophenyl)amino)-6-methylthieno[2,3-blpyridine-3-carboxylate

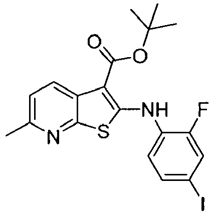

To a solution of tert-butyl 2-(2-chloro-6-methylpyridin-3-yl)acetate (3.03 g, 12.5 mmol) in tetrahydrofuran (250 mL) was added sodium *tert*-butoxide (1.26 g, 13.13 mmol) under nitrogen atmosphere. A yellow solution was formed which was stirred at room temperature for 15 min. Then 2-fluoro-4-iodo-1-isothiocyanatobenzene (3.5 g, 12.5 mmol) was added and the solution was stirred for 30 min. The solution turned light brown. Finally the mixture was stirred at reflux overnight. The reaction was cooled to room temperature and partitioned between ethyl acetate (300 mL) and water (300 mL). The organic phase was collected and the aqueous layer was extracted with ethyl acetate (300 mL). Combined organics were dried over Na₂SO₄, filtered and concentrated. The crude material was purified by column chromatography eluting with dichloromethane to afford the desired product as a yellow solid (2.5 g, 42%). UPLC-MS (Acidic Method, 2 min): rt = 1.56 min, *m*/*z* 484.9 [M+H]⁺. ¹H NMR (400 MHz, CDCl₃) δ ppm 10.70 (br s, 1H), 8.21 (d, *J*=8.3 Hz, 1H), 7.49-7.54 (m, 2H), 7.42-7.48 (m, 1H), 7.13 (d, *J*=8.5 Hz, 1H), 2.59 (s, 3H), 1.68 (s, 9H).

### Step 7: 2-((2-Fluoro-4-iodophenyl)amino)-6-methylthieno[2,3-b]pyridine-3-carboxylic acid

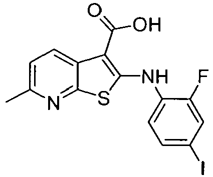

To a solution of tert-butyl 2-((2-fluoro-4-iodophenyl)amino)-6-methylthieno[2,3-*b*]pyridine-3-carboxylate (0.26 g, 0.54 mmol) in dichloromethane (5.5 mL) was added trifluoroacetic acid (0.55 mL, 7.18 mmol), and the resulting mixture was stirred at room temperature for 4 h. The reaction mixture was then concentrated to afford a residue, which was co-distilled from toluene (3 × 50 mL) to afford the desired product as a yellow solid (0.24 g, 100%). UPLC-MS (Acidic Method, 4 min): rt = 2.15 min, *m*/*z* 428.9 [M+H]⁺. ¹H NMR (400 MHz, DMSO-d₆) δ ppm 10.63 (s, 1H), 8.24 (d, *J*=8.3 Hz, 1H), 7.83 (dd, *J*=1.9, 10.2 Hz, 1H), 7.65-7.70 (m, 1H), 7.52 (t, *J*=8.6 Hz, 1H), 7.28 (d, *J*=8.2 Hz, 1H)

### Step 8: 2-((2-Fluoro-4-iodophenyl)amino)-N-(2-hydroxyethoxy)-6-methylthieno[2,3-b]pyridine-3-carboxamide

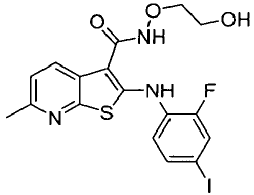

To a solution of 2-((2-fluoro-4-iodophenyl)amino)-6-methylthieno[2,3-b]pyridine-3-carboxylic acid (0.24 g, 0.56 mmol) in anhydrous tetrahydrofuran (14 mL) was added PyBOP (0.41 mg, 0.78 mmol), followed by Et₃N (0.23 mL, 1.68 mmol), and the resulting solution was stirred at room temperature for 30 min. 2-(Aminooxy)ethan-1-ol (65 mg, 0.84 mmol) was then added to the reaction, and the resulting mixture was stirred at room temperature for 12 h. Water (50 mL) was added to the reaction, forming a biphasic mixture. The aqueous phase was collected and extracted with ethyl acetate (3 × 50 mL). The combined organics were washed with brine, dried over Na₂SO₄, filtered and concentrated. The crude material was purified by prep-HPLC to afford the desired product as a pale yellow solid (115 mg, 42%). UPLC-MS (Acidic Method, 4 min): rt = 1.84 min, *m*/*z* 487.9 [M+H]⁺. ¹H NMR (400 MHz, DMSO-d₆) δ ppm 11.24 (br s, 1H), 10.1 1-10.38 (br s, 1H), 7.93-8.06 (m, 1H), 7.67-7.80 (m, 1H), 7.58 (br d, *J*=7.7 Hz, 1H), 7.36 (br t, *J*=8.6 Hz, 1H), 7.28 (br dd, *J*=4.1, 7.2 Hz, 1H), 4.59-4.84 (br s, 1H), 3.92 (t, *J*=4.9 Hz, 3H), 3.63 (br d, *J*=3.8 Hz, 3H).

### Example 63: 2-((2-fluoro-4-iodophenyl)amino)-N-(2-hydroxyethoxy)-6-methoxythieno[2,3-b]pyridine-3-carboxamide

### Step 1: (2-Chloro-6-methoxypyridin-3-yl)methanol

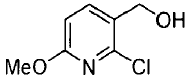

To a solution of methyl 2-chloro-6-methoxynicotinate (5.0 g, 24.8 mmol) in THF (50 mL) at 0 °C was added lithium aluminium hydride (1.88 g, 49.6 mmol) in a portion wise manner, taking care to keep the temperature below 10 °C. Upon completion of the addition, the reaction mixture was stirred at 50 °C for 3 h. The reaction mixture was then cooled to 0 °C, before being quenched by the careful addition of a saturated solution of aqueous sodium sulphate (50 mL). The resulting suspension was stirred for 20 min, before being filtered through a pad of celite, washing with ethyl acetate (2 × 50 mL). The combined organics were dried over anhydrous sodium sulphate, filtered and evaporated to dryness to give the desired product as a yellow-orange oil (3.82 g, 89%). UPLC-MS (Acidic Method, 2 mins): rt = 0.83 min, *m*/*z* 174.0 [M+H]⁺. ¹H NMR (400 MHz, CDCl₃) δ ppm 7.69 (d, *J*=8.2 Hz, 1 H) 6.69 (d, *J*=8.2 Hz, 1 H) 4.70 (s, 2 H) 3.88 (s, 3 H)

### Step 2: (2-Chloro-6-methoxypyridin-3-yl)methyl methanesulfonate

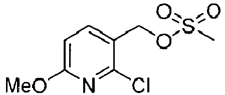

Methanesulfonyl chloride (3.4 mL, 44.0 mmol) was added to a stirred solution of (2-chloro-6-methoxypyridin-3-yl)methanol (3.82 g, 22.0 mmol) in dichloromethane (90 mL) at 0 °C, and the resulting mixture was stirred at room temperature for 3 h. The reaction mixture was poured into water (100 mL), affording a biphasic solution. The organic phase was collected and the aqueous phase extracted with dichloromethane (2 × 50 mL). The combined organics were dried over anhydrous sodium sulfate, filtered and evaporated to dryness to give the desired product as an orange solid (4.26 g, 77%). UPLC-MS (Acidic Method, 2 mins): rt = 1.13 min, *m*/*z* 252.1 [M+H]⁺. ¹H NMR (400 MHz, CDCl₃) δ ppm 7.65 (d, *J*=8.2 Hz, 1 H) 6.69 (d, *J*=8.2 Hz, 1 H) 4.64 (s, 2 H) 3.94 (s, 3 H) 3.14 (s, 3 H)

### Step 3: 2-(2-Chloro-6-methoxypyridin-3-yl)acetonitrile

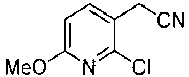

To a solution of (2-chloro-6-methoxypyridin-3-yl)methyl methanesulfonate (4.26 g, 16.9 mmol) in DMF (17 mL) was added sodium cyanide (2.48 g, 50.7 mmol) in a portion wise manner over 5 min, and the resulting mixture was stirred at room temperature for 24 h. The reaction was poured into water (100 mL), and the resulting mixture was extracted with ethyl acetate (3 × 50 mL). The combined organics were washed with brine (2 × 200 mL), dried over anhydrous sodium sulfate, filtered and evaporated to dryness. The crude was purified by flash column chromatography eluting with a gradient of methanol (0-5%) in DICHLOROMETHANE to give the desired product as a white solid (1.75 g, 57%). UPLC-MS (Acidic Method, 2 mins): rt 0.96 min, *m*/*z* 183.0 [M+H]⁺. ¹H NMR (400 MHz,CHCl₃) δ ppm 7.69 (d, *J*=8.2 Hz, 1H), 6.73 (d, *J*=8.2 Hz, 1H), 3.94 (s, 3H), 3.75 (s, 2H).

### Step 4: 2-(2-Chloro-6-methoxypyridin-3-yl)acetic acid

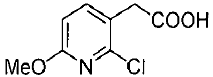

A suspension of 2-(2-chloro-6-methoxypyridin-3-yl)acetonitrile (1.75 g, 9.5 mmol) in an aqueous solution of 15% w/w NaOH (16 mL) was stirred at 60 °C for 24 h. The reaction mixture was then cooled to room temperature and acidified of pH=1 with concentrated hydrochloric acid. The resulting precipitate was collected by filtration and washed with water (2 × 20 mL), before being dried under vacuum at 40 °C to give the desired product as a white solid (1.50 g, 78%). UPLC-MS (Acidic Method, 2 mins): rt = 0.84 min, *m*/*z* 202.0 [M+H]⁺. ¹H NMR (400 MHz, DMSO-*d*₆) δ ppm 12.51 (br s, 1H), 7.76 (d, *J*=8.2 Hz, 1H), 6.84 (d, *J*=8.2 Hz, 1H), 3.85 (s, 3H), 3.66 (s, 2H).

### Step 5: tert-Butyl 2-(2-chloro-6-methoxypyridin-3-yl)acetate

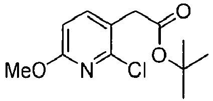

To a suspension of 2-(2-chloro-6-mthoxypyridin-3-yl)acetic acid (1.34 g, 6.65 mmol) in *tert*-butanol (15 mL) was added di-tert-butyl dicarbonate (2.3 mL, 9.97 mmol), followed by DMAP (0.082 g, 0.67 mmol), and the resulting mixture was stirred at 50 °C for 18 h. The reaction mixture was cooled to room temperature and evaporated to dryness to give a residue, which was diluted with water (20 mL) and extracted with ethyl acetate (2 × 20 mL). The combined organics were dried over anhydrous sodium sulfate, filtered and concentrated to give the crude, which was purified by flash column chromatography eluting with a gradient of ethyl acetate (20-80%) in hexane to give the desired product as a pale yellow oil (1.27 g, 69%). UPLC-MS (Acidic Method, 2 mins): rt = 1.21 min, *m*/*z* 258.1 [M+H]⁺. ¹H NMR (400 MHz, CDCl₃) δ ppm 7.49 (d, *J*=8.2 Hz, 1H), 6.65 (d, *J*=8.2 Hz, 1H), 3.92 (s, 3H), 3.58 (s, 2H), 1.45 (s, 9H).

### Step 6: tert-Butyl 2-((2-fluoro-4-iodophenyl)amino)-6-methoxythieno[2,3-b]pyridine-3-carboxylate

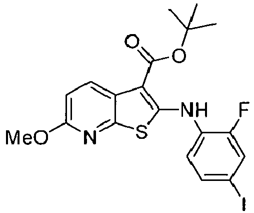

*tert*-Butyl 2-(2-chloro-6-methoxypyridin-3-yl)acetate (1.0 g, 3.88 mmol) and sodium tert-butoxide (0.41 g, 4.27 mmol) were added to a microwave vial, followed by THF (35 mL), and the resulting mixture was agitated for 5 min. A solution of 2-fluoro-4-iodo-1-isothiocyanatobenzene (1.11 g, 3.88 mmol) in THF (5 mL) was then added to the microwave vial, and the resulting mixture was stirred at room temperature for 1 h. Caesium fluoride (0.295 g, 1.94 mmol) was then added to the vial, and the reaction mixture was heated at 90 °C under microwave irradiation for 1 h. The mixture was cooled to room temperature and evaporated to dryness to afford the crude product, which was purified by flash column chromatography eluting with dichloromethane to give the desired product as a yellow solid (0.564 g, 30%). UPLC-MS (Acidic Method, 4 mins): rt = 2.95 min, *m*/*z* 500.9 [M+H]⁺. ¹H NMR (400 MHz, CDCl₃) δ ppm 10.56 (s, 1H), 8.25 (d, *J*=8.8 Hz, 1H), 7.43-7.53 (m, 3H), 6.76 (d, *J*=8.8 Hz, 1H), 3.96 (s, 3H), 1.78 (s, 9H).

### Step 7: 2-((2-fluoro-4-iodophenyl)amino)-6-methoxythieno[2,3-b]pyridine-3-carboxylic acid

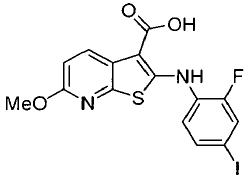

To a solution of tert-butyl 2-((2-fluoro-4-iodophenyl)amino)-6-methoxythieno[2,3-b]pyridine-3-carboxylate (0.050 g, 0.10 mmol) in dichloromethane (3 mL) was added trifluoroacetic acid (0.3 mL). The resultant mixture was stirred at room temperature under a N₂ atmosphere for 6h. The solvent was removed *in vacuo* and co-distilled with toluene (2 × 10 mL) to give the desired product as a yellow solid (0.44 g, 100%), which was used in the next step without further purification. UPLC-MS (Acidic Method, 2 mins): rt = 1.35 min, *m*/*z* 445.0 [M+H]⁺

### Step 8: 2-((2-fluoro-4-iodophenyl)amino)-N-(2-hydroxyethoxy)-6-methoxythieno[2,3-blpyridine-3-carboxamide

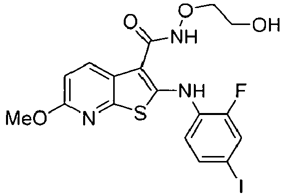

To a suspension of 2-((2-fluoro-4-iodophenyl)amino)-6-methoxythieno[2,3-b]pyridine-3-carboxylic acid (0.050 g, 0.11 mmol) in THF (2 mL) was added PyBOP (0.078g, 0.15 mmol) and triethylamine (0.05 mL, 0.33 mmol). The resultant mixture was stirred at room temperature under a N₂ atmosphere for 30 min. A solution of 2-(aminooxy)ethanol (0.01 1 mL, 0.15 mmol) in THF (0.1 mL) was added and the reaction mixture was stirred at room temperature under a N₂ atmosphere for 1h. The reaction mixture was diluted with water (10 mL) and then extracted with ethyl acetate (2 × 10 mL). The combined organics were dried over anhydrous sodium sulfate, filtered and evaporated to dryness to give the crude product, which was purified by preparatory HPLC to give the desired product as an off-white solid (0.023 g, 22%). UPLC-MS (Acidic Method, 4 mins): rt = 2.10 min, *m*/*z* 503.9 [M+H]⁺. ¹H NMR (400 MHz, DMSO-*d*₆) δ ppm 11.30 (brs, 1H), 9.77 (br s, 1H), 8.11 (br s, 1H), 7.70 (br s, 1H), 7.53 (brd, *J*=8.2 Hz, 1H), 7.26 (br d, *J*=8.2 Hz, 1H), 6.91 (br d, *J*=7.0 Hz, 1H), 4.77 (br s, 1H), 3.90 (s, 3H), 3.89 (br s, 2H), 3.62 (br s, 2H).

### Example 64: 2-((2-Fluoro-4-iodophenyl)amino)-N-(2-hydroxyethoxy)-5-methoxythieno[2,3-b]pyridine-3-carboxamide

### Step 1: (2-Chloro-5-methoxypyridin-3-yl)methanol

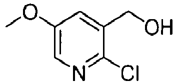

A solution of methyl 2-chloro-5-methoxynicotinate (0.5 g, 2.48 mmol) in anhydrous THF (5 mL) was cooled to 0 °C and treated with lithium aluminium hydride (0.19 g, 4.96 mmol) in a portion-wise manner. Upon completion of the addition, the mixture was heated to 50 °C for 1 h. The mixture was then cooled to 0 °C and treated with an aqueous solution of sodium sulphate, and the resulting mixture was stirred for 0.5 h at ambient temperature. The mixture was diluted with ethyl acetate (10 mL) and filtered through a pad of Celite. The pad was washed with ethyl acetate (2 × 20 mL) and the combined organic phases were dried over Na₂SO₄, filtered and concentrated under vacuum to give the crude product, which was purified by flash column chromatography eluting with a gradient of methanol (0-5%) in DICHLOROMETHANE to give the desired product as a brown oil (0.368 g, 86%). UPLC-MS (Acidic Method, 2 min): rt = 0.72 min, *m*/*z* 174.0/175.9 [M+H]⁺. ¹H NMR (400 MHz, CDCl₃) δ ppm 7.97 (d, *J*=3.0 Hz, 1H), 7.47 (dt, *J*=3.1, 0.8 Hz, 1H), 4.74 (s, 2H), 3.86 (s, 3H)

### Step 2: (2-Chloro-5-methoxypyridin-3-yl)methyl methanesulfonate

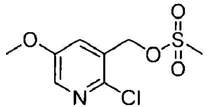

A solution of (2-chloro-5-methoxypyridin-3-yl)methanol (2.74 g, 15.84 mmol) in anhydrous dichloromethane (60 mL) was cooled to 0 °C and treated sequentially with triethylamine (4.42 mL, 31.67 mmol) methanesulfonyl chloride (2.45 mL, 31.67 mmol), and the resulting mixture was slowly warmed up to ambient temperature with stirring over 1 h. The mixture was diluted with water (30 mL) and extracted with DICHLOROMETHANE (2 × 30 mL). The combined organic phases were washed with brine (30 mL), dried over Na₂SO₄, filtered and concentrated under vacuum to afford the desired product as a brown oil (3.99 g,100%). UPLC-MS (Acidic Method, 2 min): rt = 0.87 min, *m*/*z* 252.0/254.0 [M+H]⁺ and 1.01 min, *m*/*z* 192.0/194.0 [M+H]⁺. ¹H NMR (400 MHz, CDCl₃) δ ppm 8.06-8.10 (m, 1H), 7.35-7.43 (m, 1H), 5.29 (s, 1H), 4.65 (s, 1H), 3.89 (s, 3H), 3.14 (s, 1H), 3.11 (s, 2H)

### Step 3: 2-(2-Chloro-5-methoxypyridin-3-yl)acetonitrile

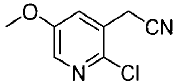

A solution of (2-Chloro-5-methoxypyridin-3-yl)methyl methanesulfonate (200 mg, 0. 80 mmol) in anhydrous DMF (1 mL) was treated with sodium cyanide (117 mg, 2.39 mmol), and the resulting mixture was stirred at ambient temperature for 1 h. The mixture was diluted with water (5 mL) and extracted with ethyl acetate (3 × 5 mL). The combined organic phases were washed sequentially with water (5 mL) and brine (5 mL), before being dried over Na₂SO₄, filtered and concentrated under vacuum to give the crude product, which was purified by flash column chromatography eluting with a gradient of methanol (0-4%) in dichloromethane to give the desired product as a yellow solid (89 mg, 61%). UPLC-MS (Acidic Method, 2 min): rt = 0.86 min, *m*/*z* 183.0/185.0 [M+H]⁺. ¹H NMR (400 MHz, CDCl₃) δ ppm 8.07 (d, *J*=2.9 Hz, 1H), 7.41-7.44 (m, 1H), 3.90 (s, 3H), 3.84 (s, 2H)

### Step 4: 2-(2-Chloro-5-methoxypyridin-3-yl)acetic acid

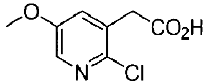

A suspension of 2-(2-chloro-5-methoxypyridin-3-yl)acetonitrile (1.19 g, 6.55 mmol) in a 15% (w/v) solution of aqueous sodium hydroxide (11.9 mL) was heated at 100 °C with stirring for 2 h, resulting in a clear solution. The reaction mixture was then cooled to 5 °C (ice bath) and cautiously treated with a 1 M solution of aqueous hydrogen chloride until the solution was acidic (pH = 1), leading to a formation of an off-white precipitate. The solid was collected by filtration and washed with water until the filtrate became pH neutral. The solid was then dried under vacuum to give the desired product as a beige solid (1.18 g, 89%). UPLC-MS (Acidic Method, 2 min): rt = 0.78 min, *m*/*z* 202.1/204.0 [M+H]⁺. ¹H NMR (400 MHz, DMSO-*d*₆) δ ppm 8.03 (d, *J*=3.0 Hz, 1H), 7.52 (d, *J*=3.0 Hz, 1H), 3.82 (s, 3H), 3.69 (s, 2H)

### Step 5: tert-Butyl 2-(2-chloro-5-methoxypyridin-3-yl)acetate

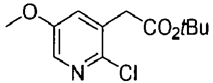

A suspension of 2-(2-chloro-5-methoxypyridin-3-yl)acetic acid (980 mg, 4.80 mmol) *in* tert-butanol (10.9 mL) was treated sequentially with di-tert-butyl dicarbonate (1.67 mL, 7.28 mmol) and 4-dimethylaminopyridine (59 mg, 0.48 mmol), and the resulting mixture was heated at 50 °C for 2 h. The mixture was cooled down to ambient temperature and concentrated under vacuum to give a residue, which was dissolved in ethyl acetate (20 mL). The solution was washed with water (20 mL) and the aqueous phase was extracted with ethyl acetate (120 mL). The combined organic layers were dried over Na₂SO₄, filtered and concentrated under vacuum to give the crude product as an oil, which was purified by flash column chromatography eluting with a gradient of ethyl acetate (0-15%) in hexanes to afford the desired product as a yellow oil (990 mg, 79%). UPLC-MS (Acidic Method, 2 min): rt = 1.16 min, *m*/*z* 258.1/260.1 [M+H]⁺. ¹H NMR (400 MHz, CDCl₃) δ ppm 8.01 (d, *J*=3.0 Hz, 1H), 7.21 (d, *J*=3.0 Hz, 1H), 3.88 (s, 3H), 3.66 (s, 2H), 1.48 (s, 9H)

### Step 6: tert-Butyl 2-((2-fluoro-4-iodophenyl)amino)-5-methoxythieno[2,3-b]pyridine-3-carboxylate

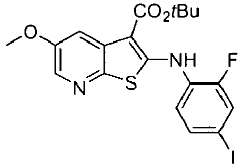

A solution of *tert*-butyl 2-(2-chloro-5-methoxypyridin-3-yl)acetate (50 mg, 0.194 mmol) in anhydrous THF (2 mL) was treated with sodium *tert*-butoxide (20 mg, 0.213 mmol), and the resulting mixture was stirred at ambient temperature for 5 min. A solution of 2-fluoro-4-iodo-1-isothiocyanatobenzene (54 mg, 0.194 mmol) in anhydrous THF (0.5 mL) was then added to the reaction, and the resulting mixture was stirred at ambient temperature for 1 .5 h, before being heated under microwave irradiation at 90 °C for 2 h. The mixture was cooled to ambient temperature and concentrated under vacuum to give the crude product as an oil, which and purified by flash column chromatography (Silica 12 g, 0-7% ethyl acetate in hexanes), followed by the trituration of the resulting solid with hexanes to afford the desired product as a pale yellow solid (33 mg, 34%). UPLC-MS (Acidic Method, 4 min): rt = 2.84 min, *m*/*z* 500.9 [M+H]⁺. ¹H NMR (400 MHz, CDCl₃) δ ppm 10.79 (br s, 1H), 8.08 (d, *J*=2.9 Hz, 1H), 7.93 (d, *J*=2.8 Hz, 1H), 7.45-7.56 (m, 3H), 3.90 (s, 3H), 1.71 (s, 9H)

### Step 7: 2-((2-Fluoro-4-iodophenyl)amino)-5-methoxythieno[2,3-b]pyridine-3-carboxylic acid

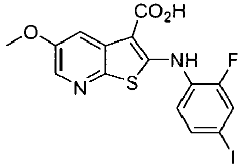

A solution of *tert*-butyl 2-((2-fluoro-4-iodophenyl)amino)-5-methoxythieno[2,3-*b*]pyridine-3-carboxylate (385 mg, 0.77 mmol) in dichloromethane (25 mL) was treated with trifluoroacetic acid (2.5 mL), and the resulting mixture was stirred at ambient temperature for 18 h. The mixture was then concentrated under vacuum to giver an oil, which was co-distilled from toluene (2 × 20 mL) to afford the desired product as yellow solid (342 mg, 100%). UPLC-MS (Acidic Method, 4 min): rt = 2.14 min, *m*/*z* 443.0 [M-H]⁻. ¹H NMR (400 MHz, DMSO-*d*₆) δ ppm 10.69 (s, 1H), 8.06 (d, *J*=2.8 Hz, 1H), 7.88 (d, *J*=2.8 Hz, 1H), 7.82 (dd, *J*=10.2, 1.9 Hz, 1H), 7.65 (d, *J*=8.7 Hz, 1H), 7.51 (t, *J*=8.6 Hz, 1H), 3.85 (s, 3H).

### Step 8: 2-((2-Fluoro-4-iodophenyl)amino)-N-(2-hydroxyethoxy)-5-methoxythieno[2,3-b]pyridine-3-carboxamide

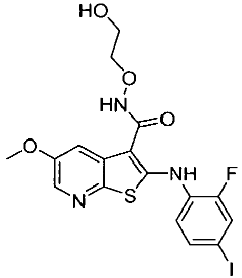

A suspension of 2-((2-fluoro-4-iodophenyl)amino)-5-methoxythieno[2,3-*b*]pyridine-3-carboxylic acid (342 mg, 0.77 mmol) in THF (15 mL) was treated sequentially with PyBOP (546 mg, 1.05 mmol) and triethylamine (0.32 mL, 2.31), and the resulting mixture was stirred at ambient temperature for 0.5 h. (2-Aminooxy)ethanol (81 mg, 1.05 mmol) was added to the reaction, and the resulting mixture was stirred at ambient temperature for 18 h. The mixture was then diluted with ethyl acetate (10 mL), washed with water (2 × 10 mL), dried over Na₂SO₄, filtered and concentrated to give the crude product, which was purified by preparatory HPLC to give the desired product as a yellow solid (131 mg, 34%). UPLC-MS (Acidic Method, 4 min): rt = 1.85 min, *m*/*z* 503.9 [M+H]⁺. ¹H NMR (400 MHz, DMSO-*d*₆) δ ppm 11.29 (s, 1H), 10.43 (br s, 1H), 8.09 (br s, 1H), 7.77 (br d, *J*=10.3 Hz, 1H) 7.57-7.64 (m, 2H), 7.41 (t, *J*=8.6 Hz, 1H), 4.78 (br s, 1H), 3.85-3.96 (m, 5H) 3.68-3.61 (m, 2H)

### Example 65: 2-((2-Fluoro-4-iodophenyl)amino)-N-(2-hydroxyethoxy)-5-fluorothieno[2,3-b]pyridine-3-carboxamide

### Step 1: (2-Chloro-5-fluoropyridin-3-yl)methanol

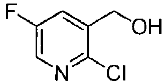

A solution of methyl 2-chloro-5-fluoronicotinate (100 mg, 0.53 mmol) in anhydrous THF (4 mL) was cooled to 0 °C and treated with sodium borohydride (120 mg, 3.17 mmol) in a portion-wise manner over a period of 5 min, and the resulting mixture was heated at 70 °C for 15 min. Methanol (0.8 mL) was then added in a dropwise manner over 15 min, resulting in considerable effervescence, and the resulting mixture was heated at 70 °C for 30 min. The mixture was cooled to ambient temperature and treated with a saturated solution of aqueous ammonium chloride (5 mL). Ethyl acetate (5 mL) was added, resulting in a biphasic mixture, which stirred for 30 min. The organic phase was collected and the aqueous layer was extracted with ethyl acetate (2 × 5 mL). The combined organic phases were then dried over Na₂SO₄, filtered and concentrated under vacuum to give the desired product as an orange glass (84 mg, 99%). UPLC-MS (Acidic Method, 2 min): rt = 0.73 min, *m*/*z* 162.0/164.0 [M+H]⁺. ¹H NMR (400 MHz, CDCl₃) δ ppm 8.17 (d, *J*=3.0 Hz, 1H), 7.71 (ddt, *J*=8.3, 3.0, 0.9, 0.9 Hz, 1H), 4.78 (s, 2H).

### Step 2: (2-Chloro-5-fluoropyridin-3-yl)methyl methanesulfonate

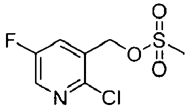

A solution of (2-chloro-5-fluoropyridin-3-yl)methanol (225 mg, 1.39 mmol) in anhydrous dichloromethane (5 mL) was cooled to 0 °C and treated with triethylamine (0.39 mL, 2.78 mmol) followed by methanesulfonyl chloride (0.22 mL, 2.78 mmol). The resulting mixture was then gradually warmed up to ambient temperature and stirred for 1 h. The mixture was diluted with water (5 mL) and extracted with dichloromethane (2 × 5 mL). The combined organic phases were washed with brine (5 mL), dried over Na₂SO₄, filtered and concentrated under vacuum to afford the desired product as a brown oil (335 mg, 100%). UPLC-MS (Acidic Method, 2 min): rt = 0.88 min, *m*/*z* 240.1/242.0 [M+H]⁺ and 1.03 min, *m*/*z* 223.2 [M+H]⁺. ¹H NMR (400 MHz, CDCl₃) δ ppm 8.24-8.30 (m, 1H), 7.62-7.68 (m, 1H), 5.29 (s, 1H), 4.65 (s, 1H), 3.15 (s, 3H).

### Step 3: 2-(2-Chloro-5-fluoropyridin-3-yl)acetonitrile

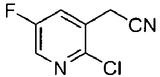

A solution of (2-chloro-5-fluoropyridin-3-yl)methyl methanesulfonate (4.93 g, 20.6 mmol) in anhydrous DMF (20.6 mL) was treated with sodium cyanide (3.0 g, 61.7 mmol), and the resulting mixture was stirred at ambient temperature for 4 h. The mixture was diluted with water (50 mL) and extracted with ethyl acetate (3 × 50 mL). The combined organic phases were washed sequentially with H₂O (50 mL) and brine (50 mL), before being dried over Na₂SO₄, filtered and concentrated under vacuum to give the crude product, which was purified by flash column chromatography eluting with a gradient of dichloromethane (0-80%) in hexanes to give the desired product as a light brown oil (1.38 g, 39%). UPLC-MS (Acidic Method, 2 min): rt = 0.84 min, *m*/*z* 171.1/173.1 [M+H]⁺. ¹H NMR (400 MHz, CDCl₃) δ ppm 8.30 (d, *J*=2.9 Hz, 1H), 7.70 (ddt, *J*=7.8, 2.9, 0.8, 0.8 Hz, 1H), 3.88 (t, *J*=0.6 Hz, 2H).

### Step 4: 2-(2-Chloro-5-fluoropyridin-3-yl)acetic acid

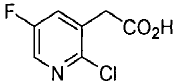

A suspension of 2-(2-chloro-5-fluoropyridin-3-yl)acetonitrile (1.38 g, 8.09 mmol) in a 15% (w/v) aqueous solution of sodium hydroxide (13.8 mL) was heated at 100 °C with stirring for 0.5 h, resulting in a clear solution. The reaction mixture was then cooled to 5 °C (ice bath) and cautiously treated with a 1 M solution of aqueous hydrogen chloride until the solution was acidic (pH = 1), leading to a formation of an off-white precipitate. The solid was collected by filtration and washed with H₂O until the filtrate became pH neutral. The solid was then dried under vacuum to give the desired product as an off-white solid (1.22 g, 80%). UPLC-MS (Acidic Method, 2 min): rt = 0.75 min, *m*/*z* 190.0/192.00 [M+H]⁺. ¹H NMR (400 MHz, DMSO-*d*₆) δ ppm 12.73 (br s, 1H), 8.38 (d, *J*=3.0 Hz, 1H), 7.91 (dd, *J*=8.8, 3.0 Hz, 1H), 3.76 (s, 2H).

### Step 5: tert-Butyl 2-(2-chloro-5-fluoropyridin-3-yl)acetate

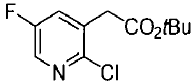

A suspension of 2-(2-chloro-5-fluoropyridin-3-yl)acetic acid (1.22 g, 6.43 mmol) *in tert*-butanol (17 mL) was treated sequentially with di-tert-butyl dicarbonate (2.22 mL, 9.65 mmol) and 4-dimethylaminopyridine (82 mg, 0.643 mmol), and the resulting mixture was heated at 50 °C with stirring for 1.5 h. The mixture was then cooled down to ambient temperature and concentrated under vacuum to give a residue, which was dissolved in ethyl acetate (20 mL). The resulting solution was washed sequentially with water (20 mL) and brine (20 mL), before being dried over Na₂SO₄, filtered and concentrated under vacuum to give the crude product as an oil, which was purified by flash column chromatography eluting with a gradient of ethyl acetate (0-10%) in hexanes to give the desired product as a pale yellow oil (1.25 g, 79%). UPLC-MS (Acidic Method, 2 min): rt = 1.16 min, *m*/*z* 246.1/248.1 [M+H]⁺. ¹H NMR (400 MHz, CDCl₃) δ ppm 8.20 (d, *J*=3.0 Hz, 1H), 7.45 (dd, *J*=8.0, 2.9 Hz, 1H), 3.69 (s, 2H), 1.47 (s, 9H).

### Step 6: tert-Butyl 2-((2-fluoro-4-iodophenyl)amino)-5-fluorothieno[2,3-b]pyridine-3-carboxylate

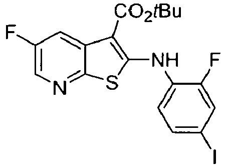

A solution of *tert*-butyl 2-(2-chloro-5-fluoropyridin-3-yl)acetate (1.15 g, 4.68 mmol) in anhydrous THF (34 mL) was treated with sodium tert-butoxide (0.49 g, 5.148 mmol), and the resulting mixture was stirred at ambient temperature for 10 min. A solution of 2-fluoro-4-iodo-1-isothiocyanatobenzene (1.31 g, 4.68 mmol) in anhydrous THF (6 mL) was then added to the reaction, and the resulting mixture was stirred at ambient temperature for 1.5 h, before being heated under microwave irradiation at 90 °C for 2 h. The mixture was cooled to ambient temperature and concentrated under vacuum to give an oil, which was purified by flash column chromatography eluting with a gradient of ethyl acetate (0-5%) in hexanes to afford the desired product as a white solid (1.39 g, 56%). UPLC-MS (Acidic Method, 2 min): rt = 1.63 min, *m*/*z* 489.0 [M+H]⁺. ¹H NMR (400 MHz, DMSO-*d*₆) δ ppm 10.44 (s, 1H), 8.34 (dd, *J*=2.8, 0.8 Hz, 1H), 7.95 (dd, *J*=10.6, 2.8 Hz, 1H), 7.85 (dd, *J*=10.0, 1.9 Hz, 1H), 7.67 (d, *J*=8.5 Hz, 1H), 7.50 (t, *J*=8.5 Hz, 1H), 1.64 (s, 9H).

### Step 7: 2-((2-Fluoro-4-iodophenyl)amino)-5-fluorothieno[2,3-b]pyridine-3-carboxylic acid

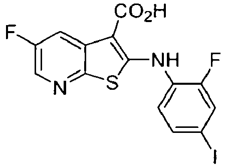

A solution of *tert*-butyl 2-((2-fluoro-4-iodophenyl)amino)-5-fluoroythieno[2,3-*b*]pyridine-3-carboxylate (1.39 g, 2.85 mmol) in dichloromethane (80 mL) was treated with trifluoroacetic acid (8 mL), and the resulting mixture was stirred at ambient temperature for 18 h. The mixture was then concentrated under vacuum to give an oil, which was co-distilled from toluene (2 × 20 mL) to afford the desired product as a pale yellow solid (1.22 g, 99%). UPLC-MS (Acidic Method, 2 min): rt = 1.30 min, *m*/*z* 431.0 [M-H]⁻. ¹H NMR (400 MHz, DMSO-*d*₆) δ ppm 10.64 (s, 1H), 8.33 (dd, *J*=2.7, 0.7 Hz, 1H), 8.07 (dd, *J*=10.42, 2.76 Hz, 1H), 7.85 (dd, *J*=10.1, 1.8 Hz, 5 H), 7.68 (dt, *J*=8.5, 1.0 Hz, 1H), 7.51 (t, *J*=8.5 Hz, 1H).

### Step 8: 2-((2-Fluoro-4-iodophenyl)amino)-N-(2-hydroxyethoxy)-5-fluorothieno[2,3-b]pyridine-3-carboxamide

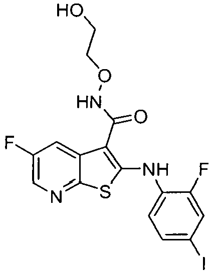

A suspension of 2-((2-fluoro-4-iodophenyl)amino)-5-fluorothieno[2,3-b]pyridine-3-carboxylic acid (500 mg, 1.16 mmol) in THF (20 mL) was treated sequentially with PyBOP (822 mg, 1.58 mmol) and triethylamine (0.49 mL, 3.48 mmol), and the resulting mixture was stirred at ambient temperature for 0.5 h. (2-Aminooxy)ethanol (122 mg, 1.58 mmol) was then added to the reaction, and the resulting mixture was stirred at ambient temperature for 18 h. The mixture was diluted with ethyl acetate (10 mL), washed with water (2 × 10 mL), dried over Na₂SO₄, filtered and concentrated under vacuum to give the crude product, which was purified by preparatory HPLC to give the desired product as a light yellow solid (115 mg, 20%). UPLC-MS (Acidic Method, 2 min): rt = 1.19 min, *m*/*z* 491.9 [M+H]⁺. ¹H NMR (400 MHz, DMSO-*d*₆) δ ppm 11.33 (s, 1H), 10.48 (s, 1H), 8.36 (s, 1H), 7.90 (d, *J*=9.9 Hz, 1H), 7.81 (dd, *J*=10.2, 1.8 Hz, 1H), 7.62 (d, *J*=8.4 Hz, 1H), 7.43 (t, *J*=8.5 Hz, 1H), 4.81 (br s, 1H), 3.95 (br t, *J*=4.7 Hz, 2H), 3.64 (br d, *J*=4.3Hz, 2H).

### Example 66: Methyl 5-acetyl-4-ethyl-2-((2-fluoro-4-iodophenyl)amino)thiophene-3-carboxylate (General Procedure for Synthesis of Tetra-substituted Thiophene-3-esters)

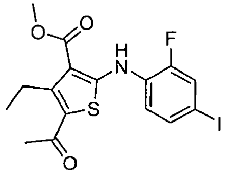

A solution of 2-fluoro-4-iodo-1-isothiocyanatobenzene (2.0 g, 7.16 mmol), methyl-3-oxovalerate (0.9 mL, 7.16 mmol) in DMF (20 mL) was stirred at room temperature and K₂CO₃ (2.0 g, 14.33 mmol) was added. Stirring was maintained for 4.5 h prior to the addition of chloroacetone (10.6 mL, 7.16 mmol) and it was further continued for the next 18 h. The reaction was quenched with H₂O (150 mL) and extracted with EtOAc (2 × 150 mL). The combined organic extracts were washed with brine (150 mL), dried over NaSO₄, filtered and the solvent removed under vacuum. The crude was purified by flash column chromatography (Silica 40 g, 5-30% EtOAC in hexane) to give the product (2.2 g, 67.3%) as an off-white solid. *m*/*z* 448.0 [M+H]⁺. ¹H NMR (400 MHz, DMSO-*d*₆): δ ppm 7.82 - 7.87 (m, 1H), 7.67 - 7.70 (m, 1H), 7.45 (m, 1H), 3.86 (s, 3H), 3.15 - 3.23 (q, *J*=7.3 Hz, 2H), 2.42 (s, 3H), 1.11 - 1.19 (t, *J*=7.3 Hz, 3H).

### Example 67: Ethyl 5-acetyl-2-((2-fluoro-4-iodophenyl)amino)-4-isopropylthiophene-3-carboxylate

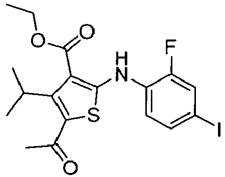

The title material was synthesised according to the general procedure for tetra-substituted thiophenes as outlined in Example 1, using 2-fluoro-4-iodo-1-isothiocyanatobenzene (2.0 g, 7.16 mmol), ethyl 4-methyl-3-oxopentanoate (1.2 mL, 7.16 mmol), DMF (20 mL), K₂CO₃ (2.0 g, 14.33 mmol), and chloroacetone (0.6 mL, 7.16 mmol). The product was isolated as a flocculant white solid (2.4 g, 69.3%) after purification by a flash column chromatography (Silica 40 g, 5-30% EtOAC in hexane). *m*/*z* 475.9 [M+H]⁺. ¹H NMR (400 MHz, DMSO-*d*₆): δ ppm 7.83 (dd, *J*=10.2, 1.8 Hz, 1H), 7.67 (d, *J*=8.5 Hz, 1H), 7.48 (t, *J*=8.6 Hz, 1H), 4.33 (q, *J*=7.0 Hz, 2H), 3.07 (sep, *J*=6.6 Hz, 1H), 1.33 (t, *J*=7.0 Hz, 3H), 1.08 (d, *J*=6.7 Hz, 6H).

### Example 68: Ethyl 5-acetyl-2-((2-fluoro-4-iodophenyl)amino)-4-methylthiophene-3-carboxylate

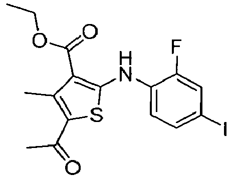

The title material was synthesized according to the general procedure for tetra-substituted thiophenes as outlined in Example 1, using 2-fluoro-4-iodo-1-isothiocyanatobenzene (1.0 g, 3.6 mmol), ethyl acetoacetate (0.5 mL, 3.6 mmol), DMF (10 mL), K₂CO₃ (1.0 g, 7.2 mmol), and chloroacetone (0.3 mL, 3.6 mmol). The product was isolated after work-up as an orange solid (1.6 g, 99%) and was used without further purification. *m*/*z* 447.9 [M+H]⁺. ¹H NMR (400 MHz, CDCl₃): δ ppm 7.50 - 7.58 (m, 2H), 7.44 - 7.50 (m, 1H), 4.43 (d, *J*=7.1 Hz, 2H), 2.79 (s, 3H), 2.53 (s, 3H), 1.44 (t, *J*=7.1 Hz, 3H).

### Example 69: 5-Acetyl-4-ethyl-2-((2-fluoro-4-iodophenyl)amino)thiophene-3-carboxylic acid (General Procedure for Ester Hydrolysis)

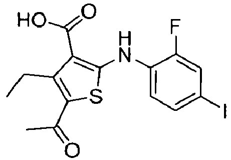

A solution of methyl 5-acetyl-4-ethyl-2-((2-fluoro-4-iodophenyl)amino)thiophene-3-carboxylate (1.8 g, 4.02 mmol), THF (72 mL), MeOH (24 mL), H₂O (48 mL) was stirred at room temperature and treated with 1M LiOH (24.1 mL, 24.1 mmol). A reflux condenser was attached and the reaction mixture was stirred at 50°C for 18 h. The cooled reaction mixture was acidified with 1M HCl, extracted with EtOAc (2 × 100 mL), and the combined organic layers were washed with brine (150 mL), dried over NaSO₄, filtered and concentrated under vacuum to give a yellow solid. The crude solid was triturated with DCM to give the product (1.75 g, quantitative) and was used without further purification. *m*/*z* 433.9 [M+H]⁺. ¹H NMR (400 MHz, DMSO-*d*₆): δ ppm 10.76 - 10.89 (br s, 1H), 7.79 - 7.81 (m, 1H), 7.65 - 7.70 (m, 1H), 7.46 - 7.52 (m, 1H), 3.20 - 3.27 (q, *J*=7.2 Hz, 2H), 2.44 (s, 3H), 1.15 (t, *J*=7.2 Hz, 3H).

### Example 70: 5-Acetyl-2-((2-fluoro-4-iodophenyl)amino)-4-isopropylthiophene-3-carboxylic acid

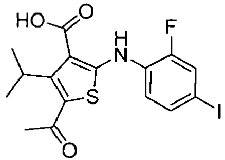

The title material was synthesized according to the general procedure for ester hydrolysis as outlined in the synthesis of Example 4, using ethyl 5-acetyl-2-((2-fluoro-4-iodophenyl)amino)-4-isopropylthiophene-3-carboxylate (2.0 g, 4.2 mmol), THF (75.7 mL), EtOH (25.2 mL), H₂O (50.5 mL) and 1M LiOH (25.2 mL, 25.2 mmol). The brown crude solid was purified by trituration with acetone to give the product as an off-white solid (1.0 g, 54.6%). *m*/*z* 447.9 [M+H]⁺. ¹H NMR (400 MHz, DMSO-*d*₆): δ ppm 10.82 (br s, 1H), 7.82 (dd, *J*=10.4, 1.8 Hz, 1H), 7.67 (d, *J*=8.5 Hz, 1H), 7.50 (t, *J*=8.6 Hz, 1H), 3.10 (sep, *J*=6.6 Hz, 1H), 2.67 - 2.74 (m, 3H), 1.09 (d, *J*=6.6 Hz, 6H).

### Example 71: 5-Acetyl-2-((2-fluoro-4-iodophenyl)amino)-4-methylthiophene-3-carboxylic acid

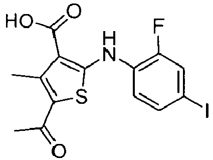

The title material was synthesized according to the general procedure for ester hydrolysis as outlined in Example 4, using ethyl 5-acetyl-2-((2-fluoro-4-iodophenyl)amino)-4-methylthiophene-3-carboxylate (1.6 g, 3.6 mmol), THF (64 mL), EtOH (21.3 mL), H₂O (42.8 mL) and 1M LiOH (21.3 mL, 21.3 mmol). The brown crude solid was purified by trituration with acetone to give the product as a brown solid (0.8 g, 68.7%). *m*/*z* 419.9 [M+H]⁺. ¹H NMR (400 MHz, DMSO-*d*₆): δ ppm 13.43 (br s, 1H), 10.82 (br s, 1H), 7.81 (dd, *J*=10.4, 1.8 Hz, 1H), 7.68 (m, 1H), 7.48 (t, *J*=8.6 Hz, 1H), 2.70 (s, 3H), 2.45 (s, 3H).

The following compounds were prepared according to the general procedure given above using the appropriate starting intermediates and reagents:

| **Ex. No.** | **Comp. No.** | **Structure** | **1H NMR (400 MHz, DMSO-d6)** | **m/z** |
|---|---|---|---|---|
| 72 | 4.007 | 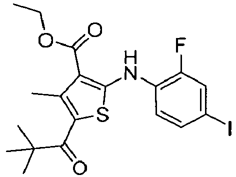 | δ 10.14 (s, 1H), 7.78 (dd, J=1.8 and 1.8 Hz, 1H), 7.64 (d, J=8.7 Hz, 1H), 7.45 (t, J=8.6 Hz, 1H), 4.30 (q, 2H), 2.49 (s, 3H), 1.30 (t,J=7.1 Hz, 3H), 1.23 (s, 9H). | 490.3 [M+1]⁺ |
| 73 | 4.008 | 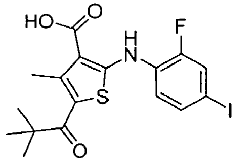 | δ 10.61 (s, 1H), 7.79 (dd, J=1.8 and 1.8 Hz, 1H), 7.65 (d, J=8.1 Hz, 1H), 7.49 (t, J=8.9 Hz, 1H), 2.52 (s, 3H), 1.24 (s, 9H). | 460.1 [M-1]⁻ |

### Example 74: 5-Acetyl-4-ethyl-2-((2-fluoro-4-iodophenyl)amino)-N-(2-hydroxyethoxy) thiophene-3-carboxamide (General Procedure for Amide Coupling)

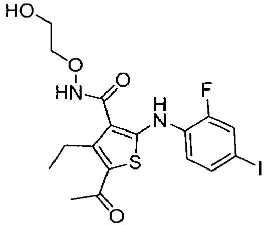

A solution of 5-acetyl-4-ethyl-2-((2-fluoro-4-iodophenyl)amino)thiophene-3-carboxylic acid (200 mg, 0.46 mmol), HATU (340 mg, 0.92 mmol), DMF (4.45 mL) in a Reacti-Vial^{™} was treated with DIPEA (0.1 5 mL, 0.92 mmol). The Reacti-Vial^{™} was sealed and the reaction mixture was heated to 50°C for 1 h, then cooled down to room temperature and 2-(aminooxy)ethanol (48 µl, 0.69 mmol) was added. The Reacti-Vial^{™} was re-sealed and the reaction mixture was stirred at room temperature for 3 days. The reaction mixture was purified by preparatory HPLC to give the product (28 mg, 12.3%) as an off-white/yellow solid. *m*/*z* 493.0 [M+H]⁺. ¹H NMR (400 MHz, DMSO-*d*₆): δ ppm 11.40 - 11.49 (br s, 1H), 8.98 - 9.10 (br s, 1H), 7.70 (br d, *J*=10.2 Hz, 1H), 7.53 (br d, *J*=8.1 Hz, 1H), 7.17 (br t, *J*=8.2 Hz, 1H), 4.78 - 4.85 (br s, 1H), 3.81 (t, *J*=4.0 Hz, 2H), 3.53 - 3.59 (t, *J*=4.0 Hz, 2H), 2.93 (m, 2H), 2.38 (s, 3H), 1.10 (t, *J*=7.4 Hz, 3H).

### Example 75: 5-Acetyl-2-((2-fluoro-4-iodophenyl)amino)-N-(2-hydroxyethoxy)-4-isopropylthiophene-3-carboxamide

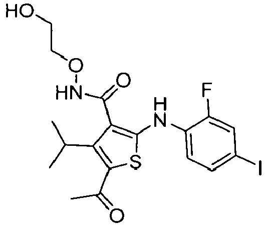

The title material was synthesized according to the general procedure for amide coupling as outlined in Example 9, using 5-acetyl-2-((2-fluoro-4-iodophenyl)amino)-4-isopropylthiophene-3-carboxylic acid (100 mg, 0.22 mmol), HATU (170 mg, 0.44 mmol), DMF (2.2 mL), DIPEA (70 µl, 0.44 mL), and 2-(aminooxy)ethanol (23 µl, 0.33 mL). The reaction mixture was purified by preparatory HPLC to give the product (9.0 mg, 7.9%) as a pale yellow/off-white solid. *m*/*z* 506.9 [M+H]⁺. ¹H NMR (400 MHz, DMSO-*d*₆): δ ppm 1 1.35 (br s, 1H), 9.45 (br s, 1H), 7.73 (br d, *J*=9.4 Hz, 1H), 7.56 (br d, *J*=8.5 Hz, 1H), 7.26 (br t, *J*=8.5 Hz, 1H), 4.81 (br s, 1H), 3.80 - 3.91 (m, 2H), 3.40 - 3.64 (m, 2H), 3.04 (sep, *J*= 6.6 Hz, 1H), 1.07 (d, *J*=6.6 Hz, 6H).

### Example 76: 5-Acetyl-N-(cyclopropylmethoxy)-2-((2-fluoro-4-iodophcnyl)amino)-4-isopropylthiophene-3-carboxamide

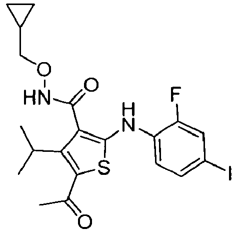

The title material was synthesized according to the general procedure for amide synthesis as outlined in the synthesis of Example 9, using 5-acetyl-2-((2-fluoro-4-iodophenyl)amino)-4-isopropylthiophene-3-carboxylic acid (100 mg, 0.22 mmol), HATU (170 mg, 0.44 mmol), DMF (2.2 mL), DIPEA (70 µl, 0.44 mL), and a solution of *O-*cyclopropylmethyl-hydroxylamine hydrochloride (41 mg, 0.33 mL) in pyridine (0.25 mL). The reaction mixture was purified by preparatory HPLC to give the product (7.2 mg, 7.8%) as a pale yellow/off-white solid. *m*/*z* 516.9 [M+H]⁺. ¹H NMR (400 MHz, CD₃CN) δ ppm 7.58 - 7.65 (m, 2H), 7.47 (t, *J*=8.6 Hz, 1H), 3.77 - 3.79 (d, *J*=7.2, 2H), 3.09 - 3.20 (m, 1H), 2.66 (s, 3H), 1.78 - 1.81 (m, 1H), 1.15 - 1.18 (d, *J*=6.6 Hz, 6H), 0.57 - 0.62 (m, 2H), 0.29 - 0.34 (m, 2H).

### Example 77: 5-Acetyl-2-((2-fluoro-4-iodophenyl)amino)-4-isopropylthiophene-3-carboxamide

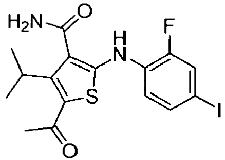

The title material was synthesized according to the general procedure for amide synthesis as outlined in the synthesis of Example 9, using 5-acetyl-2-((2-fluoro-4-iodophenyl)amino)-4-isopropylthiophene-3-carboxylic acid (100 mg, 0.22 mmol), HATU (170 mg, 0.44 mmol), DMF (2.2 mL), DIPEA (70 µl, 0.44 mL), and 0.5M NH₃ in 1,4-dioxane (0.67 mL, 0.33 mmol). The reaction mixture was purified by preparatory HPLC to give the product (13.4 mg, 13.4%) as an off-white solid. *m*/*z* 446.9 [M+H]⁺. ¹H NMR (400 MHz, CD₃CN): δ ppm 11.05 - 11.14 (br s, 1H), 7.55 - 7.62 (m, 2H), 7.43 - 7.50 (m, 1H), 6.19 - 6.30 (br s, 2H), 3.06 - 3.18 (m, 1H), 2.71 (s, 3H), 1.13 (d, *J*=6.7 Hz, 6H).

### Example 78: 5-Acetyl-2-((2-fluoro-4-iodophenyl)amino)-N-(2-hydroxyethoxy)-4-methylthiophene-3-carboxamide

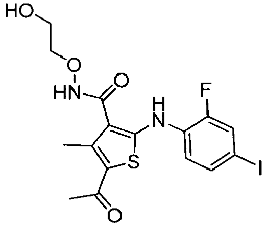

A solution of 5-acetyl-2-((2-fluoro-4-iodophenyl)amino)-4-methylthiophene-3-carboxylic acid (400 mg, 0.95 mmol), PyBOP (631 mg, 1.21 mmol), THF (4.8 mL) in a Reacti-Vial^{™} was treated with triethylamine (0.16 mL, 1.12 mmol), sealed and stirred for 30 min at room temperature. Then 2-(aminooxy)ethanol (106 mg, 1.38 mmol) was added, the Reacti-Vial^{™} was re-sealed and stirring continued for 18 h. The reaction mixture was diluted with EtOAc (150 mL), washed sequentially with H₂O (100 mL) and brine (100 mL), dried over Na₂SO₄, filtered and concentrated under vacuum. The crude product was purified by preparatory HPLC to give the product (103 mg, 22.6%) as a yellow solid. *m*/*z* 479.28 [M+H]⁺. ¹H NMR (400 MHz, CD₃CN): δ ppm 7.59 - 7.66 (m, 2H), 7.45 (t, *J*=8.8 Hz, 1H), 3.99 - 4.05 (m, 2H), 3.70 - 3.73 (m, 2H), 2.64 (s, 3H), 2.46 (s, 3H).

### Example 79: (S)-5-acetyl-N-(2,4-dihydroxybutyl)-2-((2-fluoro-4-iodophenyl)amino)-4-methylthiophene-3-carboxamide

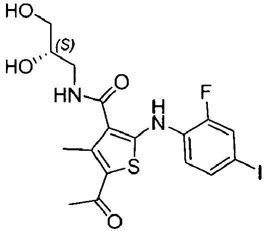

### Step 1: (S)-5-acetyl-N-((2,2-dii-nethyl-1,3-dioxolan-4-yl)methyl)-2-((2-fluoro-4-iodophenyl)amino)-4-methylthiophene-3-carboxamide

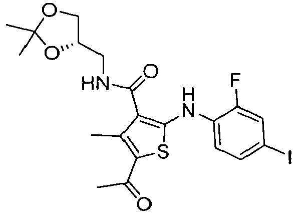

A solution of 5-acetyl-4-ethyl-2-((2-fluoro-4-iodophenyl)amino)thiophene-3-carboxylic acid (1.00 g, 2.39 mmol), PyBOP (1.61 g, 3.10 mmol) and Et₃N (0.4 mL, 2.87 mmol) in THF (12.5 mL) was stirred for 30 min at room temperature, then it was treated with (*S*)-(+)-(2,2-dimethyl-1,3-dioxolan-4-yl)methanamine (0.47 g, 3.59 mmol) and stirred for 2 h. The reaction mixture was concentrated *in vacuo,* the residue was dissolved in EtOAc (30 mL) and washed with water (30 mL), then brine (30 mL). The organic layer was dried over Na₂SO₄, filtered and concentrated *in vacuo.* The crude solid was purified by flash column chromatography (Silica 40 g, 0-35% EtOAc in hexane) to afford the desired product as an off-white solid (0.87 g, 69%). *m*/*z* 533.0 [M+H]⁺. ¹H NMR (400 MHz, DMSO-*d*₆): δ ppm 9.67 (s, 1H), 8.16 (t, *J*=5.9 Hz, 1H), 7.74 (dd, *J*=10.5, 1.8 Hz, 1H), 7.58 (d, *J*=8.4 Hz, 1H), 7.29 (t, *J*=8.7 Hz, 1H), 4.13 (quin, *J*=5.9 Hz, 1H), 3.94 (dd, *J*=8.3, 6.3 Hz, 1H), 3.67 (dd, *J*=8.3, 5.6 Hz, 1H), 3.36 - 3.44 (m, 1H), 3.24 - 3.33 (m, 1H), 2.54 (s, 3 H), 2.43 (s, 3H), 1.34 (s, 3H) 1.26 (s, 3H).

### Step 2: (S)-5-acetyl-N-(2,4-dihydroxybutyl)-2-((2-fluoro-4-iodophenyl)amino)-4-methylthiophene-3-carboxamide

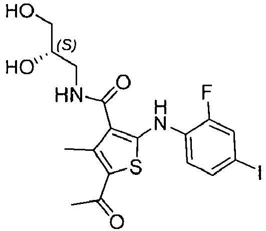

A solution of (*S*)-5-acetyl-N-((2,2-dimethyl-1,3-dioxolan-4-yl)methyl)-2-((2-fluoro-4-iodophenyl)amino)-4-methylthiophene-3-carboxamide (0.64 g, 1.20 mmol) in 1,4-dioxane (5 mL) was treated with 4M HCl in 1,4-dioxane (0.75 mmol) and stirred at room temperature for 4 h. The formed precipitate was filtered off, washed with 1,4-dioxane, Et₂O and EtOH to give the product (0.351 g, 59%) as an off-white solid. *m*/*z* 492.9 [M+H]⁺. ¹H NMR (400 MHz, DMSO-*d*₆): δ ppm 9.87 (s, 1H), 7.91 (t, *J*=5.8 Hz, 1H), 7.73 (dd, *J*=10.5, 1.9 Hz, 1H), 7.59 (d, *J*=8.4 Hz, 1H), 7.34 (t, *J*=8.7 Hz, 1H), 4.83 (d, *J*=5.0 Hz, 1H), 4.61 (t, *J*=5.7 Hz, 1H), 3.58 - 3.65 (m, 1H), 3.33 - 3.41 (m, 3H), 3.10 - 3.25 (m, 1H), 2.55 (s, 3H), 2.43 (s, 3H).

### Example 80: (R)-5-acetyl-N-(2,4-dihydroxybutyl)-2-((2-fluoro-4-iodophenyl)amino)-4-methylthiophene-3-carboxamide

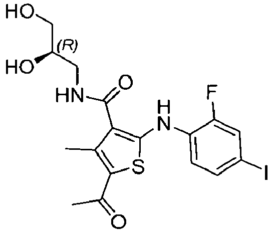

### Step 1: (R)-5-acetyl-N-((2,2-dimethyl-1,3-dioxolan-4-yl)methyl)-2-((2-fluoro-4-iodophenyl)amino)-4-methylthiophene-3-carboxamide

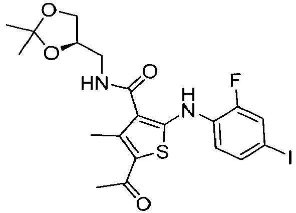

To a solution of 5-acetyl-2-((2-fluoro-4-iodophenyl)amino)-4-methylthiophene-3-carboxylic acid (1.00 g, 2.39 mmol) in THF (12.5 mL) was added Et₃N (0.4 mL, 2.87 mmol) and PyBOP (1.62 g, 3.11 mmol). The resultant mixture was stirred for 30 min, before the addition of (*R*)-(-)-(2,2-dimethyl-[1,3]dioxolan-4-yl) methylamine (471 mg, 3.59 mmol) as a solution in THF (0.5 mL). The reaction mixture was stirred for 4 h, before the solvent was removed *in vacuo* and the crude residue was partitioned between EtOAc and H₂O. The organic layer was separated, washed with brine, dried over Na₂SO₄, and the solvent removed *in vacuo.* The crude material was purified by flash column chromatography (Silica 40 g, 20-30% EtOAc in hexane) to give the product (969 mg, 76%) as a light purple solid. *m*/*z* 533.0 [M+H]⁺. ¹H NMR (400 MHz, DMSO-*d*₆): δ ppm 9.66 (s, 1 H), 8.14 (t, *J*=5.9 Hz, 1H), 7.73 (dd, *J*=10.5, 1.9 Hz, 1H), 7.57 (d, *J*=8.4 Hz, 1H), 7.28 (t, *J*=8.7 Hz, 1H), 4.13 (quin, *J*=5.9 Hz, 1H), 3.94 (dd, *J*=8.3, 6.3 Hz, 1H), 3.67 (dd, *J*=8.4, 5.6 Hz, 1H), 3.35 - 3.46 (m, 1H), 3.23 - 3.31 (m, 1H), 2.54 (s, 3H), 2.42 (s, 3H), 1.33 (s, 3H), 1.25 (s, 3H).

### Step 2: (R)-5-acetyl-N-(2,4-dihydroxybutyl)-2-((2-fluoro-4-iodophenyl)amino)-4-methylthiophene-3-carboxamide

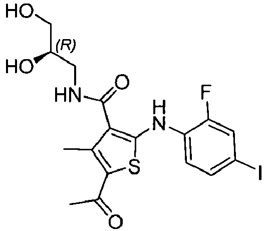

To a solution of (*R*)-5-acetyl-*N*-((2,2-dimethyl-1,3-dioxolan-4-yl)methyl)-2-((2-fluoro-4-iodophenyl)amino)-4-methylthiophene-3-carboxamide (417 mg, 0.783 mmol) in dioxane (5 mL) was added 4 N HCl in dioxane (0.49 mL, 1.96 mmol). The reaction mixture was stirred at room temperature for 18 h, resulting in the formation of a precipitate. The solid material was filtered and washed with Et₂O to give the product (331 mg, 86%) as a light purple solid. *m*/*z* 492.9 [M+H]⁺. ¹H NMR (400 MHz, DMSO-*d*₆): δ ppm 9.88 (s, 1H), 7.93 (br t, *J*=5.6 Hz, 1H), 7.74 (dd, *J*=10.4, 1.8 Hz, 1H), 7.59 (d, *J*=8.4 Hz, 1H), 7.34 (t, *J*=8.7 Hz, 1H), 4.86 (d, *J*=5.0 Hz, 1H), 4.64 (t, *J*=5.7 Hz, 1H), 3.61 (br d, *J*=6.7 Hz, 1H), 3.29 - 3.43 (m, 3H), 3.13 - 3.22 (m, 1H), 2.56 (s, 3H), 2.42 (s, 3H).

### Example 81: (R)-5-acetyl-2-((2-fluoro-4-iodophenyl)amino)-4-methyl-N-(piperidin-3-yl)thiophene-3-carboxamide

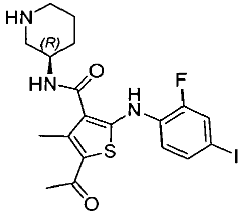

### Step 1: (R)-tert-butyl 3-(5-acetyl-2-((2-fluoro-4-iodophenyl)amino)-4-methylthiophene-3-carboxamido)piperidine-1-carboxylate

Following the general synthetic examples given above, the title compound was prepared using the appropriate starting intermediates and reagents. *m*/*z*: 600.2 [M-1]⁺.

### Step 2: (R)-5-acetyl-2-((2-fluoro-4-iodophenyl)amino)-4-methyl-N-(piperidin-3-yl)thiophene-3-carboxamide

A round bottom flask was charged with (R)-tert-butyl 3-(5-acetyl-2-((2-fluoro-4-iodophenyl)amino)-4-methylthiophene-3-carboxamido)piperidine-1-carboxylate (0.062 g, 0.1 mmol) in dichloromethane (8 mL) and 4M hydrogen chloride in dioxan (0.26 mL, 1.0 mmol) was added dropwise. The reaction mixture was stirred at room temperature overnight. The reaction was only 50% complete even after addition of more acid (0.13 mL). The solid formed was isolated by centrifuge, washed with dichloromethane 3 times, and dried by the lyophillizer to give the title compound as a light salmon colored solid (19 mg, 37%). *m*/*z* 502.2 [M+1]⁺. ¹H NMR (300 MHz, DMSO[-d6): δ 9.51 (s, 1H), 8.93-8.80 (m, 2H), 8.23 (d, J=7.8 Hz, 1H), 7.70 (d, J=9.9 Hz, 1H), 7.55 (d, J=8.7 Hz, 1H), 7.24 (t, J=8.9 Hz, 1H), 4.08-4.05 (m, 1H), 3.24-3.12 (m, 2H), 2.73-2.70 (m, 2H), 2.49 (s, 3H), 2.39 (s,3H), 1.84-1.80 (m, 2H), 1.67-163 (m, 1H), 1.48-1.45 (m, 1H).

### Example 82: (R)-5-acetyl-2-((2-fluoro-4-iodophenyl)amino)-4-methyl-N-(pyrrolidin-3-yl)thiophene-3-carboxamide

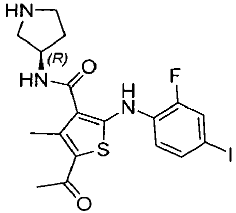

The title compound was prepared by the two step procedure according to Example 14. *m*/*z* 488.1 [M+1]⁺. ¹H NMR (300 MHz, DMSO-d6): δ 9.54 (s, 1H), 9.02-8.92 (m,2H), 8.36 (d, J=6.0 Hz, 1H), 7.73 (dd, J=1.8 and 1.8 Hz, 1H), 7.56 (d, J=9.0 Hz, 1H), 7.25 (t, J=8.4 Hz, 1H), 4.45-4.38 (m, 1H), 3.24-3.08 (m, 4H), 2.51 (s, 3H), 2.40 (s, 3H), 2.15-2.07 (m, 1H), 1.91-1.85 (m, 1H).

### Example 83: (R)-1-(4-(3-aminopyrrolidine-1-carbonyl)-5-((2-fluoro-4-iodophenyl)amino)-3-methylthiophen-2-yl)ethanone

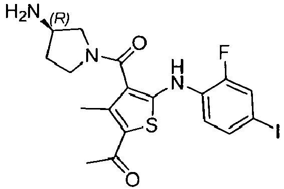

The title compound was prepared by the two step procedure according to Example 14. m/z 488.1 [M+1]⁺.

The following compounds were prepared according to the general procedure given above using the appropriate starting intermediates and reagents:

| **Ex. No.** | **Comp. No.** | **Structure** | **1H NMR (400 MHz, DMSO-d6)** | **m/z** |
|---|---|---|---|---|
| **84** | 4.019 | 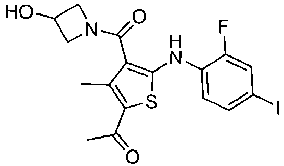 | δ 9.19 (s, 1H), 7.72 (d, J=10.5 Hz, 1H), 7.53( d, J=8.1 Hz, 1H), 7.16 (t, J=8.9 Hz, 1H), 5.72 (d, J=5.7 Hz, 1H), 4.44-4.41 (m, 1H), 4.07 (t, J=8.3 Hz, 2H), 3.70-3.68 (m, 2H), 2.39 (s, 3H), 2.36 (s, 3H) | 475.0 [M+1]⁺ |
| **85** | 4.020 | 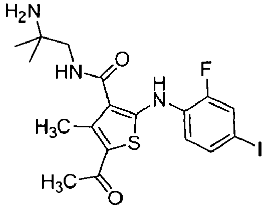 | δ 7.54 (t, J=7.7 Hz, 2H), 7.33 (t, J=10.0 Hz, 1H), 3.30 (s, 2H), 2.67 (s, 3H), 2.45 (s, 3H), 1.16 (s, 6H). (CD₃OD) | 488.2 [M+1]⁺ |
| **86** | 4.021 | 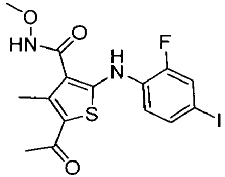 | δ 11.30 (s, 1H), 9.44 (s, 1H), 7.70 (dd, J=1.8 and 1.8 Hz, 1H), 7.55 (d, J=8.7 Hz, 1H), 7.23 (t, J=8.4 Hz, 1H), 3.62 (s, 3H), 2.47 (s, 3H), 2.38 (s, 3H). | 449.0 [M+1]⁺ |
| **87** | 4.022 | 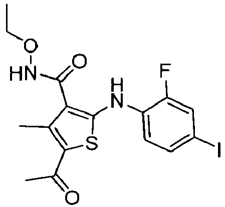 | δ 11.20 (s, 1H), 9.40 (s, 1H), 7.70 (dd, J=1.8 and 1.8 Hz, 1H), 7.54 (d, J=8.1 Hz, 1H), 7.22 (t, J=8.4 Hz, 1H), 3.83 (q, 2H), 2.47 (s, 3H), 2.38 (s, 3H), 1.15 (t, J=7.1 Hz, 3H). | 463.0 [M+1]⁺ |
| **88** | 4.023 | 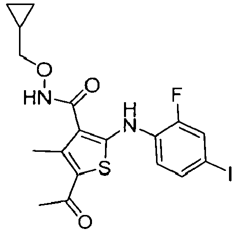 | δ 11.22 (s, 1H), 9.40 (s, 1H), 7.70 (dd, J=1.8 and 1.8 Hz, 1H), 7.53 (d, J=8.1 Hz, 1H), 7.21 (t, J=8.9 Hz, 1H), 3.57 (d, J=7.8 Hz, 2H), 2.46 (s, 3H), 2.38 (s, 3H), 1.10-1.00 (m, 1H), 0.50-0.48 (m, 2H), 0.22-0.20 (m, 2H). | 487.1 [M+1]⁺ |
| **89** | 4.024 | 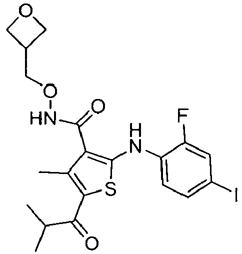 | δ 11.32 (s,1H), 9.39 (s, 1H), 7.71 (d, J=11.7 Hz, 1H), 7.54 (d, J=8.1 Hz, 1H), 7.20 (t, J=8.6 Hz, 1H), 4.63 (t, J=7.1 Hz, 2H), 4.33 (t, J=5.9 Hz, 2H), 3.98 (d, J=7.2 Hz, 2H), 3.20-3.15 (m, 1H), 2.46 (s, 3H), 2.38 (s, 3H). | 503.1 [M-1]⁻ |
| **90** | 4.025 | 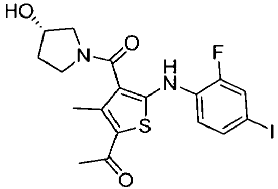 | δ 8.96 (s, 1H), 7.67 (d, J=9.9 Hz, 1H), 7.48 (d, J=9.3 Hz, 1H), 7.09 (t, J=8.1 Hz, 1H), 4.96-4.89 (m, 1H), 4.20-4.19 (m, 1H), 3.42-3.13 (m, 4H), 2.36 (s, 3H), 2.31 (s, 3H), 1.77-1.75 (m, 2H). | 489.0 [M+1]⁺ |
| **91** | 4.026 | 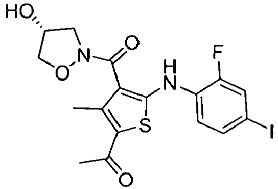 | δ 8.91 (s, 1H), 7.70 (dd, J=1.8 and 1.8 Hz, 1H), 7.54 (d, J=8.1 Hz, 1H), 7.21 (t, J=8.9 Hz, 1H), 5.55 (s, 1H), 4.65-4.63( m, 1H), 3.92-3.87 (m, 1H), 3.83-3.77 (m, 2H), 3.55-3.51 (m, 1H), 2.38 (s, 3H), 2.37 (s, 3H). | 491.1 [M+1]⁺ |

### Example 92: Ethyl 2-((2-fluoro-4-iodopheny))amino)-7-oxo-4,5,6,7-tetrahydrobenzo [b]thiophene-3-carboxylate

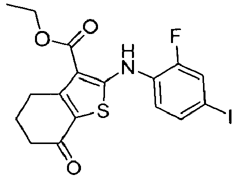

### Step 1: 2-Fluoro-4-(trimethylsilyl)aniline

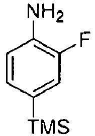

To a solution of 4-bromo-2-fluoroaniline (5.00 g, 26.31 mmol) in anhydrous THF (33 mL) under N₂ cooled down to -78 °C, *n*-BuLi (1.6M in hexanes, 65.75 mL, 105.25 mmol) was added dropwise over 20 min with internal temperature kept below -60 °C. Then the cooling bath was removed and when internal temperature reached 0 °C an ice-cold 2M HCl (150 mL) was added to the mixture and stirred vigorously for 10 min. Organic phase was separated, washed with water (100 mL) and saturated NaHCO₃ solution (100 mL). Aqueous phase was additionally extracted with DCM (4 × 100 mL) and the organic phase was washed with saturated NaHCO₃ solution (2 × 200 mL). Combine organic phases were dried over Na₂SO₄ and concentrated *in vacuo.* The crude was purified by flash column chromatography (Silica 80 g, 0-10% EtOAC in hexane) to give the product (2.71 g, 56%) as a red-brown oil. *m*/*z* 184.1 [M+H]⁺. ¹H NMR (400 MHz, DMSO-*d*₆): δ ppm 7.02 (dd, *J*=1.2, 11.8 Hz, 1H), 6.97 (dd, *J*=1.2, 7.6 Hz, 1H), 6.76 (dd, *J*=7.8, 8.9 Hz, 1H), 5.21 (br s, 2H), 0.17 (s, 9H).

### Step 2: Ethyl 2-bromo-7-oxo-4,5,6,7-tetrahydrobenzo[b]thiophene-3-carboxylate

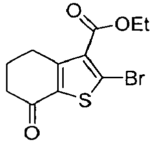

To a solution of copper(II) bromide (6.53 g, 29.25 mmol) in acetonitrile (77 mL) in a 3-neck flask under N₂ cooled to 0 °C, *tert*-butyl nitrite (90%, 3.2 mL, 24.03 mmol) was added dropwise. The mixture was stirred for 20 min at 0°C followed by a portionwise addition of solid ethyl 2-amino-7-oxo-4,5,6,7-tetrahydrobenzo[*b*]thiophene-3-carboxylate (5.00 g, 20.89 mmol) using a funnel under N₂ flow. The mixture was stirred under N₂ at 0°C for further 30 min and then partitioned between 2M HCl aqueous solution (250 mL) and EtOAc (200 mL). Aqueous phase was additionally extracted with EtOAc (4 × 100 mL). Combined organic phases were 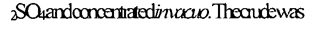 purified by flash column chromatography (Silica 120 g, 0-100% DCM in hexane) to give the product (3.85 g, 61%) as a light brown solid. *m*/*z* 304.9 [M+H]⁺. ¹H NMR (400 MHz, CDCl₃): δ ppm 4.40 (q, *J*=7.1 Hz, 2H), 3.08 (t, *J*=6.1 Hz, 2H), 2.61 (dd, *J*=7.2, 6.0 Hz, 2H), 2.01 - 2.35 (m, 2H), 1.42 (t, *J*=7.2 Hz, 3H).

### Step 3: Ethyl 2-((2-fluoro-4-(trimethylsilyl)phenyl)amino)-7-oxo-4,5,6,7-tetrahydrobenzor[b]thiophene-3-carboxylate

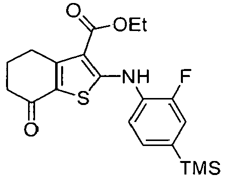

A solution of Pd₂(dba)₃ (91 mg, 0.098 mmol) and BINAP (124 mg, 0.199 mmol) in dry toluene (24 mL) in a Reacti-Vial^{™} was bubbled through with N₂ for 1 min followed by an addition of ethyl 2-bromo-7-oxo-4,5,6,7-tetrahydrobenzo[6]thiophene-3-carboxylate (600 mg, 1.979 mmol), Cs₂CO₃(904 mg, 2.774 mmol), and 2-fluoro-4-(trimethylsilyl)aniline (436 mg, 2.379 mmol) diluted with dry toluene (2 mL). The mixture was further bubbled through with N₂ for 1 min, sealed and stirred at 120 °C for 48 h. The reaction was quenched with saturated NH₄Cl aqueous solution (120 mL) and extracted with EtOAc (4 × 50 mL). Combined organic phases were washed with brine (200 mL), dried over Na₂SO₄, and concentrated *in vacuo.* The crude was purified by flash column chromatography (Silica 80 g + 12 g dry-load, 0-15% EtOAc in hexane) to give the product (716 mg, 89%) as a light yellow solid. *m*/*z* 406.1 [M+H]⁺. ¹H NMR (400 MHz, CDCl₃): δ ppm 10.93 (br s, 1H), 7.68 (t, *J*=8.0 Hz, 1H), 7.28 - 7.35 (m, 2H), 4.41 (q, *J*=7.2 Hz, 2H), 3.11 (t, *J*=6.1 Hz, 2H), 2.51 - 2.66 (m, 2H), 2.18 (quin, *J*=6.3 Hz, 2H), 1.43 (t, *J*=7.2 Hz, 3H), 0.29 (s, 9H).

### Step 4: Ethyl 2-((2-fluoro-4-iodophenyl)amino)-7-oxo-4,5,6,7-tetrahydrobenzo[b]thiophene-3-carboxylate

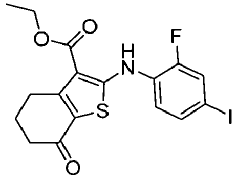

A solution of AgBF₄ (3.72 g, 19.13 mmol) in dry DCM (22 mL) under N₂ cooled to - 50 °C and protected from light was stirred for 15 min. A solution of ethyl 2-((2-fluoro-4-(trimethylsilyl)phenyl)amino)-7-oxo-4,5,6,7-tetrahydrobenzo[*b*]thiophene-3-carboxylate (2.59 g, 6.38 mmol) in dry DCM (50 mL) was added dropwise over 8 min and the mixture was further stirred at -50 °C for 30 min. Iodine monochloride (0.35 mL, 7.01 mmol) in dry DCM (10 mL) was added dropwise over 10 min and the reaction was stirred for 30 min. Reaction was quenched with saturated Na₂S₂O₃ aqueous solution (200 mL) and it was extracted with EtOAc (4 × 100 mL). Combined organic phases were washed with brine, dried over Na₂SO₄, and concentrated *in vacuo.* The crude was purified by recrystallisation from EtOH (160 mL) to give the product (2.37 g, 81%) as a creamy solid. *m*/*z* 459.9 [M+H]⁺. ¹H NMR (400 MHz, CDCl₃): δ ppm 10.92 (br s, 1H), 7.47 - 7.57 (m, 2H), 7.35 - 7.47 (m, 1H), 4.40 (q, *J*=7.2 Hz, 2H), 3.09 (t, *J*=6.1 Hz, 2H), 2.57 (dd, *J*=5.9, 7.3 Hz, 2H), 2.17 (quin, *J*=6.3 Hz, 2H), 1.42 (t, *J*=7.2 Hz, 3H).

### Example 93: 2-((2-Fluoro-4-iodophenyl)amino)-7-oxo-4,5,6,7-tetrahydrobenzo[6]thiophene-3-carboxylic acid

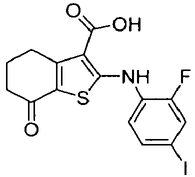

To a solution of ethyl 2-((2-fluoro-4-iodophenyl)amino)-7-oxo-4,5,6,7-tetrahydrobenzo[6]thiophene-3-carboxylate (2.37 g, 5.16 mmol) in THF (93 mL), EtOH (31 mL) and H₂O (62 mL), 1M LiOH aqueous solution (31 mL) was added. The mixture was stirred at 60 °C for 48 h. The solvents were removed *in vacuo* and the residue was sonicated with 1M HCl aqueous solution (200 mL) for 2.5h. The resulting precipitate was filtered and triturated with acetone (25 mL) to give the product (1.89 g, 85%) as a pale beige solid. *m*/*z* 431.9 [M+H]⁺. ¹H NMR (400 MHz, DMSO-*d*₆): δ ppm 13.37 (br s, 1H), 10.92 (br s, 1H), 7.81 (dd, *J*=1.9, 10.3 Hz, 1H), 7.58-7.72 (m, 1H), 7.47 (t, *J*=8.6 Hz, 1H), 3.04 (t, *J*=6.0 Hz, 2H), 2.46 (br t, *J*=6.5 Hz, 2H), 2.06 (quin, *J*=6.0 Hz, 2H).

### Example 94: 2-((2-Fluoro-4-iodophenyl)amino)-N-(2-hydroxyethoxy)-7-oxo-4,5,6,7-tetrahydrobenzo[b]thiophene-3-carboxamide

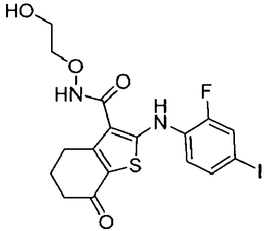

A solution of 2-((2-fluoro-4-iodophenyl)amino)-7-oxo-4,5,6,7-tetrahydrobenzo[*b*]thiophene-3-carboxylic acid (509 mg, 1.180 mmol), HATU (897 mg, 2.360 mmol), and pyridine (191 µL, 2.360 mmol) in DMF (24 mL) was stirred at 45 °C and monitored towards completion of HATU-activation of the acid. After 3 h the reaction was cooled down to room temperature, 2-aminooxyethanol (182 mg, 2.360 mmol) was added and it was stirred at room temperature for 26 h. The reaction mixture was diluted with H₂O (100 mL) and extracted with EtOAc (4 × 50 mL). Combined organic phases were washed with ice-cold brine (2 × 200 mL), dried over Na₂SO₄, and concentrated *in vacuo.* The crude was purified by preparative HPLC to give the product (238 mg, 41%) as a white solid. *m*/*z* 490.9 [M+H]⁺. ¹H NMR (400 MHz, DMSO-*d*₆): δ ppm 11.07 (br s, 1H), 10.25 (br s, 1H), 7.77 (d, *J*=9.8 Hz, 1H), 7.61 (d, *J*=7.9 Hz, 1H), 7.36 (t, *J*=8.6 Hz, 1H), 4.76 (br t, *J*=4.0 Hz, 1H), 3.90 (br t, *J*=4.6 Hz, 2H), 3.55 - 3.74 (m, 2H), 2.92 (br t, *J*=4.0 Hz, 2H), 2.45 (br t, *J*=6.1 Hz, 2H), 2.04 (br quin, *J*=6.0 Hz, 2H).

### Example 95: (R)-N-(2,3-Dihydroxypropoxy)-2-((2-fluoro-4-iodophenyl)amino)-7-oxo-4,5,6,7-tetrahydrobenzo[b]thiophene-3-carboxamide

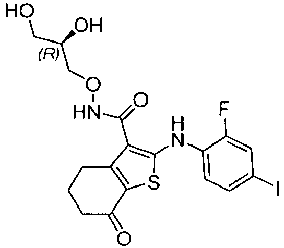

A solution of 2-((2-fluoro-4-iodophenyl)amino)-7-oxo-4,5,6,7-tetrahydrobenzo[*b*]thiophene-3-carboxylic acid (330 mg, 0.765 mmol), HATU (582 mg, 1.530 mmol), and pyridine (124 µL, 1.530 mmol) in DMF (16 mL) was stirred at 45 °C and monitored towards completion of HATU-activation of the acid. After 18 h the reaction was cooled down to room temperature, (*R*)-*O*-((2,2-dimethyl-1,3-dioxolan-4-yl)methyl)hydroxylamine (168 mg, 1.148 mmol) was added and it was stirred at room temperature for 1 h. The reaction mixture was diluted with H₂O (80 mL) and extracted with EtOAc (4 × 50 mL). Combined organic phases were washed with ice-cold brine (2 × 200 mL), dried over Na₂SO₄, and concentrated *in vacuo.* The crude residue was dissolved in MeOH (5.3 mL) followed by an addition of p-toluene sulfonic acid monohydrate (56 mg, 0.294 mmol) and ethylene glycol (210 µL, 3.722 mmol). The resultant mixture was stirred at room temperature 5 h, followed by an addition of Et₃N (105 µL, 0.767 mmol) to the reaction mixture and the solvent was removed *in vacuo.* The crude product was purified by preparatory HPLC to give the product (174 mg, 43%) as a light-yellow solid. *m*/*z* 520.9 [M+H]+. ¹H NMR (400 MHz, DMSO-*d*₆): δ ppm 11.09 (br s, 1H), 10.25 (br s, 1H), 7.77 (br d, *J*=10.5 Hz, 1H), 7.62 (d, *J*=8.4 Hz, 1H), 7.37 (t, *J*=8.6 Hz, 1H), 4.89 (br d, *J*=3.9 Hz, 1H), 4.62 (t, *J*=5.7 Hz, 1H), 3.94 (br dd, *J*=2.4, 9.1 Hz, 1H), 3.63 - 3.84 (m, 2H), 3.34 - 3.49 (m, 2H), 3.01 - 2.84 (m, 2H), 2.39 - 2.48 (m, 2H), 1.91 - 2.12 (m, 2H).

### Example 96: 2-((2-Fluoro-4-iodophenyl)amino)-N-hydroxy-7-oxo-4,5,6,7-tetrahydrobenzo[b]thiophene-3-carboxamide

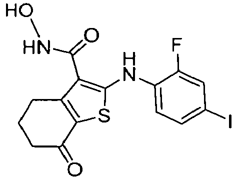

A solution of 2-((2-fluoro-4-iodophenyl)amino)-7-oxo-4,5,6,7-tetrahydrobenzo[*b*]thiophene-3-carboxylic acid (300 mg, 0.696 mmol), HATU (529 mg, 1.391 mmol), and pyridine (112 µL, 1.391 mmol) in DMF (12 mL) was stirred at 40 °C and monitored towards completion of HATU-activation of the acid. After 18 h the reaction was cooled down to room temperature, hydroxylamine hydrochloride (725 mg, 1.044 mmol) and pyridine (112 µL, 1.391 mmol) were added and it was stirred at room temperature for 2 h. The reaction mixture was diluted with H₂O (100 mL) and extracted with EtOAc (4 × 50 mL). Combined organic phases were washed with ice-cold brine (2 × 150 mL), dried over Na₂SO₄, and concentrated *in vacuo.* The crude was purified by preparative HPLC (Reach Separations, UK) to give the product (137 mg, 44%) as a light-yellow solid. *m*/*z* 447.0 [M+H]⁺. ¹H NMR (400 MHz, DMSO-*d*₆): δ ppm 10.58 (br s, 1H), 10.48 (br s, 1H), 9.16 (br s, 1H), 7.75 (br d, *J*=9.5 Hz, 1H), 7.61 (br d, *J*=8.8 Hz, 1H), 7.38 (br t, *J*=8.7 Hz, 1H), 2.92 (br t, *J*=5.0 Hz, 2H), 2.43 (br t, *J*=5.0 Hz, 2H), 1.98 - 2.06 (br m, 2H).

### Example 97: N-(Cyclopropylmethoxy)-2-((2-fluoro-4-iodophenyl)amino)-7-oxo-4,5,6,7-tetrahydrobenzo[b]thiophene-3-carboxamide

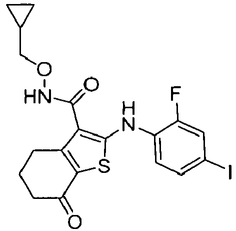

A solution of 2-((2-fluoro-4-iodophenyl)amino)-7-oxo-4,5,6,7-tetrahydrobenzo[*b*]thiophene-3-carboxylic acid (44 mg, 0.102 mmol), HATU (2 × 78 mg, 0.408 mmol), and pyridine (2 × 16 µL, 0.408 mmol) in DMF (2 mL) was stirred initially at room temperature for 18 h, and then at 40 °C for 1.5 h being monitored towards completion of HATU-activation of the acid. The reaction was cooled down to room temperature, *O*-(cyclopropylmethyl)-hydroxylamine hydrochloride (15+7+7 mg, 0.245 mmol) was added and it was stirred at room temperature for 48 h. The reaction mixture was directly purified by preparative HPLC to give the product (9 mg, 17%) as an off-white solid. *m*/*z* 500.9 [M+H]⁺. ¹H NMR (400 MHz, DMSO-*d*₆): δ ppm 11.00 (br s, 1H), 10.25 (br s, 1H), 7.69 - 7.83 (m, 1H), 7.52 - 7.66 (m, 1H), 7.21 - 7.45 (m, 1H), 3.66 (d, *J*=7.5 Hz, 2H), 2.88 - 2.96 (m, 2H), 2.38 - 2.48 (m, 2H), 1.99 - 2.12 (m, 2H), 1.02 - 1.17 (m, 1H), 0.44 - 0.60 (m, 2H), 0.21 - 0.31 (m, 2H).

### Example 98: N-(2-Aminoethoxy)-2-(2-fluoro-4-iodophenylamino)-7-oxo-4,5,6,7-tetrahydrobenzo[b]thiophene-3-carboxamide hydrochloride

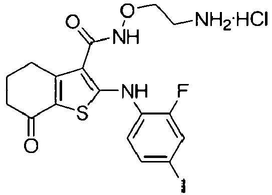

### Step 1: tert-Butyl 2-(2-(2-fluoro-4-iodophenylamino)-7-oxo-4,5,6,7-tetrahydrobenzo [b]thiophene-3-carboxamidooxy)ethylcarbamate

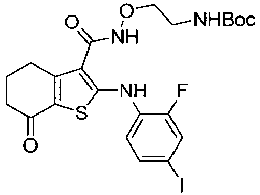

To a solution of 2-((2-fluoro-4-iodophenyl)amino)-7-oxo-4,5,6,7-tetrahydrobenzo[*b*]thiophene-3-carboxylic acid (600 mg, 1.391 mmol) and HATU (1.06 g, 2.783 mmol) in DMF (18 mL) stirred at room temperature DIPEA (0.486 ml, 2.783 mmol) was added dropwise and the reaction was monitored towards completion of HATU-activation of the acid. After 40 min *tert*-butyl 2-(aminooxy)-ethylcarbamate (368 mg, 2.086 mmol) was added to the reaction mixture and it was stirred at room temperature for 1 h. The reaction mixture was quenched with H₂O (30 mL) and a precipitate formed which was filtered and washed with H₂O. The crude was purified by flash column chromatography (Silica 40 g, 0-60% EtOAc in hexanes) to give the product (473 mg, 58%) as a creamy solid. *m*/*z* 590.1 [M+H]⁺. ¹H NMR (400 MHz, DMSO-*d*₆): δ ppm 10.95 (br s, 1H), 10.31 (br s, 1H), 7.77 (d, *J*=10.3 Hz, 1H), 7.62 (d, *J*=8.5 Hz, 1H), 7.37 (t, *J*=8.8 Hz, 1H), 6.84 (br s, 1H), 3.93 - 3.78 (m, 2H), 3.26 - 3.14 (m, 2H), 3.00 - 2.85 (m, 2H), 2.46 - 2.37 (m, 2H), 2.13 - 1.99 (m, 2H), 1.39 (d, *J*=2.5 Hz, 9H).

### Step 2: N-(2-Aminoethoxy)-2-(2-fluoro-4-iodophenylamino)-7-oxo-4,5,6,7-tetrahydrobenzo[b]thiophene-3-carboxamide hydrochloride

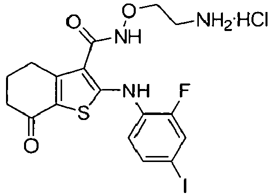

A suspension of *tert*-butyl 2-(2-(2-fluoro-4-iodophenylamino)-7-oxo-4,5,6,7-tetrahydrobenzo-[*b*]thiophene-3-carboxamidooxy)ethylcarbamate (50 mg, 84.8 µmol) in dioxane (0.5 ml) was stirred at room temperature and 4N HCl in dioxane (36 µl, 144.2 µmol) was added. After 30 min a further portion of 4N HCl in dioxane (0.5 ml, 2.0 mmol) was added to the reaction mixture. After 18 h the suspension was filtered and the collected crude material was washed sequentially with dioxane and Et₂O to give the product (40.5 mg, 98%) as a yellow solid. *m*/*z* 490.0 [M+H]⁺. ¹H NMR (400 MHz, DMSO-*d*₆): δ ppm 11.35 (br s, 1H), 10.22 (br s, 1H), 8.00 (br s, 3H), 7.79 (dd, *J*=10.4, 1.9 Hz, 1H), 7.70 - 7.59 (m, 1H), 7.38 (t, *J*=8.6 Hz, 1H), 4.08 (t, *J*=5.2 Hz, 2H), 3.09 (q, *J*=5.5 Hz, 2H), 2.95 (t, *J*=5.9 Hz, 2H), 2.50 - 2.42 (m, 2H), 2.12 - 1.96 (m, 2H).

### Example 99: (S)-N-(2,3-dihydroxypropyl)-2-((2-fluoro-4-iodophenyl)amino)-7-oxo-4,5,6,7-tetrahydrobenzo[b]thiophene-3-carboxamide

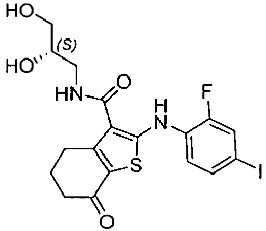

### Step 1: (S)-N-((2,2-dimethyl-1,3-dioxolan-4-yl)methyl)-2-((2-fluoro-4-iodophenyl)amino)-7-oxo-4,5,6,7-tetrahydrobenzor[b]thiophene-3-carboxamide

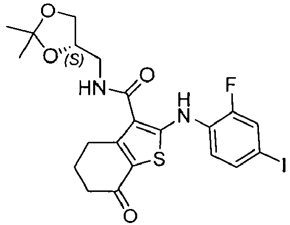

A solution of 2-((2-fluoro-4-iodophenyl)amino)-7-oxo-4,5,6,7-tetrahydrobenzo[*b*]thiophene-3-carboxylic acid (500 mg, 1.159 mmol), HATU (882 mg, 2.319 mmol), and Et₃N (320 µL, 2.319 mmol) in DMF (15 mL) was stirred at room temperature and monitored towards completion of HATU-activation of the acid (5 min). (*S*)-(+)-(2,2-Dimethyl-[1,3]-dioxolan-4-yl)-methylamine (229 mg, 1.738 mmol) was added and it was stirred at room temperature for 1 h. The reaction mixture was diluted with H₂O (50 mL) and a precipitation occurred. Solids were collected by filtration and washed with water. Aqueous filtrate was extracted with EtOAc (3 × 25 mL). Combined organic phases were washed with ice-cold brine (2 × 100 mL), dried over Na₂SO₄, and concentrated *in vacuo.* The combined crude was purified by flash column chromatography (Silica 40 g, 0-50% EtOAc in hexane) to give the product (314 mg, 50%) as a light purple solid. *m*/*z* 545.0 [M+H]⁺. ¹H NMR (400 MHz, DMSO-*d*₆): δ ppm 10.64 (d, *J*=1.1 Hz, 1H), 7.71-7.82 (m, 2H), 7.62 (d, *J*=8.4 Hz, 1H), 7.38 (t, *J*=8.7 Hz, 1H), 4.20 (quin, *J*=5.6 Hz, 1H), 3.98 (dd, *J*=6.3, 8.3 Hz, 1H), 3.69 (dd, *J*=5.6, 8.4 Hz, 1H), 3.40-3.49 (m, 1H), 3.32-3.48 (m, 1H), 2.97 (q, *J*=5.5 Hz, 2H), 2.36-2.47 (m, 2H), 2.00-2.07 (m, 2H), 1.35 (s, 3H), 1.26 (s, 3H).

### Step 2: (S)-N-(2,3-dihydroxypropyl)-2-((2-fluoro-4-iodophenyl)amino)-7-oxo-4,5,6,7-tetrahydrobenzor[b]thiophene-3-carboxamide

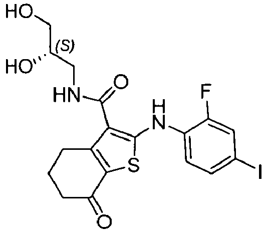

A solution of (*S*)-*N*-((2,2-dimethyl-1,3-dioxolan-4-yl)methyl)-2-((2-fluoro-4-iodophenyl)amino)-7-oxo-4,5,6,7-tetrahydrobenzo[*b*]thiophene-3-carboxamide (314 mg, 0.577 mmol) in dioxane (15 ml) was treated with 4N HCl solution in dioxane (0.36 ml, 1.442 mmol) and stirred at room temperature. After 24h additional portion of 4N HCl in dioxane (0.36 ml, 1.442 mmol) was added and reaction was transferred to a rotary evaporator for 2 h to remove forming acetone at 40 °C at 500 mbar. A precipitate formed with concentration thus additional dioxane (5 ml) and 4N HCl in dioxane (0.36 ml, 1.442 mmol) were added to the reaction stirred at room temperature for further 18h until completion. Formed precipitate was filtered and washed with dioxane and Et₂O to give the product (186 mg, 64%) as a light yellow solid. *m*/*z* 505.0 [M+H]⁺. ¹H NMR (400 MHz, DMSO-*d*₆): δ ppm 10.92 (d, *J*=1.1 Hz, 1H), 7.77 (dd, *J*=1.9, 10.5 Hz, 1H), 7.63 (d, *J*=8.4 Hz, 1H), 7.52 (t, *J*=5.7 Hz, 1H), 7.42 (t, *J*=8.7 Hz, 1H), 4.88 (d, *J*=5.1 Hz, 1H), 4.66 (t, *J*=5.7 Hz, 1H), 3.60-3.72 (m, 1H), 3.33-3.50 (m, 3H), 3.14-3.26 (m, 1H), 2.93-3.07 (m, 2H), 2.44-2.47 (m, 2H), 2.02-2.16 (m, 2H).

### Example 100: (R)-N-(2,3-dihydroxypropyl)-2-((2-fluoro-4-iodophenyl)amino)-7-oxo-4,5,6,7-tetrahydrobenzo[b]thiophene-3-carboxamide

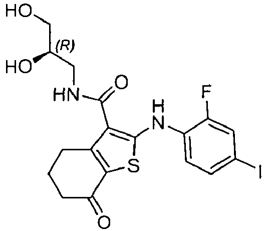

### Step 1: (R)-N-((2,2-dimethyl-1,3-dioxolan-4-yl)methyl)-2-((2-fluoro-4-iodophenyl)amino)-7-oxo-4,5,6,7-tetrahydrobenzo[b]thiophene-3-carboxamide

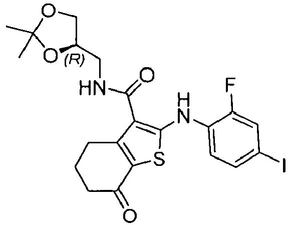

A solution of 2-((2-fluoro-4-iodophenyl)amino)-7-oxo-4,5,6,7-tetrahydrobenzo[*b*]thiophene-3-carboxylic acid (500 mg, 1.159 mmol), HATU (882 mg, 2.319 mmol), and Et₃N (320 µL, 2.319 mmol) in DMF (10 mL) was stirred at room temperature and monitored towards completion of HATU-activation of the acid (5 min). (*R*)-(-)-(2,2-Dimethyl-[1,3]-dioxolan-4-yl)-methylamine (229 mg, 1.738 mmol) was added and it was stirred at room temperature for 1 h. The reaction mixture was diluted with H₂O (40 mL) and a precipitation occurred. Solids were collected by filtration and washed with water. Aqueous filtrate was extracted with EtOAc (3 × 25 mL). Combined organic phases were washed with ice-cold brine (2 × 100 mL), dried over Na₂SO₄, and concentrated in *vacuo.* The combined crude was purified by flash column chromatography (Silica 40 g, 0-80% EtOAc in hexane) to give the product (340 mg, 54%) as a light purple solid. *m*/*z* 545.0 [M+H]⁺. ¹H NMR (400 MHz, DMSO-*d*₆): δ ppm 10.64 (s, 1H), 7.77 (dd, *J*=1.9, 10.3 Hz, 2H), 7.62 (d, *J*=8.3 Hz, 1H), 7.38 (t, *J*=8.7 Hz, 1H), 4.14-4.25 (m, 1H), 3.98 (dd, *J*=6.3, 8.3 Hz, 1H), 3.70 (dd, *J*=5.6, 8.3 Hz, 1H), 3.40-3.51 (m, 1H), 3.27-3.38 (m, 1H), 2.97 (q, *J*=5.7 Hz, 2H), 2.44-2.47 (m, 2H), 2.01-2.11 (m, 2H), 1.36 (s, 3H), 1.26 (s, 3H).

### Step 2: (R)-N-(2,3-dihydroxypropyl)-2-((2-fluoro-4-iodophenyl)amino)-7-oxo-4,5,6,7-tetrahydrobenzo[b]thiophene-3-carboxamide

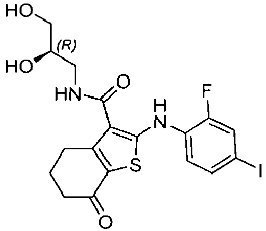

To a solution of (*R*)-N-((2,2-dimethyl-1,3-dioxolan-4-yl)methyl)-2-((2-fluoro-4-iodophenyl)amino)-7-oxo-4,5,6,7-tetrahydrobenzo[*b*]thiophene-3-carboxamide (330 mg, 0.606 mmol) in dioxane (10 mL) was added 4 N HCl in dioxane (0.38 mL, 1.52 mmol). The reaction mixture was stirred at room temperature for 18 h, resulting in the formation of a precipitate. The solid material was filtered and washed with Et₂O to give the product (214 mg, 70%) as a light yellow solid. *m*/*z* 505.0 [M+H]⁺. ¹H NMR (400 MHz, DMSO-*d*₆): δ ppm 10.92 (s, 1 H), 7.77 (dd, *J*=10.5, 1.8 Hz, 1H), 7.62 (s, 1 H), 7.52 (br t, *J*=5.5 Hz, 1H), 7.41 (t, *J*=8.7 Hz, 1H), 4.89 (d, *J*=5.0 Hz, 1H), 4.66 (t, *J*=5.7 Hz, 1H), 3.60 - 3.71 (m, 1H), 3.33 - 3.49 (m, 3H), 3.16 - 3.26 (m, 1H), 2.96 - 3.05 (m, 2H), 2.47 (br d, *J*=6.8 Hz, 2H), 2.07 (br t, *J*=5.9 Hz, 2H).

### Example 101: (R)-2-(2,3-Dihydroxypropyl)-8-(2-fluoro-4-iodophenylamino)-2,6-naphthyridin-1(2H)-one

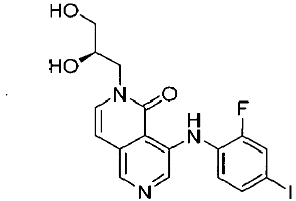

### Step 1: Methyl 3-bromo-5-fluoroisonicotinate

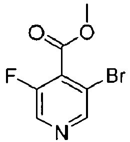

To a solution of dry diisopropylamine (8.8 mL, 62.5 mmol) in dry THF (300 mL) stirred at 0°C *n*-BuLi (2.5M in hexanes, 25 mL, 62.5 mmol) was added. The reaction mixture was stirred for 30 min at room temperature, then cooled down to -78°C and a solution of 3-bromo-5-fluoropyridine (10 g, 56.8 mmol) in dry THF (300 mL) was added. The reaction mixture was stirred for 1 h and treated with methyl chloroformate (5.3 mmol, 68.2 mmol). The reaction mixture was stirred for 1.5 h and then was quenched with a saturated NH₄Cl aqueous solution at 0°C, extracted with EtOAc (3 × 100 mL), washed with H₂O (100 mL) and brine (100 mL), dried over Na₂SO₄ and concentrated *in vacuo.* The crude material was purified by flash column chromatography (Silica, 0-7% EtOAc in hexanes) to give the product (10.43 g, 78%) as a yellowish liquid. UPLC-MS (Acidic Method, 2 min): rt 0.86 min, *m*/*z* 234.0/236.0 [M+H]⁺. ¹H NMR (400 MHz, CDCl₃): δ ppm 8.69 (t, *J*=0.5 Hz, 1H), 8.57 (d, *J*=8.4 Hz, 1H), 4.08 (s, 3H).

### Step 2: 2-(2-Ethoxyvinyl)-4,4,5,5-tetramethyl-1,3,2-dioxaborolane (the compound of formula (103))

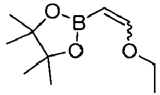

To a mixture of pinacolborane (20 g, 156 mmol) and ethyl vinyl ether (61.4 mL, 640 mmol) was added palladium(11) acetate (0.176 g, 0.781 mmol) carefully due to an exothermic process. The reaction mixture was stirred at room temperature for 18 h. Then the reaction mixture was concentrated *in vacuo* and the residue was passed through a silica plug (0-10% EtOAc in hexanes) to give the product (24.6 g, 85%) as a yellow liquid. UPLC-MS (Acidic Method, 2 min): rt 0.92 and 1.03 min, *m*/*z* 199.2 [M+H]⁺. ¹H NMR (400 MHz, CDCl₃): δ ppm (*Note:* a mixture of *E*:*Z* isomers 1.25:1) 7.03 (d, *J*=14.4 Hz, 1.25H), 6.64 (d, *J*=0.4 Hz, 1H), 4.43 (d, *J*=14.4 Hz, 1.25H), 4.11 (dd, *J*=7.8, 4.4 Hz, 1H), 3.94 (q, *J*=7.1 Hz, 2H), 3.84 (q, *J*=7.1 Hz, 2.5H), 1.28 - 1.24 (m, 31H), 0.95 - 0.84 (m, 3H).

### Step 3: Methyl 3-(2-ethoxyvinyl)-5-fluoroisonicotinate (a compound of formula (104))

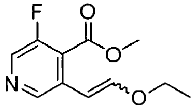

A degassed solution of methyl 3-bromo-5-fluoroisonicotinate (10 g, 42.70 mmol), 2-(2-ethoxyvinyl)-4,4,5,5-tetramethyl-1,3,2-dioxaborolane (12.7 g, 64.05 mmol), Cs₂CO₃ (48.7 g, 49.45 mmol) and PdXPhos G2 catalyst (3.4 g, 4.27 mmol) in THF/H₂O (9:1 v/v, 90:10 mL) was stirred at 85°C for 18 h. The reaction mixture was diluted with EtOAc (100 mL) and filtered through a Celite pad. The organic filtrate was washed with H₂O (100 mL), brine (100 mL), dried over Na₂SO₄, and concentrated *in vacuo.* The crude material was purified by flash column chromatography (Silica, 0-25% EtOAc in hexanes) to give the product (7.07 g, 74%) as a brown oil. UPLC-MS (Acidic Method, 2 min): rt 1.01 and 1.10 min, *m*/*z* 226.1 [M+H]⁺. ¹H NMR (400 MHz, CDCl₃): δ ppm (*Note:* a mixture of E:Z isomers 1.15 : 1) 9.18 - 9.13 (m, 1H), 8.48 (s, 1.15H), 8.30 (dd, *J*=5.2, 0.8 Hz, 2.15H), 7.03 (d, *J*=12.9 Hz, 1.15H), 6.41 (dd, *J*=7.1, 0.7 Hz, 1H), 5.91 (d, *J*=12.9 Hz, 1.15H), 5.27 (d, *J*=7.1 Hz, 1H), 4.05 (q, *J*=7.1 Hz, 2H), 4.01 - 3.90 (m, 8.75H), 1.37 (td, *J*=7.1, 2.8 Hz, 6.5H).

### Step 4: 8-Fluoro-1H-pyrano[4,3-c]pyridin-1-one hydrochloride (a compound of formula (105))

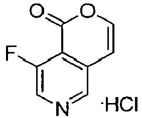

To methyl 3-(2-ethoxyvinyl)-5-fluoroisonicotinate (2.07 g, 9.2 mmol) was added 4M HCl (26 mL, 104 mmol) and the reaction mixture was stirred at 100°C for 18 h. The reaction mixture was cooled down and the formed precipitate was isolated by filtration, washed with dioxane (3 × 5 mL) and dried *in vacuo* to give the product (0.93 g, 50%) as a pale-brown solid. UPLC-MS (Acidic Method, 2 min): rt 0.60, *m*/*z* 166.0 [M+H]⁺. ¹H NMR (400 MHz, CDCl₃): δ ppm 8.69 (s, 1H), 8.63 (d, *J*=2.2 Hz, 1H), 7.41 (d, *J*=5.6 Hz, 1H), 6.60 (dd, *J*=5.6, 2.5 Hz, 1H).

### Step 5: (R)-2-((2,2-Dimethyl-1,3-dioxolan-4-yl)methyl)-8-fluoro-2,6-naphthyridin-1(2H)-one

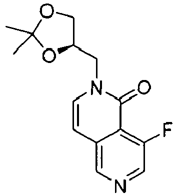

To a solution of 8-fluoro-1*H*-pyrano[4,3-*c*]pyridin-1-one hydrochloride (300 mg, 1.82 mmol) in MeOH (13 mL) was added (*R*)-(2,2-dimethyl-1,3-dioxolan-4-yl)methanamine (334 mg, 2.55 mmol) and the reaction mixture was heated at 80 °C for 72 h. The reaction mixture was concentrated *in vacuo* and the residue was dissolved in EtOAc, washed with H₂O (30 mL), brine (30 mL), dried over Na₂SO₄, and concentrated *in vacuo.* The crude material was purified by flash column chromatography (Silica, 0-80% EtOAc in hexanes) to give the product (94 mg, 19%) as an off-white solid. UPLC-MS (Acidic Method, 2 min): rt 0.84 min, *m*/*z* 279.2 [M+H]⁺. ¹H NMR (400 MHz, CDCl₃): δ 8.74 (s, 1H), 8.46 (d, *J*=3.1 Hz, 1H), 7.37 (d, *J*=7.4 Hz, 1H), 6.55 (dd, *J*=7.4, 2.3 Hz, 1H), 4.52 (qd, *J*=6.6, 3.0 Hz, 1H), 4.39 (dd, *J*=13.8, 3.0 Hz, 1H), 4.16 (dd, *J*=8.8, 6.5 Hz, 1H), 3.91 (dd, *J*=13.8, 7.0 Hz, 1H), 3.74 (dd, *J*=8.8, 6.3 Hz, 1H), 1,43 (s, 3H), 1.34 (s, 3H).

### Step 6: (R)-2-((2,2-Dimethyl-1,3-dioxolan-4-yl)methyl)-8-(2-fluoro-4-iodophenylamino)-2,6-naphthyridin-1(2H)-one

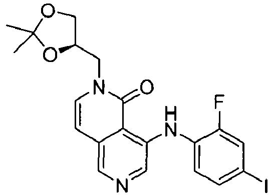

A solution of 2-fluoro-4-iodoaniline (49 mg, 0.21 mmol) in dry THF (1 mL) stirred at -78°C was treated with LiHMDS (1M in THF, 0.3 mL, 0.3 mmol) and the reaction mixture was stirred for 10 min. Then a solution of (*R*)-2-((2,2-dimethyl-1,3-dioxolan-4-yl)methyl)-8-fluoro-2,6-naphthyridin-1(2*H*)-one (60 mg, 0.216 mmol) in dry THF (1 mL) was added and the reaction mixture was stirred at -78°C for 15 min, and then let to warm up to room temperature. The reaction mixture was quenched with a saturated NH₄Cl aqueous solution (1 mL) at 0°C and extracted with EtOAc (3 × 7 mL). The combined organic phase was washed with H₂O (7 mL), brine (7 mL), dried over Na₂SO₄, and concentrated *in vacuo.* The crude material was purified by flash column chromatography (Silica, 0-50% EtOAc in hexanes) to give the product (68 mg, 64%) as a yellow solid. UPLC-MS (Acidic Method, 2 min): rt 1.17 min, *m*/*z* 496.1 [M+H]⁺. ¹H NMR (400 MHz, CDCl₃): δ 10.56 (s, 1H), 8.41 (s, 1H), 8.28 (s, 1H), 7.56 - 7.43 (m, 2H), 7.33 (t, *J*=8.4 Hz, 1H), 7.25 (d, 1H), 6.50 (d, *J*=7.4 Hz, 1H), 4.51 (qd, *J*=6.5, 3.1 Hz, 1H), 4.36 (dd, *J*=13.8, 3.1 Hz, 1H), 4.16 (dd, *J*=8.7, 6.5 Hz, 1H), 3.88 (dd, *J*=13.8, 7.0 Hz, 1H), 3.75 (dd, *J*=8.7, 6.2 Hz, 1H), 1.44 (s, 3H), 1.35 (s, 3H).

### Step 7: (R)-2-(2,3-Dihydroxypropyl)-8-(2-fluoro-4-iodophenylamino)-2,6-naphthyridin-1(2H)-one

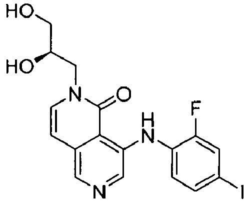

A solution of (*R*)-2-((2,2-dimethyl-1,3-dioxolan-4-yl)methyl)-8-(2-fluoro-4-iodophenylamino)-2,6-naphthyridin-1(2*H*)-one (68 mg, 0.137 mmol) in dioxane (3.5 mL) was treated with 4M HCl in dioxane (0.086 mL) and the reaction mixture was stirred at room temperature for 18 h. The reaction mixture was concentrated *in vacuo* to give the product (62 mg, 100%) as an orange solid. UPLC-MS (Acidic Method, 2 min): rt 0.87 min, *m*/*z* 456.0 [M+H]⁺. ¹H NMR (400 MHz, CD₃CN): δ 11.13 (s, 1H), 8.30 (s, 1H), 7.94 (s, 1H), 7.69 (dd, *J*=10.1, 1.9 Hz, 1H), 7.61 (dd, *J*=16.5, 7.9 Hz, 2H), 7.36 (t, *J*=8.4 Hz, 1H), 6.75 (d, *J*=7.3 Hz, 1H), 4.31 (dd, *J*=13.5, 3.2 Hz, 1H), 4.03 - 3.91 (m, 1H), 3.87 (dd, *J*=13.4, 8.2 Hz, 1H), 3.58 - 3.44 (m, 2H).

### Example 102: (S)-2-(2,3-Dihydroxypropyl)-8-(2-fluoro-4-iodophenylamino)-2,6-naphthyridin-1(2H)-one

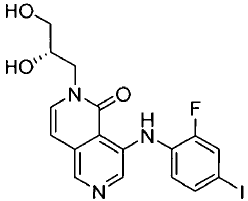

### Step 1: (S)-2-((2,2-Dimethyl-1,3-dioxolan-4-yl)methyl)-8-fluoro-2,6-naphthyridin-1(2H)-one

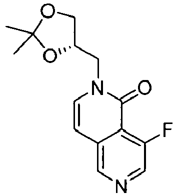

To a solution of 8-fluoro-1*H*-pyrano[4,3-*c*]pyridin-1-one hydrochloride (500 mg, 3.03 mmol) in MeOH (22 mL) was added (*S*)-(2,2-dimethyl-1,3-dioxolan-4-yl)methanamine (556 mg, 4.24 mmol) and the reaction mixture was stirred at 80°C for 72 h. The reaction mixture was concentrated *in vacuo* and the residue was dissolved in EtOAc, washed with H₂O (50 mL), brine (50 mL), dried over Na₂SO₄, and concentrated *in vacuo.* The crude material was purified by flash column chromatography (Silica, 0-100% EtOAc in hexanes) to give the product (165 mg, 20%) as a yellow solid. UPLC-MS (Acidic Method, 2 min): rt 0.76 min, *m*/*z* 279.1 [M+H]⁺. ¹H NMR (400 MHz, CDCl₃): δ 8.74 (s, 1H), 8.46 (d, *J*=3.1 Hz, 1H), 7.37 (d, *J*=7.4 Hz, 1H), 6.55 (dd, *J*=7.4, 2.3 Hz, 1H), 4.52 (qd, *J*=6.5, 3.0 Hz, 1H), 4.39 (dd, *J*=13.8, 3.0 Hz, 1H), 4.16 (dd, *J*=8.8, 6.5 Hz, 1H), 3.90 (dd, *J*=13.8, 7.0 Hz, 1H), 3.74 (dd, *J*=8.8, 6.3 Hz, 1H), 1.43 (s, 3H), 1.34 (s, 3H).

### Step-2: (S)-2-((2,2-Dimethyl-1,3-dioxolan-4-yl)methyl)-8-(2-fluoro-4-iodophenylamino)-2,6-naphthyridin-1(2H)-one

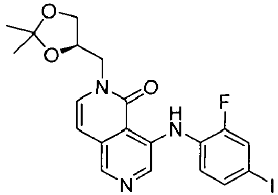

A solution of 2-fluoro-4-iodoaniline (53 mg, 0.222 mmol) in dry THF (1 mL) stirred at -78°C was treated with LiHMDS (1M in THF, 0.33 mL, 0.33 mmol) and the reaction mixture was stirred for 10 minutes. Then, a solution of (*S*)-2-((2,2-dimethyl-1,3-dioxolan-4-yl)methyl)-8-fluoro-2,6-naphthyridin-1(2*H*)-one (65 mg, 0.234 mmol) in dry THF (1 mL) was added and the reaction mixture was stirred at -78°C for 15 minutes, then let to warm up to room temperature. The reaction mixture was quenched with a saturated NH₄Cl aqueous solution (1 mL) at 0°C and extracted with EtOAc (3 × 7 mL). The combined organic phase was washed with H₂O (7 mL), brine (7 mL), dried over Na₂SO₄, and concentrated *in vacuo.* The crude material was purified by flash column chromatography (Silica, 0-50% EtOAc in hexanes) to give the product (77 mg, 66%) as a yellow solid. UPLC-MS (Acidic Method, 2 min): rt 1.14 min, *m*/*z* 496.0 [M+H]⁺. ¹H NMR (400 MHz, CDCl₃): δ 10.55 (s, 1H), 8.40 (s, 1H), 8.27 (s, 1H), 7.55 - 7.42 (m, 2H), 7.32 (t, *J*=8.4 Hz, 1H), 7.24 (d, 1H), 6.49 (d, *J*=7.3 Hz, 1H), 4.50 (qd, *J*=6.6, 3.2 Hz, 1H), 4.35 (dd, *J*=13.8, 3.1 Hz, 1H), 4.15 (dd, *J*=8.8, 6.5 Hz, 1H), 3.87 (dd, *J*=13.8, 7.0 Hz, 1H), 3.74 (dd, *J*=8.7, 6.2 Hz, 1H), 1.43 (s, 3H), 1.34 (s, 3H).

### Step 3: (S)-2-(2,3-Dihydroxypropyl)-8-(2-fluoro-4-iodophenylamino)-2,6-naphthyridin-1(2H)-one

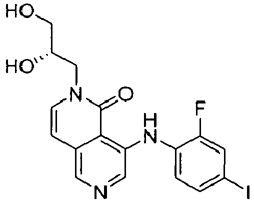

A solution of (*S*)-2-((2,2-dimethyl-1,3-dioxolan-4-yl)methyl)-8-(2-fluoro-4-(trimethylsilyl)phenyl-amino)-2,6-naphthyridin-1(2*H*)-one (67 mg, 0.136 mmol) in dioxane (3.5 mL) was treated with 4M HCl in 1,4-dioxane (85 µL) and the reaction mixture was stirred at ambient temperature for 18 h. The reaction mixture was concentrated *in vacuo* and the crude material was purified by preparatory HPLC to give the product (16 mg, 26%) as a yellow solid. UPLC-MS (Acidic Method, 2 min): rt 1.02 min, *m*/*z* 456.1 [M+H]⁺. ¹H NMR (400 MHz, CD₃CN): δ 10.73 (s, 1H), 8.35 (d, *J*=1.3 Hz, 1H), 8.32 (s, 1H), 7.62 (dd, *J*=10.4, 2.0 Hz, 1H), 7.55 (ddd, *J*=8.5, 2.1, 1.0 Hz, 1H), 7.44 (t, *J*=8.5 Hz, 1H), 7.37 (d, *J*=7.4 Hz, 1H), 6.60 (d, J--7.4 Hz, 1H), 4.23 (dd, *J*=13.4, 3.6 Hz, 1H), 3.95 (m, 1H), 3.85 (dd, *J*=13.4, 7.8 Hz, 1H), 3.54 - 3.46 (m, 2H).

### Example 103: 2-(3-Aminopropyl)-8-(2-fluoro-4-iodophenylamino)-2,6-naphthyridin-1(2H)-one hydrochloride

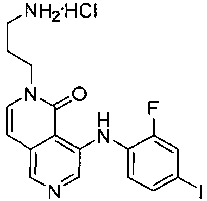

### Step 1: tert-Butyl 3-(8-fluoro-1-oxo-2,6-naphthyridin-2(1H)-yl)propylcarbamate

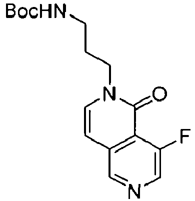

A solution of 8-fluoro-1*H*-pyrano[4,3-*c*]pyridin-1-one hydrochloride (0.50 g, 2.48 mmol) and *tert*-butyl 3-aminopropylcarbamate (0.74 g, 4.24 mmol) in MeOH (22 mL) was heated at 80°C for 18 h and then concentrated *in vacuo.* The crude residue was treated with EtOAc and the collected organic phases were washed with brine, dried over Na₂SO₄, filtered and concentrated *in vacuo* to give the crude product (1.02 g, 35% pure) that was taken to the next step without further purification. UPLC-MS (Acidic Method, 2 min): rt 0.97 min, *m*/*z* 322.1 [M+H]⁺.

### Step 2: tert-Butyl 3-(8-(2-fluoro-4-iodophenylamino)-1-oxo-2,6-naphthyridin-2(1H)-yl)propylcarbamate

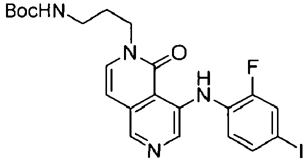

A solution of 2-fluoro-4-iodoaniline (680 mg, 2.87 mmol) in dry THF (15 mL) stirred at -78°C under N₂ was treated with LiHMDS (1M in THF, 4.23 mL, 4.23 mmol) added dropwise. The reaction mixture was stirred for 15 min at -78°C and then a suspension of *tert-*butyl 3-(8-fluoro-1-oxo-2,6-naphthyridin-2(1*H*)-yl)propylcarbamate (970 mg, 35% pure, 1.06 mmol) in dry THF (15 mL) was added. The reaction mixture was further stirred at -7 °C and then let to warm up to room temperature. After 1 h the reaction mixture was re-cooled down to -78°C and treated with additional portions of LiHMDS (1M in THF, 4.23 mL, 4.23 mmol) added dropwise; this addition was repeated once more. The reaction mixture was further stirred for 1 h and let to warm up to room temperature. Then the reaction mixture was quenched with a saturated NH₄Cl aqueous solution at 0°C and extracted with EtOAc. The combined organic phase was washed with brine, dried over Na₂SO₄, filtered and concentrated *in vacuo* to give the crude material which was purified by flash column chromatography (Silica, 0-90% EtOAc in hexane) to give the product (73 mg, 13%). UPLC-MS (Acidic Method, 2 min): rt 1.28 min, *m*/*z* 539.0 [M+H]⁺. ¹H NMR (400 MHz, CDCl₃): δ ppm 10.57 (s, 1H), 8.38 (d, *J*=1.3 Hz, 1H), 8.26 (s, 1H), 7.52 (dd, *J*=9.8, 2.0 Hz, 1H), 7.46 (dt, *J*=8.5, 1.5 Hz, 1H), 7.31 (t, *J*=8.3 Hz, 1H), 7.16 (d, *J*=7.3 Hz, 1H), 6.52 (d, *J*=7.3 Hz, 1H), 5.01 (br s, 1H), 4.03 (t, *J*=6.7 Hz, 2H), 3.18 (q, *J*=6.3 Hz, 2H), 1.96 (p, *J*=6.6 Hz, 2H), 1.44 (s, 9H).

### Step 3: 2-(3-Aminopropyl)-8-(2-fluoro-4-iodophenylamino)-2,6-naphthyridin-1(2H)-one hydrochloride

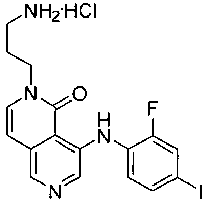

A solution of *tert*-butyl 3-(8-(2-fluoro-4-iodophenylamino)-1-oxo-2,6-naphthyridin-2(1*H*)-yl)propylcarbamate (73 mg, 0.136 mmol) in dry dioxane (1 mL) stirred at room temperature was treated with HCl solution (4 N in dioxane, 50 µl, 0.195 mmol). After 1.5 h an additional portion of HCl solution (4 N in dioxane, 2 × 50 µl, 0.390 mmol) was added and the reaction mixture was further stirred for 18 h. The reaction mixture was then concentrated *in vacuo* to give the product (62 mg, 97%) as a yellow solid. UPLC-MS (Acidic Method, 2 min): rt 0.85 min, *m*/*z* 439.0 [M+H]⁺. ¹H NMR (400 MHz, DMSO-*d*₆): δ ppm 10.83 (s, 1H), 8.45 (d, *J*=1.6 Hz, 1H), 8.31 (s, 1H), 7.89 (br s, 3H), 7.77 (dd, *J*=10.4, 1.9 Hz, 1H), 7.72 (d, *J*=7.7 Hz, 1H), 7.58 (dd, *J*=8.3, 2.0 Hz, 1H), 7.50 (t, *J*=8.5 Hz, 1H), 6.81 (dd, *J*=7.2, 1.3 Hz, 1H), 4.07 (t, *J*=6.9 Hz, 2H), 2.84 (q, *J*=7.2, 6.8 Hz, 2H), 2.01 (p, *J*=7.0 Hz, 2H).

### Example 104: 2-(8-(2-Fluoro-4-iodophenylamino)-1-oxo-2,6-naphthyridin-2(1H)-yl)acetic acid

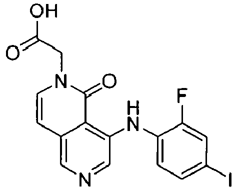

### Step 1: Methyl 2-(8-fluoro-1-oxo-2,6-naphthyridin-2(1H)-yl)acetate

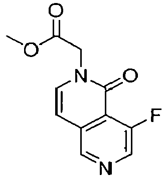

A suspension of glycine methyl ester hydrochloride (0.53 g, 4.24 mmol) in MeOH (5 mL) was treated with Et₃N (0.59 mL, 4.24 mmol) and the resultant solution was added to a solution of 8-fluoro-1*H*-pyrano[4,3-*c*]pyridin-1-one hydrochloride (0.50 g, 2.48 mmol) in MeOH (17 mL). The reaction mixture was heated at 80°C for 72 h and then concentrated *in vacuo.* The crude residue was treated with EtOAc multiple times and the collected organic phases were washed with water, brine, dried over Na₂SO₄, filtered and concentrated *in vacuo* to give the product (465 mg, 80%) that was taken to the next step without further purification. UPLC-MS (Acidic Method, 2 min): rt 0.71 min, *m*/*z* 237.1 [M+H]⁺. ¹H NMR (400 MHz, CDCl₃): δ ppm 8.75 (s, 1H), 8.48 (d, *J*=3.0 Hz, 1H), 7.16 (d, *J*=7.4 Hz, 1H), 6.59 (dd, *J*=7.3, 2.3 Hz, 1H), 4.69 (s, 2H), 3.80 (s, 3H).

### Step 2: 2-(8-Fluoro-1-oxo-2,6-naphthyridin-2(1H)-yl)acetic acid

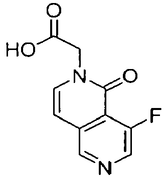

A solution of methyl 2-(8-fluoro-1-oxo-2,6-naphthyridin-2(1*H*)-yl)acetate (465 mg, 1.97 mmol) in MeOH (12 mL) and H₂O (12 mL) stirred at 0°C was treated with 1M LiOH aqueous solution (3.9 mL, 3.94 mmol). The reaction mixture was stirred for 30 min and let to warm up to room temperature. Then the reaction mixture was concentrated *in vacuo* and the residue was extracted with EtOAc. The aqueous phase was acidified with citric acid aqueous solution (pH 2) to reach pH 3 and then was extracted with EtOAc. The combined organic phase was washed with brine, dried over Na₂SO₄, filtered and concentrated *in vacuo* to give the product (153 mg, 50%) as an off-white solid. UPLC-MS (Acidic Method, 2 min): rt 0.17 min, *m*/*z* 223.0 [M+H]⁺. ¹H NMR (400 MHz, DMSO-*d*₆): δ ppm 13.09 (s, 1H), 8.91 (s, 1H), 8.55 (d, *J*=3.3 Hz, 1H), 7.67 (d, *J*=7.3 Hz, 1H), 6.80 (dd, *J*=7.4, 2.4 Hz, 1H), 4.69 (s, 2H).

### Step 3: 2-(8-(2-Fluoro-4-iodophenylamino)-1-oxo-2,6-naphthyridin-2(1H)-yl)acetic acid

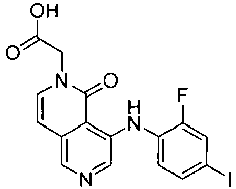

A solution of 2-fluoro-4-iodoaniline (155 mg, 0.655 mmol) in dry THF (2 mL) stirred at -78°C under N₂ was treated with LiHMDS (1M in THF, 1.65 mL, 1.65 mmol) added dropwise. The reaction mixture was stirred for 10 min at -78°C and then a suspension of 2-(8-fluoro-1-oxo-2,6-naphthyridin-2(1*H*)-yl)acetic acid (153 mg, 0.689 mmol) in dry THF (3 mL) was added. The reaction mixture was further stirred and let to warm up to room temperature. After 2.5 h the reaction mixture was re-cooled down to -78°C and treated with additional portions of LiHMDS (1M in THF, 2 × 0.8 mL, 1.60 mmol) added dropwise. The reaction mixture was further stirred for 18 h and let to warm up to room temperature. Then the reaction mixture was quenched with a saturated NH₄Cl aqueous solution at 0°C and extracted with EtOAc. The aqueous phase was acidified with citric acid aqueous solution (pH 2) to reach pH 3 and then was extracted with EtOAc. The combined organic phase was washed with brine, dried over Na₂SO₄, filtered and concentrated *in vacuo* to give the crude material which was purified by preparative HPLC purification to give the product (32 mg, 11%) as a yellow solid. UPLC-MS (Acidic Method, 2 min): rt 1.01 min, *m*/*z* 440.1 [M+H]⁺. ¹H NMR (400 MHz, DMSO-*d*₆): δ ppm 13.16 (s, 1H), 10.56 (s, 1H), 8.39 (s, 1H), 8.30 (d, *J*=1.2 Hz, 1H), 7.75 (dd, *J*=10.4, 1.9 Hz, 1H), 7.60 (d, *J*=7.3 Hz, 1H), 7.58 - 7.53 (m, 1H), 7.49 (t, *J*=8.5 Hz, 1H), 6.73 (d, J=7.3 Hz, 1H), 4.70 (s, 2H).

### Example 105: Methyl 2-(8-(2-fluoro-4-iodophenylamino)-1-oxo-2,6-naphthyridin-2(1H)-yl)acetate

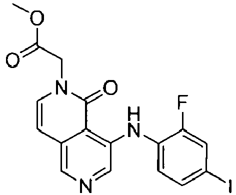

A solution of 2-(8-(2-fluoro-4-iodophenylamino)-1-oxo-2,6-naphthyridin-2(1*H*)-yl)acetic acid (200 mg, 0.46 mmol) in MeOH (1 mL) stirred at 0°C was treated with SOCl₂ (0.12 mL, 1.61 mmol) added dropwise. The reaction mixture was stirred for 18 h and let to warm up to room temperature. The reaction mixture was concentrated *in vacuo* to give the crude material which was purified by preparative HPLC purification to give the product (11 mg, 8%). UPLC-MS (Acidic Method, 2 min): rt 1.14 min, *m*/*z* 454.0 [M+H]⁺. ¹H NMR (400 MHz, DMSO-*d*₆): δ ppm 10.46 (s, 1H), 8.39 (s, 1H), 8.30 (d, *J*=1.3 Hz, 1H), 7.75 (dd, *J*=10.4, 1.9 Hz, 1H), 7.61 (d, *J*=7.3 Hz, 1H), 7.59 - 7.53 (m, 1H), 7.48 (t, *J*=8.5 Hz, 1H), 6.76 (d, *J*=7.3 Hz, 1H), 4.82 (s, 2H), 3.71 (s, 3H).

### Example 106: 8-(2-Fluoro-4-iodophenylamino)-2-hydroxy-2,6-naphthyridin-1(2H)-one

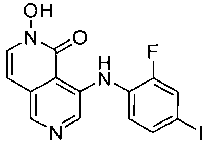

### Step 1: 8-Fluoro-2-hydroxy-2,6-naphthyridin-1(2H)-one

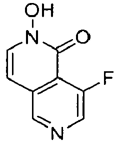

A suspension of hydroxyamine hydrochloride (0.29 g, 4.24 mmol) in MeOH (5 mL) was treated with Et₃N (0.59 mL, 4.24 mmol) and the resultant solution was added to a solution of 8-fluoro-1*H*-pyrano[4,3-*c*]pyridin-1-one hydrochloride (0.50 g, 2.48 mmol) in MeOH (17 mL) with added 4 Å molecular sieves. The reaction mixture was heated at 80°C for 18 h and then at room temperature for 72 h. The reaction mixture was concentrated *in vacuo,* the crude residue was treated with EtOAc and the collected organic phases were concentrated *in vacuo* to give the product (0.26 g, 58%) that was taken to the next step without further purification. UPLC-MS (Acidic Method, 2 min): rt 0.17 min, *m*/*z* 181.0 [M+H]⁺. ¹H NMR (400 MHz, DMSO-*d*₆): δ ppm 10.75 (s, 1H), 8.52 (d, *J*=0.9 Hz, 1H), 8.49 (s, 1H), 7.42 (t, *J*=5.3 Hz, 1H), 6.76 (t, *J*=5.2 Hz, 1H).

### Step 2: 8-(2-Fluoro-4-iodophenylamino)-2-hydroxy-2,6-naphthyridin-1(2H)-one

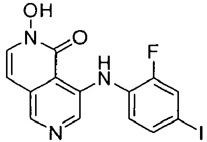

A solution of 2-fluoro-4-iodoaniline (95 mg, 0.40 mmol) in dry THF (2 mL) stirred at -78°C under N₂ was treated with LiHMDS (1M in THF, 1.00 mL, 1.00 mmol) added dropwise. The reaction mixture was stirred for 10 min at -78°C and then a suspension of 8-fluoro-2-hydroxy-2,6-naphthyridin-1(2*H*)-one (76 mg, 0.42 mmol) in dry THF (1 mL) was added. The reaction mixture was further stirred at -78°C for 15 min and then let to warm up to room temperature. After 30 min the reaction mixture was re-cooled down to -78°C and treated with additional portions of LiHMDS (1M in THF, 1.00 mL, 1.00 mmol) added dropwise. The reaction mixture was further stirred and let to warm up to room temperature then it was quenched with a saturated NH₄Cl aqueous solution at 0°C. The aqueous phase was extracted with EtOAc. The combined organic phase was washed with brine, dried over Na₂SO₄, filtered and concentrated *in vacuo* at room temperature to give the crude material which was purified by preparative HPLC purification to give the product (9.5 mg, 6%) as a yellow solid. UPLC-MS (Acidic Method, 2 min): rt 1.00 min, m/z 398.0 [M+H]⁺. ¹H NMR (400 MHz, DMSO-*d*₆): δ ppm 11.98 (br s, 1H), 10.70 (br s, 1H), 8.43 (s, 1H), 8.30 (s, 1H), 7.84 (d, *J*=7.5 Hz, 1H), 7.75 (dd, *J*=10.4, 1.9 Hz, 1H), 7.59 - 7.46 (m, 2H), 6.72 (d, *J*=7.5 Hz, 1H).

### Example 107: 8-(2-Fluoro-4-iodophenylamino)-2-(2-hydroxyethoxy)-2,6-naphthyridin-1(2H)-one

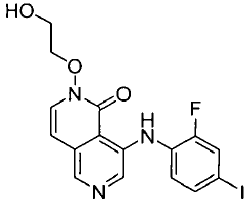

### Step 1: 2-(2-(tert-Butyldiphenylsilyloxy)ethoxy)-8-(2-fluoro-4-iodophenylamino)-2,6-naphthyridin-1(2H)-one

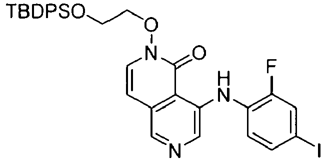

To a solution of *O*-(2-(*tert*-butyl-diphenylsilyloxy)ethyl)hydroxylamine (0.70 g, 2.22 mmol), Et₃N (0.31 mL, 2.22 mmol) and HCl (4N in dioxane, 1.1 mL, 4.44 mmol) in dioxane (5 mL) stirred at room temperature for 15 min was added methyl 3-(2-ethoxyvinyl)-5-fluoroisonicotinate (0.50 g, 2.22 mmol). The reaction mixture was stirred at 50°C for 18 h. The reaction mixture was cooled down to room temperature, was treated with LiHMDS (1M in THF, 7.1 mL, 7.10 mmol) added dropwise and stirred for 30 min. Then 2-fluoro-4-iodoaniline (0.53 g, 2.22 mmol) was added to the reaction mixture followed by LiHMDS (1M in THF, 2.7 mL, 2.66 mmol) added dropwise and it was further stirred at room temperature. After 45 min an additional portion of LiHMDS (1M in THF, 1.3 mL, 1.33 mmol) was added and the reaction mixture was stirred for 30 min. Then the reaction mixture was quenched with a saturated NH₄Cl aqueous solution and extracted with EtOAc. The combined organic phase was washed with brine, dried over Na₂SO₄, filtered and concentrated *in vacuo* to give the crude material which was purified by flash column chromatography (Silica, 0-30% EtOAc in hexane + 1% Et₃N) to give the product (250 mg, 16%) as a yellow glass. UPLC-MS (Acidic Method, 2 min): rt 1.61 min, *m*/*z* 680.2 [M+H]⁺. ¹H NMR (400 MHz, DMSO-*d*₆): δ ppm 10.45 (s, 1H), 8.42 (s, 1H), 8.30 (d, *J*=1.3 Hz, 1H), 7.82 (d, *J*=7.6 Hz, 1H), 7.77 (dd, *J*=10.4, 1.9 Hz, 1H), 7.65 - 7.50 (m, 5H), 7.50 - 7.33 (m, 7H), 6.74 (d, *J*=7.7 Hz, 1H), 4.49 - 4.34 (m, 2H), 3.97 (t, *J*=4.3 Hz, 2H), 0.93 (s, 9H).

### Step 2: 8-(2-Fluoro-4-iodophenylamino)-2-(2-hydroxyethoxy)-2,6-naphthyridin-1(2H)-one

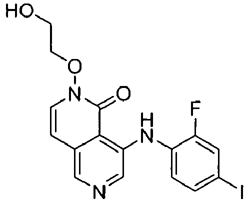

To a solution of 2-(2-(*tert*-butyldiphenylsilyloxy)ethoxy)-8-(2-fluoro-4-iodophenylamino)-2,6-naphthyridin-1(2*H*)-one (250 mg, 0.368 mmol) in THF (5 mL) stirred at room temperature TBAF (1M in THF, 0.37 mL, 0.368 mmol) was added. After 30 min reaction was complete and a saturated NaHCO₃ aqueous solution was added. The mixture was extracted twice with EtOAc, the organic phase was washed with H₂O and brine, dried over Na₂SO₄, filtered and concentrated *in vacuo.* A half of the crude material was purified by preparative HPLC purification followed by SFC purification to give the product (30 mg, 64%). UPLC-MS (Acidic Method, 2 min): rt 1.03 min, *m*/*z* 442.0 [M+H]⁺. ¹H NMR (400 MHz, DMSO-*d*₆): δ ppm 10.42 (s, 1H), 8.42 (s, 1H), 8.29 (d, *J*=1.3 Hz, 1H), 7.86 (d, *J*=7.6 Hz, 1H), 7.76 (dd, *J*=10.4, 1.9 Hz, 1H), 7.60 - 7.53 (m, 1H), 7.49 (t, *J*=8.5 Hz, 1H), 6.74 (d, *J*=7.7 Hz, 1H), 4.97 (t, *J*=5.5 Hz, 1H), 4.28 (dd, *J*=5.2, 4.1 Hz, 2H), 3.68 (q, *J*=5.1 Hz, 2H).

### Example 108: 8-(2-Fluoro-4-iodophenylamino)-2-isopropoxy-3,4-dihydro-2,6-naphthyridin-1(2H)-one

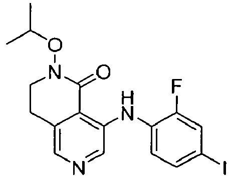

### Step 1: Methyl 3-fluoro-5-(2-(isopropoxyimino)ethyl)isonicotinate

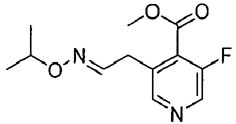

To a solution of *O*-isopropylhydroxylamine hydrochloride (495 mg, 4.44 mmol), Et₃N (0.62 mL, 4.44 mmol) and HCl (4N in dioxane, 1.1 mL, 4.44 mmol) in dioxane (8 mL) placed in a pressure tube was added a solution of methyl 3-(2-ethoxyvinyl)-5-fluoroisonicotinate (1.0 g, 4.44 mmol) in dioxane (2 mL). The reaction mixture was sealed and stirred at 50°C for 18 h. *Note:* the reaction mixture is in a form of a dense suspension of formed NH₄Cl salt during the process and should be efficiently stirred for best results. Then the reaction mixture was concentrated *in vacuo.* The crude material was dry loaded on Celite and was purified by flash column chromatography (40 g silica, 0-15% EtOAc in hexanes modified with 1% Et₃N) to give the product (795 mg, 70%, mixture of two isomers) as a pale oil. UPLC-MS (Acidic Method, 2 min): rt 1.07 min, *m*/*z* 255.1 [M+H]⁺ . ¹H NMR (400 MHz, DMSO-*d*₆): δ ppm 8.66 (dd, *J*=4.0, 1.1 Hz, 2H), 8.53 (s, 1H), 8.49 (s, 1H), 7.47 (t, *J*=5.4 Hz, 1H), 6.84 (t, *J*=5.1 Hz, 1H), 4.27 (p, *J*=6.2 Hz, 1H), 4.15 (p, *J*=6.2 Hz, 1H), 3.91 (s, 3H), 3.90 (s, 3H), 3.72 (d, *J*=5.1 Hz, 2H), 3.68 (d, *J*=5.4 Hz, 2H), 1.17 (d, *J*=6.2 Hz, 6H), 1.11 (d, *J*=6.2 Hz, 6H).

### Step 2: 8-Fluoro-2-isopropoxy-3,4-dihydro-2,6-naphthvridin-1(2H)-one

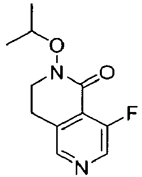

To a solution of methyl 3-fluoro-5-(2-(isopropoxyimino)ethyl)isonicotinate (400 mg, 1.575 mmol) in MeOH (4 mL) stirred at room temperature under N₂ flow, with an output to a Drechsel bottle with a solution of bleach and 1M NaOH, NaCNBH₃ (297 mg, 4.724 mmol) was added at once followed by 1M HCl aqueous solution (1.57 mL, 1.575 mmol) added dropwise. The reaction mixture was stirred for 5 days at room temperature. Reaction was quenched with H₂O (50 mL) and extracted with EtOAc (6 × 25 mL). The organic phase was washed with brine, dried over Na₂SO₄, filtered and concentrated *in vacuo* to give the product (275 mg, 78%) as a white soft solid used in the next step without further purification. UPLC-MS (Acidic Method, 2 min): rt 0.78 min, *m*/*z* 225.1 [M+H]⁺. ¹H NMR (400 MHz, DMSO-*d*₆): δ ppm 8.56 (d, *J*=3.0 Hz, 1H), 8.48 (s, 1H), 4.33 (p, *J*=6.2 Hz, 1H), 3.82 (t, *J*=6.6 Hz, 2H), 3.19 (t, *J*=6.6 Hz, 2H), 1.22 (d, *J*=6.2 Hz, 6H).

### Step 3: 8-(2-Fluoro-4-iodophenylamino)-2-isopropoxy-3,4-dihydro-2,6-naphthyridin-1(2H)-one

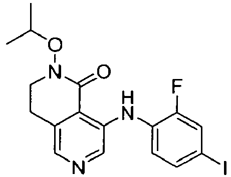

A solution of 2-fluoro-4-iodoaniline (106 mg, 0.45 mmol) in dry THF (1 mL) stirred at -78°C under N₂ was treated with LiHMDS (1M in THF, 0.45 mL, 0.45 mmol) added dropwise and the reaction mixture was stirred for 15 min. Then a solution of 2-(cyclopropylmethoxy)-8-fluoro-3,4-dihydro-2,6-naphthyridin-1(2*H*)-one (100 mg, 0.45 mmol) in dry THF (1 mL) was added and the reaction mixture was further stirred at and let to warm up to room temperature. After 18 h the reaction mixture was re-cooled to -78°C and LiHMDS (1M in THF, 0.45 mL, 0.45 mmol) was added dropwise and the reaction mixture was further stirred at and let to warm up to room temperature. After 18 h the reaction mixture was quenched with a saturated NH₄Cl aqueous solution (15 mL) and extracted with EtOAc (3 × 10 mL). The combined organic phase was washed with brine (10 mL), dried over Na₂SO₄, and concentrated *in vacuo.* The crude material was purified by flash column chromatography (Silica, 0-60% EtOAc in hexanes) to give the product (61 mg, 31%) as an orange gum. UPLC-MS (Acidic Method, 4 min): rt 1.21 min, *m*/*z* 442.1 [M+H]⁺. ¹H NMR (400 MHz, DMSO-*d*₆): δ ppm 10.13 (s, 1H), 8.34 (d, *J*=1.4 Hz, 1H), 7.98 (s, 1H), 7.73 (dd, *J*=10.4, 1.9 Hz, 1H), 7.53 (dt, *J*=8.5, 1.4 Hz, 1H), 7.36 (t, *J*=8.6 Hz, 1H), 4.36 (p, *J*=6.2 Hz, 1H), 3.82 (t, *J*=6.7 Hz, 2H), 3.12 (t, J=6.6 Hz, 2H), 1.24 (d, *J*=6.2 Hz, 6H).

### Example 109: 2-(Cyclopropylmethoxy)-8-(2-fluoro-4-iodophenylamino)-3,4-dihydro-2,6-naphthyridin-1(2H)-one

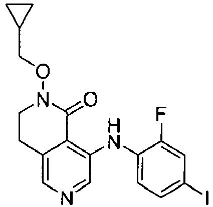

### Step 1: Methyl 3-(2-(cyclopropylmethoxyimino)ethyl)-5-fluoroisonicotinate

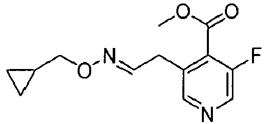

To a solution of *O*-(cyclopropylmethyl)hydroxylamine hydrochloride (546 mg, 4.44 mmol), Et₃N (0.62 mL, 4.44 mmol) and HCl (4N in dioxane, 1.1 mL, 4.44 mmol) in dioxane (8 mL) placed in a pressure tube was added a solution of methyl 3-(2-ethoxyvinyl)-5-fluoroisonicotinate (1.0 g, 4.44 mmol) in dioxane (2 mL). The reaction mixture was sealed and stirred at 50 °C for 18 h. *Note:* the reaction mixture is in a form of a dense suspension of formed NH₄Cl salt during the process and should be efficiently stirred for best results. An additional portion of Et₃N (0.62 mL, 4.44 mmol), HCl (4N in dioxane, 1.1 mL, 4.44 mmol) and *O-*(cyclopropylmethyl)hydroxylamine hydrochloride (273 mg, 2.22 mmol) were added to the reaction mixture stirred at 50°C in further 30 h. Then the reaction mixture was concentrated *in vacuo.* The crude material was dry loaded on Celite and was purified by flash column chromatography (45 g silica, 0-10% EtOAc in hexanes modified with 1% Et₃N) to give the product (791 mg, 67%, mixture of two isomers) as a pale oil. UPLC-MS (Acidic Method, 2 min): rt 1.07 min, *m*/*z* 267.1 [M+H]⁺. ¹H NMR (400 MHz, DMSO-*d*₆): δ ppm 8.66 (dd, *J*=2.4, 1.0 Hz, 2H), 8.54 (s, 1H), 8.50 (s, 1H), 7.52 (t, *J*=5.4 Hz, 1H), 6.85 (t, *J*=5.2 Hz, 1H), 3.91 (s, 3H), 3.90 (s, 3H), 3.86 (d, *J*=7.1 Hz, 2H), 3.76 (d, *J*=5.2 Hz, 2H), 3.72 (d, *J*=7.1 Hz, 2H), 3.68 (d, *J*=5.4 Hz, 2H), 1.14 - 0.91 (m, 2H), 0.57 - 0.38 (m, 4H), 0.23 (dt, *J*=6.1, 4.3 Hz, 2H), 0.18 (dt, *J*=6.1, 4.3 Hz, 2H).

### Step 2: 2-(Cyclopropylmethoxy)-8-fluoro-3,4-dihydro-2,6-naphthyridin-1(2H)-one

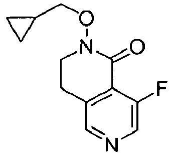

To a solution of methyl 3-(2-(cyclopropylmethoxyimino)ethyl)-5-fluoroisonicotinate (600 mg, 2.253 mmol) in MeOH (6 mL) stirred at room temperature under N₂ flow, with an output to a Drechsel bottle with a solution of bleach and 1M NaOH, NaCNBH₃ (425 mg, 6.760 mmol) was added at once followed by 1M HCl aqueous solution (2.25 mL, 2.253 mmol) added dropwise. The reaction mixture was stirred for 5 days at room temperature. Reaction was quenched with H₂O (50 mL) and extracted with EtOAc (6 × 25 mL). The organic phase was washed with brine, dried over Na₂SO₄, filtered and concentrated *in vacuo* to give the product (582 mg, 96%) as a light-yellow oil used in the next step without further purification. UPLC-MS (Acidic Method, 2 min): rt 0.81 min, *m*/*z* 237.1 [M+H]⁺. ¹H NMR (400 MHz, DMSO-*d*₆): δ ppm 8.56 (d, *J*=2.9 Hz, 1H), 8.47 (s, 1H), 3.90 (t, *J*=6.6 Hz, 2H), 3.82 (d, *J*=7.3 Hz, 2H), 3.19 (t, *J*=6.6 Hz, 2H), 1.19 - 1.03 (m, 1H), 0.55 (dd, *J*=8.1, 1.9 Hz, 2H), 0.30 (dd, *J*=4.7, 1.7 Hz, 2H).

### Step 3: 2-(Cyclopropylmethoxy)-8-(2-fluoro-4-iodophenylamino)-3,4-dihydro-2,6-naphthyridin-1(2H)-one

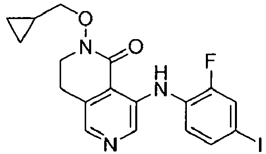

A solution of 2-fluoro-4-iodoaniline (201 mg, 0.85 mmol) in dry THF (2 mL) stirred at -78°C under N₂ was treated with LiHMDS (1M in THF, 0.85 mL, 0.85 mmol) added dropwise and the reaction mixture was stirred for 15 min. Then a solution of 2-(cyclopropylmethoxy)-8-fluoro-3,4-dihydro-2,6-naphthyridin-1(2*H*)-one (200 mg, 0.85 mmol) in dry THF (2 mL) was added and the reaction mixture was further stirred at and let to warm up to room temperature. After 18 h the reaction mixture was re-cooled to -78°C and LiHMDS (1M in THF, 0.21 mL, 0.21 mmol) was added dropwise and the reaction mixture was further stirred at and let to warm up to room temperature. After 18 h the reaction mixture was quenched with a saturated NH₄Cl aqueous solution (20 mL) and extracted with EtOAc (3 × 15 mL). The combined organic phase was washed with brine (20 mL), dried over Na₂SO₄, and concentrated *in vacuo.* The crude material was purified by flash column chromatography (Silica, 0-60% EtOAc in hexanes) to give the product (132 mg, 34%) as an orange gum. UPLC-MS (Acidic Method, 4 min): rt 1.22 min, *m*/*z* 454.1 [M+H]⁺. ¹H NMR (400 MHz, DMSO-*d*₆): δ ppm 10.11 (s, 1H), 8.34 (s, 1H), 7.98 (s, 1H), 7.73 (dd, *J*=10.5, 1.9 Hz, 1H), 7.53 (dt, *J*=8.3, 1.3 Hz, 1H), 7.36 (t, *J*=8.6 Hz, 1H), 3.90 (t, *J*=6.8 Hz, 2H), 3.84 (d, *J*=7.3 Hz, 2H), 3.11 (t, *J*=6.7 Hz, 2H), 1.20 - 1.09 (m, 1H), 0.64 - 0.50 (m, 2H), 0.41 - 0.23 (m, 2H).

### Example 110: 8-(2-Fluoro-4-iodophenylamino)-2-(2-hydroxyethoxy)-3,4-dihydro-2,6-naphthyridin-1(2H)-one

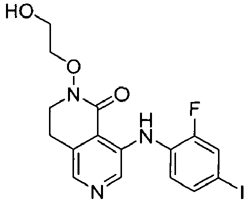

### Step 1: Methyl 3-(9,9-dimethyl-8,8-diphenyl-4,7-dioxa-3-aza-8-siladec-2-enyl)-5-fluoroisonicotinate

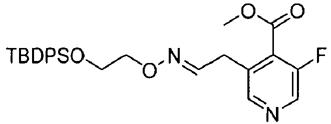

A solution of methyl 3-(2-ethoxyvinyl)-5-fluoroisonicotinate (1.0 g, 4.44 mmol) in dioxane (10 mL) was treated with HCl (4 N in dioxane, 2.2 mL, 8.88 mmol) and stirred at 45°C for 18 h. Then the reaction mixture was cooled down to room temperature and a solution of O-(2-(tert-butyl-diphenylsilyloxy)ethyl)hydroxylamine (1.75 g, 5.55 mmol) and Et₃N (0.62 mL, 4.44 mmol) in dioxane (2 mL) was added. The reaction mixture was stirred for 3 days at room temperature and then concentrated *in vacuo.* The crude material was dry loaded on Celite and was purified by flash column chromatography (80 g silica, 0-10% MeOH in DCM) to give the product (760 mg, 35%, mixture of two isomers) as a light-yellow oil. UPLC-MS (Acidic Method, 4 min): rt 2.65, 2.67 min (two isomers 1:1), *m*/*z* 495.2 [M+H]⁺. ¹H NMR (400 MHz, DMSO-*d*₆): δ ppm 8.66 (dd, *J*=2.4, 1.0 Hz, 2H), 8.50 (d, *J*=0.9 Hz, 1H), 8.46 (d, *J*=0.8 Hz, 1H), 7.68 - 7.57 (m, 8H), 7.54 (t, *J*=5.5 Hz, 1H), 7.49 - 7.33 (m, 12H), 6.90 (t, *J*=5.2 Hz, 1H), 4.21 - 4.14 (m, 2H), 4.06 - 4.00 (m, 2H), 3.85 (s, 3H), 3.88 - 3.84 (m, 2H), 3.83 (s, 3H), 3.81 - 3.77 (m, 2H), 3.76 (d, *J*=5.1 Hz, 2H), 3.66 (d, *J*=5.5 Hz, 2H), 0.99 (s, 9H), 0.96 (s, 9H).

### Step 2: 2-(2-(tert-Butyldiphenylsilyloxy)ethoxy)-8-fluoro-3,4-dihydro-2,6-naphthyridin-1(2H)-one

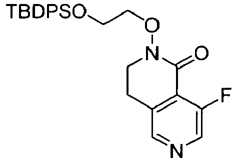

To a solution of methyl 3-(9,9-dimethyl-8,8-diphenyl-4,7-dioxa-3-aza-8-siladec-2-enyl)-5-fluoroisonicotinate (2.82 g, 5.71 mmol) in MeOH (28 mL) stirred at room temperature under N₂ flow, with an output to a Drechsel bottle with a solution of bleach and 1M NaOH, NaCNBH₃ (1.07 g, 17.1 mmol) was added at once followed by 1M HCl aqueous solution (2.86 mL, 2.86 mmol) added dropwise. After 7 h additional portion of 1M HCl aqueous solution (1.43 mL, 1.43 mmol) was added and the reaction mixture was stirred further for 3 days. Reaction was quenched with 1M NaOH aqueous solution and extracted twice with EtOAc. The organic phase was washed with H₂O and brine, dried over Na₂SO₄, filtered and concentrated *in vacuo.* The crude material was purified by flash column chromatography (Silica, 20-50% EtOAc in heptane) to give the product (1.90 g, 72%) as a light-yellow solid. UPLC-MS (Acidic Method, 2 min): rt 1.37 min, *m*/*z* 465.0 [M+H]⁺. ¹H NMR (400 MHz, DMSO-*d*₆): δ ppm 8.56 (d, *J*=2.9 Hz, 1H), 8.46 (s, 1H), 7.71 - 7.60 (m, 4H), 7.51 - 7.33 (m, 6H), 4.15 (dd, *J*=5.5, 4.0 Hz, 2H), 3.93 - 3.83 (m, 4H), 3.16 (t, *J*=6.6 Hz, 2H), 1.00 (s, 9H).

### Step 3: 2-(2-(tert-Butyldiphenylsilyloxy)ethoxy)-8-(2-fluoro-4-iodophenylamino)-3,4-dihydro-2,6-naphthyridin-1(2H)-one

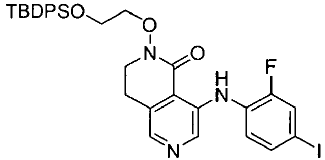

To a solution of 2-fluoro-4-iodoaniline (512 mg, 2.16 mmol) in THF (2.5 mL) stirred at room temperature LiHMDS (1M in THF, 2.6 mL, 2.59 mmol) was added. The mixture was stirred for 15 min and then added dropwise to a solution of 2-(2-(*tert*-butyldiphenylsilyloxy) ethoxy)-8-fluoro-3,4-dihydro-2,6-naphthyridin-1(2*H*)-one (536 mg, 1.08 mmol) in THF (2.5 mL) stirred at room temperature. The reaction mixture was stirred for 18 h at room temperature. The reaction was then quenched with a saturated NH₄Cl aqueous solution and extracted twice with EtOAc. The organic phase was washed with brine, dried over Na₂SO₄, filtered and concentrated *in vacuo.* The crude material was purified by flash column chromatography (Silica, 10-40% EtOAc in heptane) to give the product (316 mg, 43%). UPLC-MS (Acidic Method, 2 min): rt 1.58 min, *m*/*z* 682.0 [M+H]⁺. ¹H NMR (400 MHz, DMSO-*d*₆): δ ppm 10.10 (s, 1H), 8.35 (d, *J*=1.4 Hz, 1H), 7.97 (s, 1H), 7.73 (dd, *J*=10.4, 2.0 Hz, 1H), 7.68 - 7.62 (m, 4H), 7.53 (ddd, *J*=8.4, 2.0, 0.9 Hz, 1H), 7.49 - 7.39 (m, 6H), 7.35 (t, *J*=8.6 Hz, 1H), 4.21 - 4.13 (m, 2H), 3.95 - 3.85 (m, 4H), 3.09 (t, *J*=6.7 Hz, 2H), 1.00 (s, 9H).

### Step 4: 8-(2-Fluoro-4-iodophenylamino)-2-(2-hydroxvethoxv)-3,4-dihydro-2,6-naphthyridin-1(2H)-one

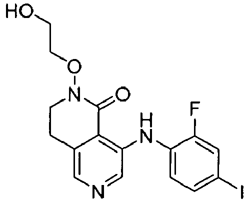

To a solution of 2-(2-(*tert*-butyldiphenylsilyloxy)ethoxy)-8-(2-fluoro-4-iodophenylamino)-3,4-dihydro-2,6-naphthyridin-1(2*H*)-one (360 mg, 0.530 mmol) in THF (4 mL) stirred at room temperature TBAF (1M in THF, 0.53 mL, 0.530 mmol) was added. After 10 min reaction was complete and a saturated NaHCO₃ aqueous solution was added. The mixture was extracted twice with EtOAc, the organic phase was washed with H₂O and brine, dried over Na₂SO₄, filtered and concentrated *in vacuo.* A half of the crude material (200 mg) was purified by preparative HPLC purification to give the product (75.8 mg, 64%) as a yellow solid. UPLC-MS (Acidic Method, 2 min): rt 0.99 min, *m*/*z* 444.0 [M+H]⁺. ¹H NMR (400 MHz, DMSO-*d*₆): δ ppm 10.07 (s, 1H), 8.34 (d, *J*=1.4 Hz, 1H), 7.98 (s, 1H), 7.73 (dd, *J*=10.5, 2.0 Hz, 1H), 7.54 (dt, J=8.4, 1.4 Hz, 1H), 7.36 (t, *J*=8.6 Hz, 1H), 4.80 (t, *J*=5.5 Hz, 1H), 4.05 (dd, *J*=5.8, 3.9 Hz, 2H), 3.90 (t, *J*=6.7 Hz, 2H), 3.63 (q, *J*=5.1 Hz, 2H), 3.12 (t, *J*=6.7 Hz, 2H).

### Example 111: 2-ethoxy-8-((2-fluoro-4-iodophenyl)amino)-3,4-dihydro-2,6-naphthyridin-1(2H)-one

Compound 5.01 1 can be prepared as described in Example 108, replacing the *O-*isopropylhydroxylamine hydrochloride in Step 1 with an appropriate *O*-ethylhydroxylamine which is commercially available or prepared using conditions known to one of ordinary skill in the art.

| **Comp. No.** | **Structure** |
|---|---|
| 5.011 | 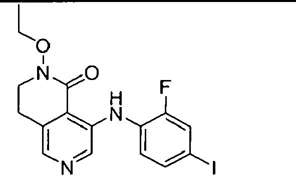 |

### Example 112: 8-((2-Fluoro-4-iodophenyl)amino)-3,4-dihydro-2,6-naphthyridin-1(2H)-one

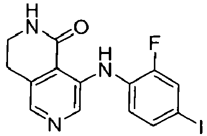

A solution of 2-ethoxy-8-(2-fluoro-4-iodophenylamino)-3,4-dihydro-2,6-naphthyridin-1(2*H*)-one (40 mg, 93.6 µmol) in dry THF (1.1 mL) stirred at room temperature under N₂ was treated with SmI2 (0.1M in THF, 3.74 mL, 0.374 mmol) added dropwise and the reaction mixture was stirred for 5 min. Then the reaction mixture was quenched with a saturated Na₂S₂O₄ aqueous solution (10 mL) and extracted with EtOAc (3 × 10 mL). The combined organic phase was washed with brine, dried over Na₂SO₄, and concentrated *in vacuo.* The crude material (31.4 mg) was purified by preparative HPLC purification to give the product (8.1 mg, 23%) as a yellow solid.

Alternatively, a solution of 2-(cyclopropylmethoxy)-8-(2-fluoro-4-iodophenylamino)-3,4-dihydro-2,6-naphthyridin-1(2*H*)-one (40 mg, 88.2 µmol) in dry THF (1.1 mL) stirred at room temperature under N₂ was treated with SmI₂ (0.1M in THF, 3.74 mL, 0.374 mmol) added dropwise and the reaction mixture was stirred for 5 min. Then the reaction mixture was quenched with a saturated Na₂S₂O₄ aqueous solution (10 mL) and extracted with EtOAc (3 × 10 mL). The combined organic phase was washed with brine, dried over Na₂SO₄, and concentrated *in vacuo.* The crude material (31.6 mg) was purified by preparative HPLC purification to give the product (14.6 mg, 43%) as a yellow solid.

UPLC-MS (Acidic Method, 2 min): rt 1.00 min, *m*/*z* 383.9 [M+H]⁺. ¹H NMR (400 MHz, DMSO-*d*₆) δ ppm 10.30 (s, 1H), 8.50 (br s, 1H), 8.40 (s, 1H), 8.00 (s, 1H), 7.71 (dd, *J*=10.5, 2.0 Hz, 1H), 7.51 (dd, *J*=8.4, 1.7 Hz, 1H), 7.37 (t, *J*=8.6 Hz, 1H), 3.41 (td, *J*=6.6, 2.8 Hz, 2H), 2.87 (t, *J*=6.6 Hz, 2H).

Although the foregoing invention has been described in some detail by way of illustration and example for purposes of clarity of understanding, one of skill in the art will appreciate that certain changes and modifications may be practiced within the scope of the appended claims. In addition, each reference provided herein is incorporated by reference in its entirety to the same extent as if each reference was individually incorporated by reference. Where a conflict exists between the instant application and a reference provided herein, the instant application shall dominate.

The following are numbered aspects of the invetion:
1. A method of treating a birthmark, the method comprising administering to a subject in need thereof a therapeutically effective amount of a compound selected from the group consisting of formula (1), (II), (111), (IV), and (V): 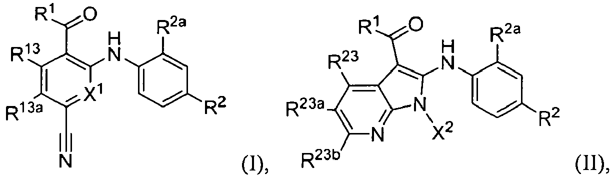 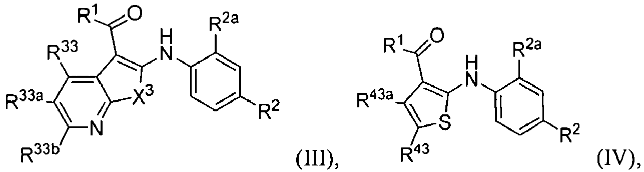 and 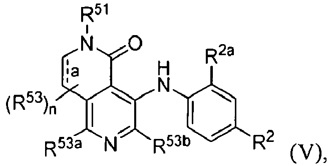 or a N-oxide, stereoisomer, mixture of stereoisomers, and/or a pharmaceutically acceptable salt thereof,
   wherein:
   X¹ is -CR^{13b} or N; X² is C₁-C₆ alkyl; X³ is S or O;
   subscript n is an integer from 0 to 2;
   bond "a" is a single or double bond;
   R¹ is -OR⁴, -NR⁵R^{5a}, -N(OR^{5b})R^{5a}, or a N-linked heterocycloalkyl which is unsubstituted or substituted with one or two R⁶;
   R² is halo, C₁-C₆ alkyl, -S-C₁-C₆ alkyl, C₃-C₈ cycloalkyl, C₂-C₆ alkenyl, or C₂-C₆ alkynyl;
   R^{2a} is halo or C₁-C₆ alkyl;
   R⁴, R⁵, and R^{5b} are each independently hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-Ci-Ce alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶;
   R^{5a} is hydrogen or C₁-C₆ alkyl;
   each R⁶ is independently halo, hydroxy, oxo, C₁-C₆ alkyl, C₃-C₈ cycloalkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl;
   R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino;
   R¹³, R^{13a}, and R^{13b} are each independently hydrogen, halo, C₁-C₆ alkyl, C₂-C₆ alkenyl, or C₂-C₆ alkynyl;
   R²³, R^{23a}, and R^{23b} are each independently hydrogen, halo, C₁-C₆ alkyl, C₂-C₆ alkenyl, C₂-C₆ alkynyl, C₁-C₆ alkoxy, or C₃-C₈ cycloalkoxy;
   R³³, R^{33a}, and R^{33b} are each independently hydrogen, halo, C₁-C₆ alkyl, C₂-C₆ alkenyl, C₂-C₆ alkynyl, C₁-C₆ alkoxy, or C₃-C₈ cycloalkoxy;
   R⁴³ is cyano, -C(O)NR⁴⁸R^{48a}, or -C(O)R⁴⁶;
   R^{43a} is hydrogen, halo, C₁-C₆ alkyl; or
   R⁴³ and R^{43a} together form -CH₂CH₂C(O)- or -CH₂CH₂CH₂C(O)-, each of which is unsubstituted or substituted with one or two R⁴⁹;
   R⁴⁶ is hydrogen, C₁-C₆ alkyl, C₁-C₆ haloalkyl, C₃-C₈ cycloalkyl or heterocycloalkyl;
   R⁴⁸ and R^{48a} are independently hydrogen or C₁-C₆ alkyl; and
   each R⁴⁹ is independently C₁-C₆ alkyl, C₃-C₈ cycloalkyl, or C₃-C₈ cycloalkyl-C₁-C₆ alkyl;
   R⁵¹ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, R⁷-C(O)-C₁-C₆ alkyl, or -OR⁵⁴, wherein each of the C₃-C₈ cycloalkyl and heterocycloalkyl groups is unsubstituted or substituted with one to six R⁶;
   each R⁵³ is independently halo or C₁-C₆ alkyl;
   R^{53a} and R^{53b} are each independently hydrogen, halo, C₁-C₆ alkyl, C₂-C₆ alkenyl, or C₂-C₆ alkynyl; and
   R⁵⁴ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl, wherein each of the C₃-C₈ cycloalkyl and heterocycloalkyl groups is unsubstituted or substituted with one to six R⁶.
**2.** The method of **1,** wherein the compound has formula (Ia) or (Ib): 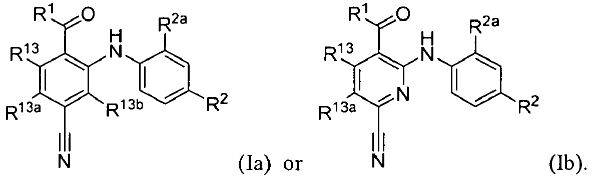
**3.** The method of **1** or **2**, wherein R¹³, R^{13a}, and R^{13b} are each independently hydrogen, halo, or C₁-C₆ alkyl.
**4.** The method of any one of **1-3**, wherein R¹³ is hydrogen.
**5.** The method of any one of **1-4,** wherein R^{13a} is hydrogen.
**6.** The method of any one of **1-5,** wherein R^{13b} is hydrogen.
**7.** The method of any one of **1-5,** wherein R^{13b} is halo.
**8.** The method of any one of **1-5** and **7,** wherein R^{13b} is fluoro.
**9.** The method of **1,** wherein X² of formula (II) is C₁-C₃ alkyl.
**10.** The method of **1** or **9,** wherein X² is methyl.
11. The method of any one of **1, 9-10,** wherein R²³, R^{23a}, and R^{23b} are each independently hydrogen, halo, C₁-C₆ alkyl, or C₁-C₆ alkoxy.
**12.** The method of any one of **1 and 9-11,** wherein R²³ and R^{23b} are each hydrogen and R^{23a} is halo, C₁-C₆ alkyl, or C₁-C₆ alkoxy.
**13.** The method of any one of **1, 9-11,** and **12,** wherein R²³ and R^{23b} are each hydrogen and R^{23a} is fluoro, methyl, or methoxy.
**14.** The method of any one of **1** and **9-11,** wherein R²³, R^{23a}, and R^{23b} are each hydrogen.
**15.** The method of 1, wherein the compound has formula (IIIa) or (IIIb): 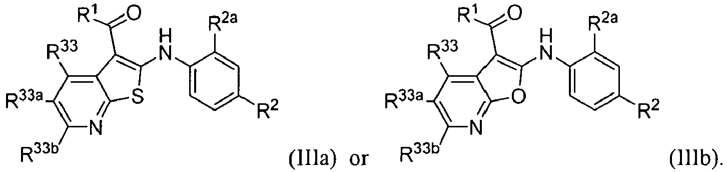
**16.** The method of **1** or **15**, wherein R³³, R^{33a}, and R^{33b} are each independently hydrogen, halo, C₁-C₆ alkyl, or C₁-C₆ alkoxy.
**17.** The method of any one of **1** and **15-16**, wherein R³³, R^{33a}, and R^{33b} are each independently fluoro, methyl, or methoxy.
**18.** The method of any one of **1** and **15-17**, wherein R^{33a} and R^{33b} are each hydrogen and R³³ is fluoro, methyl, or methoxy.
**19.** The method of any one of **1 and 15-17,** wherein R³³ and R^{33b} are each hydrogen and R^{33a} is fluoro, methyl, or methoxy.
**20.** The method of any one of **1** and **15-17,** wherein R³³ and R^{33a} are each hydrogen and R^{33b} is fluoro, methyl, or methoxy.
**21.** The method of any one of **1** and **15-16**, wherein R³³, R^{33a}, and R^{33b} are each hydrogen.
**22.** The method of **1**, wherein the compound has the formula selected from the group consisting of: 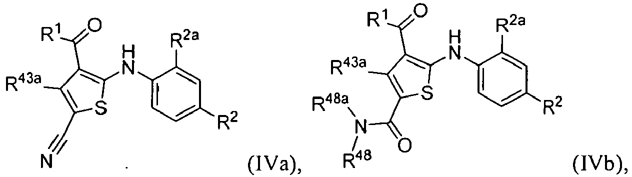 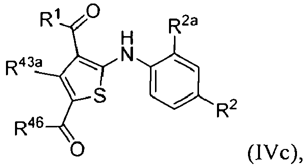 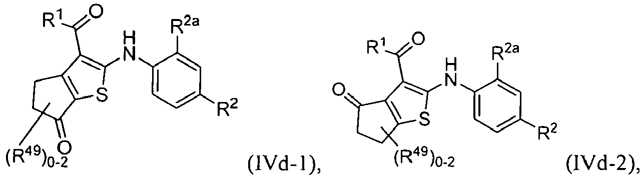 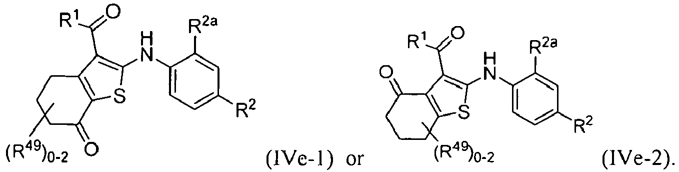
**23.** The method of **1** or **22**, wherein R⁴⁸ and R^{48a} are hydrogen.
**24.** The method of **1** or **22**, wherein R⁴⁶ is hydrogen, C₁-C₆ alkyl, or C₁-C₆ haloalkyl.
**25.** The method of any one of **1, 22,** and **24,** wherein R⁴⁶ is C₁-C₆ alkyl.
**26.** The method of any one of **1, 22,** and **24-25,** wherein R⁴⁶ is methyl, ethyl, propyl, or butyl.
**27.** The method of any one of **1** and **22-26,** wherein R^{43a} is hydrogen.
**28.** The method of any one of **1** and **22-26,** wherein R^{43a} is halo.
**29.** The method of any one of **1** and **22-26,** wherein R^{43a} is C₁-C₆ alkyl.
**30.** The method of any one of **1, 22-26** and **29,** wherein R^{43a} is methyl.
**31.** The method of **1** or **22,** wherein each R⁹ is independently C₁-C₆ alkyl.
**32.** The method of **1** or **22,** wherein R⁹ is absent.
**33.** The method of any one of **1-32,** wherein R¹ is -OR⁴, -NR⁵R^{5a}, or -N(OR^{5b})R^{5a}.
**34.** The method of any one of **1-33,** wherein R¹ is -OR⁴; R⁴ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl.
**35.** The method of any one of **1-34,** wherein R¹ is selected from the group consisting of -OH, 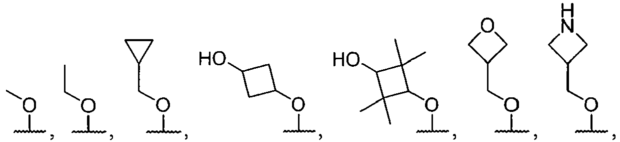 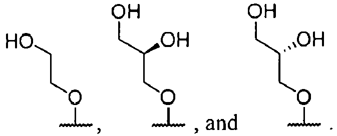
**36.** The method of any one of **1-33,** wherein R¹ is -NR⁵R^{5a}; R⁵ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl.
**37.** The method of any one of **1-33** and **36,** wherein R⁵ is selected from the group consisting of hydrogen, 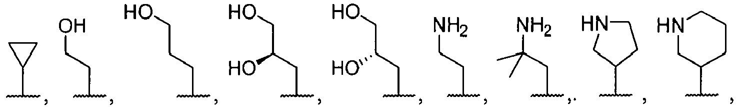 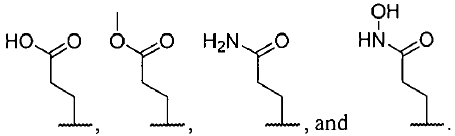
**38.** The method of any one of **1-33,** wherein R¹ is -NR⁵R^{5a} and R⁵ is -OR^{5b}.
**39.** The method of any one of **1-33** and **38,** wherein R¹ is -N(OR^{5b})R^{5a}; R^{5b} is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl.
**40.** The method of any one of **1-33** and **38-39,** wherein -OR^{5b} is selected from the group consisting of: 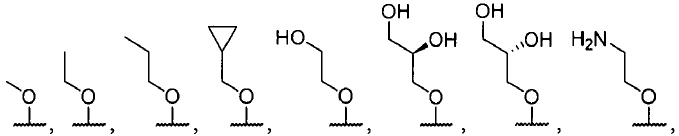 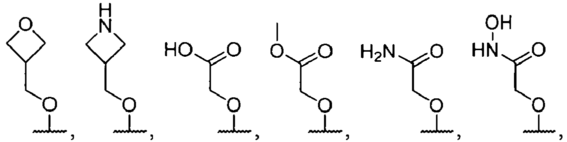 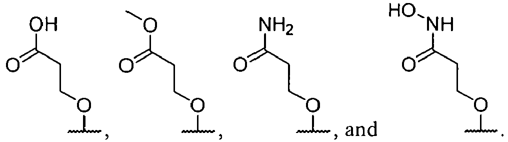
**41.** The method of any one of **1-33** and **36-40,** wherein R¹ is -NR⁵R^{5a} or -N(OR^{5b})R^{5a}, and R^{5a} is hydrogen.
**42.** The method of any one of **1-33** and **36-40,** wherein R¹ is -NR⁵R^{5a} or -N(OR^{5b})R^{5a}, and R^{5a} is C₁-C₆ alkyl.
**43.** The method of any one of **1-32,** wherein R¹ is a N-linked heterocycloalkyl which is unsubstituted or substituted with one or two R⁶.
**44.** The method of any one of **1-32** and **43,** wherein the N-linked heterocycloalkyl is N-linked azetidinyl, N-linked pyrrolidinyl, or N-linked isoxazolidinyl.
**45.** The method of any one of **1-32** and **43-44,** wherein each R⁷ is independently hydroxyl, oxo, or amino.
**46.** The method of **1,** wherein the compound has formula (Va) or (Vb): 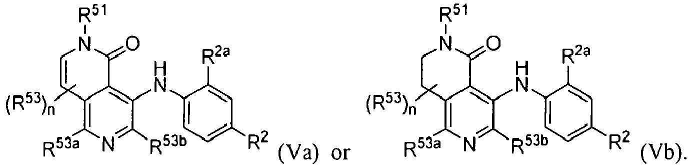
**47.** The method of **1 or 46,** wherein each R⁵³ is independently halo or C₁-C₃ alkyl.
**48.** The method of **1** or **46,** wherein subscript n is 0.
**49.** The method of any one of **1-48,** wherein R^{53a} and R^{53b} are each independently hydrogen, halo, or C₁-C₆ alkyl.
**50.** The method of any one of **1** and **46-49,** wherein R^{53a} is hydrogen.
**51.** The method of any one of **1** and **46-50,** wherein R^{53b} is hydrogen.
**52.** The method of any one of **1** and **46-50,** wherein R^{53b} is halo.
**53.** The method of any one of **1, 46-50, 52,** wherein R^{53b} is fluoro.
**54.** The method of any one of **1** and **46-53,** wherein R⁵¹ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl.
**55.** The method of any one of **1** and **46-53,** wherein R⁵¹ is -OR^{54b}; R^{54b} is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl.
**56.** The method of any one of **1** and **46-53,** wherein R⁵¹ is selected from the group consisting of hydrogen, -OH, 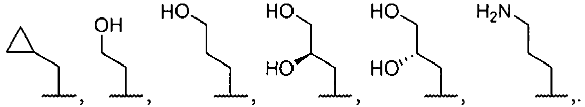 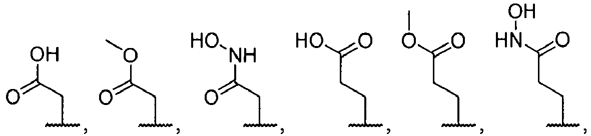 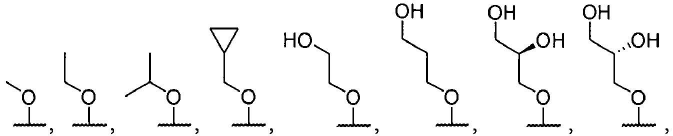 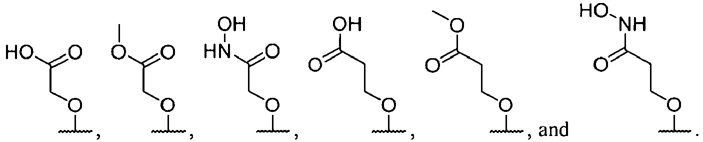
**57.** The method of any one of **1-56,** wherein R² is halo C₁-C₆ alkyl, -S-C₁-C₆ alkyl, or C₂-C₆ alkynyl.
**58.** The method of any one of **1-57,** wherein R² is halo.
**59.** The method of any one of **1-58,** wherein R² is iodo.
**60.** The method of any one of **1-57,** wherein R² is C₁-C₆ alkyl.
**61.** The method of any one of **1-57** and **60,** wherein R² is CH₃.
**62.** The method of any one of **1-57,** wherein R² is -S-C₁-C₆ alkyl.
**63.** The method of any one of **1-57** and **60,** wherein R² is -SCH₃.
**64.** The method of any one of **1-57,** wherein R² is C₂-C₆ alkynyl.
**65.** The method of any one of **1-57** and **60,** wherein R² is acetylenyl.
**66.** The method of any one of **1-61,** wherein R^{2a} is halo.
**67.** The method of any one of **1-66,** wherein R^{2a} is fluoro.
**68.** The method of any one of **1-61,** wherein R^{2a} is C₁-C₆ alkyl.
**69.** The method of any one of **1-61** and **68,** where R^{2a} is methyl.
**70.** The method of any one of **1-69,** wherein the compound is selected from the group consisting of Compounds in Tables 1 to 5.
**71.** The method of any one of **1-70,** wherein the compound is administered with a pharmaceutically acceptable carrier in a pharmaceutical composition.
**72.** The method of any one of **1-71,** wherein the birthmark is selected from the group consisting of port-wine stains/capillary malformations, melanocytic nevus, displastic nevi, epidermal nevi, nevus sebaceous, nevus spilus, arterio-venous malformations, lymphatic malformations, and congenital melanocytic nevus.
**73.** The method of any one of **1-72,** wherein the birthmark is associated with activation of p-ERK.
**74.** The method of **72,** wherein the birthmark associated with activation of p-ERK is selected from the group consisting of epidermal nevi, nevus sebaceous, nevus spilus, arterio-venous malformations, capillary malformations/port-wine stains, congenital melanocytic nevus, and lymphatic malformations.
75. The method of any one of **1-74,** wherein the compound having any one of formulae (I) to (V) or the pharmaceutical composition thereof is administered topically, subcutaneously, intradermally, or intralesionally.
76. The method of any one of **1**-75, wherein the compound having any one of formulae (I) to (V) or the pharmaceutical composition thereof is administered as a lotion, a spray, an ointment, a cream, a gel, a paste, and a patch.

## Claims

1. A compound for use in a method of treating a birthmark, the method comprising administering to a subject in need thereof a therapeutically effective amount of the compound, wherein the compound is represented by formula (IV): 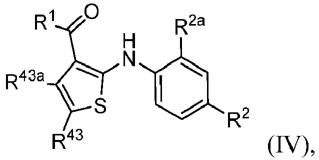 or a N-oxide, stereoisomer, mixture of stereoisomers, and/or a pharmaceutically acceptable salt thereof, wherein:
R¹ is -OR⁴, -NR⁵R^{5a}, -N(OR^{5b})R^{5a}, or a N-linked heterocycloalkyl which is unsubstituted or substituted with one or two R⁶;
R² is halo, C₁-C₆ alkyl, -S-C₁-C₆ alkyl, C₃-C₈ cycloalkyl, C₂-C₆ alkenyl, or C₂-C₆ alkynyl;
R^{2a} is halo or C₁-C₆ alkyl;
R⁴, R⁵, and R^{5b} are each independently hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or k⁷-C(O)-C₁-C₆ alkyl, wherein each of the C₃-C₈ cycloalkyl and heterocycloalkyl groups is unsubstituted or substituted with one to six R⁶;
R^{5a} is hydrogen or C₁-C₆ alkyl;
each R⁶ is independently halo, hydroxy, oxo, C₁-C₆ alkyl, C₃-C₈ cycloalkyl, C₁-C₆ alkoxy, C₁-C₆-hydroxyalkyl, C₁-C₆ haloalkyl, amino, C₁-C₆ alkylamino, di-(C₁-C₆ alkyl)amino, amino-C₁-C₆ alkyl, C₁-C₆ alkylamino-C₁-C₆ alkyl, or di-(C₁-C₆ alkyl)amino-C₁-C₆ alkyl;
R⁷ is hydroxy, C₁-C₆ alkoxy, amino, C₁-C₆ alkylamino, di-(C₁-C₆ alkyl)amino, hydroxyamino, or *N*-C₁-C₆ alkyl hydroxyamino;
R⁴³ and R^{43a} together form -CH₂CH₂C(O)- or -CH₂CH₂CH₂C(O)-, each of which is unsubstituted or substituted with one or two R⁴⁹; or
R⁴³ is cyano, -C(O)NR⁴⁸R^{48a}, or -C(O)R⁴⁶;
R^{43a} is hydrogen, halo, C₁-C₆ alkyl;
R⁴⁶ is hydrogen, C₁-C₆ alkyl, C₁-C₆ haloalkyl, C₃-C₈ cycloalkyl or heterocycloalkyl;
R⁴⁸ and R^{48a} are independently hydrogen or C₁-C₆ alkyl; and
each R⁴⁹ is independently C₁-C₆ alkyl, C₃-C₈ cycloalkyl, or C₃-C₈ cycloalkyl-C₁-C₆ alkyl.

2. The compound for use of claim 1, wherein the compound is represented by formula (IVe-1) or (IVe-2): 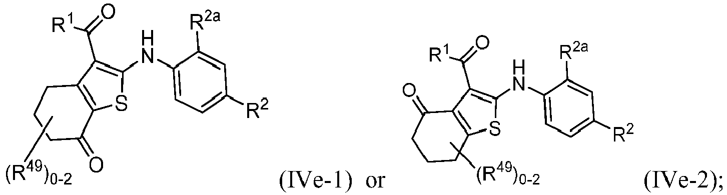 preferably wherein each R⁴⁹ is independently C₁-C₆ alkyl; or R⁴⁹ is absent.

3. The compound for use of claim **1,** wherein the compound is represented by formula (IVd-1) or (IVd-2): 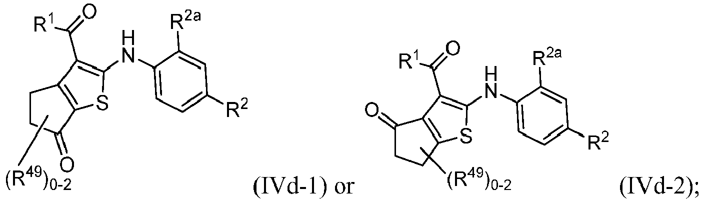 preferably wherein each R⁴⁹ is independently C₁-C₆ alkyl; or R⁴⁹ is absent.

4. The compound for use of claim **1,** wherein the compound is represented by the formula selected from the group consisting of: 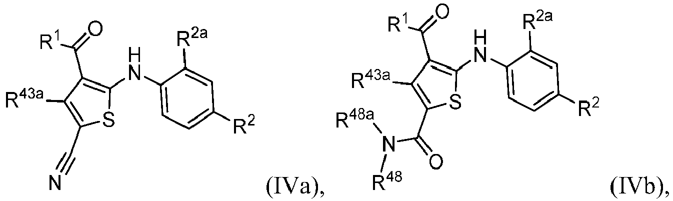 and 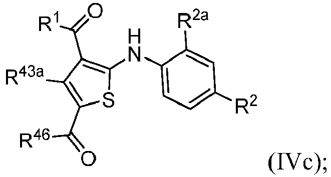 preferably wherein
(a) R⁴⁸ and R^{48a} are hydrogen;
(b) R⁴⁶ is hydrogen, C₁-C₆ alkyl, or C₁-C₆ haloalkyl, preferably wherein R⁴⁶ is C₁-C₆ alkyl, more preferably wherein R⁴⁶ is methyl, ethyl, propyl, or butyl; and/or
(c) R^{43a} is hydrogen, halo, C₁-C₆ alkyl, preferably wherein R^{43a} is hydrogen, halo, or methyl.

5. The compound for use of any one of claims **1-4,** wherein R¹ is -OR⁴, -NR⁵R^{5a}, or -N(OR^{5b})R^{5a}.

6. The compound for use of any one of claims **1-5,** wherein:
(a) R¹ is -OR⁴; R⁴ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl, preferably wherein R¹ is selected from the group consisting of -OH, 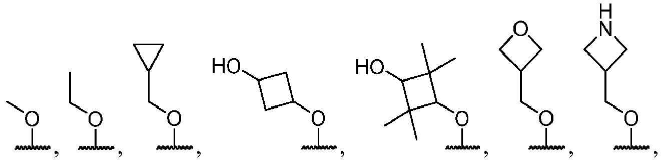 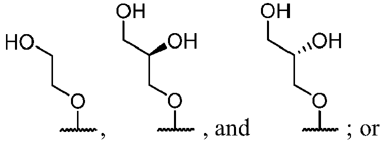
(b) R¹ is -NR⁵R^{5a}; R⁵ is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl, preferably wherein R⁵ is selected from the group consisting of hydrogen, 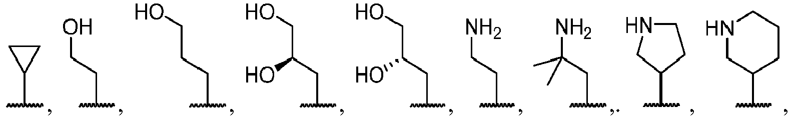 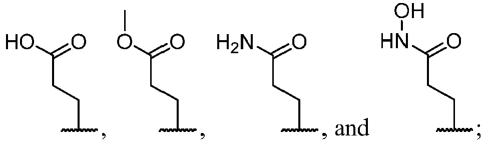 or
(c) R¹ is -NR⁵R^{5a} and R⁵ is -OR^{5b}, preferably wherein R¹ is -N(OR^{5b})R^{5a}; R^{5b} is hydrogen, C₁-C₆ alkyl, C₃-C₈ cycloalkyl, C₃-C₈ cycloalkyl-C₁-C₆ alkyl, C₁-C₆ hydroxyalkyl, C₁-C₆ alkoxy-C₁-C₆ alkyl, amino-C₁-C₆ alkyl, C₁-C₆ alkylamino-C₁-C₆ alkyl, di-(C₁-C₆ alkyl)amino-C₁-C₆ alkyl, heterocycloalkyl, heterocycloalkyl-C₁-C₆ alkyl, or R⁷-C(O)-C₁-C₆ alkyl; R⁷ is hydroxy, C₁-C₆ alkoxy, amino, or hydroxyamino; and each of the C₃-C₈ cycloalkyl and heterocycloalkyl groups is unsubstituted or substituted with one to six R⁶ and each R⁶ is independently hydroxy or C₁-C₆ alkyl, more preferably wherein -OR^{5b} is selected from the group consisting of: 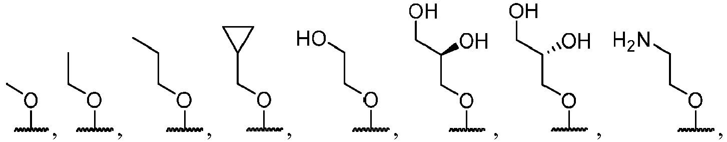 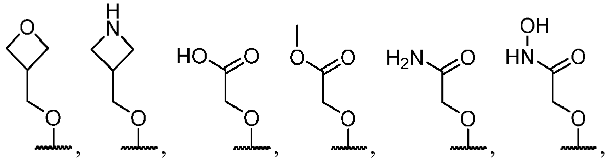 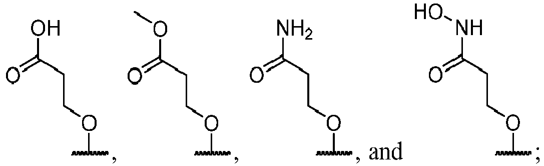 or
(d) R¹ is -NR⁵R^{5a} or -N(OR^{5b})R^{5a}, and R^{5a} is hydrogen or C₁-C₆ alkyl.

7. The compound for use of any one of claims **1-4,** wherein (a) R¹ is a N-linked heterocycloalkyl which is unsubstituted or substituted with one or two R⁶, wherein the N-linked heterocycloalkyl is preferably N-linked azetidinyl, N-linked pyrrolidinyl, or N-linked isoxazolidinyl; and/or (b) each R⁷ is independently hydroxyl, oxo, or amino.

8. The compound for use of any one of claims **1-7,** wherein R² is halo, C₁-C₆ alkyl, -S-C₁-C₆ alkyl, C₃-C₈ cycloalkyl, C₂-C₆ alkenyl, or C₂-C₆ alkynyl.

9. The compound for use of any one of claims **1-8,** wherein:
(a) R² is halo, preferably wherein R² is iodo; or
(b) R² is C₁-C₆ alkyl, preferably wherein R² is CH₃; or
(c) R² is -S-C₁-C₆ alkyl, preferably wherein R² is -SCH₃; or
(d) R² is C₂-C₆ alkynyl, preferably wherein R² is acetylenyl.

10. The compound for use of any one of claims **1-9,** wherein (a) R^{2a} is halo, preferably wherein R^{2a} is fluoro; (b) R^{2a} is C₁-C₆ alkyl, preferably where R^{2a} is methyl.

11. The compound for use of claim **1,** wherein the compound is selected from the group consisting of:
| **No.** | **Structure** | **No.** | **Structure** |
|---|---|---|---|
| **4.001** | 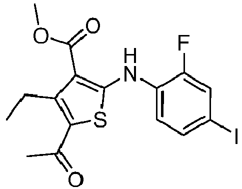 | **4.002** | 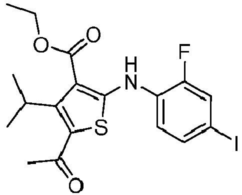 |
| **4.003** | 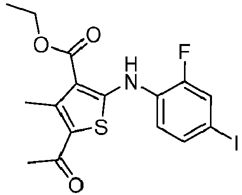 | **4.004** | 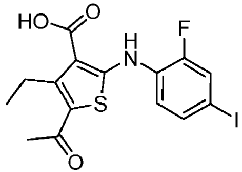 |
| **4.005** | 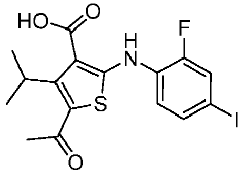 | **4.006** | 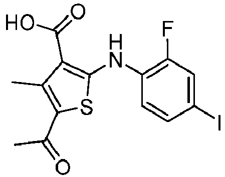 |
| **4.007** | 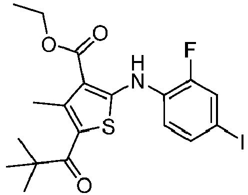 | **4.008** | 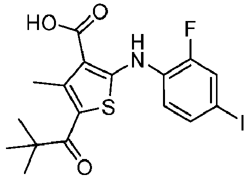 |
| **4.009** | 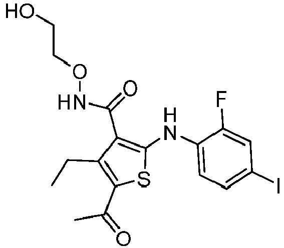 | **4.010** | 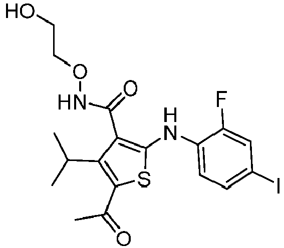 |
| **4.011** | 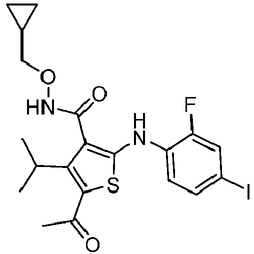 | **4.012** | 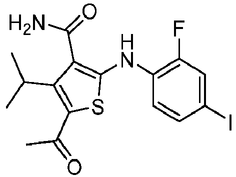 |
| **4.013** | 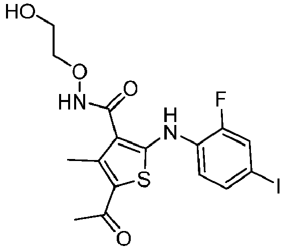 | **4.014** | 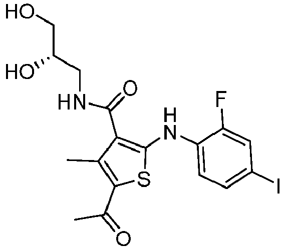 |
| **4.015** | 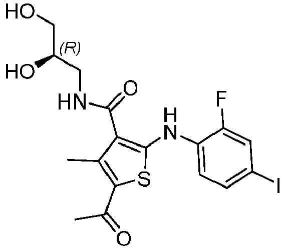 | **4.016** | 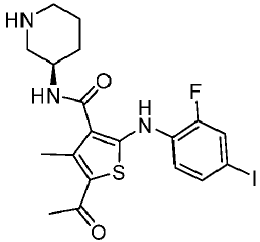 |
| **4.017** | 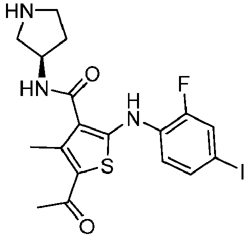 | **4.018** | 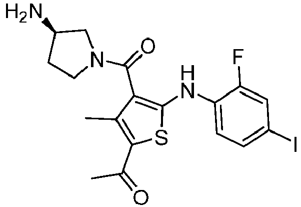 |
| **4.019** | 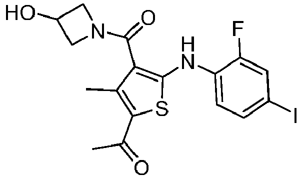 | **4.020** | 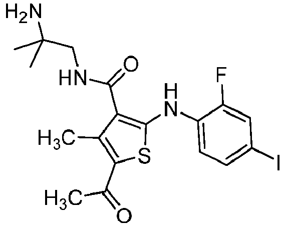 |
| **4.021** | 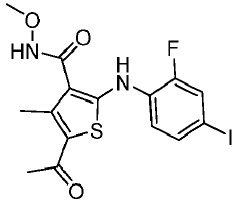 | **4.022** | 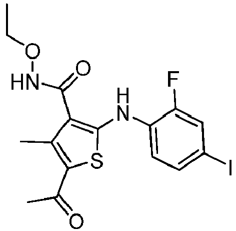 |
| **4.023** | 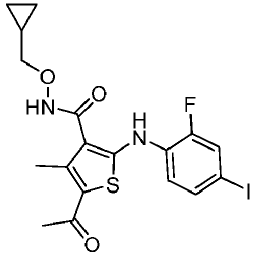 | **4.024** | 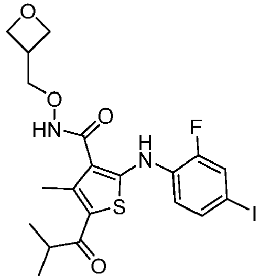 |
| **4.025** | 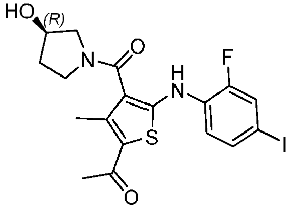 | **4.026** | 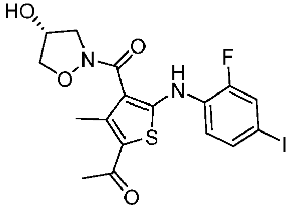 |
| **4.027** | 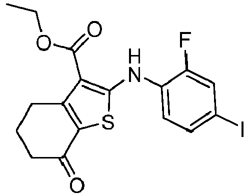 | **4.028** | 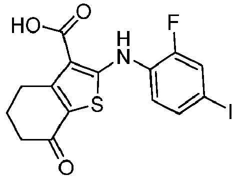 |
| **4.029** | 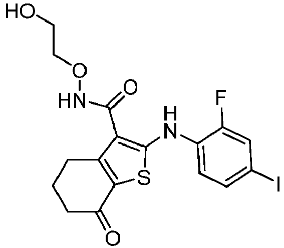 | **4.030** | 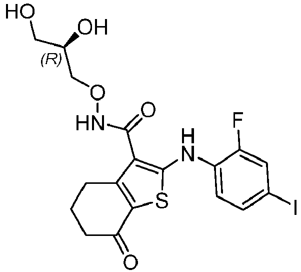 |
| **4.031** | 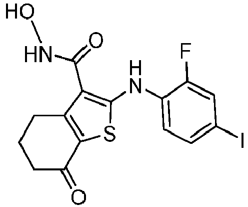 | **4.032** | 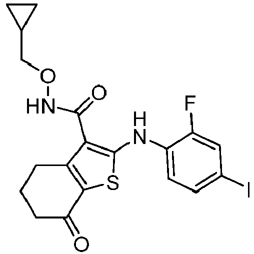 |
| **4.033** | 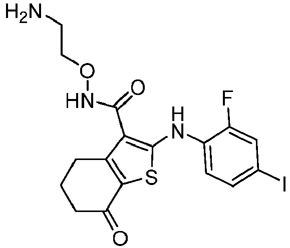 | **4.034** | 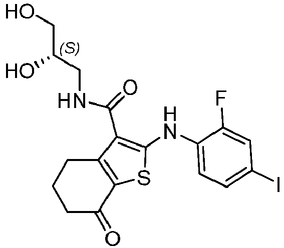 |
| **4.035** | 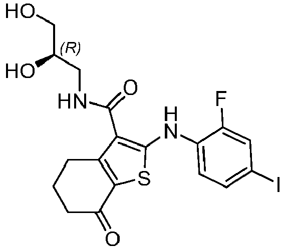 | **4.036** | 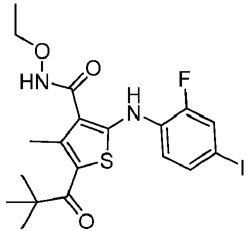 |
| **4.037** | 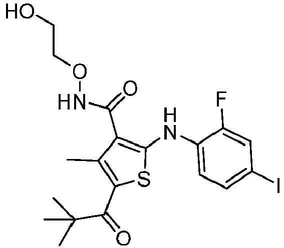 | **4.038** | 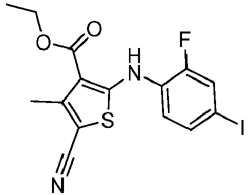 |
| **4.039** | 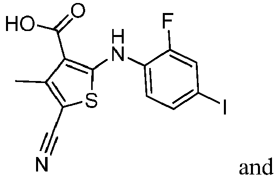 | **4.040** | 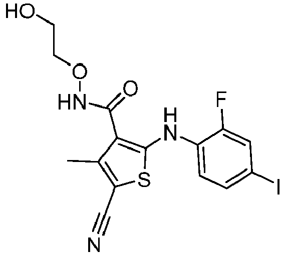 |

12. The compound for use of claim **11,**wherein the compound is represented by the formula: 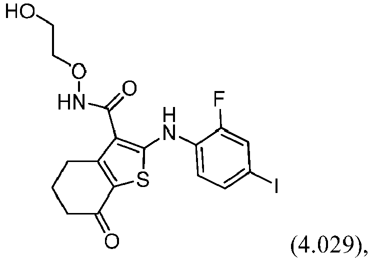 or a pharmaceutically acceptable salt thereof.

13. The compound for use of claim **11,**wherein the compound is represented by the formula: 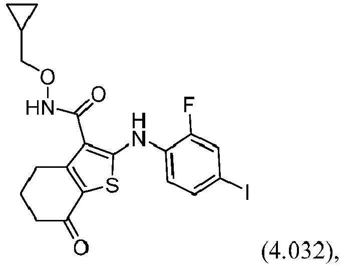 or a pharmaceutically acceptable salt thereof.

14. The compound for use of claim **11**,wherein the compound is represented by the formula: 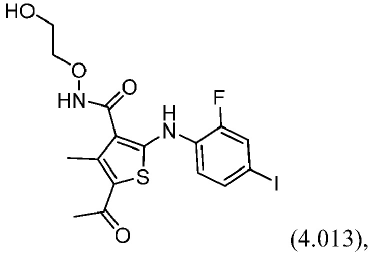 or a pharmaceutically acceptable salt thereof.

15. The compound for use of any one of claims **1-14**, wherein:
(a) the compound is administered with a pharmaceutically acceptable carrier in a pharmaceutical composition; and /or
(b) wherein one or more of (i) to (iv) appplies:
(i) the birthmark is selected from the group consisting of port-wine stains/capillary malformations, melanocytic nevus, displastic nevi, epidermal nevi, nevus sebaceous, nevus spilus, arterio-venous malformations, lymphatic malformations, and congenital melanocytic nevus
(ii) the birthmark is associated with activation of p-ERK, preferably wherein the birthmark associated with activation of p-ERK is selected from the group consisting of epidermal nevi, nevus sebaceous, nevus spilus, arterio-venous malformations, capillary malformations/port-wine stains, congenital melanocytic nevus, and lymphatic malformations
(iii) the compound having formula (IV) or the pharmaceutical composition thereof is for administration topically, subcutaneously, intradermally, or intralesionally
(iv) the compound having formula (IV) or the pharmaceutical composition thereof is for administration as a lotion, a spray, an ointment, a cream, a gel, a paste, and a patch.
